# EUROPEAN PATENT APPLICATION

(11) **EP 4 459 280 A1**
(43) Date of publication of application: **06.11.2024**
(21) Application number: 23171736.4
(22) Date of filing: 04.05.2023
(51) Int. Cl.: G01N 33/50

(54) **SCREENING AND USES OF GLUTATHIONE SYNTHETASE MODULATORS**

(71) Applicant: Max-Planck-Gesellschaft zur Förderung der Wissenschaften e.V., 80539 München (DE)
(72) Inventor: Martins Garcia, Bruna, Köln (DE); Farah Pernas, Lena, Köln (DE)
(74) Representative: Arth, Hans-Lothar

(57) **Abstract**

The present invention relates to an in vitro bioassay to identify glutamine synthetase activators and inhibitors on the basis of one or more glutamine markers such as citrate, a metabolite derived from the mevalonate pathway, a glutamine responsive mRNA or proteins, such as HMGCR or SREBP2.
Disclosed herein are also glutamine synthetase activators and inhibitors identified by the inventive method for use to stimulate or inhibit the synthesis of a metabolite derived from the mevalonate pathway, such as cholesterol, and to treat a disease associated with deregulated levels of said metabolite.

## Description

The present invention relates to an in vitro bioassay to identify glutamine synthetase activators and inhibitors on the basis of one or more glutamine markers selected from citrate, a metabolite derived from the mevalonate pathway, a fatty acid-derived lipid, and a glutamine responsive mRNA or proteins, such as HMGCR or SREBP2.

Disclosed herein are also glutamine synthetase activators and inhibitors identified by the inventive method for use to stimulate or inhibit the synthesis of a metabolite derived from the mevalonate pathway, such as cholesterol, or to stimulate or inhibit the synthesis of a fatty acid-derived lipid, in order to treat a disease associated with altered levels of said metabolite.

### Background of the invention

The mevalonate pathway is one of the most important metabolic networks in the cell as it generates essential cell constituents, such as cholesterol, and key metabolites by its branches, such as geranyl geranyl pyrophosphate and farnesyl pyrophosphate, necessary for normal cell metabolism.

The mevalonate pathway, as described above, generates a key intermediate for cholesterol production, a fundamental constituent of cell membranes. Moreover, cholesterol is also converted to steroid hormones, regulating different cellular pathways, vitamin D and bile acid production. The mevalonate pathway is also important for the production of farnesyl-pyrophosphate (FPP). FPP is converted in dolichols, used to assemble carbohydrate chains in glycoproteins, or in ubiquinones (or coenzyme Q10), electron transporters in mitochondria; or it is used to farnesylate or geranylate proteins, thus targeting them to cell membranes. Therefore, the mevalonate pathway is responsible for numerous cellular processes.

Approximately 39% of adults globally suffer from high cholesterol, which is a major risk factor for heart disease, heart attack and stroke. Statins, a class of molecules that inhibit HMGCR were the most used class of drugs that reduce lipid levels and mitigate the risk of cardiovascular events increased in 75% of countries between 2008 and 2018 for an estimated total of 173 million users. However, statins also inhibit the synthesis of several essential molecules derived from the mevalonate pathway, such as coenzyme Q which is essential for mitochondrial function. Statins are also associated with a higher risk of diabetes and muscle-pain related symptoms in 10% of users, other than with renal failure. Statin therapy is contraindicated in pregnant women and patients with liver disorders. Moreover, treatment of statins is not ever effective, and therefore there is still a need for novel agents to lower cholesterol levels.

Thus, other strategies to regulate cholesterol levels are required, and could be used in diseases such as cancer that require cholesterol to fuel cancer cell proliferation.

Therefore there is a need for a method to identify substances that can stimulate or inhibit the synthesis of cholesterol and other mevalonate pathway derived metabolites in order to treat diseases associated with altered levels of cholesterol or of other mevalonate pathway derived metabolites.

It is the objective of the present invention to provide a method to identify substances modulating glutamine synthetase, based on the activity of the mevalonate pathway and its sub-branches including the synthesis of farnesyl-PP, geranylgeranyl-PP, cholesterol, as well as on the activity of fatty acid synthesis and incorporation into phospholipids and triglycerides. Therefore, the present invention also provides useful glutamine synthetase activators and inhibitors that can be used to regulate the levels of cholesterol, or of other metabolites derived from the mevalonate pathway, or of lipids derived from fatty acid synthesis. Such glutamine synthetase activators and inhibitors could be advantageously used to treat diseases associated with a deficiency or an excess of cholesterol or of said metabolites derived from the mevalonate pathway, or of said lipids derived from fatty acid synthesis.

The objective of the present invention is solved by the teaching of the independent claims. Further advantageous features, aspects and details of the invention are evident from the dependent claims, the description, the figures, and the examples of the present application.

### Brief description of the invention

The objective of the present invention is solved by an *in vitro* method for evaluating whether a substance is a glutamine synthetase modulator in mammal cells, said method comprising:
(a) culturing mammal cells in presence or absence of at least one candidate substance whose ability to act as glutamine synthetase modulator is sought to be determined;
(b) stopping the culturing of the cells of step (a);
(c) determining levels of one or more glutamine markers;
(d) comparing levels of said one or more glutamine marker in the cells cultured with the candidate substance to those cells cultured without said candidate substance;
(e) identifying the candidate substance as glutamine synthetase modulator on the basis of the comparison at step (d), and
wherein the glutamine synthetase modulator is a glutamine synthetase inhibitor or a glutamine synthetase activator.

In some embodiments of the inventive method, the one or more glutamine marker is selected from the group comprising citrate, a fatty acid-derived lipid, and a metabolite derived from the mevalonate pathway downstream of HMGCR, and step (e) comprises:
identifying the tested candidate substance as a glutamine synthetase inhibitor if the level of citrate in the cells cultured with said candidate substance is at least 10% lower compared to cells cultured without said candidate substance, and/or the level of said fatty acid-derived lipid in the cells cultured with said candidate substance is at least 10% lower compared to cells cultured without said candidate substance and/or the level of the metabolite derived from the mevalonate pathway downstream of HMGCR in the cells cultured with said candidate substance is at least 10% lower compared to cells cultured without said candidate substance, or
identifying the tested candidate substance as a glutamine synthetase activator if the level of citrate in the cells cultured with said candidate substance is at least 10% higher to cells cultured without said candidate substance, and/or the level of said fatty acid-derived lipid in the cells cultured with said candidate substance is at least 10% higher compared to cells cultured without said candidate substance and/or the level of the metabolite derived from the mevalonate pathway downstream of HMGCR in the cells cultured with said candidate substance is at least 10% higher compared to cells cultured without said candidate substance.

In some embodiments, step (b) of stopping the culturing of the cells of step (a) is performed after at least 24 hours.

In some other embodiments of the inventive method, the metabolite derived from the mevalonate pathway downstream of HMGCR is selected from the group comprising: HMG-CoA, mevalonate, mevalonate-5-P, mevalonate-5-PP, Isopentyl-5-PP, farnesyl-PP, squalene, lanosterol, dihydrolanosterol (DHL), desmosterol, epoxycholesterol (EC), dehydrocholesterols, cholesterol, geranyl-PP, geranylgeranyl-PP, dolicyl-PP, dolichol, coenzyme Q10, Vitamin K2, Vitamin D, steroid hormones, bile acid, Heme A; and/or
the fatty acid-derived lipid is selected from the group comprising a fatty acid with a carbon chain length between 16 and 24 carbon atoms, a phospholipid comprising acyl groups with a carbon chain length between 16 and 24 carbon atoms, a diacylglycerol comprising acyl groups with a carbon chain length between 16 and 24 carbon atoms, a triglyceride comprising acyl groups with a carbon chain length between 16 and 24 carbon atoms, and a sphingolipid comprising acyl groups with a carbon chain length between 16 and 24 carbon atoms.

In alternative embodiments of the inventive method, the fatty acid-derived lipid is selected from the group comprising palmitic acid, a phosphatidylcholine, phosphatidylserine, phosphatidylethanoline, phosphatidylglycerol, phosphatidylinositol, diacylglycerol, triglyceride, sphingomyelin, wherein said phosphatidylcholine, phosphatidylserine, phosphatidylethanoline, phosphatidylglycerol, phosphatidylinositol, diacylglycerol, triglyceride, and sphingomyelin, comprise acyl groups with a carbon chain length between 16 and 24 carbon atoms.

In some other embodiments of the inventive method, the one or more glutamine marker is a "glutamine stimulated mRNA" selected from the group comprising *FASN,* HSPA1A, CAVIN2, HSPA1B, HMGCR, JAKMIP2, NYNRIN, SH3TC2, INSIG1, ZBED2, CD180, TNFRSF11B, FAM111B, PNPLA3, THBS1, BMP4, MIAT, HSPA8, SORBS2, MAP1A, EPHB3, NES, LINC02615, SLC2A12, DPYSL2, HSPB1, CENPI, SAMD9L, TMEM97, FDFT1, HMMR, GAS6-AS1, ANXA6, BLACAT1, RHOBTB1, SQLE, PCYT2, TET1, TRIB2, MSMO1, NSDHL, ITGB8, CRISPLD1, UACA, ROR1, LSS, SYNC, LPCAT1, IDI1, PIK3R3, SOX2, MVK, PDGFB, SNAI3-AS1, DUBR, CSGALNACT1, PLD5P1, TUBA1A, IGSF10, SAMD9, SNX21, TNFSF9, ACSS2, PARP9, RTKN, PLCH1, NREP, ZMYM3, HEXIM1, MAGED1, TTC3P1, PGP, SLCO4C1, USP51, DHCR24, and/or a "glutamine inhibited mRNA" selected from the group comprising TRIB3, UPP1, SPX, TSLP, MAFF, CLDN1, MYC, ATF3, RAB39B, CREB5, PPP1R15A, ERRFI1, ALOXE3, GADD45A, CDC42EP1, CTH, SESN2, ARC, DDIT3, CHRNA9, BTG1, KDM7A, SLC22A15, N4BP3, EGR1, DDIT4, NR1D1, ESRP1, INHBE, CXCL8, PTGS2, HK2, KDM6B, AREG, TNFRSF9, MYCT1, GEM, CHIC2, SNHG17, ASNS, FOSL1, FLNC, CD274, FHL2, FAM107B, SERPINE1, CCL4, ZNF697, EPAS1, PER2, ITPRIP, MTHFD2, ROCK1P1, MXD1, CEBPB, UNC5B, MAP2K3, RELB, ARHGEF2, PTX3, AKNATRIM25, NSC, SLC7A1, LIMA1, CHAC1, GPAT3, SLC38A1, EGFR, CNKSR3, RPLP0P2, LAT2, BEND7, TNFAIP3, ETV5, UHRF1BP1, SLC3A2, NFIL3, ABCB1, FIBIN, LINC01667, GPT2, RASGEF1C, LDAF1, IL1RL1, GDF15, NGF, NR4A1, TAF4B, SNHG1, GOT1, SLC6A9, ARID5A, SPNS2, CEBPG, LETM2, ADM2, EPB41L4A-AS1, MMP3, SNAPC1, DUSP2, NUPR1, VEGFA, OSBPL6, MAP1LC3B, SYNE1, FAM83G, PKD1P6, KCTD16, LINC00973, DUSP1, EBF3, HRK, PER1, CLUHP4, RAPH1, SPHK1, PKD1P1, PSAT1, SARS1, FGF21, DUSP5, IL6R, PRKCE, ABL2, GARS1, TSC22D3, PXK, TXNIP, SINHCAF, FGF2, FAM167A, NFATC1, SH3TC1, UAP1, RCL1, SLC1A5, SPRR2D, SLC1A4, SLC30A1, C3orf52, SMOX, CECR2, GABARAPL1, SYP, CBARP, ETS1, SNHG15, PFDN2, NIPAL4, LONRF2, IL31RA, EIF1B, CDO1, IL11, SERPINB8, SLC9A1, CSRNP1, CARS1, FUT1, RND3, TNFRSF10B, OLIG2, FAM241A, WDR43, DDR2, VGLL3, CYTOR, HBEGF, DUSP8, KLF10, ATXN1, DAGLB, AVPI1, GTPBP2, TMEM268, SH2B3, FBXO31, B3GALT5, SPOCD1, TIMM44, PHLDA2, SDC4, AEN, EGR2, MIDEAS, TRIB1, CYLD, CDK7, MOCOS, STK40, PTHLH, PCID2, ZFPM2-AS1, GPR137B, GAS5, SGMS2, DDX21, EREG, FYN, ZFAS1, BEX2, FOXO3, YARS1, ICOSLG, FKBP9, DUX4L26, IRF2BP2, PSPH, and step (e) further comprises:
identifying the tested candidate substance as glutamine synthetase inhibitor if the level of a glutamine inhibited mRNA in the cells cultured with the candidate substance is higher than + 1.5 and/or the level of a glutamine stimulated mRNA in the cells cultured with the candidate substance is lower than 1.5 compared to cells cultured without said candidate substance; or
identifying the tested candidate substance as glutamine synthetase activator if the level of a glutamine inhibited mRNA in the cells cultured with the candidate substance is lower than - 1.5 and/or the level of a glutamine stimulated mRNA in the cells cultured with the candidate substance is higher than 1.5 compared to cells cultured without said candidate substance; and
wherein the level of said mRNA is calculated as log2FC between the cells cultured with said candidate substance and the cells cultured without said candidate substance.

In further embodiments, the one or more glutamine marker is glutamine stimulated mRNA *HMGCR.*

In an aspect of the inventive method, the one or more glutamine marker is a glutamine stimulated protein selected from SREBP2, HMGCR, FDFT1, c-MYC, FASN, HMGCS1, FDPS, LDLR and LSS, and
step (e) further comprises:
identifying the tested candidate substance as a glutamine synthetase inhibitor if the level of said "glutamine- stimulated proteins" is at least 10% lower compared to cells cultured without said candidate substance, or
identifying the tested candidate substance as a glutamine synthetase activator if the level of said "glutamine-- stimulated proteins" is at least 10% higher compared to cells cultured without said candidate substance.

In a particular aspect of the inventive method wherein the one or more glutamine marker is a glutamine stimulated protein,
step (c) further comprises determining proteolytic activation of the glutamine stimulated protein and/or the glutamine stimulated protein is SREBP2.

In a further particular aspect of the inventive method, the mammal cells are mammal cell lines having a glutamine synthetase protein relative expression value higher than 0.5 and/or a glutamine synthetase mRNA expression value higher than 2.0, wherein the mRNA expression value is calculated as log2 of transcripts per million.

In a further more particular aspect of the inventive method, the mammal cells are HepG2.

A **second embodiment** of the present invention is directed to a glutamine synthetase inhibitor identifiable by the *in vitro* method described above, for use in treatment of a disease associated with high levels of a mevalonate pathway derived metabolite, and/or with high levels of a fatty acid-derived lipid.

In a preferred aspect of the second embodiment, the disease is selected from the group comprising or consisting of steatosis, cirrhosis, renal dysfunction, testosterone deficiency, glioblastoma, multiple myeloma, leukemia, cancer, premature aging, infectious disease, neurodegenerative disease including Alzheimer's disease, arthritis, cardiovascular disease, Hutchinson-Gilford progeria syndrome (HGPS), cerebral cavernous malformations (CCMs), neuroinflammatory disorders, autoimmune disorders, diabetes, atherosclerosis, hyperlipidemia, immuno-inflammatory diseases, acquired immune deficiency syndrome (AIDS), allograft rejection, adult respiratory distress syndrome, arthritis, asthma, atherosclerosis, autoimmune disorders, cerebral palsy, eczema, glomerulonephritis, heart failure, herpes dementia, immune complex diseases, inflammatory bowel disease, ischemia , metabolic syndrome, migraine, multiple sclerosis, myocarditis, organ transplant/bypass disorders, osteoporosis, oxidant induced lung injury, Paget's disease, hyperimmunoglobulin D syndrome, mevalonate kinase deficiency, pain, peritonitis, reperfusion injury, retinitis, sepsis, septic shock, sickle cell anemia, stroke, toxic shock syndrome, uveitis, X-adenoleukodystrophy (X-ALD), Alzheimer's disease, Parkinson's disease, Landry-Guillain-Barre-Strohl syndrome, multiple sclerosis, viral encephalitis, AIDS-related dementia, amyotrophic lateral sclerosis, brain trauma, spinal cord disorders, atherosclerosis, coronary artery insufficiency, coronary heart disease, myocardial infarction, hypertriglyceridemia, lymphoma, Cushing syndrome, porphyria, hypothyroidism, renal disease, cholestatic liver disorders, lymphoma, bipolar disorders, schizophrenia, neurodegenerative diseases, Niemann-Pick and Huntington diseases, multiple sclerosis, lipid storage disease.

In a more preferred aspect of the second embodiment, the glutamine synthetase inhibitor is selected from L-methionine sulfoximine, a phosphinothricin analogue, a derivative of aminomethylenebisphosphonic acid, and γ-hydroxylysine.

In a further aspect of the second embodiment, the mevalonate pathway derived metabolite is an isoprenoid, and/or a sterol, and/or cholesterol. In another aspect of the second embodiment, the fatty acid-derived lipid is selected from the group comprising a phospholipid, a diacylglycerol, a triglyceride, and a sphingolipid.

A **third embodiment** of the present invention is directed to a glutamine synthetase activator identifiable by the *in vitro* method described above, for use in treatment of a disease associated with low levels of a mevalonate pathway derived metabolite, and/or with low levels of a fatty acid-derived lipid. In an aspect of the third embodiment, the fatty acid-derived lipid is selected from the group comprising a phospholipid, a diacylglycerol, a triglyceride, and a sphingolipid.

A further embodiment of the invention is directed to a **pharmaceutical composition** comprising a glutamine synthetase modulator identifiable by the described *in vitro* method, together with at least one pharmaceutically acceptable carrier, excipient and/or diluent.

### Description of the invention

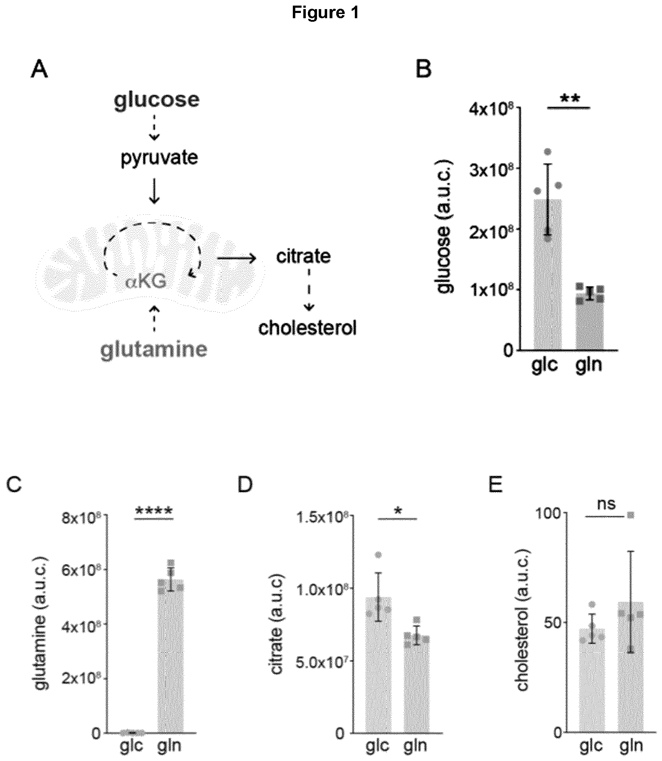

When looking for advantegeous modulators of the mevalonate pathway, the investigator surprisingly found that mammal cells cultured *in vitro* with glucose but not glutamine had 20% higher citrate levels than cells cultured with only glutamine for 8 hours (**Fig.1B-D**). Opposite to citrate however, cholesterol trended ~20% lower in cells cultured with only glucose (**Fig. 1E**). This result was unexpected given that cholesterol synthesis depends on citrate, and raised the question of how glutamine-deprivation reduced cholesterol levels (**Example 1**).

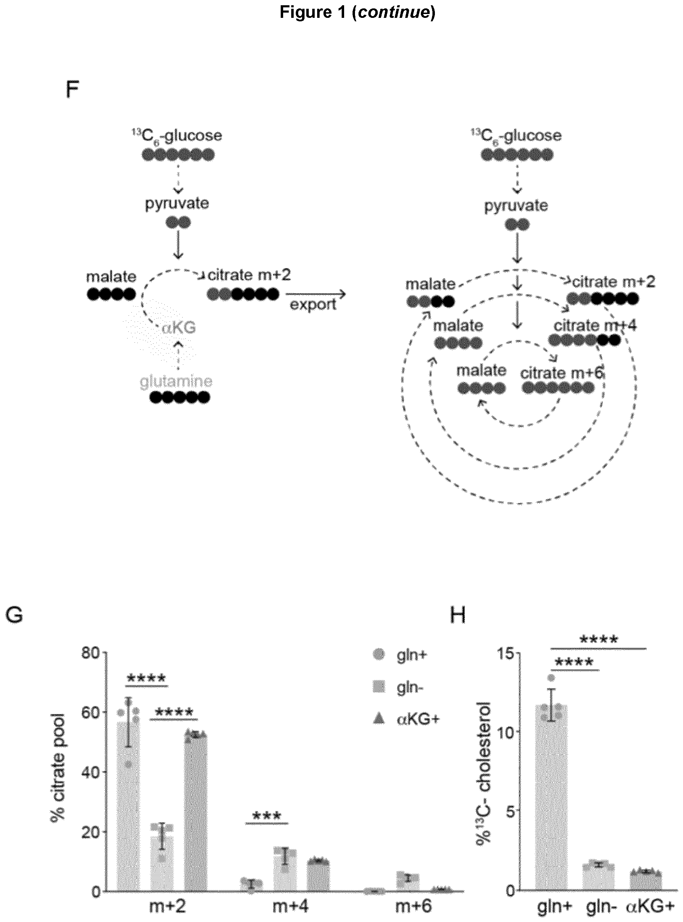

Moreover, it was shown that in the absence of glutamine, glucose-derived citrate was oxidized to sustain the TCA cycle, thereby restricting its export from mitochondria and availability for cholesterol synthesis (**Fig. 1F**; right).

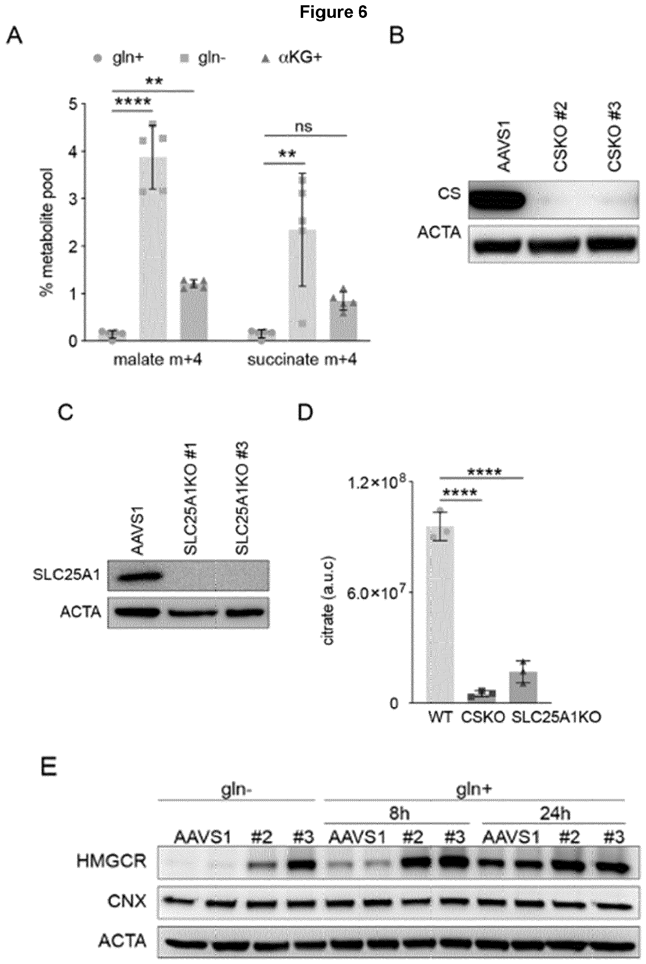

In cells fed with glutamine (gln+) and ¹³C-glucose, the majority of citrate had 2 labelled carbons (m+2), which is the expected pattern following its generation in and export from mitochondria (**Fig. 1F-G**). Glutamine-starved (gln-) cells however had a three-fold decrease in m+2 citrate while m+4 and m+6 citrate were increased by ~5- and ~100-fold, respectively. A similar trend was observed for the TCA cycle intermediates malate and succinate (**Fig. 6A**). Moreover, there was a >5-fold decrease in ¹³C-incorporation into cholesterol in cells cultured without glutamine (**Fig. 1H**), showing that glutamine is required for cholesterol synthesis independently of its role in anaplerosis.

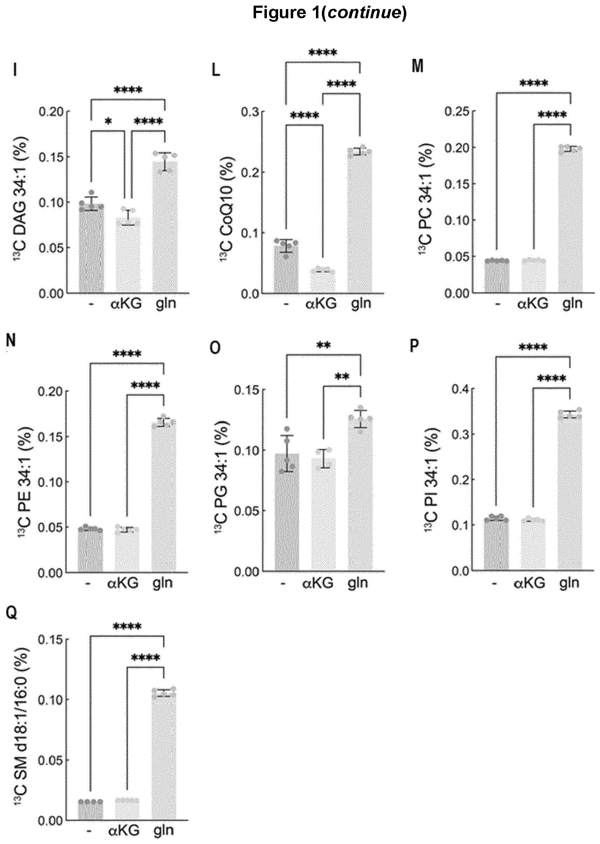

Surprisingly, glutamine-starvation also inhibited the synthesis of abundant species of fatty acids, which are downstream of citrate, and of fatty acid-derived lipids such as diacylgerol (precursor to trigylcerides), phospholipids, sphingomyelin, and coenzyme Q10 (**Figure 1 I-Q**).

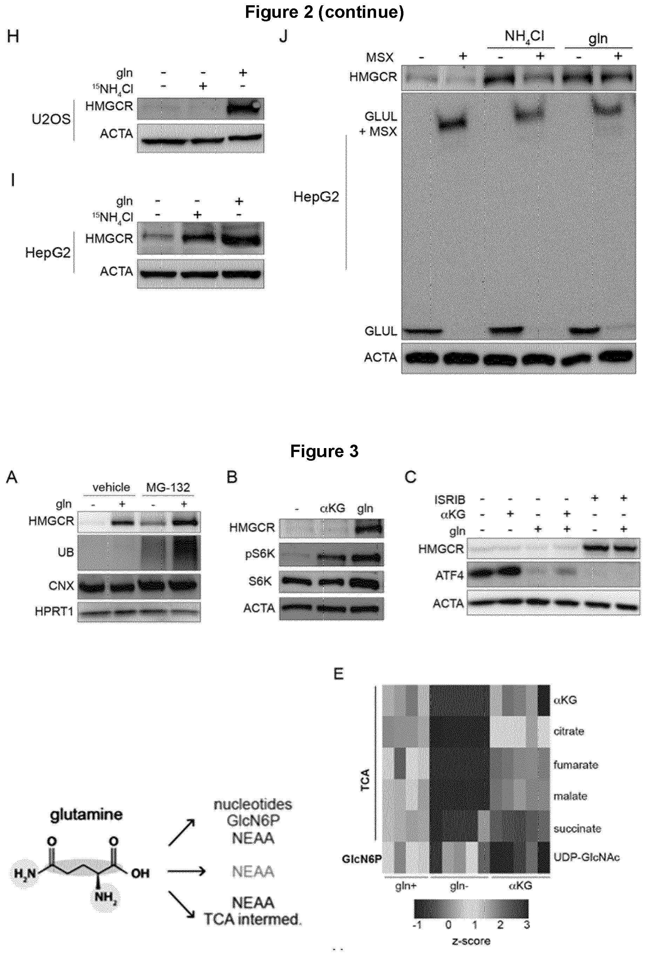

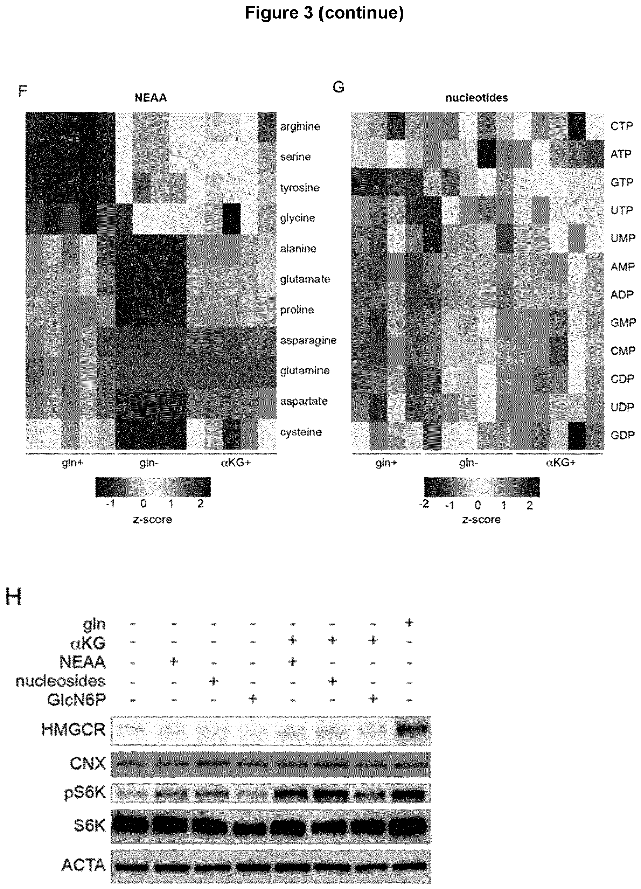

Moreover, the following metabolites were decreased after 24 hours of glutamine starvation: TCA-cycle intermediates citrate, fumarate, malate and succinate (Fig. 3E), uridine diphosphate N-acetylglucosamine (UDP-GlcNAc) (Fig. 3E), non-essential amino acids (NEAAs) (Fig. 3F), and nucleotides (Fig. 3G).

Therefore, the present invention is directed to an *in vitro* method for evaluating whether a substance is a glutamine synthetase modulator in mammal cells, said method comprising:
(a) culturing mammal cells in presence or absence of at least one candidate substance whose ability to act as glutamine synthetase modulator is sought to be determined;
(b) stopping the culturing of the cells of step (a);
(c) determining levels of one or more glutamine markers;
(d) comparing levels of said one or more glutamine marker in the cells cultured with the candidate substance to those cells cultured without said candidate substance;
(e) identifying the candidate substance as glutamine synthetase modulator on the basis of the comparison at step (d), and
wherein the glutamine synthetase modulator is a glutamine synthetase inhibitor or a glutamine synthetase activator.

In a particular aspect of the *in vitro* method for evaluating whether a substance is a glutamine synthetase modulator in mammal cells, the one or more glutamine marker is selected from the group comprising citrate, a fatty acid-derived lipid, and a metabolite derived from the mevalonate pathway downstream of HMGCR, and step (e) comprises:
identifying the tested candidate substance as a glutamine synthetase inhibitor if the level of citrate in the cells cultured with said candidate substance is at least 10%, or 15%, or 20%, or 25%, or 30%, or 35%, or 40%, or 45%, or 50%, or 55%, and more preferably 10 % lower compared to cells cultured without said candidate substance, and/or the level of said fatty acid-derived lipid in the cells cultured with said candidate substance is at least 10%, or 15%, or 20%, or 25%, or 30%, or 35%, or 40%, or 45%, or 50%, or 55%, and more preferably 10 % lower compared to cells cultured without said candidate substance and/or the level of the metabolite derived from the mevalonate pathway downstream of HMGCR in the cells cultured with said candidate substance is at least 10%, or 15%, or 20%, or 25%, or 30%, or 35%, or 40%, or 45%, or 50%, or 55%, and more preferably 10 % lower compared to cells cultured without said candidate substance, or
identifying the tested candidate substance as a glutamine synthetase activator if the level of citrate in the cells cultured with said candidate substance is at least 10%, or 15%, or 20%, or 25%, or 30%, or 35%, or 40%, or 45%, or 50%, or 55%, and more preferably 10 % higher to cells cultured without said candidate substance, and/or the level of said fatty acid-derived lipid in the cells cultured with said candidate substance is at least 10%, or 15%, or 20%, or 25%, or 30%, or 35%, or 40%, or 45%, or 50%, or 55%, and more preferably 10 % higher compared to cells cultured without said candidate substance and/or the level of the metabolite derived from the mevalonate pathway downstream of HMGCR in the cells cultured with said candidate substance is at least 10%, or 15%, or 20%, or 25%, or 30%, or 35%, or 40%, or 45%, or 50%, or 55%, and more preferably 10 % higher compared to cells cultured without said candidate substance.

Therefore, the present invention is directed to an *in vitro* method for evaluating whether a substance is a glutamine synthetase modulator in mammal cells, said method comprising:
(a) culturing mammal cells in presence or absence of at least one candidate substance whose ability to act as glutamine synthetase modulator is sought to be determined;
(b) stopping the culturing of the cells of step (a);
(c) determining levels of one or more glutamine markers;
(d) comparing levels of said one or more glutamine marker in the cells cultured with the candidate substance to those cells cultured without said candidate substance;
(e) identifying the candidate substance as glutamine synthetase modulator on the basis of the comparison at step (d), and

wherein the glutamine synthetase modulator is a glutamine synthetase inhibitor or a glutamine synthetase activator, and
wherein the one or more glutamine marker is selected from the group comprising citrate, a fatty acid-derived lipid, and a metabolite derived from the mevalonate pathway downstream of HMGCR, and
wherein step (e) comprises:
   identifying the tested candidate substance as a glutamine synthetase inhibitor if the level of citrate in the cells cultured with said candidate substance is at least 10 % lower compared to cells cultured without said candidate substance, and/or the level of said fatty acid-derived lipid in the cells cultured with said candidate substance is at least 10 % lower compared to cells cultured without said candidate substance and/or the level of the metabolite derived from the mevalonate pathway downstream of HMGCR in the cells cultured with said candidate substance is at least 10 % lower compared to cells cultured without said candidate substance, or
   identifying the tested candidate substance as a glutamine synthetase activator if the level of citrate in the cells cultured with said candidate substance is at least 10 % higher to cells cultured without said candidate substance, and/or the level of said fatty acid-derived lipid in the cells cultured with said candidate substance is at least 10% higher compared to cells cultured without said candidate substance and/or the level of the metabolite derived from the mevalonate pathway downstream of HMGCR in the cells cultured with said candidate substance is at least 10 % higher compared to cells cultured without said candidate substance.

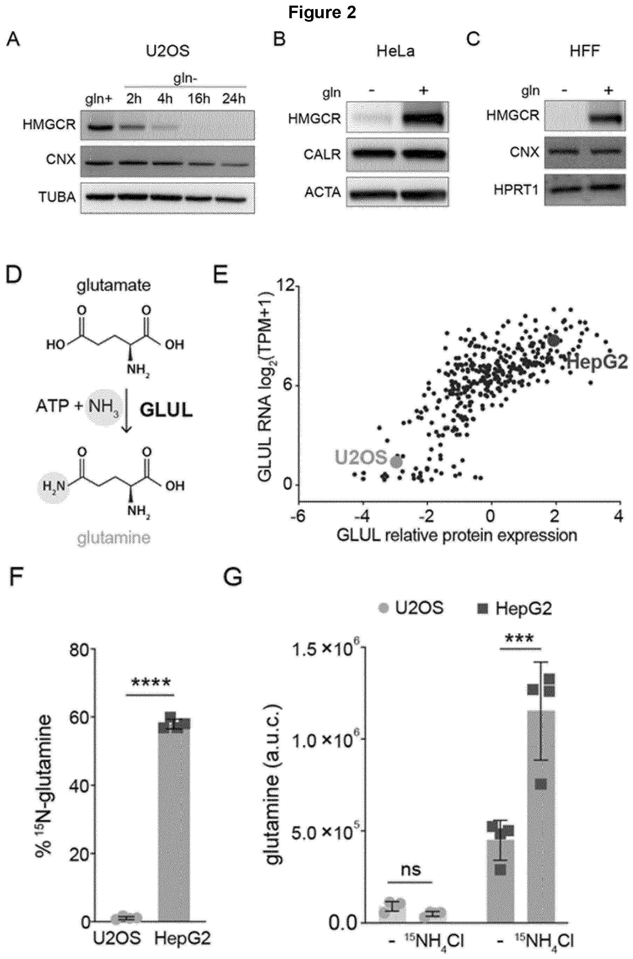

Glutamine starvation reduced levels of HMGCR, the rate-limiting enzyme in cholesterol synthesis, in various human cell lines (**Fig. 2A-2C**). Because HMGCR loss paralleled a decrease in cholesterol synthesis, HMGCR was also used as a marker for mevalonate pathway activity.

The importance of glutamine synthetase in the regulation of HMGCR was shown in experiments comparing HMGCR levels in U2OS, expressing very low *GLUL* mRNA, and HepG2, expressing *GLUL* at high levels. In HepG2 cells cultured without glutamine, HMGCR resulted maintained at low levels and increased following ammonia treatment along with newly synthetized glutamine (**Fig 2F-I**). Differently, in U2OS cells, neither glutamine nor HMGCR increased following ammonia treatment. Importantly, MSX treatment of HepG2 cells further decreased HMGCR during glutamine starvation and prevented the ammonium chloride-induced increase in HMGCR (**Fig. 2J**).
Thus, glutamine starvation reduced HMGCR levels, thus inhibiting the mevalonate pathway, independently of mTORC1 and the integrated stress response (ISR) (**Fig. 3B-C**).

The Cancer Genome Atlas (https://depmap.org/portal/ccle/) reports the levels of GLUL mRNA expression based on RNAseq and of GLUL protein expression of 347 cell lines (**Table 4** and **Table 5,** **Fig. 2E**).

In some embodiments of the *in vitro* method for evaluating whether a substance is a glutamine synthetase modulator in mammal cells, the mammal cells are mammal cell lines having a glutamine synthetase protein relative expression value higher than 0.5, or 0.6, or 0.7, or 0.8, or 0.9, or 1.0, or 1.1, or 1.2, or 1.3, or 1.4, or 1.5, or 1.6, or 1.7, or 1.8, or 1.9, or 2.0, or 2.1, or 2.2, or 2.3, or 2.4, or 2.5, or 3.0, and more preferably higher than 0.5.

In some embodiments of the *in vitro* method for evaluating whether a substance is a glutamine synthetase modulator in mammal cells, the mammal cells are mammal cell lines having a glutamine synthetase mRNA expression value higher than 2.0, or 2.5, or 3.0, or 3.5, or 4.0, or 4.5, or 5.0, or 5.5, or 6.0, or 6.5, or 7.0, or 7.5, or 8.0, or 8.5, or 9.0, or 9.5, or 10.0, and more preferably higher than 2.0, wherein the mRNA expression value is calculated as log2 of transcripts per million. Therefore, the present invention is particularly directed to an *in vitro* method for evaluating whether a substance is a glutamine synthetase modulator in mammal cells, said method comprising:
(a) culturing mammal cells in presence or absence of at least one candidate substance whose ability to act as glutamine synthetase modulator is sought to be determined;
(b) stopping the culturing of the cells of step (a);
(c) determining levels of one or more glutamine markers;
(d) comparing levels of said one or more glutamine marker in the cells cultured with the candidate substance to those cells cultured without said candidate substance;
(e) identifying the candidate substance as glutamine synthetase modulator on the basis of the comparison at step (d),

wherein the glutamine synthetase modulator is a glutamine synthetase inhibitor or a glutamine synthetase activator, and
wherein the mammal cells are mammal cell lines having a glutamine synthetase protein relative expression value higher than 0.5 and/or a glutamine synthetase mRNA expression value higher than 2.0, wherein the mRNA expression value is calculated as log2 of transcripts per million.

The present invention is also directed to an *in vitro* method for evaluating whether a substance is a glutamine synthetase modulator in mammal cells, said method comprising:
(a) culturing mammal cells in presence or absence of at least one candidate substance whose ability to act as glutamine synthetase modulator is sought to be determined;
(b) stopping the culturing of the cells of step (a);
(c) determining levels of one or more glutamine markers;
(d) comparing levels of said one or more glutamine marker in the cells cultured with the candidate substance to those cells cultured without said candidate substance;
(e) identifying the candidate substance as glutamine synthetase modulator on the basis of the comparison at step (d),

wherein the glutamine synthetase modulator is a glutamine synthetase inhibitor or a glutamine synthetase activator, and
wherein the mammal cells are HepG2.

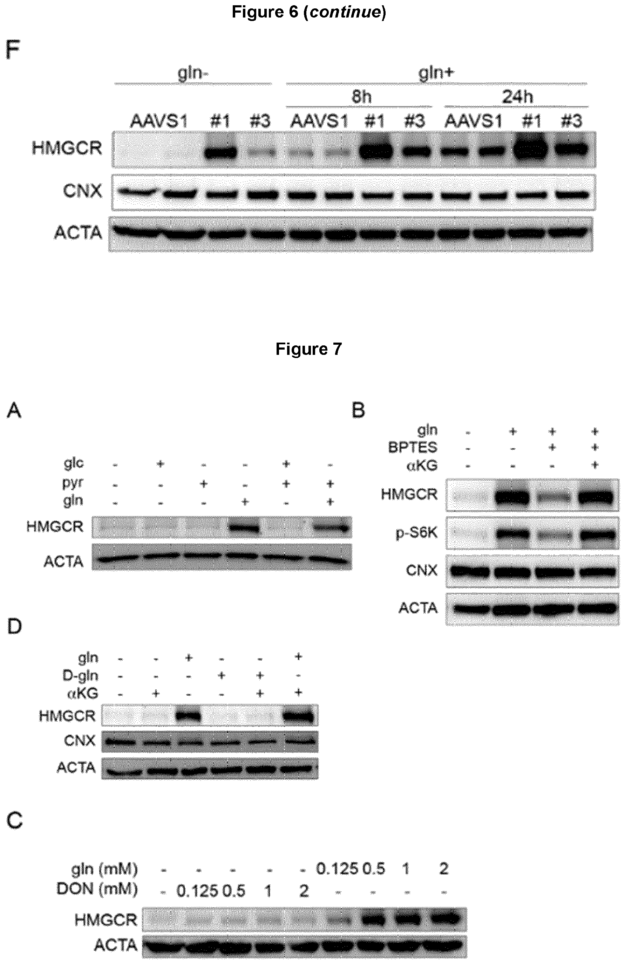

Further experiments demonstrated that TCA cycle metabolites are not involved in glutamine mediated HMGCR regulation **(****Fig. 6B-E****).** Supplementation with NEAAs, nucleotides, or UDP-GlcNAc did not rescue HMGCR levels during glutamine starvation (**Fig. 3H**). Furthermore, neither glutamine analog DON (6-diazo-5-oxo-L-norleucine) nor D-glutamine, a stereoisomer of glutamine, rescued levels of HMGCR during glutamine starvation (**Fig. 7C-D**). Thus, these results indicate that glutamine per se is sensed by the mevalonate pathway, and that glutamine positively regulates the mevalonate pathway.

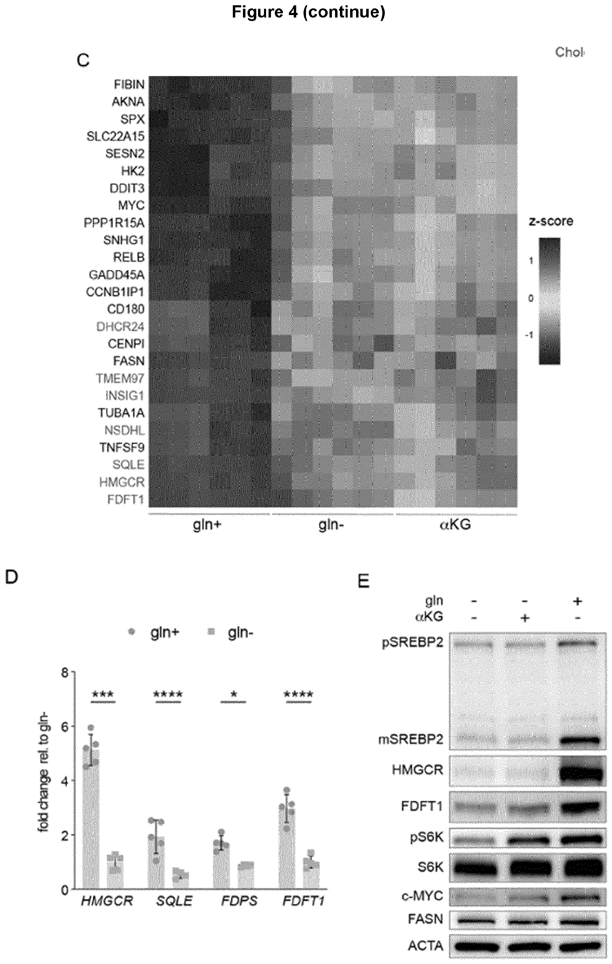

Transcriptome analysis revealed that among the top 25 glutamine-regulated mRNAs were those encoding for key enzymes in the cholesterol biosynthesis pathway including *HMGCR, SQLE, and FDFT1*-which were found reduced in glutamine starved cells, and then induced after glutamine refeeding(**Fig. 4C-D**). In line with the RNAseq data, levels of HMCGR and FDFT1 protein were decreased in cells starved of glutamine for 8 hours, despite αKG supplementation (**Fig. 4E**).

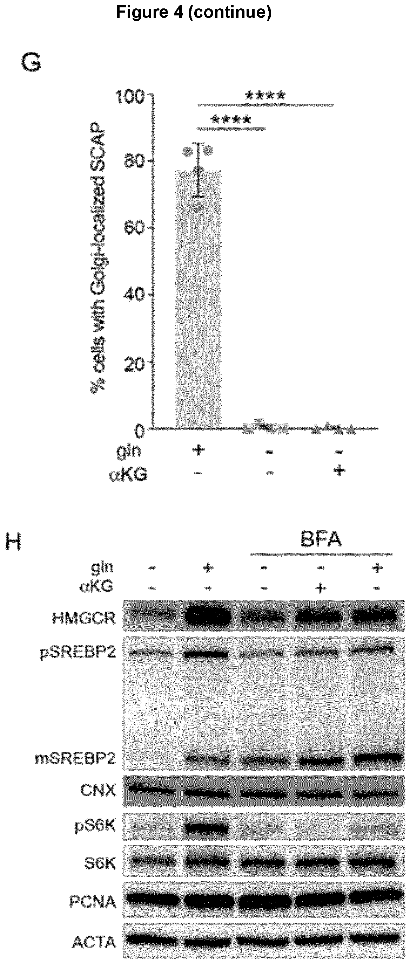

Surprisingly, glutamine was required for the proteolytic activation of the master regulator of the mevalonate pathway SREBP2 (**Fig. 4E**) and this was due to glutamine mediated activation of ER-to-Golgi trafficking of SCAP and SREBP2 (**Fig. 4H**), where SREBP2 is proteolytically cleaved by S1P and S2P to yield active fragments.

The above results suggest that glutamine act as a regulator for cholesterol production and fatty acid synthesis, and suggest that glutamine-sensing acts as an anabolic toggle-switch that coordinates precursor availability with lipid biosynthesis.

Therefore, reducing in vivo glutamine levels by reducing GLUL expression or activity could be used to lower levels of cholesterol or of other mevalonate pathway-derived metabolites, as well as of fatty acids, phospholipids, and/or triglycerides in conditions associated to higher levels of cholesterol or of said mevalonate pathway-derived metabolites or of said fatty acids, phospholipids, and/or triglycerides.

More in particular, modulating the mevalonate pathway using a glutamine synthetase inhibitor could lower lipidemia in patients suffering from high cholesterol or high fatty acid-related lipids such as triglycerides, or as an anticancer therapy.

Also more importantly, modulating the mevalonate pathway using a glutamine synthetase inhibitor could modulate production of other sterols, and isoprenoids, playing a role in the treatments of isoprenoid associated conditions.

Moreover, modulating the mevalonate pathway using a glutamine synthetase activator could increase the levels cholesterol in patients in need thereof, such as those suffering from hypocholesterolemia, or of fatty-acid derived lipids in patient suffering from a disease associated with hypolipidemia.

Thus, the present invention is directed to an *in vitro* method for evaluating whether a substance is a glutamine synthetase modulator in mammal cells, said method comprising:
(a) culturing mammal cells in presence or absence of at least one candidate substance whose ability to act as glutamine synthetase modulator is sought to be determined;
(b) stopping the culturing of the cells of step (a)
(c) determining levels of one or more glutamine regulated markers;
(d) comparing levels of said one or more glutamine regulated markers in the cells cultured with the candidate substance to those of cells cultured without said candidate substance;
(e) identifying the candidate substance as glutamine synthetase modulator on the basis of the comparison at step (d), and
wherein the glutamine synthetase modulator is a glutamine synthetase inhibitor or a glutamine synthetase activator.

In other words, the present invention is directed to an *in vitro* method for evaluating whether a substance is a glutamine synthetase modulator in mammal cells, said method comprising:
(a) culturing mammal cells in presence or absence of at least one candidate substance whose ability to act as glutamine synthetase modulator is sought to be determined;
(b) stopping the culturing of the cells of step (a)
(c) determining levels of one or more glutamine regulated markers;
(d) comparing levels of said one or more glutamine regulated markers in the cells cultured with the candidate substance to those of cells cultured without said candidate substance;
(e) assessing on the basis of the comparison at step (d) whether the tested candidate substance is a glutamine synthetase modulator, and
wherein the glutamine synthetase modulator is a glutamine synthetase inhibitor or a glutamine synthetase activator.

Reformulating, the present invention is directed to an *in vitro* method for screening for glutamine synthetase modulators in mammal cells, said method comprising:
(a) culturing mammal cells in presence or absence of at least one candidate substance whose ability to act as glutamine synthetase modulator is sought to be determined;
(b) stopping the culturing of the cells of step (a)
(c) determining levels of one or more glutamine markers;
(d) comparing levels of said one or more glutamine markers in the cells cultured with the candidate substance to those of cells cultured without said candidate substance;
(e) identifying the candidate substance as glutamine synthetase modulator on the basis of the comparison at step (d), and
wherein the glutamine synthetase modulator is a glutamine synthetase inhibitor or a glutamine synthetase activator.

Initial experiments showed that mammal cells cultured in vitro with glucose but not glutamine had 20% higher citrate levels than cells cultured with only glutamine for 8 hours (Fig.1B-D). However, citrate and other TCA-cycle intermediates significantly decreased after 24 hours of glutamine starvation (Fig. 3E).

Therefore, in some embodiments of the *in vitro* method for evaluating whether a substance is a glutamine synthetase modulator in mammal cells described herein, step (b) of stopping the culturing of the cells of step (a) is performed after at least 8 hours, or at least 9 hours, or at least 10 hours, or at least 11 hours, or at least 12 hours, or at least 13 hours, or at least 14 hours, or at least 15 hours, or at least 16 hours, or at least 17 hours, or at least 18 hours, or at least 19 hours, or at least 20 hours, or at least 21 hours, or at least 22 hours, or at least 23 hours, or at least 24 hours, or at least 25 hours, or at least 26 hours, or at least 27 hours, and more preferably after at least 24 hours.

### DEFINITIONS

### Glutamine synthetase (GS; EC 6.3.1.2)

The enzyme glutamine synthetase is responsible alone for *de novo* synthesis of glutamine. This ligase catalyses, under normal conditions, the formation of glutamine from glutamate and ammonia:

**Glutamate + NH₃ + ATP → L-glutamine + ADP + phosphate;**

GS is a large polymeric enzyme consisting of twelve subunits in case of bacterial proteins and ten in eukaryotic ones. There are twelve or ten active sites (respectively) located between two subunits. In vitro, GS converts around 90% of glutamate to glutamine when it is incubated with equal concentrations of L-glutamate, ammonium ion and ATP, in presence of Mg²⁺, at pH 7.0 and 37°C. GS activity depends on the concentrations of its substrates and products, of divalent cations like Mg²⁺ and Mn²⁺, and of ADP/ATP.

Beyond generating glutamine, GS plays a role in ammonia and glutamate detoxification, acid-base homeostasis, cell signaling, and cell proliferation. Glutamine is an abundant amino acid, and is important to the biosynthesis of several amino acids, pyrimidines, and purines.

The protein glutamine synthetase (GS) is also known as Glutamate-Ammonia Ligase (GLUL). This protein is encoded by the gene *GLUL,* Glutamate-Ammonia Ligase, NCBI Entrez Gene: 2752 Ensembl: ENSG00000135821. Mutations in *GLUL* are associated with congenital glutamine deficiency, and overexpression of *GLUL* was observed in some primary liver cancer samples.

Some compounds are able to bind GS active sites and partially block the action of the enzyme. These are for example amino acids like alanine, glycine, histidine, serine, tryptophane, or compounds such as adenosine monophosphate, carbamyl phosphate, cytidine triphosphate, glucosamine 6-phosphate, ... At increasing concentrations of these "compounds", more and more GS sites are occupied, eventually blocking GS enzymatic activity.

"Glutamine synthetase inhibitors", "glutamine synthetase activators" and "glutamine synthetase modulators" are used herein to refer to inhibitory, activating, or modulating substances identified by using the in vitro methods based on the principles of the invention. "Glutamine synthetase inhibitors", "glutamine synthetase activators" and "glutamine synthetase modulators" are also used herein to refer to inhibitory, activating, or modulating molecules identified by using the in vitro methods based on the principles of the invention

A "**GS modulator**" is either a substance acting as "**GS activator**" or a substance acting as "**GS inhibitor**".

A **GS activator** is defined as a substance that increases GS activity by stimulating a process selected from: *GLUL* expression, *GLUL* mRNA transcription, GS protein expression, *GLUL* mRNA stability, GS post-translational modification, GS conformational change, GS enzymatic activity, thereby increasing glutamine synthesis in the presence of GS substrates ammonia and glutamate, and of ATP and divalent cations.

A **GS inhibitor** is defined as a substance that inhibits GS activity by partially or totally preventing or blocking a process selected from: *GLUL* expression, *GLUL* mRNA transcription, GS protein expression, *GLUL* mRNA stability, GS post-translational modification, GS conformational change, GS enzymatic activity, thereby inhibiting GS enzymatic activity and thus blocking the synthesis of glutamine despite the presence of GS substrates ammonia and glutamate, and of ATP and divalent cations.

Although most of the known GS inhibitors are analogues of glutamate. Bisphosphonates and derivatives constitute the only known example of synthetic inhibitors which interacts most probably with the ATP binding site.

**L-methionine sulfoximine** (L-MSO or MSX), which has the systematic name 2-amino-4-(S-methylsulfonimidosyl) butanoic acid or L-s-[3-amino-3-carboxypropyl]-s-methylsulfoximine (C₅H₁₂N₂O₃S; F.W.: 180.22), is a rare aminoacid with a structural resemblance to glutamate. It occurs in nature and as a by-product of some forms of food processing.

Further known GS inhibitors have been described by Berlicki et al., (Mini-Reviews in Medicinal Chemistry, 2008, Vol. 8, No. 9) and include phosphinothricin analogues, derivatives of aminomethylenebisphosphonic acid, and γ-hydroxylysine.

A further GS inhibitor is L-α-Aminoadipic Acid.

### "Glutamine marker"

The term **"glutamine marker"** or **"glutamine level marker"** or **"glutamine synthetase marker"** refers to a metabolite, to a lipid, to a mRNA or to a protein, which level changes in conditions of glutamine reduction, of glutamine starvation, or of glutamine increase. Glutamine reduction or starvation can be due, for example, to a reduction or loss of glutamine synthetase activity or to a lower expression of *GLUL.*

Therefore, in some embodiments, the term **"glutamine marker"** refers to a "glutamine regulated mRNA". In some embodiments the term **"glutamine marker"** refers to a "glutamine regulated protein". In some embodiments the term **"glutamine marker"** refers to a "glutamine regulated lipid", which is preferably a fatty acid-derived lipid. In some embodiments the term **"glutamine marker"** refers to a "glutamine regulated metabolite", which is preferably a metabolite derived from the mevalonate pathway downstream of HMGCR.

**"Gene expression"** refers to the conversion of the information, contained in a gene, into a gene product. A gene product can be the direct transcriptional product of a gene (e.g. mRNA, tRNA, rRNA, antisense RNA, shRNAs, microRNAs (miRNAs) ribozyme, structural RNA), and preferentially mRNA, or a protein produced by translation of a mRNA.

**"Regulation of gene expression"** refers to a change in the expression of a gene. Regulation of expression can include, but is not limited to, gene activation and gene repression.

### Glutamine-regulated mRNA and proteins as "glutamine markers"

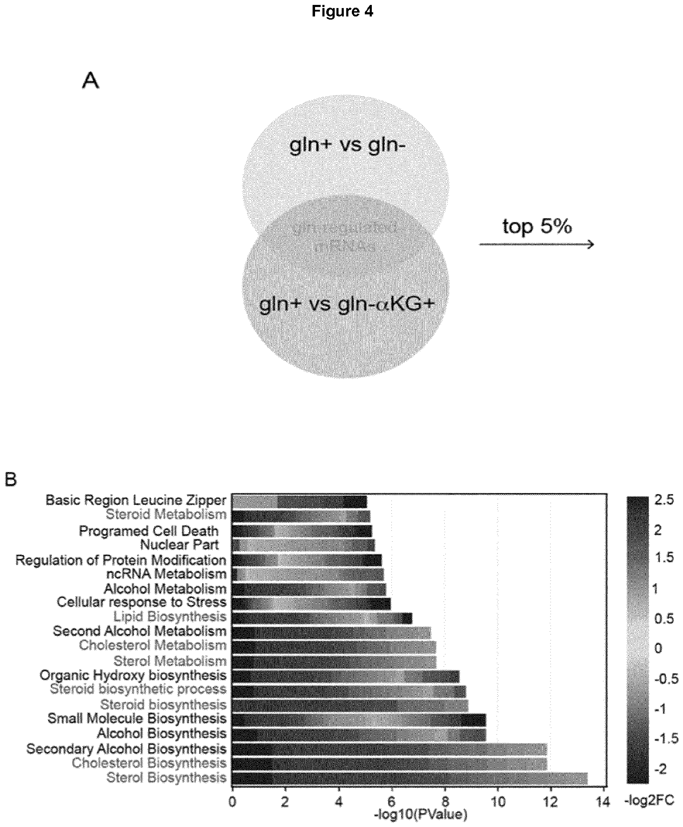

Glutamine-regulated genes (also referred to as "glutamine-responsive genes") are defined as genes differentially regulated between cell supplemented with glutamine and cells not supplemented (starved) with glutamine (Table 2 and 3, Figure 4 A-D).
Therefore, "glutamine-regulated genes" are also defined as genes which expression is induced or inhibited by a glutamine synthetase modulator such as a glutamine synthetase activator or inhibitor.

The expression of the "glutamine-regulated genes" used as "glutamine marker" can be measured at mRNA level, i.e. in terms of "glutamine-regulated mRNA" or "glutamine mRNA marker", or at protein level, i.e. in terms of "glutamine-regulated protein " or "glutamine protein marker ".

The glutamine-regulated mRNAs to be used for the inventive method to identify a glutamine synthetase activator or inhibitor are listed in Tables 2 and 3. The glutamine-regulated mRNAs characterized by a log2FC higher than +1.5 are referred to as "negative glutamine-regulated mRNAs" or "glutamine-inhibited mRNAs" (Table 2) and their level is stimulated by glutamine starvation or by GS inhibitor or inhibited by a GS activator or inhibited by glutamine.

In some embodiments, a "negative glutamine-regulated mRNAs" or "glutamine-inhibited mRNAs" (Table 2) refers to a glutamine-regulated mRNA characterized by a log2FC higher than +1.5, +1.6, +1.7, +1.8, +2.0, +2.3, +2.4, +2.5, and more preferably higher than +1.5.

The glutamine-regulated mRNAs characterized by a log2FC lower than -1.5 are referred to as "positive glutamine-regulated mRNAs " or "glutamine-stimulated mRNAs" (Table 3) and their level is inhibited by glutamin starvation or by a glutamin synthetase inhibitor or is stimulated by glutamin synthetase activator or by glutamin.
In some embodiments, a " positive glutamine-regulated mRNAs" or "glutamine-stimulated mRNAs" (Table 2) refers to a glutamine-regulated mRNA characterized by a log2FC lower than -1.5, -1.6, -1.7, -1.8, -2.0, -2.3, -2.4, -2.5, and more preferably lower than -1.5.

In other words, a tested candidate substance is identifed as glutamine synthetase inhibitor if the level of a glutamine inhibited mRNA in the cells cultured with the candidate substance is higher than + 1.5 and/or the level of a glutamine stimulated mRNA in the cells cultured with the candidate substance is lower than -1.5 compared to cells cultured without said candidate substance; or
identifying the tested candidate substance as glutamine synthetase activator if the level of a glutamine inhibited mRNA in the cells cultured with the candidate substance is lower than - 1.5 and/or the level of a glutamine stimulated mRNA in the cells cultured with the candidate substance is higher than +1.5 compared to cells cultured without said candidate substance; wherein the level of said mRNA is calculated as log2FC between the cells cultured with said candidate substance and the cells cultured without said candidate substance.

Therefore, a preferred "glutamine stimulated mRNA" for the inventive method is selected from the group comprising *FASN,* HSPA1A, CAVIN2, HSPA1B, HMGCR, JAKMIP2, NYNRIN, SH3TC2, INSIG1, ZBED2, CD180, TNFRSF11B, FAM111B, PNPLA3, THBS1, BMP4, MIAT, HSPA8, SORBS2, MAP1A, EPHB3, NES, LINC02615, SLC2A12, DPYSL2, HSPB1, CENPI, SAMD9L, TMEM97, FDFT1, HMMR, GAS6-AS1, ANXA6, BLACAT1, RHOBTB1, SQLE, PCYT2, TET1, TRIB2, MSMO1, NSDHL, ITGB8, CRISPLD1, UACA, ROR1, LSS, SYNC, LPCAT1, IDI1, PIK3R3, SOX2, MVK, PDGFB, SNAI3-AS1, DUBR, CSGALNACT1, PLD5P1, TUBA1A, IGSF10, SAMD9, SNX21, TNFSF9, ACSS2, PARP9, RTKN, PLCH1, NREP, ZMYM3, HEXIM1, MAGED1, TTC3P1, PGP, SLCO4C1, USP51, DHCR24. A preferred "glutamine inhibited mRNA" for the inventive method is selected from the group comprising TRIB3, UPP1, SPX, TSLP, MAFF, CLDN1, MYC, ATF3, RAB39B, CREB5, PPP1R15A, ERRFI1, ALOXE3, GADD45A, CDC42EP1, CTH, SESN2, ARC, DDIT3, CHRNA9, BTG1, KDM7A, SLC22A15, N4BP3, EGR1, DDIT4, NR1D1, ESRP1, INHBE, CXCL8, PTGS2, HK2, KDM6B, AREG, TNFRSF9, MYCT1, GEM, CHIC2, SNHG17, ASNS, FOSL1, FLNC, CD274, FHL2, FAM107B, SERPINE1, CCL4, ZNF697, EPAS1, PER2, ITPRIP, MTHFD2, ROCK1P1, MXD1, CEBPB, UNC5B, MAP2K3, RELB, ARHGEF2, PTX3, AKNATRIM25, NSC, SLC7A1, LIMA1, CHAC1, GPAT3, SLC38A1, EGFR, CNKSR3, RPLP0P2, LAT2, BEND7, TNFAIP3, ETV5, UHRF1BP1, SLC3A2, NFIL3, ABCB1, FIBIN, LINC01667, GPT2, RASGEF1C, LDAF1, IL1RL1, GDF15, NGF, NR4A1, TAF4B, SNHG1, GOT1, SLC6A9, ARID5A, SPNS2, CEBPG, LETM2, ADM2, EPB41L4A-AS1, MMP3, SNAPC1, DUSP2, NUPR1, VEGFA, OSBPL6, MAP1LC3B, SYNE1, FAM83G, PKD1P6, KCTD16, LINC00973, DUSP1, EBF3, HRK, PER1, CLUHP4, RAPH1, SPHK1, PKD1P1, PSAT1, SARS1, FGF21, DUSP5, IL6R, PRKCE, ABL2, GARS1, TSC22D3, PXK, TXNIP, SINHCAF, FGF2, FAM167A, NFATC1, SH3TC1, UAP1, RCL1, SLC1A5, SPRR2D, SLC1A4, SLC30A1, C3orf52, SMOX, CECR2, GABARAPL1, SYP, CBARP, ETS1, SNHG15, PFDN2, NIPAL4, LONRF2, IL31RA, EIF1B, CDO1, IL11, SERPINB8, SLC9A1, CSRNP1, CARS1, FUT1, RND3, TNFRSF10B, OLIG2, FAM241A, WDR43, DDR2, VGLL3, CYTOR, HBEGF, DUSP8, KLF10, ATXN1, DAGLB, AVPI1, GTPBP2, TMEM268, SH2B3, FBXO31, B3GALT5, SPOCD1, TIMM44, PHLDA2, SDC4, AEN, EGR2, MIDEAS, TRIB1, CYLD, CDK7, MOCOS, STK40, PTHLH, PCID2, ZFPM2-AS1, GPR137B, GAS5, SGMS2, DDX21, EREG, FYN, ZFAS1, BEX2, FOXO3, YARS1, ICOSLG, FKBP9, DUX4L26, IRF2BP2, PSPH.

The value log2FC was calculated in RNAseq experiments comparing gene expression between glutamine supplemented cells (gln+) and glutamine starved cells, wherein the group "glutamine starved cells" comprises glutamine starved cells (gln-) and glutamine starved cells supplemented with aKG (aKG+gln-) (Example 4, Table 2).
Therefore, the gene expression level can be calculated according to the present method as log2FC by performing RNAseq experiments and applying the method of Love, MI, et al., 2014.

Further gene expression and RNA quantification methods are also contemplated herein, such as Northern Blot, real-time polymerase chain reaction (qPCR), microarray, serial analysis of gene expression (SAGE) as described in the state of the art, for example in *Current Protocols in Molecular Biology,* John Wiley & Sons, Inc..

Therefore, a particular aspect of the present invention is directed to an *in vitro* method for evaluating whether a substance is a glutamine synthetase modulator in mammal cells, said method comprising:
(a) culturing mammal cells in presence or absence of at least one candidate substance whose ability to act as glutamine synthetase modulator is sought to be determined;
(b) stopping the culturing of the cells of step (a);
(c) determining levels of one or more glutamine markers;
(d) comparing levels of said one or more glutamine marker in the cells cultured with the candidate substance to those cells cultured without said candidate substance;
(e) identifying the candidate substance as glutamine synthetase modulator on the basis of the comparison at step (d),

wherein the glutamine synthetase modulator is a glutamine synthetase inhibitor or a glutamine synthetase activator, and
wherein the one or more glutamine marker is a "glutamine stimulated mRNA" selected from the group comprising *FASN,* HSPA1A, CAVIN2, HSPA1B, HMGCR, JAKMIP2, NYNRIN, SH3TC2, INSIG1, ZBED2, CD180, TNFRSF11B, FAM111B, PNPLA3, THBS1, BMP4, MIAT, HSPA8, SORBS2, MAP1A, EPHB3, NES, LINC02615, SLC2A12, DPYSL2, HSPB1, CENPI, SAMD9L, TMEM97, FDFT1, HMMR, GAS6-AS1, ANXA6, BLACAT1, RHOBTB1, SQLE, PCYT2, TET1, TRIB2, MSMO1, NSDHL, ITGB8, CRISPLD1, UACA, ROR1, LSS, SYNC, LPCAT1, IDI1, PIK3R3, SOX2, MVK, PDGFB, SNAI3-AS1, DUBR, CSGALNACT1, PLD5P1, TUBA1A, IGSF10, SAMD9, SNX21, TNFSF9, ACSS2, PARP9, RTKN, PLCH1, NREP, ZMYM3, HEXIM1, MAGED1, TTC3P1, PGP, SLCO4C1, USP51, DHCR24, and/or a "glutamine inhibited mRNA" selected from the group comprising TRIB3, UPP1, SPX, TSLP, MAFF, CLDN1, MYC, ATF3, RAB39B, CREB5, PPP1R15A, ERRFI1, ALOXE3, GADD45A, CDC42EP1, CTH, SESN2, ARC, DDIT3, CHRNA9, BTG1, KDM7A, SLC22A15, N4BP3, EGR1, DDIT4, NR1D1, ESRP1, INHBE, CXCL8, PTGS2, HK2, KDM6B, AREG, TNFRSF9, MYCT1, GEM, CHIC2, SNHG17, ASNS, FOSL1, FLNC, CD274, FHL2, FAM107B, SERPINE1, CCL4, ZNF697, EPAS1, PER2, ITPRIP, MTHFD2, ROCK1P1, MXD1, CEBPB, UNC5B, MAP2K3, RELB, ARHGEF2, PTX3, AKNATRIM25, NSC, SLC7A1, LIMA1, CHAC1, GPAT3, SLC38A1, EGFR, CNKSR3, RPLP0P2, LAT2, BEND7, TNFAIP3, ETV5, UHRF1BP1, SLC3A2, NFIL3, ABCB1, FIBIN, LINC01667, GPT2, RASGEF1C, LDAF1, IL1RL1, GDF15, NGF, NR4A1, TAF4B, SNHG1, GOT1, SLC6A9, ARID5A, SPNS2, CEBPG, LETM2, ADM2, EPB41L4A-AS1, MMP3, SNAPC1, DUSP2, NUPR1, VEGFA, OSBPL6, MAP1LC3B, SYNE1, FAM83G, PKD1P6, KCTD16, LINC00973, DUSP1, EBF3, HRK, PER1, CLUHP4, RAPH1, SPHK1, PKD1P1, PSAT1, SARS1, FGF21, DUSP5, IL6R, PRKCE, ABL2, GARS1, TSC22D3, PXK, TXNIP, SINHCAF, FGF2, FAM167A, NFATC1, SH3TC1, UAP1, RCL1, SLC1A5, SPRR2D, SLC1A4, SLC30A1, C3orf52, SMOX, CECR2, GABARAPL1, SYP, CBARP, ETS1, SNHG15, PFDN2, NIPAL4, LONRF2, IL31RA, EIF1B, CDO1, IL11, SERPINB8, SLC9A1, CSRNP1, CARS1, FUT1, RND3, TNFRSF10B, OLIG2, FAM241A, WDR43, DDR2, VGLL3, CYTOR, HBEGF, DUSP8, KLF10, ATXN1, DAGLB, AVPI1, GTPBP2, TMEM268, SH2B3, FBXO31, B3GALT5, SPOCD1, TIMM44, PHLDA2, SDC4, AEN, EGR2, MIDEAS, TRIB1, CYLD, CDK7, MOCOS, STK40, PTHLH, PCID2, ZFPM2-AS1, GPR137B, GAS5, SGMS2, DDX21, EREG, FYN, ZFAS1, BEX2, FOXO3, YARS1, ICOSLG, FKBP9, DUX4L26, IRF2BP2, PSPH,
and wherein step (e) further comprises:
identifying the tested candidate substance as glutamine synthetase inhibitor if the level of a glutamine inhibited mRNA in the cells cultured with the candidate substance is higher than + 1.5 and/or the level of a glutamine stimulated mRNA in the cells cultured with the candidate substance is lower than - 1.5 compared to cells cultured without said candidate substance; or
identifying the tested candidate substance as glutamine synthetase activator if the level of a glutamine inhibited mRNA in the cells cultured with the candidate substance is lower than - 1.5 and/or the level of a glutamine stimulated mRNA in the cells cultured with the candidate substance is higher than + 1.5 compared to cells cultured without said candidate substance; and
wherein the level of said mRNA is calculated as log2FC between the cells cultured with said candidate substance and the cells cultured without said candidate substance.

A still more particular aspect of the present invention is directed to an *in vitro* method for evaluating whether a substance is a glutamine synthetase modulator in mammal cells, said method comprising:
(a) culturing mammal cells in presence or absence of at least one candidate substance whose ability to act as glutamine synthetase modulator is sought to be determined;
(b) stopping the culturing of the cells of step (a);
(c) determining levels of one or more glutamine markers;
(d) comparing levels of said one or more glutamine marker in the cells cultured with the candidate substance to those cells cultured without said candidate substance;
(e) identifying the candidate substance as glutamine synthetase modulator on the basis of the comparison at step (d),

wherein the glutamine synthetase modulator is a glutamine synthetase inhibitor or a glutamine synthetase activator, and
wherein the one or more glutamine marker is a glutamine stimulated mRNA *HMGCR,* and
wherein step (e) further comprises:
   identifying the tested candidate substance as glutamine synthetase inhibitor if the level of a glutamine inhibited mRNA in the cells cultured with the candidate substance is higher than + 1.5 and/or the level of a glutamine stimulated mRNA in the cells cultured with the candidate substance is lower than - 1.5 compared to cells cultured without said candidate substance; or
   identifying the tested candidate substance as glutamine synthetase activator if the level of a glutamine inhibited mRNA in the cells cultured with the candidate substance is lower than - 1.5 and/or the level of a glutamine stimulated mRNA in the cells cultured with the candidate substance is higher than + 1.5 compared to cells cultured without said candidate substance; and
wherein the level of said mRNA is calculated as log2FC between the cells cultured with said candidate substance and the cells cultured without said candidate substance.

**"Glutamine-regulated proteins"** are proteins which expression or activity is induced or inhibited by a glutamine synthetase modulator such as a glutamine synthetase activator or inhibitor. Preferred **"glutamine-regulated proteins"** are SREBP2, HMGCR, FDFT1, c-MYC, FASN, HMGCS1, FDPS, LDLR and LSS. The expression of SREBP2, HMGCR, FDFT1, c-MYC, FASN, HMGCS1, FDPS, LDLR and LSS is induced by glutamine supplementation (**Figure 4E**), and therefore they are **"glutamine-stimulated proteins".**

Therefore, a tested candidate substance is identifed as a glutamine synthetase inhibitor if the level of said "glutamine-stimulated proteins" selected from SREBP2, HMGCR, FDFT1, S6K, c-MYC, FASN, HMGCS1, FDPS, LDLR and LSS, is at least 10%, or 15 %, or 20 %, or 25%, or 30%, or 35%, or 40%, or 45%, or 50%, and more preferably 10% lower compared to cells cultured without said candidate substance, or a tested candidate substance is identifed as a glutamine synthetase activator if the level of said "glutamine-stimulated proteins" selected from SREBP2, HMGCR, FDFT1, c-MYC, FASN, HMGCS1, FDPS, LDLR and LSS, is at least 10%, or 15 %, or 20 %, or 25%, or 30%, or 35%, or 40%, or 45%, or 50%, and more preferably 10% higher compared to cells cultured without said candidate substance.

Methods to quantify protein expression are known in the state of the art and include, but are not limited to, immunoblotting, ELISA, mass-spectrometry as described in the state of the art, for example in *Current Protocols in Molecular Biology,* John Wiley & Sons, Inc..

Thus, an aspect of the present invention is directed to an *in vitro* method for evaluating whether a substance is a glutamine synthetase modulator in mammal cells, said method comprising:
(a) culturing mammal cells in presence or absence of at least one candidate substance whose ability to act as glutamine synthetase modulator is sought to be determined;
(b) stopping the culturing of the cells of step (a);
(c) determining levels of one or more glutamine markers;
(d) comparing levels of said one or more glutamine marker in the cells cultured with the candidate substance to those cells cultured without said candidate substance;
(e) identifying the candidate substance as glutamine synthetase modulator on the basis of the comparison at step (d),

wherein the glutamine synthetase modulator is a glutamine synthetase inhibitor or a glutamine synthetase activator, and
wherein the one or more glutamine marker is a glutamine stimulated **protein** selected from SREBP2, HMGCR, FDFT1, c-MYC, FASN, HMGCS1, FDPS, LDLR and LSS, and wherein step (e) further comprises:
   identifying the tested candidate substance as a glutamine synthetase inhibitor if the level of said "glutamine- stimulated proteins" is at least 10% lower compared to cells cultured without said candidate substance, or identifying the tested candidate substance as a glutamine synthetase activator if the level of said "glutamine-- stimulated proteins" is at least 10% higher compared to cells cultured without said candidate substance.

A more particular aspect of the present invention is directed to an *in vitro* method for evaluating whether a substance is a glutamine synthetase modulator in mammal cells, said method comprising:
(a) culturing mammal cells in presence or absence of at least one candidate substance whose ability to act as glutamine synthetase modulator is sought to be determined;
(b) stopping the culturing of the cells of step (a);
(c) determining levels of one or more glutamine markers;
(d) comparing levels of said one or more glutamine marker in the cells cultured with the candidate substance to those cells cultured without said candidate substance;
(e) identifying the candidate substance as glutamine synthetase modulator on the basis of the comparison at step (d),

wherein the glutamine synthetase modulator is a glutamine synthetase inhibitor or a glutamine synthetase activator, and
wherein the one or more glutamine marker is a glutamine stimulated **protein** selected from SREBP2, HMGCR, FDFT1, c-MYC, FASN, HMGCS1, FDPS, LDLR and LSS, and
   wherein step (e) further comprises:
   identifying the tested candidate substance as a glutamine synthetase inhibitor if the level of said "glutamine- stimulated proteins" is at least 10% lower compared to cells cultured without said candidate substance, or identifying the tested candidate substance as a glutamine synthetase activator if the level of said "glutamine-- stimulated proteins" is at least 10% higher compared to cells cultured without said candidate substance, and
wherein step (c) further comprises determining proteolytic activation of the glutamine stimulated protein and/or the glutamine stimulated protein is SREBP2.

### "Mevalonate pathway"

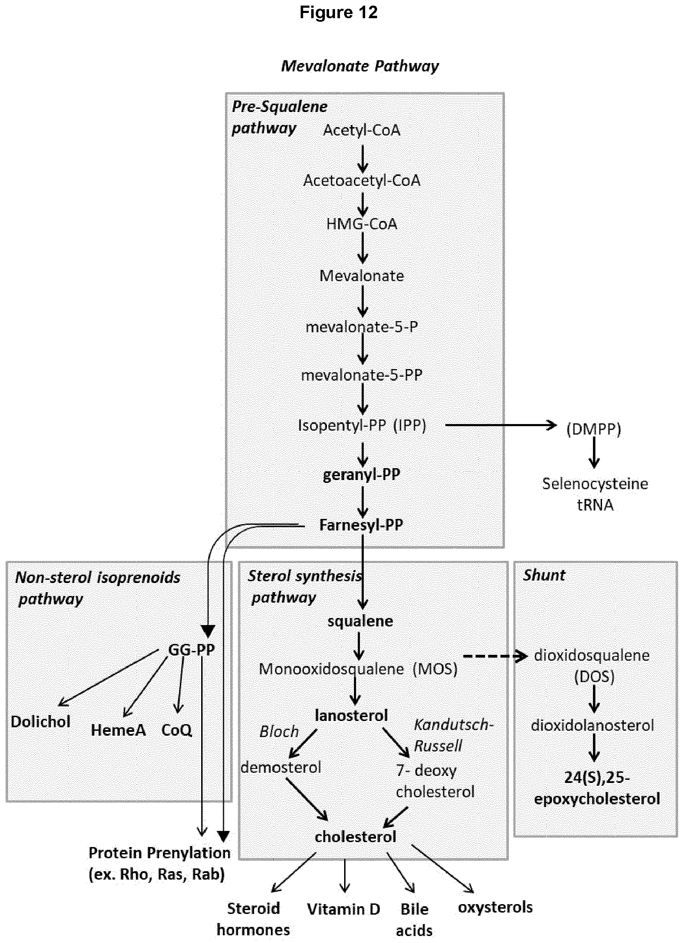

The **mevalonate pathway** (MEV pathway) is a biosynthetic pathway responsible for the synthesis of mevalonate (**Figure 12**), which act as precursor of isoprenoid groups that are incorporated into different classes of end-products, including 1) sterols, such as cholesterol, 2) heme A and 3) ubiquinone, involved in electron transport; 4) dolichol, required for glycoprotein synthesis; 5) isopentenyladenine, present in some transfer RNAs; and intercellular messengers, such as cytokines in plants, 6) farnesylated mating factors in fungi, 7) juvenile hormones in insects and 8) steroid hormones, bile acids, and oxysterols in animals.

Non-sterol isoprenoids such as ubiquinone and geranyl-geranyl-PP are in high demand by rapidly dividing cells.

The **mevalonate pathway** converts acetyl-CoA to isopentenyl pyrophosphate (IPP). The mevalonate pathway comprises the following steps: (a) condensing two molecules of acetyl-CoA to acetoacetyl-CoA; (b) condensing acetoacetyl-CoA with acetyl-CoA to form HMG-CoA; (c) converting HMG-CoA to mevalonate; (d) phosphorylating mevalonate to mevalonate 5-phosphate; (e) converting mevalonate 5-phosphate to mevalonate 5-pyrophosphate; and (f) converting mevalonate 5-pyrophosphate to IPP.

IPP and its isomer DMAPP are conjugated into the 10-carbon geranyl diphosphate/pyrophosphate (GPP) and sequential additions of IPP are added to GPP to generate a 15-carbon unit called farnesyl diphosphate/pyrophosphate (FPP). Both GPP and FPP are synthesized by the activity of FPP synthase (FPPS, farnesyl diphosphate synthase, FDPS). The FPP molecule serves as a major branch point in the pathway where it either is converted to squalene entering the **"sterol synthesis pathway"** or continues into the **"nonsterol isoprenoid pathway or branch"** (**Figure 12**). The word **"pyrophosphate"** is used interchangeably herein with **"diphosphate."**

The nonsterol isoprenoid branch of the mevalonate pathway is thus responsible for the production of: dolichol, a glycosyl lipid carrier for glycosylation of proteins, Heme A, an iron-chelating cofactor of cytochrome c oxidase involved in mitochondrial respiratory chain; ubiquinone-10 (CoQ10), which serves as an antioxidant by preventing mitochondrial oxidative stress; FPP and GGPP comprising prenyl groups for prenylation of proteins possessing a characteristic C-terminal motif; geranylgeranyl diphosphate/pyrophosphate (GGPP) is also used to prenylate menadione (MD, vitamin K3) released from dietary vitamin K1 to produce a subtype of vitamin K2 called menaquinone-4(MK-4).

Protein prenylation plays a pivotal regulatory role by targeting proteins to membranes subsequently stimulating downstream signaling pathways of proteins.

The **sterol synthesis pathway** downstream of the mevalonate pathway is responsible for the synthesis of: squalene, lanosterol, desmosterol, epoxycholesterol (EC), dihydrolanosterol (DHL), dehydrocholesterols, and cholesterol, which serves as precursor for oxysterols, steroid hormones, vitamin D, and bile acids.

Cholesterol is a hydrophobic lipid inserted in the phospholipid bilayer of biological membranes including the plasma membrane and other organelles such as the endoplasmic reticulum (ER), Golgi apparatus, mitochondria, nuclear membrane, etc. Cholesterol regulates the fluidity and rigidity of membranes and dynamically changes in response to environmental conditions. Cells obtain cholesterol by two mechanisms: 1) de novo synthesis from the mevalonate pathway; and 2) uptake of extracellular low-density lipoprotein (LDL) by the LDL receptor.

Because cholesterol is essential for organismal homeostasis but insoluble in aqueous environments, any imbalance in its levels can cause serious disease. A deficiency of cholesterol due to defects in genes involved in its synthesis causes malformation syndromes such as SLOS, Lathosterolosis, Desmosterolosis, CDPX2, CHILD Syndrome, SC4MOL, Antley-Bixler, HEM dysplasia. Moreover, the condition of total cholesterol levels below 160 mg/dL or 4.1 mmol/L is classified as **"hypocholesterolemia",** which can be due to different causes or disorders, such as hyperthyroidism, adrenal insufficiency, liver disease, malabsorption, malnutrition, abetalipoproteinemia, hypobetalipoproteinemia, manganese deficiency, Smith-Lemli-Opitz syndrome, Marfan syndrome, leukemias.

Conversely, the accumulation of cholesterol can decrease membrane fluidity, disrupt membrane micro-domains, alter membrane protein function, and ultimately lead to cell dysfunction and death, indicating a toxic effect of cholesterol accumulation.

A preferred **glutamine regulated metabolite** usable as **"glutamine marker"** is a **"metabolite derived from the mevalonate pathway downstream of HMGCR"** that is differentially regulated between cell supplemented with glutamine and cells not supplemented with glutamine, i.e. cells starved of glutamine (Fig. 1D, 1E, 1G, 1H).

Table 1 lists **"metabolites derived from the mevalonate pathway downstream of HMGCR"** such as mevalonate pathway intermediates, products, and derived metabolites, which can be used as **"glutamine markers".**

The term **"metabolite derived from the mevalonate pathway downstream of HMGCR" or " mevalonate pathway derived metabolite"** is used herein to refer to **"intermediates and products of the mevalonate pathway downstream of HMGCR"** as well as to **"mevalonate pathway derived metabolite".**

Thus, the term **"metabolite derived from the mevalonate pathway downstream of HMGCR"** refers to a substance selected from the group comprising HMG-CoA, mevalonate, mevalonate-5-P, mevalonate-5-PP, Isopentyl-5-PP, farnesyl-PP, squalene, lanosterol, dihydrolanosterol (DHL), desmosterol, epoxycholesterol (EC), dehydrocholesterols, cholesterol, geranyl-PP, geranylgeranyl-PP, dolicyl-PP, dolichols; coenzyme Q10, Vitamin K2, Vitamin D, steroid hormones, bile acid, Heme A

The terms **"isoprenoid,"** "isoprenoid compound," "terpene," "terpene compound," "terpenoid," and "terpenoid compound" are used interchangeably herein. Isoprenoid compounds are made up of isoprene (C5) units. The number of C-atoms present in the isoprenoids is typically evenly divisible by five, e.g., C5, C1O, C15, C20, C25, C30 and C40), but also irregular isoprenoids and polyterpenes have been reported.

Isoprenoid compounds include, but are not limited to, monoterpenes C₁₀H₁₆; sesquiterpenes, C₁₅H₂₄, diterpenes, C₂₀H₃₂; triterpenes, C₃₀H₄₈; tetraterpenes, C₄₀H₆₄; and polyterpenes, (C₅H₈)*ₙ*.

Therefore, a particular aspect of the present invention is directed to an *in vitro* method for evaluating whether a substance is a glutamine synthetase modulator in mammal cells, said method comprising:
(a) culturing mammal cells in presence or absence of at least one candidate substance whose ability to act as glutamine synthetase modulator is sought to be determined;
(b) stopping the culturing of the cells of step (a);
(c) determining levels of one or more glutamine markers;
(d) comparing levels of said one or more glutamine marker in the cells cultured with the candidate substance to those cells cultured without said candidate substance;
(e) identifying the candidate substance as glutamine synthetase modulator on the basis of the comparison at step (d), and
   wherein the glutamine synthetase modulator is a glutamine synthetase inhibitor or a glutamine synthetase activator, and
   wherein step (e) comprises:
      identifying the tested candidate substance as a glutamine synthetase inhibitor if the level of citrate in the cells cultured with said candidate substance is at least 10% lower compared to cells cultured without said candidate substance, and/or the level of said fatty acid-derived lipid in the cells cultured with said candidate substance is at least 10% lower compared to cells cultured without said candidate substance and/or the level of the metabolite derived from the mevalonate pathway downstream of HMGCR in the cells cultured with said candidate substance is at least 10% lower compared to cells cultured without said candidate substance, or
      identifying the tested candidate substance as a glutamine synthetase activator if the level of citrate in the cells cultured with said candidate substance is at least 10% higher to cells cultured without said candidate substance, and/or the level of said fatty acid-derived lipid in the cells cultured with said candidate substance is at least 10% higher compared to cells cultured without said candidate substance and/or the level of the metabolite derived from the mevalonate pathway downstream of HMGCR in the cells cultured with said candidate substance is at least 10% higher compared to cells cultured without said candidate substance,
      wherein the metabolite derived from the mevalonate pathway downstream of HMGCR is selected from the group comprising: HMG-CoA, mevalonate, mevalonate-5-P, mevalonate-5-PP, Isopentyl-5-PP, farnesyl-PP, squalene, lanosterol, dihydrolanosterol (DHL), desmosterol, epoxycholesterol (EC), dehydrocholesterols, cholesterol, geranyl-PP, geranylgeranyl-PP, dolicyl-PP, dolichol, coenzyme Q10, Vitamin K2, Vitamin D, steroid hormones, bile acid, Heme A.

### Fatty acids

The term **"fatty acid"** refers to a compound of the formula RCOOH, where R is a hydrocarbon. An unsaturated fatty acid refers to a compound where R includes at least one carbon-carbon double bond (**Table 1b**). A polyunsaturated fatty acid refers to a compound where R includes a plurality of carbon-carbon double bonds. A saturated fatty acid refers to a compound where R is a saturated aliphatic group (**Table 1a**).

The pathway **"fatty acid synthesis"** is responsible for the synthesis of fatty acids from acetyl-CoA and NADPH through the action of fatty acid synthases. This process takes place in the cytoplasm of the cell. Citrate from the mitochondrial matrix is shuttled to the cytosol via the citrate shuttle. This citrate is then converted by ATP citrate lyase into Acetyl CoA, which then can be used for the "Fatty acid synthesis".

The term **"lipid"** refers to organic compounds that are insoluble in water and soluble in organic solvents. Lipids are esters of fatty acids, and comprise diglycerides, triglycerides, phospholipids, glycerophospholipids, ether phospholipids (plasmalogens), sphingolipids, and isoprenoids (cholesterol, ubiquinone, dolichol).

The term "**fatty acid-derived lipid"** as used herein refers to a substance selected from the group comprising diglycerides, triglycerides, phospholipids, sphingolipids, and steroids. In particular, the term "**fatty acid-derived lipid"** as used herein refers to a substance selected from the group comprising
- a fatty acid with a carbon chain length between 16 and 24 carbon atoms as listed in Table 1a, and b,
- a phospholipid containing at least one acyl group with a carbon chain length between 16 and 24 carbon atoms as listed in Table 1a, and b,
- a diacylglicerol containing at least one acyl group with a carbon chain length between 16 and 24 carbon atoms as listed in Table 1a, and b,
- a triglyceride containing at least one acyl group with a carbon chain length between 16 and 24 carbon atoms as listed in Table 1a, and b, and
- a sphingolipid containing at least one acyl group with a carbon chain length between 16 and 24 carbon atoms as listed in Table 1a, and b.

In chemistry, an "**acyl group**" is a moiety derived by the removal of one or more hydroxyl groups from an oxoacid, including inorganic acids. Normally, the acyl group is derived from a naturally occurring fatty acid and the fatty acid usually contains an even number of carbon atoms and is unbranched.

Preferred fatty acids for carry out the present invention are "unsaturated and saturated fatty acids with a carbon chain length between 16 and 24 carbon atoms as listed in Table 1a, and b.

The number of carbon atoms in the unsaturated and saturated fatty acids for carry out the present invention is preferably from 16 to 24, more preferably from 18 to 24, still more preferably from 20 to 24, more preferably from 14 to 24, more preferably from 16 to 24, more preferably from 18 to 24, still more preferably from 20 to 24. preferably from 16 to 22, more preferably from 18 to 22, still more preferably from 20 to 22, more preferably from 16 to 20, more preferably from 18 to 20. Thus, preferred fatty acids for carry out the present invention are "fatty acids with a carbon chain length between 16 and 24 carbon atoms, more preferably from 18 to 24 carbon atoms, still more preferably from 20 to 24 carbon atoms, more preferably from 14 to 24 carbon atoms, more preferably from 16 to 24 carbon atoms, more preferably from 18 to 24 carbon atoms, still more preferably from 20 to 24 carbon atoms. preferably from 16 to 22 carbon atoms, more preferably from 18 to 22 carbon atoms, still more preferably from 20 to 22 carbon atoms, more preferably from 16 to 20 carbon atoms, more preferably from 18 to 20 carbon atoms. Of those, Palmitic acid, Stearic acid, Arachidic acid, Behenic acid, Lignoceric acid, α-Linolenic acid , Stearidonic acid, Eicosapentaenoic acid, Cervonic acid, Linoleic acid, Linolelaidic acid, γ-Linolenic acid, Dihomo-γ-linolenic acid, Arachidonic acid, Docosatetraenoic acid, Palmitoleic acid, Vaccenic acid, Paullinic acid, Oleic acid, Elaidic acid, Gondoic acid, Erucic acid, Nervonic acid, Mead acid are preferred.

Thus, a particular aspect of the present invention is directed to an *in vitro* method for evaluating whether a substance is a glutamine synthetase modulator in mammal cells, said method comprising:
(a) culturing mammal cells in presence or absence of at least one candidate substance whose ability to act as glutamine synthetase modulator is sought to be determined;
(b) stopping the culturing of the cells of step (a);
(c) determining levels of one or more glutamine markers;
(d) comparing levels of said one or more glutamine marker in the cells cultured with the candidate substance to those cells cultured without said candidate substance;
(e) identifying the candidate substance as glutamine synthetase modulator on the basis of the comparison at step (d), and

wherein the glutamine synthetase modulator is a glutamine synthetase inhibitor or a glutamine synthetase activator, and
wherein step (e) comprises:
   identifying the tested candidate substance as a glutamine synthetase inhibitor if the level of citrate in the cells cultured with said candidate substance is at least 10% lower compared to cells cultured without said candidate substance, and/or the level of said fatty acid-derived lipid in the cells cultured with said candidate substance is at least 10% lower compared to cells cultured without said candidate substance and/or the level of the metabolite derived from the mevalonate pathway downstream of HMGCR in the cells cultured with said candidate substance is at least 10% lower compared to cells cultured without said candidate substance, or
   identifying the tested candidate substance as a glutamine synthetase activator if the level of citrate in the cells cultured with said candidate substance is at least 10% higher to cells cultured without said candidate substance, and/or the level of said fatty acid-derived lipid in the cells cultured with said candidate substance is at least 10% higher compared to cells cultured without said candidate substance and/or the level of the metabolite derived from the mevalonate pathway downstream of HMGCR in the cells cultured with said candidate substance is at least 10% higher compared to cells cultured without said candidate substance, and
   wherein the fatty acid-derived lipid is selected from the group comprising a fatty acid with a carbon chain length between 16 and 24 carbon atoms, a phospholipid comprising acyl groups with a carbon chain length between 16 and 24 carbon atoms, a diacylglycerol comprising acyl groups with a carbon chain length between 16 and 24 carbon atoms, a triglyceride comprising acyl groups with a carbon chain length between 16 and 24 carbon atoms, and a sphingolipid comprising acyl groups with a carbon chain length between 16 and 24 carbon atoms.

Thus, a more particular aspect of the present invention is directed to an *in vitro* method for evaluating whether a substance is a glutamine synthetase modulator in mammal cells, said method comprising:
(a) culturing mammal cells in presence or absence of at least one candidate substance whose ability to act as glutamine synthetase modulator is sought to be determined;
(b) stopping the culturing of the cells of step (a);
(c) determining levels of one or more glutamine markers;
(d) comparing levels of said one or more glutamine marker in the cells cultured with the candidate substance to those cells cultured without said candidate substance;
(e) identifying the candidate substance as glutamine synthetase modulator on the basis of the comparison at step (d), and

wherein the glutamine synthetase modulator is a glutamine synthetase inhibitor or a glutamine synthetase activator, and
wherein step (e) comprises:
   identifying the tested candidate substance as a glutamine synthetase inhibitor if the level of citrate in the cells cultured with said candidate substance is at least 10% lower compared to cells cultured without said candidate substance, and/or the level of said fatty acid-derived lipid in the cells cultured with said candidate substance is at least 10% lower compared to cells cultured without said candidate substance and/or the level of the metabolite derived from the mevalonate pathway downstream of HMGCR in the cells cultured with said candidate substance is at least 10% lower compared to cells cultured without said candidate substance, or
   identifying the tested candidate substance as a glutamine synthetase activator if the level of citrate in the cells cultured with said candidate substance is at least 10% higher to cells cultured without said candidate substance, and/or the level of said fatty acid-derived lipid in the cells cultured with said candidate substance is at least 10% higher compared to cells cultured without said candidate substance and/or the level of the metabolite derived from the mevalonate pathway downstream of HMGCR in the cells cultured with said candidate substance is at least 10% higher compared to cells cultured without said candidate substance, and
   wherein the metabolite derived from the mevalonate pathway downstream of HMGCR is selected from the group comprising: HMG-CoA, mevalonate, mevalonate-5-P, mevalonate-5-PP, Isopentyl-5-PP, farnesyl-PP, squalene, lanosterol, dihydrolanosterol (DHL), desmosterol, epoxycholesterol (EC), dehydrocholesterols, cholesterol, geranyl-PP, geranylgeranyl-PP, dolicyl-PP, dolichol, coenzyme Q10, Vitamin K2, Vitamin D, steroid hormones, bile acid, Heme A; and/or
   wherein the fatty acid-derived lipid is selected from the group comprising a fatty acid with a carbon chain length between 16 and 24 carbon atoms, a phospholipid comprising acyl groups with a carbon chain length between 16 and 24 carbon atoms, a diacylglycerol comprising acyl groups with a carbon chain length between 16 and 24 carbon atoms, a triglyceride comprising acyl groups with a carbon chain length between 16 and 24 carbon atoms, and a sphingolipid comprising acyl groups with a carbon chain length between 16 and 24 carbon atoms.

A further particular aspect of the present invention is directed to an *in vitro* method for evaluating whether a substance is a glutamine synthetase modulator in mammal cells, said method comprising:
(a) culturing mammal cells in presence or absence of at least one candidate substance whose ability to act as glutamine synthetase modulator is sought to be determined;
(b) stopping the culturing of the cells of step (a);
(c) determining levels of one or more glutamine markers;
(d) comparing levels of said one or more glutamine marker in the cells cultured with the candidate substance to those cells cultured without said candidate substance;
(e) identifying the candidate substance as glutamine synthetase modulator on the basis of the comparison at step (d),

wherein the glutamine synthetase modulator is a glutamine synthetase inhibitor or a glutamine synthetase activator, and
wherein the fatty acid -derived lipid is selected from the group comprising palmitic acid, a phosphatidylcholine, phosphatidylserine, phosphatidylethanoline, phosphatidylglycerol, phosphatidylinositol, diacylglycerol, triglyceride, sphingomyelin, wherein said phosphatidylcholine, phosphatidylserine, phosphatidylethanoline, phosphatidylglycerol, phosphatidylinositol, diacylglycerol, triglyceride, and sphingomyelin, comprise acyl groups with a carbon chain length between 16 and 24 carbon atoms, and preferably
wherein step (e) comprises:
   identifying the tested candidate substance as a glutamine synthetase inhibitor if the level of citrate in the cells cultured with said candidate substance is at least 10% lower compared to cells cultured without said candidate substance, and/or the level of said fatty acid-derived lipid in the cells cultured with said candidate substance is at least 10% lower compared to cells cultured without said candidate substance and/or the level of the metabolite derived from the mevalonate pathway downstream of HMGCR in the cells cultured with said candidate substance is at least 10% lower compared to cells cultured without said candidate substance, or
      identifying the tested candidate substance as a glutamine synthetase activator if the level of citrate in the cells cultured with said candidate substance is at least 10% higher to cells cultured without said candidate substance, and/or the level of said fatty acid-derived lipid in the cells cultured with said candidate substance is at least 10% higher compared to cells cultured without said candidate substance and/or the level of the metabolite derived from the mevalonate pathway downstream of HMGCR in the cells cultured with said candidate substance is at least 10% higher compared to cells cultured without said candidate substance..

Thus, preferred saturated free fatty acids from which the acyl groups may be derived from include, but are not limited to: hexadecanoic (palmitic); octadecanoic (stearic); eicosanoic (arachidic); docosanoic (behenic); tetracosanoic (lignoceric). Preferred unsaturated fatty acids from which the acyl groups may be derived from include, but are not limited to: α-Linolenic acid , Stearidonic acid, Eicosapentaenoic acid, Cervonic acid, Linoleic acid, Linolelaidic acid, γ-Linolenic acid, Dihomo-γ-linolenic acid, Arachidonic acid, Docosatetraenoic acid, Palmitoleic acid, Vaccenic acid, Paullinic acid, Oleic acid, Elaidic acid, Gondoic acid, Erucic acid, Nervonic acid, Mead acid.

The term **"acyl group"** may also be referred to by the letter C followed by the number of carbons in the linear aliphatic chain, as, for example, C16 refers to an acyl group of 16 carbons in length. An acyl group includes a functionalized acyl group as defined herein.

The term **"sn"** in reference to a Carbon atom refers to a stereospecific numbering system used for naming glycerolipid molecules. Sn as a prefix, such as "sn glycerol" refers to the position number of the Carbon (C) atom in the glycerol backbone of a lipid molecule.

The terms "**diacylglycerol**", "**DAG**", "**diglyceride**" refer to a molecule comprising a glycerol backbone to which two acyl groups are esterified. Typically, the acyl groups are esterified to the sn-1 and sn-2 positions (Formula (**I**)), although the acyl groups may also be esterified to the sn-1 and sn-3 positions (Formula(**II**)), or to the sn-2 and sn-3 positions; the remaining position is unesterified and contains a hydroxyl group.

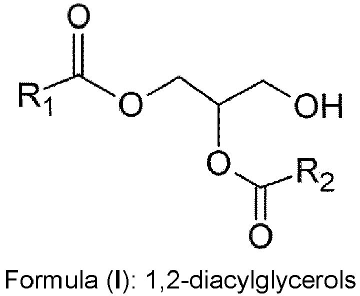

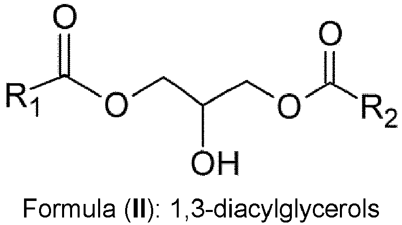

Preferred fatty-acid derived lipids for carry out the present invention are diacylglicerols containing at least one acyl group with a carbon chain length between 16 and 24 carbon atoms as listed in Table 1a, and b.

Thus, the herein preferred **diacylglicerols** have the following general formula (**I**): 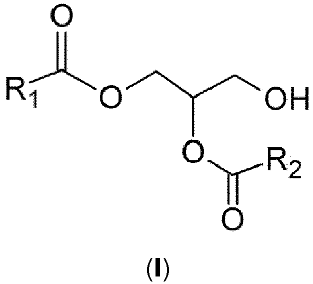 wherein R₁, and R₂ are acyl groups selected independently of each other from Palmitic acid, Stearic acid, Arachidic acid, Behenic acid, Lignoceric acid, α-Linolenic acid , Stearidonic acid, Eicosapentaenoic acid, Cervonic acid, Linoleic acid, Linolelaidic acid, γ-Linolenic acid, Dihomo-γ-linolenic acid, Arachidonic acid, Docosatetraenoic acid, Palmitoleic acid, Vaccenic acid, Paullinic acid, Oleic acid, Elaidic acid, Gondoic acid, Erucic acid, Nervonic acid, Mead acid (Table 1a and 1b).

Further preferred **diacylglicerols** have the following general formula (**II**): 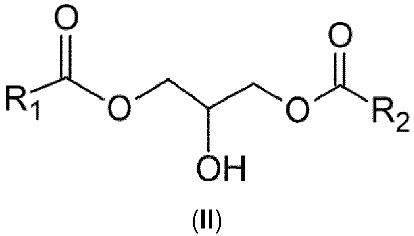 wherein R₁, and R₂ are independently of each other selected from Palmitic acid, Stearic acid, Arachidic acid, Behenic acid, Lignoceric acid, α-Linolenic acid , Stearidonic acid, Eicosapentaenoic acid, Cervonic acid, Linoleic acid, Linolelaidic acid, γ-Linolenic acid, Dihomo-γ-linolenic acid, Arachidonic acid, Docosatetraenoic acid, Palmitoleic acid, Vaccenic acid, Paullinic acid, Oleic acid, Elaidic acid, Gondoic acid, Erucic acid, Nervonic acid, Mead acid (Table 1a and 1b).

The term ""**triacylglycerol**", or "**triglyceride"** as used herein, refers to a chemical compound of glycerol (glycerin) esterified with three fatty acids, i.e., triple esterified glycerols (glycerins). Triglyceride or glycerol triester are synonymous terms of triacylglycerol.

Triglycerides have the following general formula (**III**): 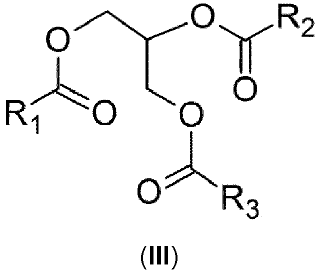 wherein R₁, R₂ and R₃ represent acyl groups. The structure of triglycerides is diverse, since R₁, R₂ and R₃ allow many different fatty acids and thus a high number of possible combinations. All are non-polar, i.e. lipophilic. In the case of triglycerides, a further distinction can be made between medium-chain and long-chain triglycerides. Medium-chain triglycerides have fatty acids with an average length of 6 to 12 carbon atoms, and long-chain triglycerides have fatty acids with a length of 14 to 24 carbon atoms. Thereby, two types of triglycerides can arise: simple and mixed triglycerides. In simple triglycerides, the fatty acid residues R¹, R² and R³ are identical; in mixed ones, at least one of the fatty acid residues R¹, R² and R³ is different from the other two.

Preferred **fatty-acid derived lipids** for carry out the present invention are triglycerides containing at least one acyl group with a carbon chain length between 16 and 24 carbon atoms as listed in Table 1a, and b. Therefore, the preferred triglycerides are long-chain triglycerides comprising fatty acids with a length of 16 to 24 carbon atoms.

Thus, the herein preferred triglycerides have the following general formula (**III**): 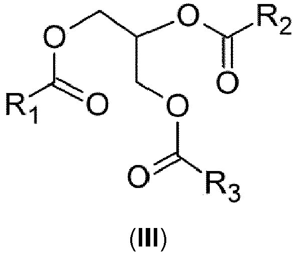 wherein R₁, R₂ and R₃ are acyl groups selected independently of each other from Palmitic acid, Stearic acid, Arachidic acid, Behenic acid, Lignoceric acid, α-Linolenic acid , Stearidonic acid, Eicosapentaenoic acid, Cervonic acid, Linoleic acid, Linolelaidic acid, γ-Linolenic acid, Dihomo-γ-linolenic acid, Arachidonic acid, Docosatetraenoic acid, Palmitoleic acid, Vaccenic acid, Paullinic acid, Oleic acid, Elaidic acid, Gondoic acid, Erucic acid, Nervonic acid, Mead acid (Table 1a and 1b).

**"Phospholipids"** are formed from glycerol bound to two fatty acids, whereas the third alcohol group is phosphorylated with a group such as phosphatidylcholine.

Preferred **fatty-acid derived lipids** for carry out the present invention are phospholipids containing two acyl groups, wherein each acyl group has a carbon chain length between 16 and 24 carbon atoms as listed in Table 1a, and b. Preferred fatty-acid derived lipids for carry out the present invention are phospholipids wherein each acyl group is independently of each other selected from Palmitic acid, Stearic acid, Arachidic acid, Behenic acid, Lignoceric acid, α-Linolenic acid , Stearidonic acid, Eicosapentaenoic acid, Cervonic acid, Linoleic acid, Linolelaidic acid, γ-Linolenic acid, Dihomo-γ-linolenic acid, Arachidonic acid, Docosatetraenoic acid, Palmitoleic acid, Vaccenic acid, Paullinic acid, Oleic acid, Elaidic acid, Gondoic acid, Erucic acid, Nervonic acid, Mead acid (Table 1a and 1b).

In particular, preferred phospholipids are selected from the group comprising phosphatidylcholine, phosphatidylserine, phosphatidylethanoline, phosphatidylglycerol, phosphatidylinositol. More particularly, preferred phospholipids are selected from the group comprising phosphatidylcholine, phosphatidylserine, phosphatidylethanoline, phosphatidylglycerol, phosphatidylinositol, wherein said phospholipids comprise two acyl groups, wherein each acyl group has a carbon chain length between 16 and 24 carbon atoms as listed in Table 1a, and b.

The herein preferred phospholipid has the following general formula (**IX**): 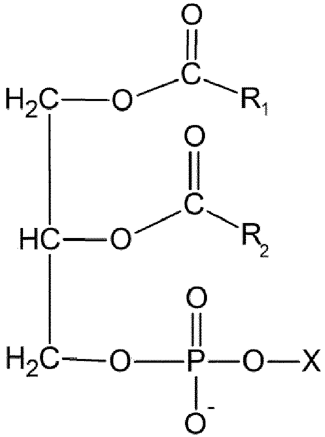
wherein X is selected from choline, serine, ethanolamine, glycerol, and inositol, and
wherein R₁ and R₂ are acyl groups selected independently of each other from Palmitic acid, Stearic acid, Arachidic acid, Behenic acid, Lignoceric acid, α-Linolenic acid , Stearidonic acid, Eicosapentaenoic acid, Cervonic acid, Linoleic acid, Linolelaidic acid, γ-Linolenic acid, Dihomo-γ-linolenic acid, Arachidonic acid, Docosatetraenoic acid, Palmitoleic acid, Vaccenic acid, Paullinic acid, Oleic acid, Elaidic acid, Gondoic acid, Erucic acid, Nervonic acid, Mead acid (Table 1a and 1b).

The herein preferred phosphatidylcholine has the following general formula (**IV**): 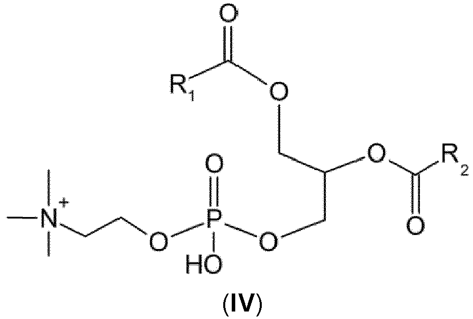 wherein R₁ and R₂ are acyl groups selected independently of each other from Palmitic acid, Stearic acid, Arachidic acid, Behenic acid, Lignoceric acid, α-Linolenic acid , Stearidonic acid, Eicosapentaenoic acid, Cervonic acid, Linoleic acid, Linolelaidic acid, γ-Linolenic acid, Dihomo-γ-linolenic acid, Arachidonic acid, Docosatetraenoic acid, Palmitoleic acid, Vaccenic acid, Paullinic acid, Oleic acid, Elaidic acid, Gondoic acid, Erucic acid, Nervonic acid, Mead acid (Table 1a and 1b).

Thus, the herein preferred phosphatidylserine has the general formula (**V**): 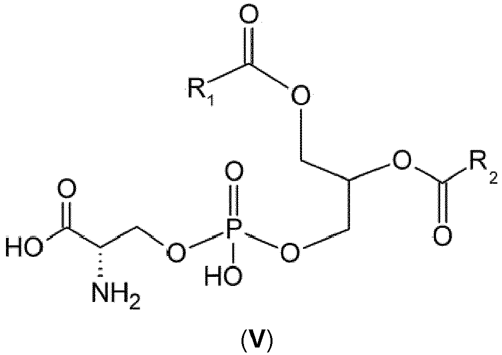 wherein R₁ and R₂ are acyl groups selected independently of each other from Palmitic acid, Stearic acid, Arachidic acid, Behenic acid, Lignoceric acid, α-Linolenic acid , Stearidonic acid, Eicosapentaenoic acid, Cervonic acid, Linoleic acid, Linolelaidic acid, γ-Linolenic acid, Dihomo-γ-linolenic acid, Arachidonic acid, Docosatetraenoic acid, Palmitoleic acid, Vaccenic acid, Paullinic acid, Oleic acid, Elaidic acid, Gondoic acid, Erucic acid, Nervonic acid, Mead acid (Table 1a and 1b).

Thus, the herein preferred **phosphatidylethanoline** has the following general formula (**VI**): 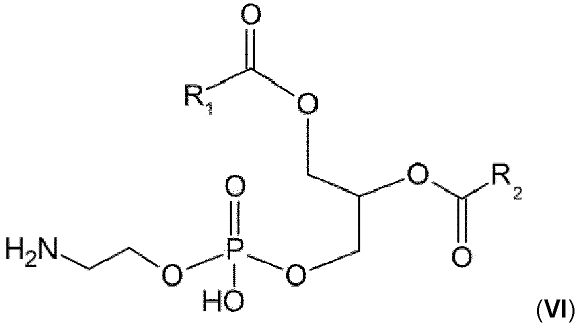 wherein R₁ and R₂ are acyl groups selected independently of each other from Palmitic acid, Stearic acid, Arachidic acid, Behenic acid, Lignoceric acid, α-Linolenic acid , Stearidonic acid, Eicosapentaenoic acid, Cervonic acid, Linoleic acid, Linolelaidic acid, γ-Linolenic acid, Dihomo-γ-linolenic acid, Arachidonic acid, Docosatetraenoic acid, Palmitoleic acid, Vaccenic acid, Paullinic acid, Oleic acid, Elaidic acid, Gondoic acid, Erucic acid, Nervonic acid, Mead acid (Table 1a and 1b).

Thus, the herein preferred **phosphatidylglycerol** has the following general formula (**VII**): 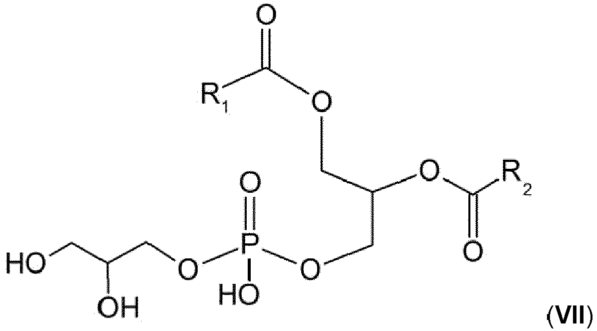 wherein R₁ and R₂ are acyl groups selected independently of each other from Palmitic acid, Stearic acid, Arachidic acid, Behenic acid, Lignoceric acid, α-Linolenic acid , Stearidonic acid, Eicosapentaenoic acid, Cervonic acid, Linoleic acid, Linolelaidic acid, γ-Linolenic acid, Dihomo-γ-linolenic acid, Arachidonic acid, Docosatetraenoic acid, Palmitoleic acid, Vaccenic acid, Paullinic acid, Oleic acid, Elaidic acid, Gondoic acid, Erucic acid, Nervonic acid, Mead acid (Table 1a and 1b).

Thus, the herein preferred **phosphatidylinositol** has the following general formula (**VIII**): 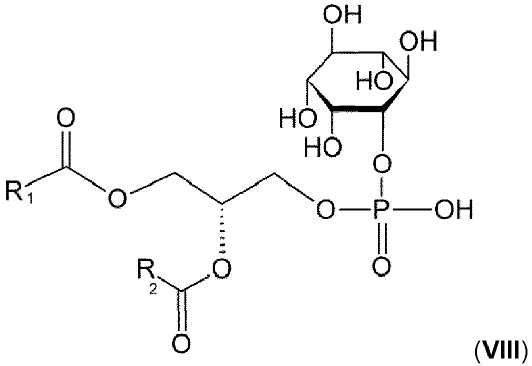 wherein R₁ and R₂ are acyl groups selected independently of each other from Palmitic acid, Stearic acid, Arachidic acid, Behenic acid, Lignoceric acid, α-Linolenic acid , Stearidonic acid, Eicosapentaenoic acid, Cervonic acid, Linoleic acid, Linolelaidic acid, γ-Linolenic acid, Dihomo-γ-linolenic acid, Arachidonic acid, Docosatetraenoic acid, Palmitoleic acid, Vaccenic acid, Paullinic acid, Oleic acid, Elaidic acid, Gondoic acid, Erucic acid, Nervonic acid, Mead acid (Table 1a and 1b).

In other words, the herein preferred phospholipids are selected from the group comprising:
a phosphatidylcholine comprising two acyl groups, wherein each acyl group is independently of each other selected from Palmitic acid, Stearic acid, Arachidic acid, Behenic acid, Lignoceric acid, α-Linolenic acid , Stearidonic acid, Eicosapentaenoic acid, Cervonic acid, Linoleic acid, Linolelaidic acid, γ-Linolenic acid, Dihomo-γ-linolenic acid, Arachidonic acid, Docosatetraenoic acid, Palmitoleic acid, Vaccenic acid, Paullinic acid, Oleic acid, Elaidic acid, Gondoic acid, Erucic acid, Nervonic acid, Mead acid;
a phosphatidylserine comprising two acyl groups, wherein each acyl group is independently of each other selected from Palmitic acid, Stearic acid, Arachidic acid, Behenic acid, Lignoceric acid, α-Linolenic acid , Stearidonic acid, Eicosapentaenoic acid, Cervonic acid, Linoleic acid, Linolelaidic acid, γ-Linolenic acid, Dihomo-γ-linolenic acid, Arachidonic acid, Docosatetraenoic acid, Palmitoleic acid, Vaccenic acid, Paullinic acid, Oleic acid, Elaidic acid, Gondoic acid, Erucic acid, Nervonic acid, Mead acid;
a phosphatidylethanoline comprising two acyl groups, wherein each acyl group is independently of each other selected from Palmitic acid, Stearic acid, Arachidic acid, Behenic acid, Lignoceric acid, α-Linolenic acid , Stearidonic acid, Eicosapentaenoic acid, Cervonic acid, Linoleic acid, Linolelaidic acid, γ-Linolenic acid, Dihomo-γ-linolenic acid, Arachidonic acid, Docosatetraenoic acid, Palmitoleic acid, Vaccenic acid, Paullinic acid, Oleic acid, Elaidic acid, Gondoic acid, Erucic acid, Nervonic acid, Mead acid;
a phosphatidylglycerol comprising two acyl groups, wherein each acyl group is independently of each other selected from Palmitic acid, Stearic acid, Arachidic acid, Behenic acid, Lignoceric acid, α-Linolenic acid , Stearidonic acid, Eicosapentaenoic acid, Cervonic acid, Linoleic acid, Linolelaidic acid, γ-Linolenic acid, Dihomo-γ-linolenic acid, Arachidonic acid, Docosatetraenoic acid, Palmitoleic acid, Vaccenic acid, Paullinic acid, Oleic acid, Elaidic acid, Gondoic acid, Erucic acid, Nervonic acid, Mead acid;
a phosphatidylinositol comprising two acyl groups, wherein each acyl group is independently of each other selected from Palmitic acid, Stearic acid, Arachidic acid, Behenic acid, Lignoceric acid, α-Linolenic acid , Stearidonic acid, Eicosapentaenoic acid, Cervonic acid, Linoleic acid, Linolelaidic acid, γ-Linolenic acid, Dihomo-γ-linolenic acid, Arachidonic acid, Docosatetraenoic acid, Palmitoleic acid, Vaccenic acid, Paullinic acid, Oleic acid, Elaidic acid, Gondoic acid, Erucic acid, Nervonic acid, Mead acid.

"**Sphingolipids**" are composed of a long-chain sphingosine base (LCB) backbone, linked to a fatty acid (FA) with an amine bond, and a polar head group. Sphingolipids have the following general formula (X):

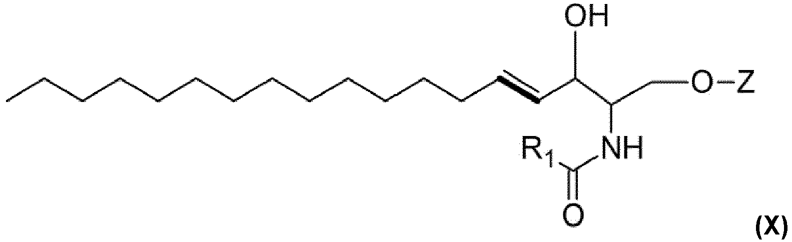

On the basis of the residue -Z in the side chain, sphingolipids are categorized in ceramides (Z=H), sphingomyelins (Z=phosphocholine) and glycosphingolipids (Z= sugar).

A preferred **fatty-acid derived lipid** for carry out the present invention is a **sphingolipid** of general formula (X): 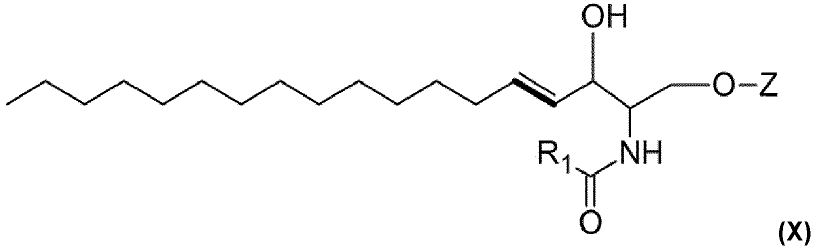
wherein Z is -H or phosphocholine C₅H₁₅NO₄P⁺, and
wherein R₁ is an acyl group selected from palmitic acid, stearic acid, Arachidic acid, Behenic acid, Lignoceric acid, α-Linolenic acid , Stearidonic acid, Eicosapentaenoic acid, Cervonic acid, Linoleic acid, Linolelaidic acid, γ-Linolenic acid, Dihomo-γ-linolenic acid, Arachidonic acid, Docosatetraenoic acid, Palmitoleic acid, Vaccenic acid, Paullinic acid, Oleic acid, Elaidic acid, Gondoic acid, Erucic acid, Nervonic acid, Mead acid.

More preferably a fatty-acid derived lipid for carry out the present invention is a sphingomielin of general formula (XI): 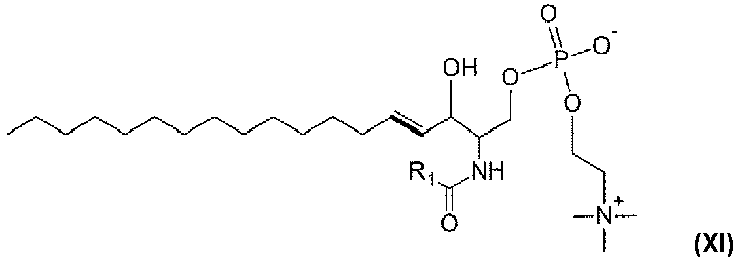 wherein R₁ is an acyl group selected from palmitic acid, stearic acid, Arachidic acid, Behenic acid, Lignoceric acid, α-Linolenic acid , Stearidonic acid, Eicosapentaenoic acid, Cervonic acid, Linoleic acid, Linolelaidic acid, γ-Linolenic acid, Dihomo-γ-linolenic acid, Arachidonic acid, Docosatetraenoic acid, Palmitoleic acid, Vaccenic acid, Paullinic acid, Oleic acid, Elaidic acid, Gondoic acid, Erucic acid, Nervonic acid, Mead acid.

Therefore, a preferred "**fatty acid-derived lipid**" for carry out the present invention is selected from the group comprising:
- a fatty acid with a carbon chain length between 16 and 24 carbon atoms as listed in Table 1a, and b,
- a phospholipid of general formula (IX): 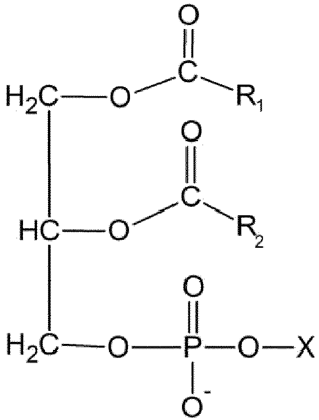 wherein X is selected from choline, serine, ethanolamine, glycerol, and inositol,
- a diacylglicerol having the general formula (**I**): 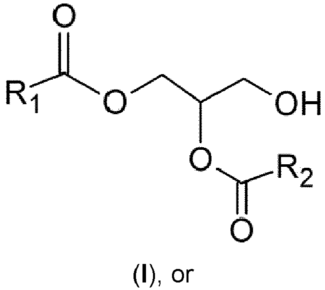 having the general formula (**II**): 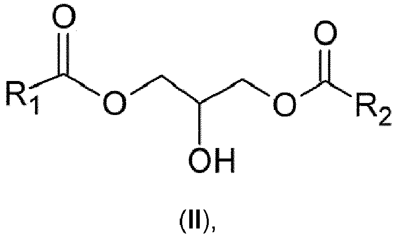 a triglyceride having the following general formula (**III**): 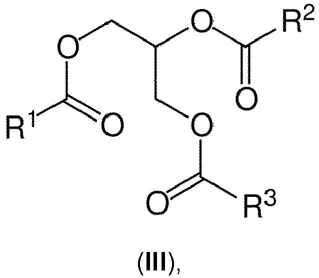
- a sphingomielin of general formula (XI): 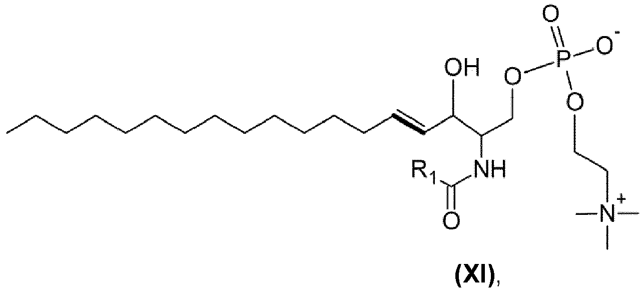
wherein R₁, R₂, and R₃ are acyl groups selected independently of each other from Palmitic acid, Stearic acid, Arachidic acid, Behenic acid, Lignoceric acid, α-Linolenic acid , Stearidonic acid, Eicosapentaenoic acid, Cervonic acid, Linoleic acid, Linolelaidic acid, γ-Linolenic acid, Dihomo-γ-linolenic acid, Arachidonic acid, Docosatetraenoic acid, Palmitoleic acid, Vaccenic acid, Paullinic acid, Oleic acid, Elaidic acid, Gondoic acid, Erucic acid, Nervonic acid, Mead acid,

More in particular, a preferred **"fatty acid-derived lipid"** for carry out the present invention is selected from the group comprising palmitic acid, phosphatidylcholine, phosphatidylserine, phosphatidylethanoline, phosphatidylglycerol, phosphatidylinositol, diacylglycerol, sphingomielin, wherein said phosphatidylcholine, phosphatidylserine, phosphatidylethanoline, phosphatidylglycerol, phosphatidylinositol, diacylglycerol, and sphingomielin comprise acyl groups selected independently of each other from palmitic acid, stearic acid, arachidic acid, behenic acid, lignoceric acid, α-linolenic acid , stearidonic acid, eicosapentaenoic acid, cervonic acid, linoleic acid, linolelaidic acid, γ-linolenic acid, dihomo-γ-linolenic acid, arachidonic acid, docosatetraenoic acid, palmitoleic acid, vaccenic acid, paullinic acid, oleic acid, elaidic acid, gondoic acid, erucic acid, nervonic acid, mead acid.

Still more in particular, a preferred **"fatty acid-derived lipid"** for carry out the present invention is selected from the group comprising palmitic acid, 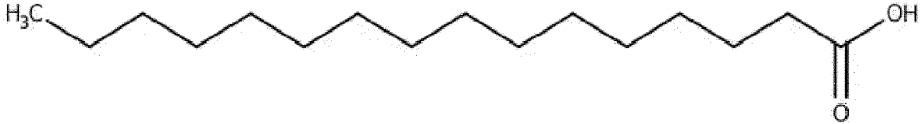 a phosphatidylcholine of formula (**IV**), 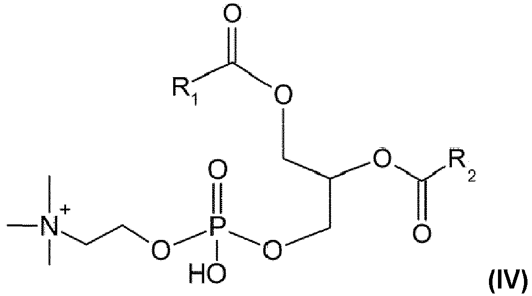 a phosphatidylserine of formula (**V**), 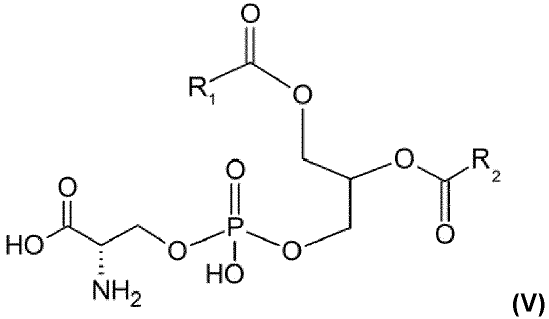 a phosphatidylethanoline of formula (**VI**): 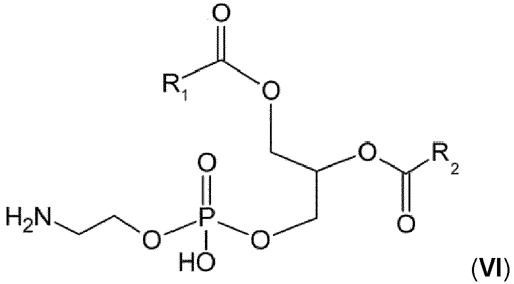 a phosphatidylglycerol of formula (**VII**): 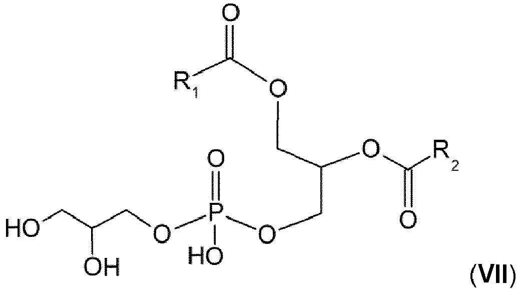 a phosphatidylinositol of formula (**VIII**): 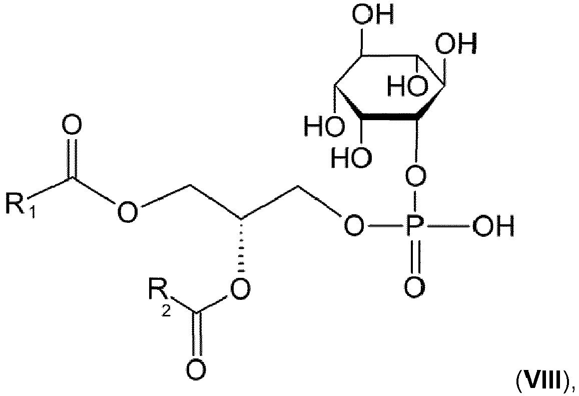 a diacylglycerol of formula (I) or (II): 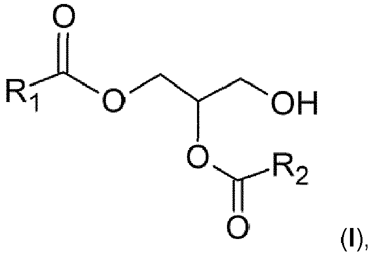 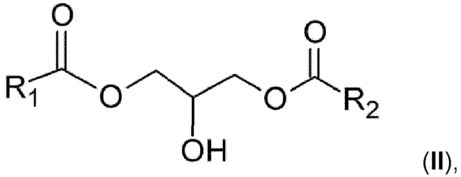 a sphingomielin of formula (**XI**): 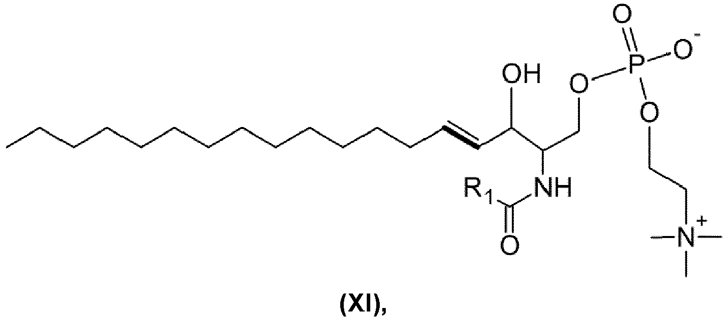 wherein R₁, and R₂ are acyl groups selected independently of each other from palmitic acid, stearic acid, arachidic acid, behenic acid, lignoceric acid, α-linolenic acid, stearidonic acid, eicosapentaenoic acid, cervonic acid, linoleic acid, linolelaidic acid, γ-linolenic acid, dihomo-γ-linolenic acid, arachidonic acid, docosatetraenoic acid, palmitoleic acid, vaccenic acid, paullinic acid, oleic acid, elaidic acid, gondoic acid, erucic acid, nervonic acid, mead acid.

Still more in particular the term **""fatty acid-derived lipid"** refers to a substance selected from the group comprising palmitic acid C16:0, phosphatidylcholine C34:1, phosphatidylserine, phosphatidylethanoline C34:1, phosphatidylglycerol C34:1, phosphatidylinositol C34:1, diacylglycerol 34:1, and sphingomielin d18:1/16:0.

### USES

The glutamine synthetase modulator identifiable by the *in vitro* method disclosed herein modulates the mevalonate pathway and/or the fatty acid synthesis and is thus useful to treat a disease associated with a deregulated mevalonate pathway and/or a deregulated fatty acid synthesis.

Therefore, a **second embodiment** of the present invention is directed to a glutamine synthetase inhibitor identifiable by the *in vitro* method described above, for use in treatment of a disease associated with high levels of a mevalonate pathway derived metabolite, and/or with high levels of a fatty acid-derived lipid.

Moreover, the present invention relates to a glutamine synthetase activator identifiable by the *in vitro* method disclosed herein, for use in treatment of a disease associated with low levels of a mevalonate pathway derived metabolite, and/or with low levels of a fatty acid-derived lipid.

Preferred embodiments relate to a glutamine synthetase inhibitor identifiable by the *in vitro* method disclosed herein, for use in treatment of a disease associated with high levels of a mevalonate pathway derived metabolite, wherein the mevalonate pathway derived metabolite is an isoprenoid, and/or a sterol, and/or cholesterol.

Also described herein is a **method for the treatment** of a disease associated with low levels of a mevalonate pathway derived metabolite, and/or with low levels of a fatty acid-derived lipid, comprising administering to a patient suffering from said disease a therapeutically effective amount of a glutamine synthetase **activator** identifiable by the *in vitro* method disclosed herein or a therapeutically effective amount of a pharmaceutical composition comprising said glutamine synthetase **activator.**

Moreover is also described a **method for the treatment** of a disease associated with high levels of a mevalonate pathway derived metabolite, and/or with high levels of a fatty acid-derived lipid, comprising administering to a patient suffering from said disease a therapeutically effective amount of a glutamine synthetase **inhibitor** identifiable by the *in vitro* method disclosed herein or a therapeutically effective amount of a pharmaceutical composition comprising said glutamine synthetase **inhibitor.**

Moreover is also described a **method for the treatment** of a disease associated with high levels of a mevalonate pathway derived metabolite, and/or with high levels of a fatty acid-derived lipid, comprising administering to a patient suffering from said disease a therapeutically effective amount of a glutamine synthetase **inhibitor** identifiable by the *in vitro* method disclosed herein or a therapeutically effective amount of a pharmaceutical composition comprising said glutamine synthetase **inhibitor,** wherein the disease is selected from the group comprising or consisting of steatosis, cirrhosis, renal dysfunction, testosterone deficiency, glioblastoma, multiple myeloma, leukemia, cancer, premature aging, infectious disease, neurodegenerative disease including Alzheimer's disease, arthritis, cardiovascular disease, Hutchinson-Gilford progeria syndrome (HGPS), cerebral cavernous malformations (CCMs), neuroinflammatory disorders, autoimmune disorders, diabetes, atherosclerosis, hyperlipidemia, immuno-inflammatory diseases, acquired immune deficiency syndrome (AIDS), allograft rejection, adult respiratory distress syndrome, arthritis, asthma, atherosclerosis, autoimmune disorders, cerebral palsy, eczema, glomerulonephritis, heart failure, herpes dementia, immune complex diseases, inflammatory bowel disease, ischemia , metabolic syndrome, migraine, multiple sclerosis, myocarditis, organ transplant/bypass disorders, osteoporosis, oxidant induced lung injury, Paget's disease, hyperimmunoglobulin D syndrome, mevalonate kinase deficiency, pain, peritonitis, reperfusion injury, retinitis, sepsis, septic shock, sickle cell anemia, stroke, toxic shock syndrome, uveitis, X-adenoleukodystrophy (X-ALD), Alzheimer's disease, Parkinson's disease, Landry-Guillain-Barre-Strohl syndrome, multiple sclerosis, viral encephalitis, AIDS-related dementia, amyotrophic lateral sclerosis, brain trauma, spinal cord disorders, atherosclerosis, coronary artery insufficiency, coronary heart disease, myocardial infarction, hypertriglyceridemia, lymphoma, Cushing syndrome, porphyria, hypothyroidism, renal disease, cholestatic liver disorders, lymphoma, bipolar disorders, schizophrenia, neurodegenerative diseases, Niemann-Pick and Huntington diseases, multiple sclerosis, lipid storage disease.

It is also described a **method for the treatment** of a disease associated with high levels of a mevalonate pathway derived metabolite, and/or with high levels of a fatty acid-derived lipid, comprising administering to a patient suffering from said disease a therapeutically effective amount of a glutamine synthetase **inhibitor** identifiable by the *in vitro* method disclosed herein or a therapeutically effective amount of a pharmaceutical composition comprising said glutamine synthetase **inhibitor,** wherein said glutamine synthetase inhibitor is selected from L-methionine sulfoximine, a phosphinothricin analogue, a derivative of aminomethylenebisphosphonic acid, and γ-hydroxylysine.

### Diseases associated with high levels of isoprenoids

A variety of important intracellular proteins undergo prenylation, including almost all members of small GTPase superfamilies as well as heterotrimeric G protein subunits and nuclear lamins. These prenylated proteins are involved in regulating a wide range of cellular processes and functions, such as cell growth, differentiation, cytoskeletal organization, and vesicle trafficking. Therefore, prenylated proteins are also implicated in the pathogenesis of different types of diseases and disorders, such as cancer, premature aging, infectious disease, neurodegenerative disease including Alzheimer's disease.

For example, dysregulated prenylation of small GTPases due to a disregulated isoprenoid synthesis is linked to different processes pertinent to the pathogenesis of **Alzheimer's disease,** including alteration of APP (amyloid-β precursor protein) trafficking/processing, increase in tau phosphorylation, microglial activation contributing to enhanced neuroinflammation, dendritic/synaptic loss leading to cognitive impairment.
Moreover, it has been shown that inhibitor of isoprenoid synthesis can treat bone diseases such as Paget's bone disease and osteoporosis, arthritis, cardiovascular disease, Hutchinson-Gilford progeria syndrome (HGPS), cerebral cavernous malformations (CCMs), neuroinflammatory disorders such as irritable bowel syndrome, schizophrenia, bipolar disorder, depression, anxiety including anxiety disorder, obsessive-compulsive disorder and post-traumatic stress disorder, Alzheimer's disease, dementia, or autism spectrum disorder including autism, asperger's disorder, pervasive developmental disorder, childhood disintegrative disorder; and **autoimmune disorders** such as ankylosing spondylitis, arthritis, rheumatoid arthritis, osteoarthritis, Chagas disease, dermatomyositis, diabetes mellitus type 1, endometriosis, Goodpasture's syndrome, Graves' disease, Guillain- Barre syndrome, Hashimoto's thyroiditis disease, Hidradenitis suppurativa, Kawasaki disease, IgA nephropathy, Idiopathic thrombocytopenic purpura, inflammatory bowel disease, Celiac's disease, Crohn's disease, eosinophilic gastroenteritis, ulcerative colitis, collagenous colitis, lymphocytic colitis, ischemic colitis, diversion colitis, Behcet's syndrome, infective colitis, indeterminate colitis interstitial cystitis, lupus, systemic lupus erythematosus, discoid lupus, drug-induced lupus, neonatal lupus, mixed connective tissue disease, morphea, multiple sclerosis, myasthenia gravis, narcolepsy, neuromyotonia, pemphigus vulgaris, pernicious anemia, psoriasis, psoriatic arthritis, polymyositis, primary biliary cirrhosis, relapsing polychondritis,, scleroderma, Sjgren's syndrome, Stiff person syndrome, temporal arteritis (also known as giant cell arteritis), vasculitis, vitiligo, Wegener's granulomatosis, alopecia areata, sarcoidosis, Addison's disease, or autoimmune hemolytic anemia.

Hutchinson-Gilford progeria syndrome (HGPS) is caused by mutations in the nuclear lamin A protein and subsequent accumulation of a prenylated form of the mutant lamin A. Cerebral cavernous malformations (CCMs) are common abnormal vascular formations that occur in the brain, spinal cord, and sometimes the retina.

Therefore, the term **"disease associated with high levels of isoprenoids"** refers to a disease selected from the group comprising cancer, premature aging, infectious disease, neurodegenerative disease including Alzheimer's disease, bone diseases, arthritis, cardiovascular disease, Hutchinson-Gilford progeria syndrome (HGPS), cerebral cavernous malformations (CCMs), neuroinflammatory disorders, autoimmune disorders.

### Diseases associated with high levels of cholesterol

**Hypercholesterolemia,** or elevated blood cholesterol levels due to high concentration of cholesterol in the cells and plasma, is an important risk factor connected with potentially deadly cardiovascular disease, including atherosclerosis, coronary artery insufficiency, coronary heart disease, myocardial infarction, stroke, myocardial infarction. Further diseases that have been associated to excess cholesterol are metabolic syndrome, steatosis, cirrhosis, renal dysfunction, hypothyroidism, osteoporosis, testosterone deficiency, osteoarthritis, Alzheimer's disease, immune dysfunction.

The term **"metabolic syndrome"** as used herein refers to a condition characterized by a combination of diabetes, high blood pressure (hypertension) and obesity. Subjects with **"metabolic syndrome"** have **high triglycerides in blood, low HDL cholesterol and high LDL cholesterol,** which can cause plaque formation in artery walls, elevated blood pressure, insulin resistance or glucose intolerance, prothrombotic state, and proinflammatory state such as elevated C-reactive protein in the blood. Subjects with metabolic syndrome are at increased risk of coronary heart disease (CHD) and other diseases related to plaque formation in artery walls.

Therefore, the term **"disease associated with high levels of cholesterol"** refers to a disease selected from the group comprising cardiovascular disease, atherosclerosis, coronary artery insufficiency, coronary heart disease, myocardial infarction, stroke, myocardial infarction, metabolic syndrome, steatosis, cirrhosis, renal dysfunction, hypothyroidism, osteoporosis, testosterone deficiency, osteoarthritis, Alzheimer's disease, immune dysfunction.

In addition to cholesterol, also **sterol intermediates and oxysterols** can contribute to various human diseases including diabetes mellitus, cancer, cardiovascular disease, multiple sclerosis, osteoporosis, lung cancer, breast cancer, infertility, neurodegenerative disorders such as Alzheimer's disease and infection. Examplary cancers associated with higher levels of sterols due to activation of SREBPs: glioblastoma, prostate cancer, breast cancer, melanoma, pancreatic cancer. Moreover, defects in enzymes involved in conversion of lanosterol to cholesterol cause syndromes with **severe malformations:** mevalonic aciduria (MVA); hyper-lgD and periodic fever syndrome (HIDS); Congenital Hemidysplasia with Icthyosiform nevus and Limb Defects (CHILD), NAD(P) dependent steroid dehydrogenase-like (NSDHL); Sterol-C4-methyl oxidase deficiency, (SC4MOL), Sterol-C4methyl oxidase-like (SC4MOL); X-linked Dominant Chondrodysplasia Punctata 2 (CDPX2); Smith Lemli Opitz Syndrome (SLOS).

### Disease associated with upregulated mevalonate pathway

In general, a **deregulated mevalonate pathway** is associated to metabolic disorders, which can cause diabetes (including type 1 diabetes mellitus and type 2 diabetes mellitus), cardiovascular disease, atherosclerosis, and hyperlipidemia.

As used herein, the term "dysregulated ", or **"altered"** refers to a level of a metabolite or of a lipid that is higher or lower than the level of said metabolite or lipid in a normal, wild-type, healthy organism or cell of reference (control).

Several cancer types demonstrate an upregulated **mevalonate pathway,** and can thus be treated with a glutamine synthetase inhibitor. Exemplary cancers are pancreatic cancer, breast cancer, prostate cancer, esophageal cancer, hepatic cancer, leukemia, and lung cancer (Guerra et al. 2021, Targeting Mevalonate Pathway in Cancer, Frontiers in Oncology).

**Further diseases associated with deregulated mevalonate pathway** are immuno-inflammatory diseases, acquired immune deficiency syndrome (AIDS), allograft rejection, adult respiratory distress syndrome, arthritis, asthma, atherosclerosis, autoimmune disorders (such as, for example. Addison's disease, autoimmune hepatitis Celiac disease, Crohn's Disease, giant cell arteritis, Goodpasture's syndrome, Grave's disease, Guillain-Barre syndrome, Hashimoto's thyroiditis, juvenile rheumatoid arthritis, lupus, polymyalgia rheumatica, psoriasis, primary biliary cirrhosis, rheumatoid arthritis, scleroderma, sclerosing cholangitis, Sjogren's syndrome, temporal arteritis, type 1 diabetes mellitus, ulcerative colitis, Wegener's granulomatosis, and combinations thereof), cerebral palsy, eczema, glomerulonephritis, heart failure, herpes dementia, immune complex diseases, inflammatory bowel disease, ischemia (including ischemic brain edema), **metabolic syndrome,** migraine, multiple sclerosis, myocarditis, organ transplant/bypass disorders, **osteoporosis,** oxidant induced lung injury, Paget's disease, hyperimmunoglobulin D syndrome, mevalonate kinase deficiency, pain, peritonitis, reperfusion injury, retinitis, sepsis and/or septic shock, sickle cell anemia, stroke, toxic shock syndrome, uveitis, X-adenoleukodystrophy (X-ALD), Alzheimer's disease, Parkinson's disease, Landry-Guillain-Barre-Strohl syndrome, multiple sclerosis, viral encephalitis, AIDS-related dementia, amyotrophic lateral sclerosis, brain trauma, spinal cord disorders, and combinations thereof.

It is noted that some of ordinary skill in the art would classify multiple sclerosis as an autoimmune disorder and others would not.

Therefore, the term **"disease associated with high levels of a mevalonate pathway derived metabolite"** refers to a disease selected from the group comprising: cancer, premature aging, infectious disease, neurodegenerative disease, Alzheimer's disease, arthritis, Hutchinson-Gilford progeria syndrome (HGPS), cerebral cavernous malformations (CCMs), neuroinflammatory disorders, autoimmune disorders, diabetes, cardiovascular disease, atherosclerosis, hyperlipidemia, coronary artery insufficiency, coronary heart disease, myocardial infarction, stroke, steatosis, cirrhosis, renal dysfunction, hypothyroidism, osteoporosis, testosterone deficiency, osteoarthritis, immune dysfunction, immuno-inflammatory diseases, acquired immune deficiency syndrome (AIDS), allograft rejection, adult respiratory distress syndrome, asthma, cerebral palsy, eczema, glomerulonephritis, heart failure, herpes dementia, immune complex diseases, inflammatory bowel disease, ischemia, **metabolic syndrome,** migraine, multiple sclerosis, myocarditis, organ transplant/bypass disorders, oxidant induced lung injury, Paget's disease, hyperimmunoglobulin D syndrome, mevalonate kinase deficiency, peritonitis, reperfusion injury, retinitis, sepsis, septic shock, sickle cell anemia, toxic shock syndrome, uveitis, X-adenoleukodystrophy (X-ALD), Parkinson's disease, Landry-Guillain-Barre-Strohl syndrome, viral encephalitis, AIDS-related dementia, amyotrophic lateral sclerosis, brain trauma, spinal cord disorders.

Therefore, a preferred embodiment is directed to a **glutamine synthetase inhibitor** identifiable by the *in vitro* method disclosed herein, for use in treatment of a disease associated with high levels of a **mevalonate pathway derived metabolite,** wherein said disease is selected from the group comprising cancer, premature aging, infectious disease, neurodegenerative disease, Alzheimer's disease, arthritis, Hutchinson-Gilford progeria syndrome (HGPS), cerebral cavernous malformations (CCMs), neuroinflammatory disorders, autoimmune disorders, diabetes, cardiovascular disease, atherosclerosis, hyperlipidemia, coronary artery insufficiency, coronary heart disease, myocardial infarction, stroke, steatosis, cirrhosis, renal dysfunction, hypothyroidism, osteoporosis, testosterone deficiency, osteoarthritis, immune dysfunction, immuno-inflammatory diseases, acquired immune deficiency syndrome (AIDS), allograft rejection, adult respiratory distress syndrome, asthma, cerebral palsy, eczema, glomerulonephritis, heart failure, herpes dementia, immune complex diseases, inflammatory bowel disease, ischemia, **metabolic syndrome,** migraine, multiple sclerosis, myocarditis, organ transplant/bypass disorders, oxidant induced lung injury, Paget's disease, hyperimmunoglobulin D syndrome, mevalonate kinase deficiency, peritonitis, reperfusion injury, retinitis, sepsis, septic shock, sickle cell anemia, toxic shock syndrome, uveitis, X-adenoleukodystrophy (X-ALD), Parkinson's disease, Landry-Guillain-Barre-Strohl syndrome, viral encephalitis, AIDS-related dementia, amyotrophic lateral sclerosis, brain trauma, spinal cord disorders.

Preferred cancers that can be treated with a glutamine synthetase inhibitor disclosed herein are glioblastoma, prostate cancer, pancreatic cancer, lung cancer, breast cancer, melanoma, esophageal cancer, hepatic cancer, leukemia.

A particularly preferred embodiment is directed to a **glutamine synthetase inhibitor** identifiable by the *in vitro* method disclosed herein, for use in treatment of a disease associated with high levels of **isoprenoids,** wherein said disease is selected from the group comprising cancer, premature aging, infectious disease, neurodegenerative disease including Alzheimer's disease, bone diseases, arthritis, cardiovascular disease, Hutchinson-Gilford progeria syndrome (HGPS), cerebral cavernous malformations (CCMs), neuroinflammatory disorders, autoimmune disorders.

A more preferred embodiment is directed to a **glutamine synthetase inhibitor** identifiable by the *in vitro* method disclosed herein, for use in treatment of a disease associated with high levels of **cholesterol,** wherein said disease is selected from the group comprising cardiovascular disease, atherosclerosis, coronary artery insufficiency, coronary heart disease, myocardial infarction, stroke, myocardial infarction, metabolic syndrome, steatosis, cirrhosis, renal dysfunction, hypothyroidism, osteoporosis, testosterone deficiency, osteoarthritis, Alzheimer's disease, immune dysfunction.

A further preferred embodiment is directed to a **glutamine synthetase inhibitor** identifiable by the *in vitro* method disclosed herein, for use in treatment of a disease associated with high levels of **sterols,** wherein said disease is selected from the group comprising diabetes mellitus, cancer, glioblastoma, prostate cancer, pancreatic cancer, lung cancer, breast cancer, melanoma, cardiovascular disease, multiple sclerosis, osteoporosis, infertility, neurodegenerative disorders.

### Diseases associated with high levels of fatty acid-derived lipids

**Hyperlipidemia** refers to a group of serious lipid disorders caused by an abnormally high level of unwanted lipids in the blood. Hyperlipidemia comprises **hypercholesterolemia** and **hypertriglyceridemia.**

Examplary diseases associated with **deregulated lipid levels** are coronary heart disease (CHD), diabetes mellitus, lymphoma, Cushing syndrome, porphyria, cardiovascular disorders, hypothyroidism (LDL hypercholesterolemia), renal diseases (hypertriglyceridemia, mixed hyperlipoproteinemia, lipoprotein elevation), and cholestatic liver disorders, lymphoma, Cushing syndrome, and porphyria.

Phospholipid imbalance has been implicated with many **neurological disorders,** including bipolar disorders, schizophrenia, and neurodegenerative diseases such as Alzheimer's, Parkinson's, Niemann-Pick and Huntington diseases. Moreover, phospholipid imbalance has been implicated with various human diseases such as cancer, diabetes, cardiovascular and infectious diseases.

Phospholipids and sphingolipids are involved in multiple sclerosis.

**"Lipid storage diseases"** (also known as **lipidoses**) are a group of inherited metabolic disorders in which harmful amounts of fatty materials (lipids) accumulate in various cells and tissues in the body. People with these disorders either do not produce enough of one of the enzymes needed to break down (metabolize) lipids or they produce enzymes that do not work. Over time, excessive storage of fats can cause permanent cellular and tissue damage, particularly in the brain, peripheral nervous system, liver, spleen, and bone marrow. Type of lipid storage diseases: Gaucher's disease, Niemann-Pick disease, Fabry disease, Farber's disease (ceramidase defect), Acid lipase deficiency.

Therefore, the term **"disease associated with high levels of a fatty acid-derived lipid"** refers to a disease selected from the group comprising metabolic syndrome, hypertriglyceridemia, coronary heart disease (CHD), diabetes mellitus, lymphoma, Cushing syndrome, and porphyria, hypothyroidism, renal disease, cholestatic liver disorders, lymphoma, Cushing syndrome, and porphyria, bipolar disorders, schizophrenia, and neurodegenerative diseases such as Alzheimer's, Parkinson's, Niemann-Pick and Huntington diseases, cancer, cardiovascular diseases, multiple sclerosis, lipid storage disease.

Therefore, a preferred embodiment is directed to a **glutamine synthetase inhibitor** identifiable by the method in vitro method disclosed herein, for use in treatment of a disease associated with high levels of a **fatty acid-derived lipid,** wherein said disease is selected from the group comprising metabolic syndrome, hypertriglyceridemia, coronary heart disease (CHD), diabetes mellitus, lymphoma, Cushing syndrome, and porphyria, hypothyroidism, renal disease, cholestatic liver disorders, lymphoma, Cushing syndrome, and porphyria, bipolar disorders, schizophrenia, and neurodegenerative diseases such as Alzheimer's, Parkinson's, Niemann-Pick and Huntington diseases, cancer, cardiovascular diseases, multiple sclerosis, lipid storage disease.

Therefore, a more preferred aspect of the second embodiment is directed to a **glutamine synthetase inhibitor** identifiable by the method in vitro method disclosed herein, for use in treatment of a disease associated with high levels of a mevalonate pathway derived metabolite and/or with high levels of a fatty acid-derived lipid, wherein said disease is selected from the group comprising cancer, premature aging, infectious disease, neurodegenerative disease, Alzheimer's disease, arthritis, Hutchinson-Gilford progeria syndrome (HGPS), cerebral cavernous malformations (CCMs), neuroinflammatory disorders, autoimmune disorders, diabetes, cardiovascular disease, atherosclerosis, hyperlipidemia, coronary artery insufficiency, coronary heart disease, myocardial infarction, stroke, steatosis, cirrhosis, renal dysfunction, hypothyroidism, osteoporosis, testosterone deficiency, osteoarthritis, immune dysfunction, immuno-inflammatory diseases, acquired immune deficiency syndrome (AIDS), allograft rejection, adult respiratory distress syndrome, asthma, cerebral palsy, eczema, glomerulonephritis, heart failure, herpes dementia, immune complex diseases, inflammatory bowel disease, ischemia, **metabolic syndrome,** migraine, multiple sclerosis, myocarditis, organ transplant/bypass disorders, oxidant induced lung injury, Paget's disease, hyperimmunoglobulin D syndrome, mevalonate kinase deficiency, peritonitis, reperfusion injury, retinitis, sepsis, septic shock, sickle cell anemia,, toxic shock syndrome, uveitis, X-adenoleukodystrophy (X-ALD), Parkinson's disease, Landry-Guillain-Barre-Strohl syndrome, viral encephalitis, AIDS-related dementia, amyotrophic lateral sclerosis, brain trauma, spinal cord disorders, hypertriglyceridemia, lymphoma, cushing syndrome, porphyria, hypothyroidism, renal disease, cholestatic liver disorders, lymphoma, bipolar disorders, schizophrenia, neurodegenerative diseases, Niemann-Pick disease, and Huntington diseases, multiple sclerosis, lipid storage disease.

A still more preferred aspect of the second embodiment is directed to a glutamine synthetase inhibitor identifiable by the *in vitro* method described above, for use in treatment of a disease associated with high levels of a mevalonate pathway derived metabolite, and/or with high levels of a fatty acid-derived lipid, wherein the glutamine synthetase inhibitor is selected from L-methionine sulfoximine, a phosphinothricin analogue, a derivative of aminomethylenebisphosphonic acid, and γ-hydroxylysine.

In a further aspect of the second embodiment, the mevalonate pathway derived metabolite is an isoprenoid, and/or a sterol, and/or cholesterol. In another aspect of the second embodiment, the fatty acid-derived lipid is selected from the group comprising a phospholipid, a diacylglycerol, a triglyceride, and a sphingolipid.

Low levels of phosphatidylcholine or absence of phosphatidylcholine are associated with the disease **"respiratory distress syndrome".**

Diseases associated with **low levels of cholesterol** are Smith-Lemli-Opitz-Syndrom (SLOS), Lathosterolosis, Desmosterolosis, X-linked dominant chondrodysplasia punctata (CDPX2), CHILD Syndrome, SC4MOL, Antley-Bixler, HEM dysplasia.

Therefore, a third embodiment of the present invention is directed to a **glutamine synthetase activator** identifiable by the in vitro method disclosed herein, for use in treatment of a disease associated with low levels of a mevalonate pathway derived metabolite, and/or with low levels of a fatty acid-derived lipid, and preferably wherein said disease is selected from the group comprising hypocholesterolemia, respiratory distress syndrome, SLOS, Lathosterolosis, Desmosterolosis, CDPX2, CHILD Syndrome, SC4MOL, Antley-Bixler, HEM dysplasia. In an aspect of the third embodiment, the mevalonate pathway derived metabolite is an isoprenoid, and/or a sterol, and/or cholesterol. In another aspect of the third embodiment, the fatty acid-derived lipid is selected from the group comprising a phospholipid, a diacylglycerol, a triglyceride, and a sphingolipid,.

The terms **"individual"** and **"subject"** are used herein interchangeably to refer to a human or another mammal (e.g., mouse, rat, rabbit, dog, cat, cattle, swine, sheep, horse or primate) that can be afflicted with or is susceptible to a disease or disorder but may or may not have the disease or disorder. In many embodiments, the subject is a human being. In many embodiments, the subject is a patient. Unless otherwise stated, the terms "individual" and "subject" do not denote a particular age, and thus encompass adults, children, and newborns.

### Dosages

A glutamine synthetase modulator or a pharmaceutical composition thereof, will generally be administered in such amounts, e.g., as a unit dose, and for such a time as is necessary or sufficient to achieve at least one desired result.
A dosing regimen according to the present invention may consist of a single dose or a plurality of doses over a period of time. Administration may be one or multiple times daily, or weekly or on an intermittent schedule. For example, a dosing regimen may comprise administration of a single dose of a glutamine synthetase modulator or administration of 2 doses, 3 doses, 4 doses, 5 doses, 6 doses or more than 6 doses. Two consecutive doses may be administered at 1 day interval, 2 days interval, 3 days interval, 4 days interval, 5 days interval, 6 days interval, 7 days interval, or more than 7 days interval. The exact amount of a glutamine synthetase modulator, or pharmaceutical composition thereof, to be administered will vary from subject to subject and may depend on several factors.

Depending on the route of administration, effective amounts may be calculated according to the body weight; body surface area; nature of disease or condition; tissues affected; etc. of the subject to be treated. Optimization of the appropriate dosages can readily be made by one skilled in the art in light of pharmacokinetic data observed in human clinical trials. The final dosage regimen may be determined by the attending physician or other clinician, considering various factors which modify the action of the glutamine synthetase modulator.
Typical effective amounts comprise between about 0.01 mg/kg body weight to about 1000 mg/kg body weight. A therapeutically effective amount may vary from 0.01 mg/kg to about 1000 mg/kg. In some embodiments, effective amounts range from about 0.1 mg/kg to about 200 mg/kg and/or from about 0.2 mg/kg to about 20 mg/kg. In some embodiments, a therapeutically effective amount ranges from about 0.5 mg/kg/day to about 10 mg/kg/day.

### Routes of Administration and Pharmaceutical Compositions

Glutamine synthetase inhibitors and activators, or pharmaceutical compositions thereof, may be administered using any administration route effective for achieving the desired therapeutic effect. The suitability of particular routes of administration may depend on the subject and/or the purpose. A route of administration may be selected on the basis of a parameter such as the particular type of modulator (small molecule, mRNA, protein, antibody, etc..) being used, the severity of the condition being treated, the dosage required for therapeutic efficacy, etc. Methods of the invention may generally be practiced using any mode of administration that is medically acceptable so that it produces effective levels of the active compounds without causing clinically unacceptable adverse effects. Routes of administration include, but are not limited to, topical (including epicutaneous, enema, eye drops, ear drops, intranasal, vaginal, etc.), enteral (including oral, feeding tube, rectal, etc.), parenteral (including intravenous, intraarterial, intramuscular, intracardiac, subcutaneous, intraosseous, intradermal, intrathecal, intraperitoneal, transdermal, transmucosal, inhalational, infusion etc.), epidural, and intravitreal.

Oral administration may be particularly suitable for subjects having a condition selected from the group consisting of hypertension, diabetes, gangrene, wounds, Buerger's syndrome, and other conditions characterized by reduction or obstruction in microvasculature, though any route of administration is possible.

Compositions suitable for oral administration may be presented as discrete units, such as capsules, or tablets, or may be presented as suspensions in aqueous liquids or non-aqueous liquids such as a syrup, elixir or an emulsion.

In some embodiments, the glutamine synthetase inhibitor or activator is delivered locally to a tissue or to some tissues where a particular biological activity (e.g., angiogenesis, vasculogenesis, improved vascular function, reduce inflammation, immunomodulation, etc.) is desired, for example by inserting a device such as a stent into the tissue of interest.

In some embodiments, local administration comprises administering to the subject a pharmaceutical composition containing a glutamine synthetase inhibitor or activator, together with at least one pharmaceutically acceptable carrier, excipient and/or diluent. In some embodiments, the pharmaceutical composition is suitable for topical application or internal applications. In some such embodiments, the composition is formulated as a salve, a gel or a patch. The pharmaceutical composition may comprise a controlled release matrix.

Pharmaceutical compositions contemplated for use in the present invention include compositions wherein the glutamine synthetase inhibitor or activator is contained in an amount effective to achieve the intended purpose. Determination of an effective amount is well within the capability of those skilled in the art, especially in light of the detailed disclosure provided herein.

In addition to the glutamine synthetase inhibitor or activator (i.e. the active ingredient or agent), the pharmaceutical compositions comprise at least one pharmaceutically acceptable carrier, excipient and/or diluent, which facilitates processing of the active compounds into preparations which can be used pharmaceutically.

**"Pharmaceutically acceptable carrier"** refers to an ingredient in a pharmaceutical formulation, other than an active ingredient, which is nontoxic to the subject to whom it is administered. A pharmaceutically acceptable carrier includes, but is not limited to, a buffer, stabilizer, or preservative.

Examples of suitable carrier or excipients include, without limitation, lactose, dextrose, sucrose, glucose, powdered sugar, sorbitol, mannitol, xylitol, starches, acacia gum, xanthan gum, guar gum, tara gum, mesquite gum, fenugreek gum, locust bean gum, ghatti gum, tragacanth gum, inositol, molasses, maltodextrin, extract of Irish moss, panwar gum, mucilage of isapol husks, Veegum, larch arabogalactan, calcium silicate, calcium phosphate, dicalcium phosphate, calcium sulfate, kaolin, sodium chloride, polyethylene glycol, alginates, gelatine, microcrystalline cellulose, polyvinylpyrrolidone, cellulose, water, saline, syrup, methylcellulose, ethylcellulose, hydroxypropylnethylcellulose, carboxymethylcellulose, polyacrylic acids such as Carbopols, such as Carbopol941, Carbopol980, Carbopol981,and gum bases such as Pharmagum^{™} (SPI Pharma Group; New Castle, Del.), and similar. Typically, the compositions of the present invention comprise from about 10% to about 90% by weight of the carrier, the excipient or combinations thereof.

Preferably, the pharmaceutical composition contains from about 0.001 % to about 90%, preferably from about 0.01 % to about 75%, more preferably from about 0.1% to 50%, and still more preferably from about 0.1% to 10% by weight of the glutamine synthetase inhibitor or activator of the present invention, with the remainder consisting of suitable pharmaceutical carriers, excipients, and/or diluents.

The pharmaceutical composition can be formulated into powders, granules, tablets, capsules, suspensions, emulsions, syrups, oral dosage form, external preparation, suppository or in the form of sterile injectable solutions, such as aerosolized in a usual manner, respectively. When formulated, it can be prepared using a **diluent** or **excipient** such as generally used fillers, extenders, binders, wetting agents, disintegrating agents, surface active agents.

In the pharmaceutical composition, the solid preparation for oral administration may be a tablet, pill, powder, granule, or capsule. The solid preparation may further comprise an excipient. Excipients may be, for example, starch, calcium carbonate, sucrose, lactose, or gelatine. In addition, the solid preparation may further comprise a lubricant, such as magnesium stearate, or talc. In the pharmaceutical composition, liquid preparations for oral administration may be best suspensions, solutions, emulsions, or syrups. The liquid formulation may comprise water, or liquid paraffin. The liquid formulation may, for excipients, for example, include wetting agents, sweeteners, aromatics or preservatives. For the purposes of parenteral administration, compositions containing the glutamine synthetase inhibitor or activator of the invention are preferably dissolved in distilled water and the pH preferably adjusted to about 6 to 8.

Useful preparations of the compositions of the invention for parenteral administration also include sterile aqueous and non-aqueous solvents, suspensions and emulsions. Examples of useful non-aqueous solvents include propylene glycol, polyethylene glycol, vegetable oil, fish oil, and injectable organic esters.

Thus, a further embodiment of the invention is directed to a **pharmaceutical composition** comprising a glutamine synthetase modulator identifiable by the described *in vitro* method, together with at least one pharmaceutically acceptable carrier, excipient and/or diluent. More in particular, a further embodiment of the invention is directed to a **pharmaceutical composition** comprising a glutamine synthetase inhibitor identifiable by the described *in vitro* method, together with at least one pharmaceutically acceptable carrier, excipient and/or diluent. Still more in particular, a further embodiment of the invention is directed to a **pharmaceutical composition** comprising a glutamine synthetase activator identifiable by the described *in vitro* method, together with at least one pharmaceutically acceptable carrier, excipient and/or diluent.

### Description of the figures:

- Figure 1: shows that glutamine is required for cholesterol synthesis. (**A**) Main nutrients used to generate the citrate used for cholesterol synthesis are glucose via oxidation of pyruvate and glutamine via reductive carboxylation of alpha-ketoglutarate (αKG) ; dotted arrow represents 1+ steps. The abundance of total (**B**) glucose (**C**) glutamine (**D**) citrate and (**E**) cholesterol in U2OS cells cultured with only glucose (glc) or only glutamine (gln) for 8h. Data are mean ±s.d. of n=5 independent cultures, *p<0.05; **p<0.01; ****p<0.0001 for Glc vs. Gln by unpaired t-test; a.u.c. area under the curve. (**F**) Left: Simplified model of efflux of m+2 citrate derived from ¹³C₆-glucose when anaplerosis is supplied by glutamine-derived αKG; right: oxidation of glucose feeds the TCA cycle in the absence of glutamine. Red circles represent ¹³C-carbons and black circles represent ¹²C-carbons; dotted arrow represents 1+ steps. (**G**) Mass isotopologues of citrate in U2OS cells cultured for 24h with 25 mM ¹³C₆-glucose in the presence of 2 mM glutamine (gln+) or its absence ±1 mM αKG (gln-;αKG+ respectively). Data are mean ±s.d. of n=5 independent cultures; ns: not significant; ****p<0.0001 by two-way ANOVA. (**H**) Percentage of ¹³C-cholesterol in U2OS cells treated as in (**G**). Data are mean ±s.d. of n=5 independent cultures; ****p<0.0001 for one-way ANOVA. (**I - Q**) show that glutaminestarvation inhibits the synthesis of abundant species of diacylgerol (precursor to trigylcerides), phospholipids, and coenzyme Q10. Percentage of ¹³C of indicated metabolites in U2OS cells cultured for 24h without glutamine, followed by 25 mM ¹³C₆-glucose in the presence of 2 mM glutamine (gln+) or its absence ±1 mM αKG (gln-; αKG+ respectively). Data are mean ± s.d. of n=5 independent cultures; ns: not significant; *p<0.05, **p<0.01, ****p<0.0001 by one-way ANOVA. Outliers identified via ROUT Q=1% removed; DAG: Diacylglycerol, CoQ: coenzyme Q, PC: phosphatidylcholine, PE: phosphoethanolamine, PG: Phosphatidylglycerol, PI: Phosphatidylinositol, SM: Sphingomyelin.
- Figure 2: shows that glutamine starvation shuts down the mevalonate pathway. (A) U2OS cells were cultured ± glutamine (gln+,gln-) for the indicated times and analyzed by immunoblotting for HMGCR, calnexin (CNX), and tubulin (TUBA). (B) Hela cells and (C) Primary human foreskin fibroblasts (HFFs) were cultured w/ or w/o glutamine (gln+,gln-) for 24h. (D) Glutamine synthetase (GLUL or GS) generates gln from ammonia and glutamate. (E) GLUL relative protein expression and RNA log2 TPM+1 in 347 cancer cell lines; data were obtained from previously generated data available at https://sites.broadinstitute.org/ccle/. (F) Percentage of ¹⁵N-glutamine in U2OS and HepG2 cells cultured for 24h w/o gln and supplemented for 24h with 10 mM ¹⁵NH₄Cl. Data are mean ±s.d. of n=4 independent cultures, ****p<0.0001 by unpaired t-test. (G) Total pool size of glutamine from (F); ***p<0.001, by two-way ANOVA. (H) U2OS and (I) HepG2 cells were starved of glutamine for 24h, then treated for 24 h w/ gln or w/o gln ± 10 mM ¹⁵NH₄Cl. Samples were harvested and analyzed by immunoblotting for HMGCR and actin (ACTA). (J) HepG2 cells were starved of gln for 24h, then treated w/gln or w/out gln ± 10 mM NH₄CI ± 500 µM methionine sulfoximine (MSX) for 8h and analyzed by immunoblotting for HMGCR, GLUL, and ACTA. For all experiments, gln was used at 2 mM.
- Figure 3: shows that glutamine-sensing is required for HMGCR expression. Glutamine-fed U2OS cells were cultured (A) ± glutamine (gln+, gln-) and MG-132 (10 µM) for 8h; (B) for 24h gln+ or gln- ± αKG as indicated; (C) ± gln in ± ISRIB (200nM) ± αKG as indicated for 8h. (A-C) Samples were analyzed by immunoblotting for HMGCR, calnexin (CNX), hypoxanthine phosphoribosyltransferase 1 (HPRT1), ubiquitin (UB), p70 S6 kinase (S6K), p70 phospho-S6K (pS6K), ATF4; and actin (ACTA). (D) Glutamine supplies carbons and nitrogens for the biosynthesis of TCA metabolites, glucosamine-6-phosphate (GlcN6P) that is used for UDP-GIcNAc synthesis, non-essential amino acids (NEAA) and nucleotides. Heat map of z-score normalization of the total pool size of (E) TCA-cycle metabolites and UDP-GIcNAc, (F) non-essential amino acids (NEAA), and (G) nucleotides in U2OS cells cultured for 24h with 25 mM ¹³C₆-glucose in gln+ or gln- ± αKG (gln-; αKG+ respectively) conditions. Data are mean ±s.d. of n=5 independent cultures. (H) U2OS cells were cultured w/o gln for 8 hours w/the indicated supplements and concentrations; gln: 2 mM; αKG: 1mM; NEAA: 100 µM (glycine, alanine, asparagine, aspartate, glutamate, proline and serine), nucleosides (cytidine 7.3 mg/L, guanosine 8.5 mg/L, uridine 7.3 mg/L, adenosine 8 mg/L, and thymidine 2.4 mg/L), and glucosamine (precursor to UDP-GIcNAc): 1 mM. Samples were analyzed by immunoblotting for HMGCR, CNX, pS6K, and ACTA. For all experiments, glutamine was used at 2 mM, αKG at 1 mM.

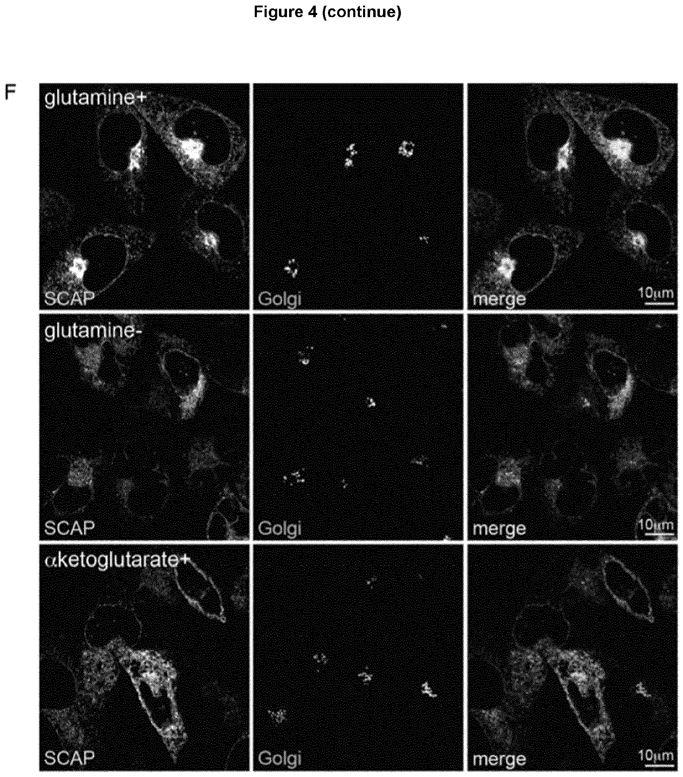

- Figure 4: shows that glutamine-sensing licenses SREBP2 proteolysis-dependent activation. (**A**) U2OS cells were cultured with glutamine (gln+) or without glutamine ± αKG (gln-, αKG+) for 8h and analyzed by RNAseq analysis. Glutamine-responsive genes were those differentially regulated (FDR adjusted p-value of <0.01) between gln+ vs. gln- and gln+ vs. αKG+, but not significantly different between gln- vs. αKG+ sets; n=6 independent replicates. (**B**) GO analysis was performed on top 5% of glutamine-responsive genes from analysis in (**A**); red text corresponds to cholesterol-related processes. (**C**) Heat map of z-score of expression values of the top 25 glutamine-regulated genes from the subset as described in (**A**); genes in red are SREBP2 targets. (**D**) *HMGCR, SQLE, FOPS, and FDFT1* mRNA levels in U2OS cells that were cultured w/o gln for 24h, followed by gln- and gln+ culture for 8h. mRNA expression was measured by the standard curve method and normalized to *HPRT1;* y-axis depicts the transcript levels relative to gln-. (**E**) U2OS cells treated as in (**A**) were harvested and analyzed by immunoblotting for: HMGCR, precursor (p) SREBP2, **mature (m) SREBP2, FDFT1, S6K, pS6K, and actin (ACT). (F)** Representative images of eGFP-SCAP-expressing CHO cells cultured w/o gln for 24h, and then treated w/gln (gln+) or w/o gln (gln-), or w/o gln w/αKG (gln-, αKG+) in the presence of methionine sulfoximine (MSX; 500 µM) for 24h and processed for immunofluorescence analysis of the Golgi (golgin-97). Panels show localization of eGFP-SCAP relative to the Golgi, scale bar 10 µm. (**G**) Percentage of cells with Golgi-localized eGFP-SCAP from experiments as in (**F**); data are mean ± s.d. of >100 cells counted from n=4 biological replicates; ****p<0.0001 for gln- vs. gln+ and gln- vs. aKG+ via one-way ANOVA. (**H**) Hep G2s were starved of glutamine for 24h and treated w/ brefeldin A: 0.5 µg/ml or gln for indicated time points. Samples were analyzed by immunoblotting for HMGCR, SREBP2, and ACTA. For all experiments, glutamine was used at 2 mM and αKG at 1 mM.

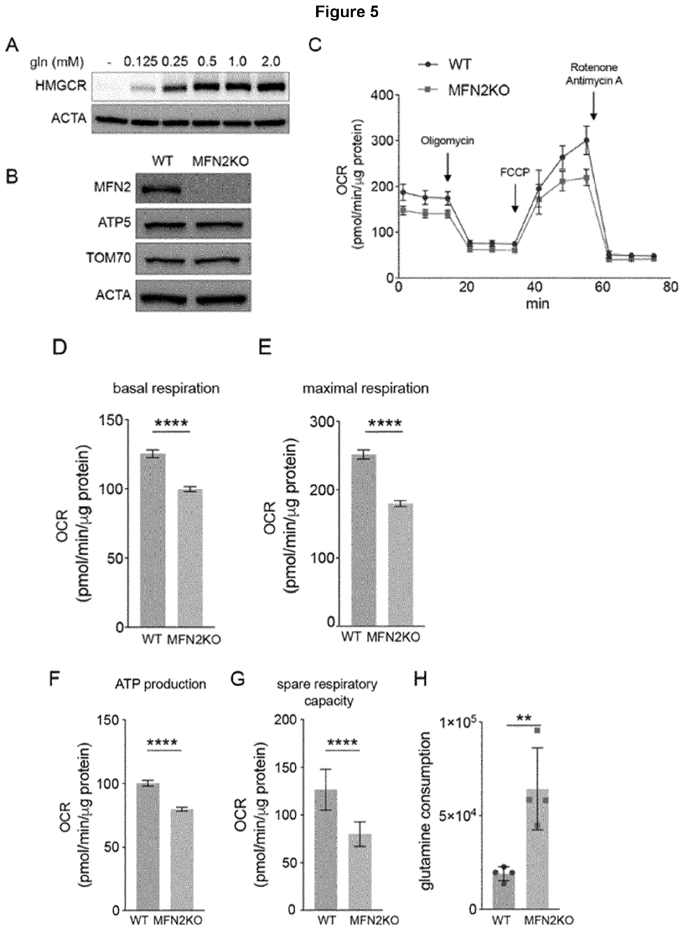

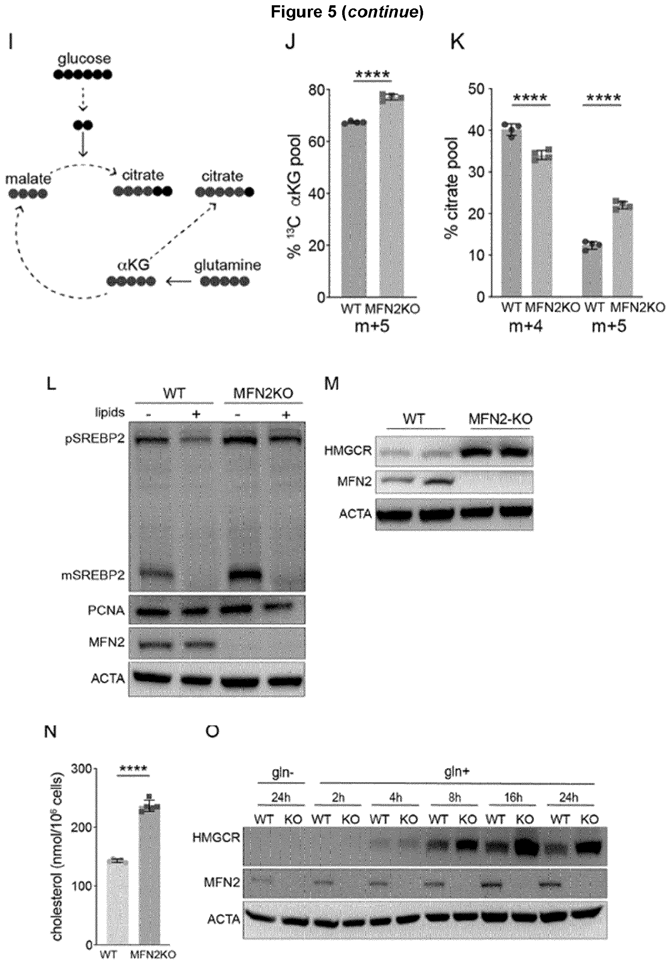

- Figure 5: shows that chronic mitochondrial dysfunction increases glutamine consumption and mevalonate pathway activation. (**A**) U2OSs were cultured w/o glutamine (gln) for 24h and treated with indicated gln concentrations for 8h. Samples were analyzed by immunoblotting for HMGCR and actin (ACTA). (**B**) WT and MFN2 knockout (KO) U2OS cells were serum-starved for 24h and analyzed by immunoblot analysis for MFN2, TOM70, ACTA, and ATP synthase F1 subunit beta (ATP5) and in parallel for (**C**) oxygen consumption analyses including (**D**) basal respiration; (**E**) maximal respiration; (**F**) ATP production; (**G**) spare respiratory capacity. (**H**) Total glutamine in DMEM versus glutamine in media 8 hours after culture with WT or MFN2 KO cells. (**I**) During mitochondrial dysfunction glutamine feeds citrate synthesis via reductive carboxylation of αKG. (**J**) m+5 αKG and (**K**) m+4/5 citrate in U2OSs cultured for 24h with 1 mM ¹³C₅-glutamine. Data are mean ±s.d. of n=4 independent cultures; ****p<0.0001 by unpaired t-test (**J**) or (**K**) two-way ANOVA. (L) WT and MFN2-KO U2OSs cultured ± lipids were analyzed by immunoblotting for SREBP2, the nuclear protein PCNA, and ACTA. (**M**) WT and MFN2KO U2OSs in lipid-free culture for 24h were analyzed by immunoblotting for HMGCR, MFN2, and ACTA, and for (**N**) total cholesterol levels. (**O**) WT and MFN2KO U2OSs in gln-free media for 24h were refed gln for indicated times and analyzed by immunoblotting for HMGCR and ACTA. Glutamine is used at 2 mM unless otherwise stated.
- Figure 6: shows that glutamine anaplerosis limits catabolic oxidation of glucose. (**A**) m+4 mass isotopologues of malate and succinate in U2OS cells cultured for 24h with 25 mM ¹³C₆-glucose in the presence of 2 mM glutamine (gln+) or its absence ± 1 mM αKG (gln-; αKG+ respectively). Data are mean ±s.d. of n=5 independent cultures, ns not-significant; **p<0.01; ****p<0.0001 by one-way ANOVA. (**B**,**C**) U2OS cells of indicated genootype were cultured with glutamine and analyzed by immunoblotting for citrate synthase (CS), solute carrier family 25 (SLC25A1), and actin (ACTA). (**D**) The abundance of total citrate in indicated U2OS genotypes. Data are mean ±s.d. of n=3 independent cultures, ****p<0.0001 by one-way ANOVA. (**E-F**) U2OSs with indicated genotypes were cultured in gln-free media for 24h, refed gln and harvested at indicated times for analysis by immunoblotting for HMGCR, CNX, and ACTA. For all experiments, glutamine was used at 2 mM and αKG at 1 mM.
- Figure 7: shows that inhibition of glutaminolysis does not affect HMGCR in glutamine-fed cells; pyruvate, DON and D-glutamine do not rescue HMGCR in glutamine-starved cells. (**A**) U2OS cells were cultured w/o glutamine for 24h and treated as indicated for 24h. Concentrations used: 1mM sodium pyruvate (pyr), 2 mM glutamine (gln) and 25mM glucose (glc). Samples were analyzed by immunoblotting for HMGCR and actin (ACTA). (**B**) U2OS cells were cultured as indicated for 8h. Concentrations used: 30µM BPTES, 2 mM gln, 1 mM αKG. Samples were analyzed by immunoblotting for HMGCR, calnexin (CNX), ACTA, and phospho-p70 S6 kinase (pS6K). (**C**) U2OS cells were cultured w/o glutamine for 24h and treated as indicated for 24h with gln or 6-diazo-5-oxo-L-norleucine (DON) with the indicated concentrations. Samples were analyzed by immunoblotting for HMGCR and ACTA. (**D**) U2OS cells were cultured w/o glutamine for 24h and treated as indicated for 24h with 2mM gln, 2 mM D-gln or 1 mM αKG. Samples were analyzed by immunoblotting for HMGCR, ACTA, and CNX.

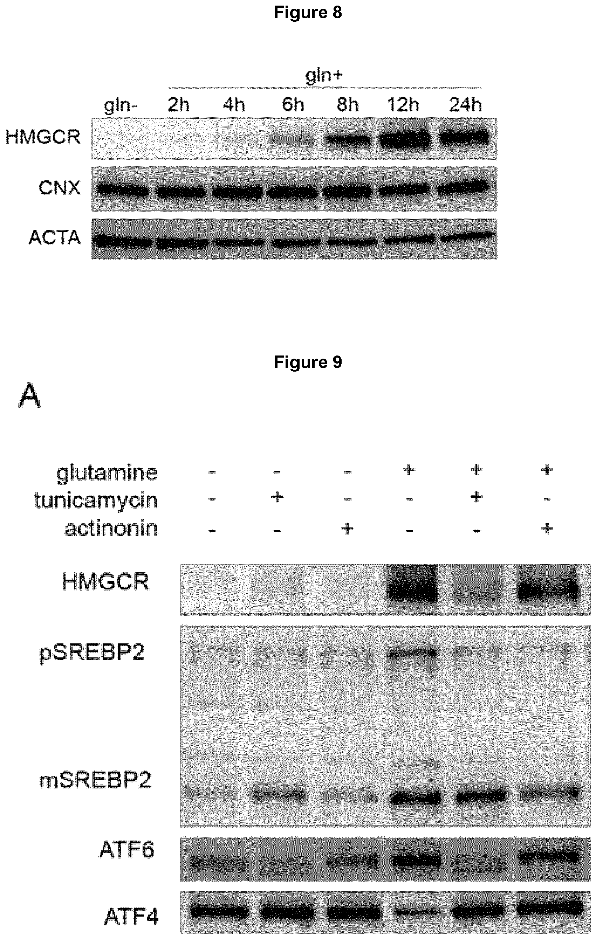

- Figure 8: shows HMGCR following glutamine-starvation and refeeding. U2OS cells were cultured w/o glutamine for 24h and refeed with 2 mM glutamine at indicated time points. Samples were analyzed by immunoblotting for HMGCR, calnexin (CNX), and actin (ACTA).

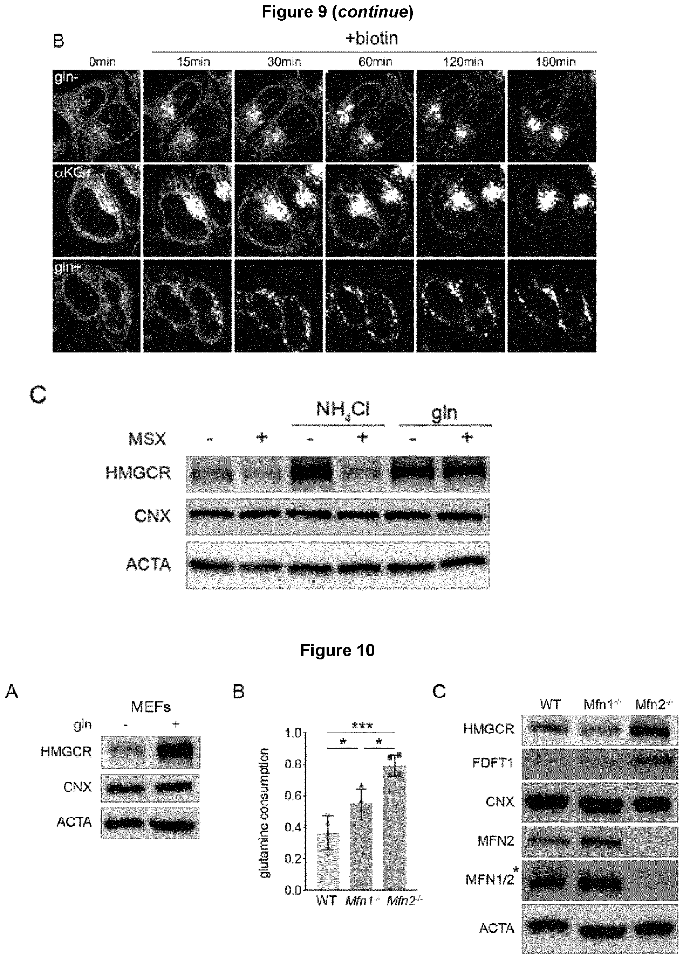

- Figure 9: shows that glutamine starvation does not globally affect S1P and S2P activity or ER-to-Golgi trafficking. (**A**) U2OS cells were cultured w/o glutamine for 24h and treated as indicated for 8 hours. Concentrations used: 2 mM glutamine (gln), 15 µg/ml tunicamycin,100µM actinonin. Samples were analyzed by immunoblotting for HMGCR, precursor and mature SREBP2 (pSREBP2, mSREBP2), ATF6, and ATF4. (**B**) HeLas stably expressing mannosidase II-mCherry (MANII) fused to a streptavidin-binding peptide, and streptavidin fused KDEL which retains it in the ER, were cultured without glutamine for 24h. Following 8h of treatment w/ glutamine (gln+) or w/o glutamine ± 1 mM αKG (gln-, αKG+, respectively). Representative live-cell images of mCherry-MANII at indicated time points following 40 µM biotin addition. Scale bar 10 µm. (**C**) eGFP-SCAP-expressing CHO cells were starved of gln for 24h, then treated w/gln or w/o gln ± 10 mM NH₄Cl ± 500 µM methionine sulfoximine (MSX) for 8h and analyzed by immunoblotting for HMGCR, glutamine synthetase (GLUL), and actin (ACTA). For all experiments, gln was used at 2 mM.
- Figure 10: shows that MFN2 loss drives an increase in HMGCR independently of mitochondrial fusion. (**A**) WT MEFs were cultured without glutamine for 8h and analyzed by immunoblotting for HMGCR, calnexin and actin (ACTA). (**B**) Media from WT, *Mfn1*^{*-*/}*⁻, Mfn2*^{*-*/*-*} MEFs cultures were analyzed for glutamine consumption after 24h. Data are mean ± s.d. of n=4 independent cultures; *p<0.05; ***p<0.001 by one-way ANOVA. (**C**) WT, *Mfn1*^{*-*/*-*} and *Mfn2⁻¹⁻* MEFs cultured without lipids for 24h were analyzed by immunoblotting for HMGCR, CNX, MFN1, MFN2, and ACTA.

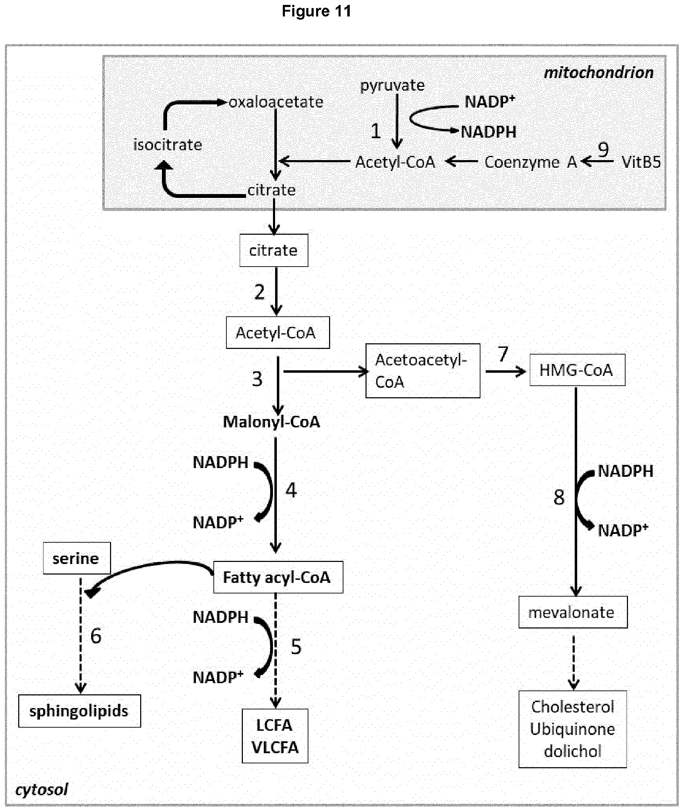

- Figure 11: shows fatty acid synthesis and MVA pathway relationships. At high energy fed state mitochondrial pyruvate is oxidized to acetyl-CoA and NADH to produce citrate (1). Citrate is exported to the cytosol where it is split by citrate lyase back to acetylCoA (2) and oxaloacetate. Citrate activates acetyl CoA carboxylase (3) for malonyl CoA synthesis. This results in saturated fatty acid synthesis by fatty acid synthase up to palmitate (C16) using NADPH (4). Further elongation of fatty acids depends on a set of elongases (ELOV 1-6) (5). Glycosphingolipids synthesis starts with condensation of L-serine and palmitoyl-CoA by L-serine palmitoyl transferase (6). Isoprenoids synthesis (cholesterol, ubiquinone, dolichol, etc ) starts in the cytoplasm from acetyl CoA to HMGCoA formation (7). HMGCoA is further reduced by NADPH to mevalonate by the highly regulated HMGCoA reductase (8). Finally, coenzyme A is provided by vitamin B5 through the initial step of intramitochondrial pantothenate kinase 2 (9).
- Figure 12: shows MVA pathway and its components: pre-squalene pathway, sterol synthesis pathway, shunt pathway and non-sterols isoprenoids pathway.

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the scope of the invention.

Further modifications and alternative embodiments of various aspects of the invention will be apparent to those skilled in the art in view of this description. Accordingly, this description is to be construed as illustrative only and is for the purpose of teaching those skilled in the art the general manner of carrying out the invention. It is to be understood that the forms of the invention shown and described herein are to be taken as examples of embodiments. Elements and materials may be substituted for those illustrated and described herein, parts and processes may be reversed, and certain features of the invention may be utilized independently, all as would be apparent to one skilled in the art after having the benefit of this description of the invention. Changes may be made in the elements described herein without departing from the scope of the invention as described in the following claims.

### EXAMPLES

### Methods

### Materials and Methods:

### Cell culture and cell lines

HeLa adenocarcinoma cells, HepG2 hepatocellular carcinoma, and Wild-type (WT) MEFs as well as *Mfn1*^{*-*/}*⁻,* and *Mfn2*^{*-*/*-*} mouse embryonic fibroblasts (MEFs) were obtained from ATCC (CCL-2, HB-8065, CRL-2991, CRL-2992, and CRL-2993, respectively). eGFP-SCAP-expressing CHO Chinese hamster ovary cells were provided by Drs. J Goldstein and M Brown (U of Texas Southwestern Medical Center); WT and MFN2 KO U2OS cells were provided by Dr. Edward A Fon (McGill University, Montreal, Canada). All cell lines were cultured in complete DMEM (cDMEM: DMEM + 10% heat-inactivated FBS + 1%P/S) at 5% CO₂ except for CHO cells which were cultured in DMEM/F12 media containing 5% heat-inactivated FBS. For all the experiments, cells were plated and cultivated without FBS (DMEM and 10% H₂0 for cell culture + 1%P/S) to promote cholesterol synthesis. Cells were tested frequently for *Mycoplasma* infection by PCR.

### CRISPR Knockout cell lines

To generate CS and SLC25A1 CRISPR KO cell lines, U2OS cells were transduced with lentiviral particles containing the following sgRNAs cloned into the pLenti CRISPRv2 (Addgene #5296): CS guide 1 F: CAACATGGCAAGACGGTGGT (SEQ ID NO 1); CS guide 1 R: ACCACCGTCTTGCCATGTTG (SEQ ID NO 2); CS guide 2 F: TTTTCCAAACCTTACCGTGG (SEQ ID NO 3); CS guide 2 R: CCACGGTAAGGTTTGGAAAA (SEQ ID NO 4); SLC25A1 guide 1 F: AGATCTCGATGCCACCCGCC (SEQ ID NO 5); SLC25A1 guide 1 R: GGCGGGTGGCATCGAGATCT (SEQ ID NO 6); SLC25A1 guide 2 F: CTACGGTTCCATCCCCAAGG (SEQ ID NO 7); SLC25A1 guide 2 R: CCTTGGGGATGGAACCGTAG (SEQ ID NO 8), and AAVS1 F: CACCGGGGGCCACTAGGGACAGGAT (SEQ ID NO 9); AAVS1 R: AAACATCCTGTCCCTAGTGGCCCCC (SEQ ID NO 10). Cells were selected in 3 ug/ml puromycin, clones were isolated by FACs sorting, and validated by immunoblotting for CS and SLC25A1.

### Immunofluorescence Assay and antibodies

For immunofluorescence (IF) analysis of eGFP-SCAP-expressing CHOs, .25 × 10⁴ cells were plated in a 24-well glass-bottom sensoplate, starved of glutamine for 24h, and incubated with methionine sulfoximine at 0.5 mM with either no glutamine, 2 mM glutamine, or 1mM a-ketoglutarate. After 24h, cells were fixed in 4% paraformaldehyde (fresh) in prewarmed DMEM/F12 for 20 min at 37C, permeabilized for 20 min at RT with 0.2% triton in 1X PBS, blocked in 3% bovine serum albumin (BSA) in 1X PBS for 30 min, and incubated in golgin-97 (CST #13192) at 1:250 in 3% BSA O/N, rinsed 3X in 1X PBS, and incubated with secondary antibody anti-rabbit Alexa Fluor Plus 594 at 1:1000 for 45 minutes. Following 3X rinses in 1X PBS, images were taken using an Olympus IXplore SpinSR spinning disk confocal microscope. All images were taken with a 100X objective and excitation with 488 and 561 laser lines and processed via cellSens software.

### Immunoblotting and antibodies

For all experiments, unless otherwise indicated, cells were plated in complete DMEM. The next day cell monolayers were rinsed 2X with 1X PBS and cultured in DMEM overnight, followed by treatments as indicated in text. Whole cells were harvested in chilled lysis buffer (50mM Hepes-KOH pH 7.4, 40mM NaCl, 2mM EDTA, 1.5mM NaVO4, 50mM NaF, 10mM NaPyrophosphate, 10mM, NaBetaGlycerophosphate (disodium salt pentahydrate), 1% Triton X-100) containing 0.25U/uL. Lysates were subsequently shaken at 1500 rpm at 37°C for 15min, centrifuged for 5 min at 1200 rpm at 4°C, and the supernatant was transferred into a fresh tube. Protein concentration was quantified using Pierce^{™} BCA Protein Assay Kit and after diluted with 5X SDS added to a final of 1X SDS. 10 ug of protein was applied in an SDS-PAGE gel and transferred to PVDF membranes. Membranes were blocked with PBS-0.05% Tween 20 (PBS-T) and 5% non-fat milk for 30min. Primary antibodies were incubated in PBS-T overnight. Following incubation, blots were washed three times in PBS-T and then incubated with horseradish peroxidase (HRP)-conjugated anti-mouse IgG (CST #7076) or anti-rabbit IgG (CST #7074) at a 1:10000 dilution for 60 minutes ar RT and developed using a chemiluminescence system (Pierce^{™} ECL Western Blotting Substrate or Pierce SuperSignal^{™} West Atto Ultimate Sensitivity Substrate; ThermoFisher Scientific). The following antibodies were used: MFN1/2 (abcam ab57602), MFN2 (Abnova 157H00009927-M03J), CALR (CST #12238), ACTB (CST #4970 and Proteintech 66009-1-lg), Golgin-97 (CST #13192S), TOMM70 (Sigma HPA048020), HMGCR (Sigma AMAB90619), FDFT1 (Sigma HPA008874), SREBP2 (BD Biosciences 557037), TUBA (Proteintech 66031-1-lg), GLUL (CST #80636S), UB (CST # 3936S), HPRT1 (Thermo Fisher Scientific PA522281), pS6K (CST #97596S), S6K (CST #9202S), ATF4 (CST # 11815S), c-Myc (CST #5605S), HK2 (CST #C64G5), FASN (CST #3189S), PCNA (BD Biosciences 610664), ATF6 (CST #65880), and CNX (GeneTex GTX109669 and Proteintech 10427-2-AP).

### Metabolomics and Isotope labeling

2 × 10⁵ cells were plated in complete DMEM in 6 wells plates. The following day, cells were rinsed 2X with 1X PBS and cultured in DMEM. Treatments were started the following day as indicated in the text in 6 wells plates containing. For glucose-related isotope-labeling experiments, D-Glucose-¹³C₆ (Sigma #389374-1G) was administed at 25mM in 1 ml of glutamine-free DMEM. For ammonia-related isotope-labeling experiments, ammonium label experiments Ammonium-¹⁵N chloride (Sigma #299251-250MG) at 10mM. After the indicated treatments cells were washed twice with 75mM of ammonium carbonate pH =7.4 and the plates were frozen in liquid nitrogen and stored at -80°C until metabolite extraction. For metabolite extraction, 60% MeOH containing internal standards buffer was added to each well of the frozen plate. The cells were then scraped, transferred to a new tube containing MTBE and EquiSPLASH^{™} LIPIDOMIX^{®} (Avanti 330731-1EA), and incubated for 30 min at 1500 rpm at 4°C. The samples were centrifuged for 10 min at 21.000xg at 4°C. The supernatants were then collected in a new tube with LCMS-grade H₂O, incubated for 10 min at 1500 rpm at 15°C, and further centrifuged for 5 min at 16.000xg at 15°C to obtain a clear phase separation. The upper phase (apolar metabolites) and the down phase (polar metabolites) were collected in different tubes. After, the metabolite extracts were dried down in speed vac and stored at -80°C until further analysis by LC-HRS-MS as described further.

### Semi-targeted liquid chromatography-high-resolution mass spectrometry-based (LC-HRS-MS) analysis of amine-containing metabolites

The LC-HRMS analysis of amine-containing compounds was performed using a QE-Plus high-resolution mass spectrometer coupled to a Vanquish UHPLC chromatography system (Thermo Fisher Scientific). In brief: 50 µL of the available 150 µL of the above mentioned (AEX-MS method) polar phase were mixed with 25 µl of 100 mM sodium carbonate (Sigma), followed by the addition of 25 µl 2% [v/v] benzoylchloride (Sigma) in acetonitrile (UPC/MS-grade, Biosove, Valkenswaard, Netherlands). The derivatized samples were thoroughly mixed and kept at a temperature of 20°C until analysis. For the LC-HRMS analysis, 1 µl of the derivatized sample was injected onto a 100 × 2.1 mm HSS T3 UPLC column (Waters). The flow rate was set to 400 µl/min using a binary buffer system consisting of buffer A (10 mM ammonium formate (Sigma), 0.15% [v/v] formic acid (Sigma) in UPC-MS-grade water (Biosove, Valkenswaard, Netherlands). Buffer B consisted of acetonitrile (IPC-MS grade, Biosove, Valkenswaard, Netherlands). The column temperature was set to 40°C, while the LC gradient was: 0% B at 0 min, 0-15% B 0- 4.1min; 15-17% B 4.1 - 4.5 min; 17-55% B 4.5-11 min; 55-70% B 11 - 11.5 min, 70-100% B 11.5 - 13 min; B 100% 13 - 14 min; 100-0% B 14 -14.1 min; 0% B 14.1-19 min; 0% B. The mass spectrometer (Q-Exactive Plus) was operating in positive ionization mode recording the mass range m/z 100-1000. The heated ESI source settings of the mass spectrometer were: Spray voltage 3.5 kV, capillary temperature 300°C, sheath gas flow 60 AU, aux gas flow 20 AU at 330°C, and the sweep gas was set to 2 AU. The RF-lens was set to a value of 60. The LC-MS data analysis was performed using the TraceFinder software (Version 5.1, Thermo Fisher Scientific). The identity of each compound was validated by authentic reference compounds, which were measured at the beginning and the end of the sequence. For data analysis the area of the protonated [M + nBz + H]+ (nBz stands for the number of benzoyl moieties attached to each compound) isotopologue mass peaks of every required compound were extracted and integrated using a mass accuracy <3 ppm and a retention time (RT) tolerance of <0.05 min as compared to the independently measured reference compounds. If no independent 12C experiments were carried out, where the pool size is determined from the obtained peak area of the 12C monoisotopologue, the pool size determination was carried out by summing up the peak areas of all detectable isotopologues per compound. These areas were then normalized, as performed for un-traced 12C experiments, to the internal standards, which were added to the extraction buffer, followed by a normalization to the protein content or the cell number of the analyzed samples. The relative isotope distribution of each isotopologue was calculated from the proportion of the peak area of each isotopologue towards the sum of all detectable isotopologues. The 13C enrichment, namely the area attributed to 13C molecules traced in the detected isotopologues, was calculated by multiplying the peak area of each isotopologue with the proportion of the 13C and the 12C carbon number for the corresponding isotopologue (the 12C and 13C monoisotopologue areas were multiplied with 0 and 1 respectively). The obtained 13C area of each isotopologue are summed up, providing the peak area fraction associated to 13C atoms in the compound. Dividing this absolute 13C area by the summed area of all isotopologues provides the relative 13C enrichment factor.

### Anion-Exchange Chromatography Mass Spectrometry (AEX-MS) for the analysis of anionic metabolites

Extracted metabolites were re-suspended in 150 µl of UPLC/MS grade water (Biosolve), of which 100 µl were transferred to polypropylene autosampler vials (Chromatography Accessories Trott, Germany) before AEX-MS analysis. The samples were analysed using a Dionex ionchromatography system (Integrion Thermo Fisher Scientific) as described previously. In brief, 5 µL of the resuspended polar metabolite extract were injected in push-partial mode, using an overfill factor of 1, onto a Dionex lonPacAS11-HC column (2 mm × 250 mm, 4 µm particle size, Thermo Fisher Scientific) equipped with a Dionex lonPac AG11-HC guard column (2 mm × 50 mm, 4 µm, Thermo Fisher Scientific). The column temperature was held at 30°C, while the auto sampler temperature was set to 6°C. A potassium hydroxide gradient was generated using a potassium hydroxide cartridge (Eluent Generator, Thermo Scientific), which was supplied with deionized water (Milli-Q IQ 7000, Millipore). The metabolite separation was carried at a flow rate of 380 µL/min, applying the following gradient conditions: 0-3 min, 10 mM KOH; 3-12 min, 10-50 mM KOH; 12-19 min, 50-100 mM KOH; 19-22 min, 100 mM KOH, 22-23 min, 100-10 mM KOH. The column was re-equilibrated at 10 mM for 3 min. For the analysis of metabolic pool sizes the eluting compounds were detected in negative ion mode using full scan measurements in the mass range m/z 77 - 770 on a Q-Exactive HF high resolution MS (Thermo Fisher Scientific). The heated electrospray ionization (ESI) source settings of the mass spectrometer were: Spray voltage 3.2 kV, capillary temperature was set to 300°C, sheath gas flow 50 AU, aux gas flow 20 AU at a temperature of 330°C and a sweep gas glow of 2 AU. The S-lens was set to a value of 60. The LC-MS data analysis was performed using the TraceFinder software (Version 5.1, Thermo Fisher Scientific). The identity of each compound was validated by authentic reference compounds, which were measured at the beginning and the end of the sequence. For data analysis the area of the deprotonated [M-H+]-1 or doubly deprotonated [M-2H]-2 isotopologues mass peaks of every required compound were extracted and integrated using a mass accuracy <3 ppm and a retention time (RT) tolerance of <0.05 min as compared to the independently measured reference compounds. If no independent 12C experiments were carried out, where the pool size is determined from the obtained peak area of the 12C monoisotopologue, the pool size determination was carried out by summing up the peak areas of all detectable isotopologues per compound. These areas were then normalized, as performed for un-traced 12C experiments, to the internal standards, which were added to the extraction buffer, followed by a normalization to the protein content or the cell number of the analyzed samples. The relative isotope distribution of each isotopologue was calculated from the proportion of the peak area of each isotopologue towards the sum of all detectable isotopologues. The 13C enrichment, namely the area attributed to 13C molecules traced in the detected isotopologues, was calculated by multiplying the peak area of each isotopologue with the proportion of the 13C and the 12C carbon number for the corresponding isotopologue (the 12C and 13C monoisotopologue areas were multiplied with 0 and 1 respectively). The obtained 13C area of each isotopologue are summed up, providing the peak area fraction associated to 13C atoms in the compound. Dividing this absolute 13C area by the summed area of all isotopologues provides the relative 13C enrichment factor.

### UPLC-HRMS-based measurement of cholesterol

The UPLC-HRMS analysis of cholesterol was performed using a modified method described by McDonald et al (McDonald, Thompson et al. 2007). In brief: The lipid fraction of the two-phase metabolite extract was re-suspended in 100 µL of 95% methanol (Optima-Grade, Thermo Fisher Scientific) and incubated at 4°C for 15 min on a thermomixer. The re-suspended extract was centrifuged for 5 min at 16.000 × g at 4°C and the cleared supernatant were transferred to auto-sampler vials with 200 µL glass inserts (Chromatography Accessories Trott, Germany). For the LC-HRMS analysis, 3 µl of the sample were injected onto a 100 × 2.1 mm HSS T3 UPLC column (Waters). The flow rate was set to 300 µl/min using a binary buffer system consisting of buffer A (5 mM ammonium acetate (Sigma), in 85% LC-MS-grade methanol (Optima-Grade, Thermo Fisher Scientific)), while buffer B consisted of 5 mM ammonium acetate (Sigma), in 100% LC-MS-grade methanol. The column temperature was set to 40°C, while the LC gradient was: 0 min 0% B, 2 min, 0% B 15 min; 100% B, 25 min 100% B, 25.1 min 0% B and 30 min 0% B. The mass spectrometer (Q-Exactive HF, Thermo Fisher Scientific) was operating in positive ionization mode recording the mass range m/z 250-600. The heated ESI source settings of the mass spectrometer were: Spray voltage 3.5 kV, capillary temperature 300°C, sheath gas flow 60 AU, aux gas flow 20 AU at a temperature of 340°C and the sweep gas to 2 AU. The S-lens was set to a value of 60 arbitrary units. Semi-targeted data analysis for the samples was performed using the TraceFinder software (Version 4.1, Thermo Fisher Scientific). The identity of each compound was validated by authentic reference compounds, which were run before and after every sequence. Peak areas of [M -H2O + H]+ ions were extracted using a mass accuracy (<3 ppm) and a retention time tolerance of <0.05 min. Areas of the cellular pool sizes were normalized to the internal standards (Cholesterol D7 Avanti Polar Lipids), which were added to the extraction buffer, followed by a normalization to the cell number of the analyzed sample. Enrichment analysis and isotope distribution were calculated as described in the IC/Bz section.

### Total levels of cholesterol quantification in WT and MFN2-KO cells

Cholesterol levels were determined by Liquid Chromatography coupled to Electrospray Ionization Tandem Mass Spectrometry (LC-ESI-MS/MS). 1.5 to 5 × 10⁶ U2OS cells were homogenized in 300 µl of Milli-Q water using the Precellys 24 Homogenisator (Peqlab) at 6.500 rpm for 30 sec. The protein content of the homogenate was routinely determined using bicinchoninic acid. To 70 µl of homogenate 430 µl of Milli-Q water, 1.875 ml of chloroform/methanol/37% hydrochloric acid 5:10:0.15 (v/v/v), and 1.26 nmol of deuterated cholesterol-D7 (Avanti Polar Lipids) as internal standard were added. Lipids were extracted using the "One-Step Extraction" described in Ozbalci et al, 2013 (Ozbalci et al, Quantitative Analysis of Cellular Lipids by Nano- Electrospray Ionization Mass Spectrometry, Methods in Molecular Biology, 1033, (2013)). Dried lipid extracts were resolved in 300 µl of methanol and sonicated for 5 min. After centrifugation (12.000 × g, 5 min, 4 °C), 40 µl of the clear supernatants were transferred to autoinjector vials. LC-MS/MS analysis of cholesterol was performed as previously described (Mourier et al., Mitofusin 2 is required to maintain mitochondrial coenzyme Q levels, JCB, 208(4): 429-442, (2015))

### Glutamine consumption

For U2OS cells, cells were cultured in DMEM without FBS, pelleted, and stored at -80°C until extraction. Samples were resuspended in extraction solution (ice-cold 80% methanol), incubated for 5 minutes on ice, and centrifuged for 3 minutes, 20.000 × g, at -9 C. Supernatant was collected and kept on dry ice. Samples were further resuspended two more times with extraction solution and treated as previously described to further collect supernatants. Samples were finally dried using a Genevac EZ2 speed vac and further processed following protocols previously described (Edwards-Hicks et al., Metabolic Dynamics of In Vitro CD8+ T Cell Activation. Metabolites, 11, 12 (2021)). Targeted metabolite quantification by LC-MS was carried out using an Agilent 1290 Infinity II UHPLC in line with an Agilent 6495 QQQ-MS operating in MRM mode. MRM settings were optimized separately for all compounds using pure standards. LC separation was performed on a Phenomenex Luna propylamine column (50 × 2 mm, 3 µm particles) as described previously . Briefly, a solvent gradient of 100% buffer B (5 mM ammonium carbonate in 90% acetonitrile) to 90% buffer A (10 mM NH4 in water) was used. Flow rate was from 1000 to 750 µL/min. The autosampler temperature was 5 C and the injection volume was 2 µL. Raw data were analyzed using the R package automRm (Eilertz, Mitterer, Büscher, in preparation). Further analysis was performed using MassHunter software (Bruker). The amount of glutamine consumed by the cells was calculated as the difference between the starting glutamine concentration in DMEM and the glutamine concentration in DMEM after 8h of culturing cells of the indicated genotype. The glutamine consumption was normalized to the average cell density. For MEFs, cells between 1-2 ×10⁶ MEFs were plated in 10 cm dishes in cDMEM so as to obtain similar confluency. The next day, cells were rinsed 2X in 1X PBS and cultured in serum-free DMEM. After 24 hours, 1 ml of cultured medium was analyzed by the CEDEX Bio Analyzer according to manufacturer's instructions.

### Quantitative real-time PCR

Total RNA was prepared from U2OS treated as indicated in the text using TriZol (Thermo Fischer Scientific; #15596018) according to manufacturer's instructions. RNA was quantified by spectrophotometry using Nanodrop One (ThermoFisher) and purity was assessed by the absorbance ratios of 26-:280 and 260:230. From an equal amount of total RNA, complementary DNA was generated with SuperScript^{™} VILO^{™} Master Mix (Thermo Fischer Scientific; # 11755050). Real-time quantitative PCR (qPCR) was based on the SYBR green chemistry and carried out using the applied biosystems Real-Time PCR system. The following primer sequences were used: *HMGCR* (F: 5'-AGG CCT GTT TGC AGA TGC TAG G -3' (SEQ ID NO 11), R: 5'-GAT GTC CTG CTG CCA ATG CT-3' (SEQ ID NO 12)), *SQLE* (F: TGG GCT GCT TTC TGT ATT GTC TCC-3' (SEQ ID NO 13), R: CAC CAC TAC TGA GAA GGG CTG G-3' (SEQ ID NO 14)), *FDFT1* (F: 5'-GGA AAG GGC AAG CAG TGA CC -3' (SEQ ID NO 15), R: 5'-GGA TGG TGG AGA TGA TCT GCC TT -3' (SEQ ID NO 16)), *and HPRT1* (F: 5'-TGA CAC TGG CAA AAC AAT GCA -3' (SEQ ID NO 17), R: 5'-CGT CCT TTT CAC CAG CAA GCT-3' (SEQ ID NO 18)). Ct levels were linearized according to *HPRT1* expression levels.

### RNAseq extraction and analysis

For RNAseq analysis cells were plated and cultured as indicated in the text. Two independent experiments were performed with n=3. RNA was extracted, quantified, and purity was assessed as mentioned above. The RNAseq was performed for the Cologne Center for Genomics (CCG) using the following protocol: libraries were prepared using the Illumina^{®} Stranded TruSeq^{®} RNA sample preparation Kit. Library preparation started with 500ng total RNA. After poly-A selection using poly-T oligo-attached magnetic beads, mRNA was purified and fragmented using divalent cations under elevated temperature. The RNA fragments underwent reverse transcription using random primers. This was followed by second-strand cDNA synthesis with DNA Polymerase I and RNase H. After end repair and A-tailing, indexing adapters were ligated. The products were then purified and amplified (15 PCR cycles) to create the final cDNA libraries. After library validation and quantification (Agilent Tape Station), equimolar amounts of library were pooled. The pool was quantified by using the Peqlab KAPA Library Quantification Kit and the Applied Biosystems 7900HT Sequence Detection System. The pool was sequenced on an Illumina NovaSeq6000 sequencing instrument with a PE100 protocol. For the RNAseq analysis, Ensembl Homo sapiens release 105/ hg38 was used for genomes and annotations. rRNA transcripts were removed and cDNA fasta was generated using 'gffread' (cufflinks/2.2.1). cDNA index was build using kallisto (kallisto/0.46.1) (Bray, et al. Near-optimal probabilistic RNA-seq quantification. Nat Biotechnol 34, 525-527 (2016). 4 million reads were pseudoaligned to reference transcriptome using kallisto/0.46.1 and RSeQC/4.0.0 used to indentify mapping strand. A strand was identified by having more than 60% of reads mapped to it. Cases with less than 60% of reads in each strand were defined as unstranded. Reads were pseudoaligned to reference transcriptome and quantified using kallisto/0.46.1. After the removal of batch effects, differential expression was performed between "Control" and "Treatment" samples using limma/3.54.0 (Love, MI, et al., Moderated estimation of fold change and dispersion for RNA-seq data with DESeq2. Genome Biol 15, 550 (2014)).
Gln+ samples were classified as "Control" whereas gln- samples and aKG+ samples were classified as "Treatment". Pathway enrichment analysis was performed using the DAVID API. Data visualization performed with Flaski.

### RUSH assay

Mannll-SBP-mCherry stable cell lines were generated via lentivirus transduction and sequential puromycin selection. A second-generation lentivirus packaging system was used. The plasmids for the lentivirus production were obtained from addgene, psPAX2 was a gift from Didier Trono (Addgene plasmid # 12260; http://n2t.net/addgene:12260 ; RRID:Addgene_12260), pMD2.G was a gift from Didier Trono (Addgene plasmid # 12259; http://n2t.net/addgene:12259 ; RRID:Addgene_12259) and pCDH_Str-KDEL_Manll-SBP-mCherry was a gift from Franck Perez (Addgene plasmid # 65259 ; http://n2t.net/addgene:65259 ; RRID:Addgene_65259). For Lentivirus production, 10 million HEK 293T cells were seeded on a 15 cm plate, after 24 hours the cells were transfected with pCDH_Str-KDEL_Manll-SBP-mCherry, psPAX2 and pMD2.G mix in a ratio 4:2:1 using CaPO4 precipitation. The virus-containing medium was collected, centrifuged, and filtered with 0.2 µM filters. Two million HeLa cells were infected in a 10 cm plate on two consecutive days. Polybrene (#TR-1003-G, Sigma) at a final concentration of 10 µg/mL was added to the medium to increase the infection efficiency. After 24 hours from the last infection, the cells were selected with puromycin (#P9620-10ML, Sigma) at the final concentration of 1 µg/mL. For life imaging, HeLas stably expressing pCDH_Str-KDEL_Manll-SBP-mCherry were plated on 6-well CELLview glass-bottom cell culture dishes (Greiner Bio-One) and treated as indicated in the text and imaged at the start and following biotin addition using an Olympus IXplore SpinSR 50 mm spinning disk confocal microscope. Live cell imaging was performed in DMEM and 10% H₂0₂ for cell culture + 1%P/S with incubation at 37°C and 5% CO₂. All images were taken with a 100X/1.35 silicon oil objective and excitation with the 561 laser lines, using ORCA-Flash4.0 cameras (Hamatsu), and cellSens Software.

### OCR measurement

Oxygen consumption rate (OCR) was measured using Seahorse XF Analyzer. A total of 32,000 cells per well were plated in XF 96 cell culture microplates in DMEM medium supplemented with 10% FBS. The next day, DMEM was replaced by Seahorse XF medium supplemented with pyruvate (1 mM), glutamine (1 mM), and either glucose or galactose (10 mM). The OCR was measured according to the manufacturer's protocol (XF Cell Mito Stress Test Kit) at basal level and after injections with oligomycin (2 µM), FCCP (0.5 µM), and a combination of antimycin A (0.5 µM) and rotenone (0.5 µM). To normalize OCR data, protein concentration per well was determined using a bicinchoninic acid assay. The data were analyzed by Seahorse Wave Desktop software.

*Statistical analyses* All statistical analyses were performed using one-way ANOVA, two-way ANOVA, or an unpaired t-test in GraphPad Prism 9 software and are indicated accordingly.

### Example 1: Requirement of glutamine for de novo cholesterol synthesis

To understand the metabolic inputs that regulate the mevalonate pathway, cholesterol was measured by mass spectrometry in cells cultured in media lacking either glucose or glutamine, the main nutrients that cells use to generate citrate. Following its synthesis in the mitochondria, citrate is transported to the cytosol for use in cholesterol synthesis (Fig. 1A). It was found that cells cultured with glucose but not glutamine had 20% higher citrate levels than cells cultured with only glutamine for 8 hours (Fig.1 B-D). Opposite to citrate however, cholesterol trended ~20% lower in cells cultured with only glucose (Fig. 1E). This result was unexpected given that cholesterol synthesis depends on citrate, and raised the question of how glutamine-deprivation reduced cholesterol levels.

In many cancer cells, citrate derived from glucose exits the TCA cycle for use in synthetic reactions while glutamine fuels anaplerotic reactions that replenish intermediates of the TCA cycle (Fig. 1F; left). It was thus considered whether in the absence of glutamine, glucose-derived citrate was oxidized to sustain the TCA cycle, thereby restricting its export from mitochondria and availability for cholesterol synthesis (Fig. 1F; right). To address this possibility, it was examined the incorporation of ¹³C from uniformly labelled ¹³C-glucose into citrate and cholesterol in cells cultured with or without glutamine. In glutamine-fed (gln+) cells, the majority of citrate had 2 labelled carbons (m+2), the expected pattern following its generation in and export from mitochondria (Fig. 1F-G). Glutamine-starved (gln) cells however had a three-fold decrease in m+2 citrate while m+4 and m+6 citrate were increased by ~5- and ~1 00-fold, respectively (Fig. 1G). A similar trend was observed for the TCA cycle intermediates malate and succinate (Fig. 6A). To test whether decreased anaplerosis drove the observed shift in citrate isotopologues, we supplemented gln- cells with alpha-ketoglutarate (αKG), the main anaplerotic derivative of glutamine. The addition of αKG was sufficient to restore m+2 and m+6 citrate pools to those of gln+ cells, and blunted the increase in m+4 malate and succinate caused by glutamine starvation (Fig. 1G; Fig. 6A). It did not, however, rescue the >5-fold decrease in ¹³C-incorporation into cholesterol in cells cultured without glutamine (Fig. 1H). Thus, glutamine is required for cholesterol synthesis independently of its role in anaplerosis.

### Example 2: Glutamine starvation inhibits synthesis of fatty acid-derived lipids.

U2OS cells cultured for 24h without glutamine produced less diacylgerol (precursor to trigylcerides), phospholipids phosphatidylcholine, phosphoethanolamine, phosphatidylglycerol, phosphatidylinositol, sphingomyelin and coenzyme Q10 compared to cells cultured in presence of glutamine (Figure 11-1Q). Therefore, glutamine synthesis also regulates synthesis of fatty acids and of fatty acids -derived lipids.

### Example 3: Glutamine starvation inhibits the mevalonate pathway

Examining key enzymes of the mevalonate pathway might provide clues to the underlying mechanism in order to understand how glutamine starvation affects cholesterol levels and synthesis. To this end, an experiment was conducted to monitor levels of HMGCR, which catalyzes the conversion of HMG-CoA to mevalonate at the cytosolic face of the ER. HMGCR was decreased as early as 2 hours after glutamine withdrawal and was undetectable by 16 and 24 hours in U2OS cells (Fig. 2A). Similar results were obtained for HeLa cells and primary human foreskin fibroblasts (HFFs) cultured without glutamine for 24h (Fig. 2B-C). Thus, glutamine is required to maintain HMGCR in various human cell lines. Because HMGCR is the rate-limiting enzyme in cholesterol synthesis and its loss paralleled the decrease in cholesterol synthesis, HMGCR was used as a marker for mevalonate pathway activity (Fig. 1H) Glutamine is classified as a non-essential amino acid because it can be synthesized from ammonia and glutamate by glutamine synthetase (GLUL) (Fig. 2D). However, endogenously synthesized glutamine did not maintain HMGCR levels in U2OS cells-at least minimally-during glutamine deprivation (Fig. 2A). To address this matter, GLUL RNAseq and proteomic quantitative profiling of cancer cell lines were examined on the basis of the data available through the Cancer Genome Atlas (https://depmap.org/portal/ccle/). Of the 347 cell lines analyzed, U2OS cells were of the 10 with lowest GLUL expression, and of the 10% with lowest *GLUL* transcripts (Fig. 2E; Tables 4 and 5). This suggested that U2OS cells did not maintain HMGCR levels during glutamine starvation because they were deficient for GLUL activity. This hypothesis was tested measuring their incorporation of ¹⁵N derived from ¹⁵N-ammonia (¹⁵NH₃) into glutamine. The U2OS were compared to liver cancer cells (Hep G2s) that were among the top 10% of GLUL-expressing cell lines (Fig. 2E). After 24 hours of glutamine starvation, ¹⁵N-labelled glutamine was less than 2% of total glutamine in U2OS cells (Fig. 2F). By contrast, in Hep G2 cells the majority of glutamine was ¹⁵N-labelled, and the total glutamine pool was increased by >two-fold (Fig 2F-G). To further investigate the relationship between glutamine synthesis and the mevalonate pathway, was tested the effect of ammonia supplementation on HMGCR. In Hep G2 cells cultured without glutamine, HMGCR resulted maintained at low levels and increased following ammonia treatment, unlike in U2OS cells (Fig. 2H-I) . The role of GLUL in the regulation of HMGCR was confirmed using L-methionine sulfoximine (**MSX**), a selective and irreversible inhibitor of GLUL. MSX treatment of Hep G2 cells further decreased HMGCR during glutamine starvation and prevented the ammonium chloride-induced increase in HMGCR (Fig. 2J). In support of the inhibition of GLUL by MSX, MSX treatment led to a shift in the migration pattern of GLUL (Fig. 2J). Thus, glutamine synthesis contributes to the regulation of the mevalonate pathway in a cell-type-specific manner.

### Example 4 Glutamine-sensing is required for HMGCR expression.

Because HMGCR is regulated by the ubiquitin/proteasome system (UPS), the UPS activity was investigated during glutamine starvation. However, treatment of glutamine-fed cells with the proteasomal inhibitor MG-132 did not prevent the depletion of HMGCR following glutamine withdrawal (Fig. 3A). Furthermore, we noted that levels of the ER chaperones calnexin and calreticulin were unaffected, which argued against an autophagy-mediated loss of HMGCR (Fig. 2A-C; Fig. 3A). Next it was investigated whether cellular stress responses that affect protein synthesis contributed to the loss of HMGCR in our system. During glutamine starvation, mTORC1 is inactive and the integrated stress response (ISR) is induced, which both lead to a reduction in global protein synthesis. This question was addressed using αKG which maintains mTORC1 activity during glutamine starvation, and the ISR-inhibitor ISRIB. Although αKG restored mTORC1 phosphorylation of its target ribosomal protein S6 kinase (S6K1) and ISRIB blocked the activation of the key ISR effector ATF4, neither treatment prevented the loss of HMGCR during glutamine withdrawal (Fig. 3B-C). Thus, glutamine starvation inhibits the mevalonate pathway independently of mTORC1 and the ISR.

Carbon and nitrogen atoms derived from glutamine fuel several biosynthetic reactions (Fig. 3D) . Therefore it was investigated whether glutamine regulated the mevalonate pathway through an intermediate metabolite, as in the case of αKG signaling to mTORC1 (Fig. 3D). This possibility was addressed analyzing the metabolites that were depleted following glutamine withdrawal, with or without αKG to exclude metabolic consequences of mTORC1 inhibition. Metabolites that rely on glutamine for their synthesis were decreased after 24 hours (Fig. 3E-G), including TCA-cycle intermediates such as citrate, fumarate, malate and succinate, and uridine diphosphate N-acetylglucosamine (UDP-GIcNAc) (Fig. 3E), non-essential amino acids (NEAAs) (Fig. 3F), and nucleotides (Fig. 3G) . Neither the addition of αKG that is the main anaplerotic derivative of glutamine and partly rescued levels of TCA-intermediates, nor sodium pyruvate which fuels the TCA cycle, rescued HMGCR levels during glutamine starvation (Fig. 3B,E; Fig. 7A). Furthermore, we found that U2OS cells deficient for either citrate synthase (CS) or the mitochondrial citrate transporter (SCL25A1), and which had ~90% lower citrate levels than those of wild-type (WT) cells, maintained HMGCR at levels higher than WT U2OS cells and similarly responded to glutamine deprivation (Fig. 6B-E). We therefore excluded a regulatory role for TCA cycle metabolites. To test whether NEAAs, nucleotides, or UDP-GIcNAc sustained the activation of the mevalonate pathway in the absence of glutamine, it was monitored the effect of their addition or that of their precursors on HMGCR. Contrary to our expectation, none rescued HMGCR levels during glutamine starvation, even in the presence of mTORC1-activating αKG (Fig. 3H). In line with these findings, the inhibition of glutaminolysis by BPTES (bis-2-(5-phenylacetamido-1,3,4-thiadiazol-2-yl)ethylsulfide) did not reduce HMGCR levels in glutamine-replete conditions when mTORC1 activity was rescued by αKG addition (Fig. 7B). Furthermore, neither DON (6-diazo-5-oxo-L-norleucine), which is a reactive analog of glutamine nor D-glutamine, a stereoisomer of glutamine, rescued levels of HMGCR during glutamine starvation (Fig. 7C-D). Thus, these results indicate that glutamine per se is sensed by the mevalonate pathway, and that glutamine positively regulates the mevalonate pathway.

### Example 5: Glutamine is required for SREBP2-SCAP ER-to-Golgi trafficking

As glutamine starvation drove the loss of HMGCR in a UPS-independent manner, and HMGCR expression was restored after 6h+ hours of glutamine refeeding argued against its post-translational regulation of the mevalonate pathway by glutamine (**Fig. 3A****;** **Fig. 8**). The transcriptome of cells cultured with (gln+) were compared to cells cultured without glutamine (gln-) for 8h. Cells treated with αKG but not glutamine (gln- αKG+) were also analyzed to control for transcriptional changes that resulted from mTORC1 inhibition (**Table 2**). To identify glutamine-regulated genes, two criteria using a false discovery rate (FDR) of <0.05 were applied: 1) difference between gln+ vs. gln- treatments (gln- and gln-αKG+ were grouped together) and; 2) no difference between gln- vs. gln-αKG+ (**Fig. 4A**). Glutamine-regulated genes were identified using a threshold log2FC of +/-1.5 between gln+ vs. gln- treatments, wherein "gln- treatment" comprises both gln- and gln-aKG+ treatment. Our gene ontology (GO) term analysis of the top 5% of these genes revealed in an unbiased fashion an enrichment for the repression of genes in sterol biosynthesis-related pathways (**Fig**. **4B**). Among the top 25 glutamine-regulated mRNAs were those encoding for key enzymes in the cholesterol biosynthesis pathway including *HMGCR, SQLE, and FDFT1*-which were found induced in gln-starved cells refed glutamine (Fig. 4C-D)-and the expected ISR-dependent genes such as *DDIT3, CHOP, SESN2, and PPP1R15A* (Fig. 3C, Fig. 4D). In line with the RNAseq data, levels of HMCGR and FDFT1 protein were decreased in cells starved of glutamine for 8 hours, despite αKG supplementation (Fig. 4E). To address the effect of glutamine on other cellular drivers of anabolism, the focus was put on fatty acid synthase (*FASN*) and the *MYC* oncogene that stimulates glutamine metabolism that were among the top 25 glutamine-regulated mRNAs (Fig. 4C). Like *HMGCR,* glutamine starvation decreased levels of both *FASN* transcript and protein (Fig. 4C, 4E). Contrary to our expectation given its role in promoting cell growth, *MYC* transcripts were increased during glutamine starvation; MYC protein however was decreased (Fig. 4C, Fig. 4E). Thus, glutamine deprivation uniquely modifies the transcriptome and transcriptionally represses cholesterol synthesis.

The global decrease in mRNAs of cholesterol synthesis enzymes hinted at impaired activation of the ER-membrane bound transcription factor SREBP2 that is required for their induction. Before reaching the nucleus, SREBP2 must traffic from the ER to the Golgi, where it is proteolytically cleaved by S1P and S2P to yield active fragments. To test whether glutamine starvation inhibited SREBP2 proteolysis, its precursor and mature forms were analyzed in cells starved of glutamine for 8h. Glutamine starvation inhibited the processing of SREBP2 with or without αKG (Fig. 4E). To exclude the possibility that glutamine starvation impaired the proteolytic activity of S1P and S2P, we examined ATF6 that is activated in an S1P- and S2P-dependent manner during the unfolded protein response (UPR). Treatment with the UPR-inducer tunicamycin similarly drove ATF6 cleavage in glutamine-fed or starved cells (Fig. 9A). Thus, glutamine is required for the proteolytic activation of the master regulator of the mevalonate pathway SREBP2.

Does glutamine starvation inhibit SREBP2 activity by impairing general ER-to-Golgi transport? This question was answered monitoring the trafficking of the Golgi enzyme mannosidase II-mCherry (MANII) with the retention using selective hooks system (RUSH). In RUSH, MANII is fused to a streptavidin-binding peptide and can thus be retained in the ER through a luminal 'hook' ER protein that is fused to streptavidin. Biotin releases MANII from its 'hook' in the ER, thereby enabling its trafficking to the Golgi. Following biotin addition, cells cultured in glutamine-replete or glutamine-depleted conditions similarly accumulated Golgi-Manll, arguing against a general defect in ER-to-Golgi trafficking (Fig. 9B). To address whether glutamine more specifically affected SREBP2 transport, we turned to SCAP, a sterol sensor that binds COPII-coated vesicles in low sterol conditions to promote the translocation of SREBP2 from the ER to Golgi. Whether glutamine starvation impaired SCAP trafficking was tested monitoring its cytoplasmic distribution in chinese hamster ovary (CHO) cells that stably express GFP-SCAP, and that were treated with MSX to inhibit GLUL-mediated induction of HMGCR (Fig. 9C) . GFP-SCAP was readily visualized in the Golgi in >75% of the glutamine-fed cells examined, unlike those that were deprived of glutamine ± αKG which retained GFP-SCAP in the ER (Fig. 4F-G). If the defect in SREBP2 activity was caused by its impaired ER-to-Golgi trafficking, SREBP2 cleavage would have been seen upon enforced Golgi-to-ER retrotranslocation, such as by brefeldin A (BFA) that relocates Golgi proteins including S1P and S2P to the ER. To this end, cells cultured with or without glutamine for 24h were treated with BFA. BFA resulted to rescue SREBP2 cleavage and HMGCR in glutamine-starved cells to levels similar to those in glutamine-fed cells (Fig. 4H). Thus, glutamine activates the ER-to-Golgi trafficking of SCAP and SREBP2.

### Example 6: Mitochondrial dysfunction dvsrequlates glutamine uptake and alters cholesterol level

Does the mevalonate pathway sense physiological levels of glutamine? To address this question, HMGCR expression was examined in glutamine-starved cells refed glutamine at a range of concentrations including 0.5 mM at which it is maintained in the bloodstream, and 2 mM, which is the concentration that is optimal for cultured cells. A concentration of 0.125 mM glutamine was sufficient to induce HMGCR in U2OS cells that lack GLUL activity (Fig. 5A). However, half-maximal effects of glutamine on HMGCR induction were apparent between 0.25 and 0.5 mM (Fig. 5A). This result confirmed that the mevalonate pathway was sensitive to fluctuations in glutamine levels. This suggested that the rewiring of glutamine metabolism that occurs during certain pathophysiological conditions dysregulated cholesterol metabolism. This possibility was tested by using chronic disturbances of mitochondrial oxidative phosphorylation (OXPHOS) that drive increased glutamine uptake and usage to sustain the TCA cycle. This analysis focused on outer mitochondrial membrane (OMM) protein mitofusin 2 (MFN2) because its ablation impairs OXPHOS and mutations in *MFN2* in humans are associated with lipodystrophies and an axonal neuropathy known as Charcot-Marie-Tooth Disease type 2A. The results confirmed that U2OS cells deficient for MFN2 had decreased basal respiration, maximal respiration, ATP production and spare respiratory capacity, and consumed >2-fold more glutamine than WT cells (Fig. 5 B-H). Consistent with a mitochondrial defect, MFN2 KO cells produced more citrate m+5 through reductive carboxylation of ¹³C₅-glutamine-derived α-ketoglutarate than WT cells (Fig. 5I-K). Mevalonate pathway activation was compared between WT and MFN2 KO cells. Both the precursor and mature forms of SREBP2, and HMGCR were increased in MFN2 KO cells, consistent with the increase in total cholesterol (Fig. 5L-N). Of note, the addition of lipids (10% fetal bovine serum) repressed SREBP2 cleavage in both cell lines, indicating that sterol-mediated negative feedback is intact in MFN2 KO cells (Fig. 5L). Thus, the loss of MFN2 impairs OXPHOS, promotes glutamine uptake, and dysregulates cholesterol metabolism.

If glutamine uptake drove the differences in mevalonate pathway activity between WT and MFN2 KO cells, these differences would be attenuated early after glutamine refeeding and thus when intracellular glutamine was still low. After glutamine starvation and 4 hours post refeeding, HMGCR was similar between WT and MFN2 KO cells (Fig. 5O). By 8 hours post glutamine refeeding however, MFN2-KO cells had higher HMGCR than WT cells, a trend that continued at 16 and 24 hours (Fig. 5O). Consistent with our results in human cells, WT murine embryonic fibroblasts (MEFs) required glutamine for HMGCR expression and *Mfn2*^{*-*/*-*} MEFs had increased glutamine uptake and HMGCR relative to WT MEFs (Fig. 10A-C). Opposite to *Mfn2*^{*-*/*-*} MEFs however, *Mfn1*^{*-*/*-*} MEFs which are also deficient for mitochondrial fusion had decreased HMGCR levels relative to WT MEFs, despite that they consumed more glutamine (Fig. 10B-C). These results suggest that cholesterol homeostasis is dysregulated in a glutamine-dependent manner in a model of mitochondrial pathophysiology, but that mitochondria may communicate with the cholesterol pathways through diverse mechanisms.

**Table 1: Glutamine regulated marker: mevalonate pathway intermediates, products, derived metabolites; fatty acid synthesis-derived lipids**

| Name | | Level induced by glutamine synthetase activator | Level induced by glutamine synthetase inhibitor |
|---|---|---|---|
| Citrate | | increase | decrease |
| | | | |

| **Intermediates/ Products** | | | |
|---|---|---|---|
| Acetoacetyl CoA | | increase | decrease |
| HMG-CoA | | increase | decrease |
| mevalonate | | increase | decrease |
| mevalonate-5-P | | increase | decrease |
| mevalonate-5-PP | | increase | decrease |
| Isopentyl-5-PP (IPP) | | increase | decrease |
| Dimetylallyl-PP | | increase | decrease |
| farnesyl-PP (FPP) | | increase | decrease |
| squalene | | increase | decrease |
| (S)-2,3-oxidosqualene | | increase | decrease |
| cholesterol | | increase | decrease |
| geranyl-PP (GPP) | | increase | decrease |
| geranylgeranyl-PP (GGPP) | | increase | decrease |
| dolicyl-pp | | increase | decrease |
| dolichols | | increase | decrease |
| lanosterol | | increase | decrease |
| Cholestratriene | | increase | decrease |
| 4-methylzymosterone | | increase | decrease |
| 4-a-methylzymosterol | | increase | decrease |
| Zymosterone | | increase | decrease |
| Zymosterol | | increase | decrease |
| 7-Dehydrodesmosterol | | increase | decrease |
| dihydrolanosterol (DHL) | | increase | decrease |
| desmosterol | | increase | decrease |
| epoxycholesterol (EC) | | increase | decrease |
| dehydrocholesterols | | increase | decrease |
| Lathosterol | | increase | decrease |

| **MEV- derived metabolites** | | | |
|---|---|---|---|
| Sterols | | increase | decrease |
| steroid hormones | | increase | decrease |
| vitamin D | | increase | decrease |
| bile acid | | increase | decrease |
| oxysterols | | increase | decrease |
| Heme A | | increase | decrease |
| coenzyme Q10 | | increase | decrease |
| Vitamin K2 | | increase | decrease |
| Isopentenyl tRNA | | increase | decrease |
| prenylated proteins: Lamins, Ras, Rheb, RhoA, Rabs, Rac, Rab2b, 2c | | increase | decrease |

| **Fatty acid-derived metabolites** | | | |
|---|---|---|---|
| Phospholipids | | increase | decrease |
| | Phosphatidylcholines | increase | decrease |
| Phosphatidylethanolamines | | increase | decrease |
| | Phosphatidylglycerols | increase | decrease |
| | Phosphatidylinositols | increase | decrease |
| Glycerolipids | | increase | decrease |
| | Diacylglycerols | increase | decrease |
| | Triglycerides | increase | decrease |
| Fatty acids | | increase | decrease |
| | Palmitic Acid (C16 :0) | increase | decrease |
| Sphingolipid | | increase | decrease |
| | Sphingomyelin | increase | decrease |

| **Table 1a: Saturated Fatty Acids** | | | |
|---|---|---|---|
| **Common Name** | **Systematic Name** | **Structural Formula** | **Lipid Numbers** |
| Palmitic acid | Hexadecanoic acid | CH₃(CH₂)₁₄COOH | C16:0 |
| Stearic acid | Octadecanoic acid | CH₃(CH₂)₁₆COOH | C18:0 |
| Arachidic acid | Eicosanoic acid | CH₃(CH₂)₁₈COOH | C20:0 |
| Behenic acid | Docosanoic acid | CH₃(CH₂)₂₀COOH | C22:0 |
| Lignoceric acid | Tetracosanoic acid | CH₃(CH₂)₂₂COOH | C24:0 |

| **Table 1b: Unsaturated fatty acids** | | | | | |
|---|---|---|---|---|---|
| **ω-n** | **Common Name** | **Lipid Numbers** | **Δⁿ** | **Structural Formula** | **Trans or Cis** |
| ω-3 | α-Linolenic acid | C18:3 | Δ^{9,12,15} | CH₃CH₂CH=CHCH₂CH=CHCH₂CH=CH(CH₂)₇COOH | cis |
| ω-3 | Stearidonic acid | C18:4 | Δ^{6,9,12,15} | CH₃CH₂CH=CHCH₂CH=CHCH₂CH=CHCH₂CH=CH(CH₂)₄COOH | cis |
| ω-3 | Eicosapentaenoic acid | C20:5 | Δ^{5,8,11,14,17} | CH₃CH₂CH=CHCH₂CH=CHCH₂CH=CHCH₂CH=CHCH₂CH=CH(CH₂)₃COOH | cis |
| ω-3 | Cervonic acid | C22:6 | Δ^{4,7,10,13,16,19} | CH₃CH₂CH=CHCH₂CH=CHCH₂CH=CHCH₂CH=CHCH₂CH=CHCH₂CH=CH(CH₂)₂COOH | cis |
| ω-6 | Linoleic acid | C18:2 | Δ^{9,12} | CH₃(CH₂)₄CH=CHCH₂CH=CH(CH₂)₇COOH | cis |
| ω-6 | Linolelaidic acid | C18:2 | | CH₃(CH₂)₄CH=CHCH₂CH=CH(CH₂)₇COOH | trans |
| ω-6 | γ-Linolenic acid | C18:3 | Δ^{6,9,12} | CH₃(CH₂)₄CH=CHCH₂CH=CHCH₂CH=CH(CH₂)₄COOH | cis |
| ω-6 | Dihomo-γ-linolenic acid | C20:3 | Δ^{8,11,14} | CH₃(CH₂)₄CH=CHCH₂CH=CHCH₂CH=CH(CH₂)₆COOH | cis |
| ω-6 | Arachidonic acid | C20:4 | Δ^{5,8,11,14} | CH₃(CH₂)₄CH=CHCH₂CH=CHCH₂CH=CHCH₂CH=CH(CH₂)₃COOH | cis |
| ω-6 | Docosatetraenoic acid | C22:4 | Δ^{7,10,13,16} | CH₃(CH₂)₄CH=CHCH₂CH=CHCH₂CH=CHCH₂CH=CH(CH₂)₅COOH | cis |
| ω-7 | Palmitoleic acid | C16:1 | Δ⁹ | CH₃(CH₂)₅CH=CH(CH₂)₇COOH | cis |
| ω-7 | Vaccenic acid | C18:1 | Δ¹¹ | CH₃(CH₂)₅CH=CH(CH₂)₉COOH | trans |
| ω-7 | Paullinic acid | C20:1 | Δ¹³ | CH₃(CH₂)₅CH=CH(CH₂)₁₁COOH | cis |
| ω-9 | Oleic acid | C18:1 | Δ⁹ | CH₃(CH₂)₇CH=CH(CH₂)₇COOH | cis |
| ω-9 | Elaidic acid | C18:1 | Δ⁹ | CH₃(CH₂)₇CH=CH(CH₂)₇COOH | trans |
| ω-9 | Gondoic acid | C20:1 | Δ¹¹ | CH₃(CH₂)₇CH=CH(CH₂)₉COOH | cis |
| ω-9 | Erucic acid | C22:1 | Δ¹³ | CH₃(CH₂)₇CH=CH(CH₂)₁₁ COOH | cis |
| ω-9 | Nervonic acid | C24:1 | Δ¹⁵ | CH₃(CH₂)₇CH=CH(CH₂)₁₃COOH | cis |
| ω-9 | Mead acid | C20:3 | Δ^{5,8,11} | CH₃(CH₂)₇CH=CHCH₂CH=CHCH₂CH=CH(CH₂)₃COOH | cis |

**Table 2: Glutamine inhibited mRNA: Loq2FC >+1.5 in glutamine starved cells**

| **ensembl_gene_id** | **gene_name** | **logFC** | **ensembl_gene_id** | **gene_name** | **logFC** |
|---|---|---|---|---|---|
| ENSG00000101255 | TRIB3 | 3.321 | ENSG00000175592 | FOSL1 | 2.184 |
| ENSG00000183696 | UPP1 | 3.088 | ENSG00000128591 | FLNC | 2.183 |
| ENSG00000134548 | SPX | 3.075 | ENSG00000120217 | CD274 | 2.183 |
| ENSG00000145777 | TSLP | 2.986 | ENSG00000115641 | FHL2 | 2.181 |
| ENSG00000185022 | MAFF | 2.913 | ENSG00000065809 | FAM 107B | 2.167 |
| ENSG00000163347 | CLDN1 | 2.883 | ENSG00000106366 | SERPINE1 | 2.159 |
| ENSG00000136997 | MYC | 2.794 | ENSG00000275302 | CCL4 | 2.156 |
| ENSG00000162772 | ATF3 | 2.754 | ENSG00000143067 | ZNF697 | 2.150 |
| ENSG00000155961 | RAB39B | 2.724 | ENSG00000116016 | EPAS1 | 2.145 |
| ENSG00000146592 | CREB5 | 2.717 | ENSG00000132326 | PER2 | 2.133 |
| ENSG00000087074 | PPP1R15A | 2.708 | ENSG00000148841 | ITPRIP | 2.130 |
| ENSG00000116285 | ERRFI1 | 2.699 | ENSG00000065911 | MTHFD2 | 2.129 |
| ENSG00000179148 | ALOXE3 | 2.694 | ENSG00000263006 | ROCK1P1 | 2.129 |
| ENSG00000116717 | GADD45A | 2.649 | ENSG00000059728 | MXD1 | 2.124 |
| ENSG00000128283 | CDC42EP1 | 2.625 | ENSG00000172216 | CEBPB | 2.118 |
| ENSG00000116761 | CTH | 2.600 | ENSG00000107731 | UNC5B | 2.113 |
| ENSG00000130766 | SESN2 | 2.595 | ENSG00000034152 | MAP2K3 | 2.107 |
| ENSG00000198576 | ARC | 2.579 | ENSG00000104856 | RELB | 2.081 |
| ENSG00000175197 | DDIT3 | 2.534 | ENSG00000116584 | ARHGEF2 | 2.057 |
| ENSG00000174343 | CHRNA9 | 2.505 | ENSG00000163661 | PTX3 | 2.042 |
| ENSG00000133639 | BTG1 | 2.483 | ENSG00000106948 | AKNA | 2.025 |
| ENSG00000006459 | KDM7A | 2.460 | ENSG00000121060 | TRIM25 | 2.007 |
| ENSG00000163393 | SLC22A15 | 2.452 | ENSG00000188487 | INSC | 1.993 |
| ENSG00000145911 | N4BP3 | 2.452 | ENSG00000139514 | SLC7A1 | 1.992 |
| ENSG00000120738 | EGR1 | 2.449 | ENSG00000050405 | LIMA1 | 1.983 |
| ENSG00000168209 | DDIT4 | 2.437 | ENSG00000128965 | CHAC1 | 1.983 |
| ENSG00000126368 | NR1D1 | 2.369 | ENSG00000138678 | G PAT3 | 1.978 |
| ENSG00000104413 | ESRP1 | 2.351 | ENSG00000111371 | SLC38A1 | 1.971 |
| ENSG00000139269 | INHBE | 2.341 | ENSG00000146648 | EGFR | 1.969 |
| ENSG00000285722 | | 2.331 | ENSG00000153721 | CNKSR3 | 1.965 |
| ENSG00000169429 | CXCL8 | 2.325 | ENSG00000243742 | RPLP0P2 | 1.962 |
| ENSG00000073756 | PTGS2 | 2.311 | ENSG00000086730 | LAT2 | 1.941 |
| ENSG00000159399 | HK2 | 2.311 | ENSG00000165626 | BEND7 | 1.930 |
| ENSG00000132510 | KDM6B | 2.303 | ENSG00000118503 | TNFAIP3 | 1.927 |
| ENSG00000109321 | AREG | 2.303 | ENSG00000244405 | ETVS | 1.924 |
| ENSG00000049249 | TNFRSF9 | 2.254 | ENSG00000065060 | UHRF1BP1 | 1.913 |
| ENSG00000120279 | MYCT1 | 2.251 | ENSG00000165030 | NFIL3 | 1.904 |
| ENSG00000164949 | GEM | 2.246 | ENSG00000085563 | ABCB1 | 1.900 |
| ENSG00000109220 | CHIC2 | 2.235 | ENSG00000176971 | FIBIN | 1.886 |
| ENSG00000196756 | SNHG17 | 2.204 | ENSG00000280081 | LINC01667 | 1.885 |
| ENSG00000070669 | ASNS | 2.200 | ENSG00000166123 | GPT2 | 1.882 |
| ENSG00000146090 | RASGEF1C | 1.878 | ENSG00000138166 | DUSP5 | 1.709 |
| ENSG00000011638 | LDAF1 | 1.872 | ENSG00000160712 | IL6R | 1.707 |
| ENSG00000115602 | IL1RL1 | 1.859 | ENSG00000171132 | PRKCE | 1.706 |
| ENSG00000130513 | GDF15 | 1.855 | ENSG00000143322 | ABL2 | 1.705 |
| ENSG00000134259 | NGF | 1.852 | ENSG00000289579 | | 1.701 |
| ENSG00000123358 | NR4A1 | 1.849 | ENSG00000106105 | GARS1 | 1.695 |
| ENSG00000141384 | TAF4B | 1.849 | ENSG00000157514 | TSC22D3 | 1.688 |
| ENSG00000255717 | SNHG1 | 1.847 | ENSG00000168297 | PXK | 1.685 |
| ENSG00000120053 | GOT1 | 1.843 | ENSG00000265972 | TXNIP | 1.685 |
| ENSG00000196517 | SLC6A9 | 1.838 | ENSG00000139146 | SINHCAF | 1.683 |
| ENSG00000196843 | ARID5A | 1.835 | ENSG00000138685 | FGF2 | 1.670 |
| ENSG00000183018 | SPNS2 | 1.830 | ENSG00000154319 | FAM 167A | 1.669 |
| ENSG00000153879 | CEBPG | 1.830 | ENSG00000131196 | NFATC1 | 1.663 |
| ENSG00000165046 | LETM2 | 1.828 | ENSG00000125089 | SH3TC1 | 1.661 |
| ENSG00000286098 | | 1.826 | ENSG00000117143 | UAP1 | 1.647 |
| ENSG00000128165 | ADM2 | 1.824 | ENSG00000120158 | RCL1 | 1.641 |
| ENSG00000224032 | EPB41L4A-AS1 | 1.817 | ENSG00000105281 | SLC1A5 | 1.639 |
| ENSG00000149968 | MMP3 | 1.811 | ENSG00000163216 | SPRR2D | 1.638 |
| ENSG00000023608 | SNAPC1 | 1.811 | ENSG00000115902 | SLC1A4 | 1.636 |
| ENSG00000158050 | DUSP2 | 1.804 | ENSG00000170385 | SLC30A1 | 1.634 |
| ENSG00000176046 | NUPR1 | 1.801 | ENSG00000114529 | C3orf52 | 1.633 |
| ENSG00000112715 | VEGFA | 1.798 | ENSG00000088826 | SMOX | 1.629 |
| ENSG00000079156 | OSBPL6 | 1.797 | ENSG00000099954 | CECR2 | 1.627 |
| ENSG00000140941 | MAP1LC3B | 1.797 | ENSG00000139112 | GABARAPL1 | 1.623 |
| ENSG00000131018 | SYNE1 | 1.790 | ENSG00000102003 | SYP | 1.623 |
| ENSG00000188522 | FAM83G | 1.787 | ENSG00000099625 | CBARP | 1.620 |
| ENSG00000250251 | PKD1P6 | 1.786 | ENSG00000134954 | ETS1 | 1.620 |
| ENSG00000183775 | KCTD16 | 1.785 | ENSG00000232956 | SNHG15 | 1.619 |
| ENSG00000240476 | LINC00973 | 1.781 | ENSG00000143256 | PFDN2 | 1.618 |
| ENSG00000120129 | DUSP1 | 1.774 | ENSG00000172548 | NIPAL4 | 1.617 |
| ENSG00000108001 | EBF3 | 1.751 | ENSG00000170500 | LONRF2 | 1.615 |
| ENSG00000135116 | HRK | 1.744 | ENSG00000164509 | IL31RA | 1.611 |
| ENSG00000179094 | PER1 | 1.742 | ENSG00000114784 | EIF1B | 1.609 |
| ENSG00000249003 | CLUHP4 | 1.741 | ENSG00000129596 | CDO1 | 1.607 |
| ENSG00000286134 | | 1.733 | ENSG00000095752 | IL11 | 1.605 |
| ENSG00000173166 | RAPH1 | 1.726 | ENSG00000166401 | SERPINB8 | 1.605 |
| ENSG00000288656 | | 1.726 | ENSG00000090020 | SLC9A1 | 1.600 |
| ENSG00000176170 | SPHK1 | 1.725 | ENSG00000144655 | CSRNP1 | 1.600 |
| ENSG00000244257 | PKD1P1 | 1.718 | ENSG00000110619 | CARS1 | 1.591 |
| ENSG00000135069 | PSAT1 | 1.717 | ENSG00000174951 | FUT1 | 1.588 |
| ENSG00000031698 | SARS1 | 1.714 | ENSG00000115963 | RND3 | 1.587 |
| ENSG00000105550 | FGF21 | 1.713 | ENSG00000120889 | TNFRSF10B | 1.585 |
| ENSG00000205927 | OLIG2 | 1.585 | ENSG00000173334 | TRIB1 | 1.544 |
| ENSG00000174749 | FAM241A | 1.585 | ENSG00000083799 | CYLD | 1.541 |
| ENSG00000163811 | WDR43 | 1.584 | ENSG00000134058 | CDK7 | 1.539 |
| ENSG00000162733 | DDR2 | 1.572 | ENSG00000075643 | MOCOS | 1.536 |
| ENSG00000206538 | VGLL3 | 1.570 | ENSG00000196182 | STK40 | 1.535 |
| ENSG00000222041 | CYTOR | 1.570 | ENSG00000087494 | PTHLH | 1.533 |
| ENSG00000113070 | HBEGF | 1.570 | ENSG00000126226 | PCID2 | 1.530 |
| ENSG00000184545 | DUSP8 | 1.568 | ENSG00000251003 | ZFPM2-AS1 | 1.527 |
| ENSG00000155090 | KLF10 | 1.567 | ENSG00000077585 | GPR137B | 1.526 |
| ENSG00000124788 | ATXN1 | 1.566 | ENSG00000234741 | GAS5 | 1.525 |
| ENSG00000164535 | DAGLB | 1.563 | ENSG00000164023 | SGMS2 | 1.522 |
| ENSG00000119986 | AVPI1 | 1.563 | ENSG00000289609 | | 1.521 |
| ENSG00000172432 | GTPBP2 | 1.559 | ENSG00000165732 | DDX21 | 1.521 |
| ENSG00000157693 | TMEM268 | 1.558 | ENSG00000124882 | EREG | 1.518 |
| ENSG00000111252 | SH2B3 | 1.557 | ENSG00000010810 | FYN | 1.518 |
| ENSG00000103264 | FBXO31 | 1.556 | ENSG00000177410 | ZFAS1 | 1.517 |
| ENSG00000183778 | B3GALT5 | 1.555 | ENSG00000133134 | BEX2 | 1.514 |
| ENSG00000134668 | SPOCD1 | 1.553 | ENSG00000118689 | FOXO3 | 1.511 |
| ENSG00000104980 | TIMM44 | 1.551 | ENSG00000134684 | YARS1 | 1.511 |
| ENSG00000181649 | PHLDA2 | 1.549 | ENSG00000160223 | ICOSLG | 1.510 |
| ENSG00000124145 | SDC4 | 1.548 | ENSG00000122642 | FKBP9 | 1.508 |
| ENSG00000181026 | AEN | 1.548 | ENSG00000236138 | DUX4L26 | 1.507 |
| ENSG00000122877 | EGR2 | 1.546 | ENSG00000168264 | IRF2BP2 | 1.504 |
| ENSG00000156030 | MIDEAS | 1.545 | ENSG00000146733 | PSPH | 1.502 |
| ENSG00000258964 | | 1.545 | | | |

**Table 3: glutamine stimulated mRNA: Loq2FC <-1.5 in Gin starved cells**

| **ensembl_gene_id** | **gene_name** | **logFC** | **ensembl_gene_id** | **gene_name** | **logFC** |
|---|---|---|---|---|---|
| ENSG00000204389 | HSPA1A | -3.738 | ENSG00000121005 | CRISPLD1 | -1.659 |
| ENSG00000168497 | CAVIN2 | -3.088 | ENSG00000137831 | UACA | -1.644 |
| ENSG00000204388 | HSPA1B | -2.997 | ENSG00000185483 | ROR1 | -1.638 |
| ENSG00000113161 | HMGCR | -2.589 | ENSG00000160285 | LSS | -1.636 |
| ENSG00000176049 | JAKMIP2 | -2.405 | ENSG00000162520 | SYNC | -1.634 |
| ENSG00000205978 | NYNRIN | -2.317 | ENSG00000153395 | LPCAT1 | -1.626 |
| ENSG00000169247 | SH3TC2 | -2.278 | ENSG00000067064 | IDI1 | -1.626 |
| ENSG00000186480 | INSIG1 | -2.256 | ENSG00000117461 | PIK3R3 | -1.625 |
| ENSG00000177494 | ZBED2 | -2.167 | ENSG00000181449 | SOX2 | -1.614 |
| ENSG00000134061 | CD180 | -2.151 | ENSG00000110921 | MVK | -1.613 |
| ENSG00000164761 | TNFRSF11B | -2.136 | ENSG00000100311 | PDGFB | -1.604 |
| ENSG00000189057 | FAM111B | -2.117 | ENSG00000260630 | SNAI3-AS1 | -1.600 |
| ENSG00000100344 | PNPLA3 | -2.027 | ENSG00000243701 | DUBR | -1.597 |
| ENSG00000137801 | THBS1 | -2.025 | ENSG00000259807 | | -1.595 |
| ENSG00000125378 | BMP4 | -2.024 | ENSG00000147408 | CSGALNACT1 | -1.592 |
| ENSG00000225783 | MIAT | -2.021 | ENSG00000283930 | PLD5P1 | -1.585 |
| ENSG00000109971 | HSPA8 | -2.013 | ENSG00000167552 | TUBA1A | -1.581 |
| ENSG00000154556 | SORBS2 | -2.003 | ENSG00000152580 | IGSF10 | -1.575 |
| ENSG00000166963 | MAP1A | -1.899 | ENSG00000205413 | SAMD9 | -1.572 |
| ENSG00000182580 | EPHB3 | -1.880 | ENSG00000124104 | SNX21 | -1.560 |
| ENSG00000132688 | NES | -1.859 | ENSG00000125657 | TNFSF9 | -1.553 |
| ENSG00000169710 | FASN | -1.838 | ENSG00000131069 | ACSS2 | -1.552 |
| ENSG00000251432 | LINC02615 | -1.823 | ENSG00000138496 | PARP9 | -1.552 |
| ENSG00000146411 | SLC2A12 | -1.822 | ENSG00000114993 | RTKN | -1.551 |
| ENSG00000092964 | DPYSL2 | -1.799 | ENSG00000114805 | PLCH1 | -1.547 |
| ENSG00000106211 | HSPB1 | -1.796 | ENSG00000134986 | NREP | -1.547 |
| ENSG00000102384 | CENPI | -1.794 | ENSG00000147130 | ZMYM3 | -1.536 |
| ENSG00000177409 | SAMD9L | -1.785 | ENSG00000186834 | HEXIM1 | -1.523 |
| ENSG00000109084 | TMEM97 | -1.770 | ENSG00000173727 | | -1.522 |
| ENSG00000215146 | | -1.759 | ENSG00000179222 | MAGED1 | -1.519 |
| ENSG00000079459 | FDFT1 | -1.754 | ENSG00000215105 | TTC3P1 | -1.516 |
| ENSG00000072571 | HMMR | -1.742 | ENSG00000184207 | PGP | -1.514 |
| ENSG00000233695 | GAS6-AS1 | -1.740 | ENSG00000173930 | SLCO4C1 | -1.502 |
| ENSG00000197043 | ANXA6 | -1.729 | ENSG00000247746 | USP51 | -1.497 |
| ENSG00000281406 | BLACAT1 | -1.728 | ENSG00000116133 | DHCR24 | -1.490 |
| ENSG00000072422 | RHOBTB1 | -1.716 | | | |
| ENSG00000104549 | SQLE | -1.715 | ENSG00000105855 | ITGB8 | -1.669 |
| ENSG00000185813 | PCYT2 | -1.709 | | | |
| ENSG00000138336 | TET1 | -1.698 | | | |
| ENSG00000071575 | TRIB2 | -1.691 | | | |
| ENSG00000052802 | MSMO1 | -1.676 | | | |
| ENSG00000147383 | NSDHL | -1.670 | | | |

**Table 4: GLUL RNA expression in mammal cells as reported in Cancer Genome Atlas**

| | Log2 | | | |
|---|---|---|---|---|
| Cell Line Name | (TPM+1) | Primary Disease | Lineage Central Nervous | Lineage Subtype |
| D283MED | 11.403 | Brain Cancer | System | Medulloblastoma |
| HMEL | 10.610 | Non-Cancerous | Breast | |
| NCIH2126 | 10.608 | Lung Cancer | Lung | NSCLC |
| CMK | 10.588 | Leukemia | Blood | AML |
| NCIH211 | 10.507 | Lung Cancer | Lung | SCLC |
| RERFLCKJ | 10.497 | Lung Cancer | Lung | NSCLC |
| HCC1428 | 10.404 | Breast Cancer | Breast | Breast Carcinoma |
| LAMA84 | 10.287 | Leukemia | Blood | CML |
| NCIH526 | 10.252 | Lung Cancer | Lung | SCLC |
| NCIH82 | 10.227 | Lung Cancer | Lung | SCLC |
| OCIAML5 | 10.202 | Leukemia | Blood | AML |
| MOLT16 | 10.092 | Leukemia | Blood | ALL |
| MEG01 | 10.087 | Leukemia | Blood | CML |
| EFM192A | 9.957 | Breast Cancer | Breast | Breast Adenocarcinoma |
| K562 | 9.947 | Leukemia | Blood Central Nervous | CML |
| D341 | 9.928 | Brain Cancer | System | Medulloblastoma |
| SET2 | 9.918 | Leukemia | Blood | AML |
| ALLSIL | 9.881 | Leukemia | Blood | ALL |
| HCC95 | 9.881 | Lung Cancer | Lung | NSCLC |
| HPAC | 9.875 | Pancreatic Cancer | Pancreas | Exocrine |
| HT1376 | 9.866 | Bladder Cancer | Urinary Tract | Bladder Carcinoma |
| U937 | 9.862 | Leukemia | Blood | AML |
| M07E | 9.860 | Leukemia | Blood | AML |
| FU97 | 9.859 | Gastric Cancer Head and Neck | Gastric Upper | Gastric Adenocarcinoma |
| UPCISCC074 | 9.828 | Cancer | Aerodigestive | Upper Aerodigestive Squamous |
| HEL | 9.823 | Leukemia | Blood | AML |
| KU812 | 9.803 | Leukemia | Blood | CML |
| NCIH854 | 9.756 | Lung Cancer | Lung | NSCLC |
| NCIH1048 | 9.725 | Lung Cancer | Lung | SCLC |
| PLB985 | 9.707 | Leukemia Head and Neck | Blood Upper | AML |
| H157 | 9.692 | Cancer | Aerodigestive | Upper Aerodigestive Squamous |
| CMK115 | 9.653 | Leukemia | Blood | AML |
| MDAMB175VII | 9.648 | Breast Cancer | Breast | Breast Ductal Carcinoma |
| TF1 | 9.627 | Leukemia | Blood | AML |
| DMS153 | 9.625 | Lung Cancer | Lung | SCLC |
| LOUCY | 9.616 | Leukemia | Blood | ALL |
| AML193 | 9.609 | Leukemia | Blood | AML |
| CORL88 | 9.584 | Lung Cancer | Lung | SCLC |
| JURLMK1 | 9.563 | Leukemia | Blood | CML |
| CTV1DM | 9.531 | Leukemia | Blood | ALL |
| KYO1 | 9.530 | Leukemia | Blood | CML |
| P12ICHIKAWA | 9.530 | Leukemia | Blood | ALL |
| | | Head and Neck | Upper | |
| BICR31 | 9.524 | Cancer | Aerodigestive | Upper Aerodigestive Squamous |
| CHAGOK1 | 9.504 | Lung Cancer | Lung | NSCLC |
| OAW28 | 9.460 | Ovarian Cancer | Ovary | Ovary Adenocarcinoma |
| BT483 | 9.449 | Breast Cancer | Breast | Breast Ductal Carcinoma |
| AU565 | 9.436 | Breast Cancer | Breast | Breast Adenocarcinoma |
| NCIH810 | 9.426 | Lung Cancer | Lung | NSCLC |
| G401 | 9.422 | Rhabdoid | Kidney | Malignant Rhabdoid Tumor |
| L33 | 9.395 | Pancreatic Cancer | Pancreas | Exocrine |
| | | Head and Neck | Upper | |
| CAL27 | 9.373 | Cancer | Aerodigestive | Upper Aerodigestive Squamous |
| PF382 | 9.341 | Leukemia | Blood | ALL |
| HCC1419 | 9.337 | Breast Cancer | Breast | Breast Ductal Carcinoma |
| | | Colon/Colorectal | | |
| C10 | 9.314 | Cancer | Colorectal | Colorectal Adenocarcinoma |
| JK1 | 9.310 | Leukemia | Blood | CML |
| SHMAC4 | 9.305 | Prostate Cancer | Prostate | Prostate Adenocarcinoma |
| HCC70 | 9.290 | Breast Cancer | Breast | Breast Ductal Carcinoma |
| BXPC3 | 9.288 | Pancreatic Cancer | Pancreas | Exocrine |
| MOLM6 | 9.278 | Leukemia | Blood | CML |
| DMS454 | 9.270 | Lung Cancer | Lung | SCLC |
| NCIH2342 | 9.263 | Lung Cancer | Lung | NSCLC |
| NCO2 | 9.197 | Leukemia | Blood | CML |
| MOLM16 | 9.195 | Leukemia | Blood | AML |
| | | Head and Neck | Upper | |
| BICR22 | 9.193 | Cancer | Aerodigestive | Upper Aerodigestive Squamous |
| KE37 | 9.185 | Leukemia | Blood | ALL |
| UACC893 | 9.184 | Breast Cancer | Breast | Breast Ductal Carcinoma |
| F36P | 9.182 | Leukemia | Blood | AML |
| COV644 | 9.153 | Ovarian Cancer | Ovary | Ovary Adenocarcinoma |
| NZOV9 | 9.151 | Ovarian Cancer | Ovary | Ovary Adenocarcinoma |
| KASUMI6 | 9.149 | Leukemia | Blood | AML |
| JURKAT | 9.135 | Leukemia | Blood | ALL |
| | | Head and Neck | Upper | |
| SNU899 | 9.134 | Cancer | Aerodigestive | Upper Aerodigestive Squamous |
| CORL95 | 9.108 | Lung Cancer | Lung | SCLC |
| HCC2935 | 9.098 | Lung Cancer | Lung | NSCLC |
| NCIH446 | 9.095 | Lung Cancer | Lung | SCLC |
| EFM19 | 9.076 | Breast Cancer | Breast | Breast Ductal Carcinoma |
| HMCB | 9.065 | Skin Cancer | Skin | Melanoma |
| MOLT3 | 9.064 | Leukemia | Blood Central Nervous | ALL |
| LN319 | 9.056 | Brain Cancer | System | Glioma |
| P31FUJ | 9.056 | Leukemia | Blood | AML |
| ECC12 | 9.034 | Gastric Cancer | Gastric | Gastric Small Cell |
| COLO668 | 9.030 | Lung Cancer | Lung | SCLC |
| NCIH1781 | 9.028 | Lung Cancer | Lung | NSCLC |
| DERL2 | 9.019 | Lymphoma | Lymphocyte | Lymphoma Unspecified |
| HSC5 | 9.017 | Skin Cancer | Skin | Skin Squamous |
| SKM1 | 8.995 | Leukemia | Blood | AML |
| | | Head and Neck | Upper | |
| SNU1041 | 8.991 | Cancer | Aerodigestive | Upper Aerodigestive Squamous |
| | | Endometrial/Uteri | | |
| COLO684 | 8.990 | ne Cancer | Uterus | Endometrial Adenocarcinoma |
| EOL1 | 8.979 | Leukemia | Blood | AML |
| KE39 | 8.978 | Gastric Cancer | Gastric | Gastric Adenocarcinoma |
| EM2 | 8.975 | Leukemia | Blood | CML |
| OCIM1 | 8.970 | Leukemia | Blood | AML |
| MHHCALL3 | 8.958 | Leukemia | Blood | ALL |
| T47D | 8.953 | Breast Cancer | Breast | Breast Ductal Carcinoma |
| HUPT4 | 8.952 | Pancreatic Cancer | Pancreas | Exocrine |
| CAL29 | 8.949 | Bladder Cancer | Urinary Tract | Bladder Carcinoma |
| SNU182 | 8.938 | Liver Cancer | Liver | Hepatocellular Carcinoma |
| NCIH2087 | 8.934 | Lung Cancer | Lung | NSCLC |
| OV7 | 8.928 | Ovarian Cancer | Ovary | Ovary Adenocarcinoma |
| KCL22 | 8.918 | Leukemia | Blood | CML |
| SNU878 | 8.916 | Liver Cancer | Liver | Hepatocellular Carcinoma |
| | | Head and Neck | Upper | |
| UPCISCC154 | 8.895 | Cancer | Aerodigestive | Upper Aerodigestive Squamous |
| MDAMB361 | 8.888 | Breast Cancer | Breast | Breast Carcinoma |
| CCC5 | 8.883 | Gastric Cancer | Gastric | |
| NUGC2 | 8.883 | Gastric Cancer | Gastric | Gastric Adenocarcinoma |
| NCIH660 | 8.880 | Prostate Cancer | Prostate | Prostate Small Cell |
| KASUMI2 | 8.878 | Leukemia | Blood | ALL |
| | | Endometrial/Uteri | | |
| T3M3 | 8.876 | ne Cancer | Uterus | Choriocarcinoma |
| PK8 | 8.875 | Pancreatic Cancer | Pancreas | Exocrine |
| SHMAC5 | 8.869 | Prostate Cancer | Prostate | Prostate Adenocarcinoma |
| SW780 | 8.866 | Bladder Cancer | Urinary Tract Central Nervous | Bladder Carcinoma |
| KNS42 | 8.859 | Brain Cancer | System | Glioma |
| RT112 | 8.833 | Bladder Cancer | Urinary Tract | Bladder Carcinoma |
| BFTC905 | 8.831 | Bladder Cancer | Urinary Tract | Bladder Carcinoma |
| NCIH1092 | 8.809 | Lung Cancer | Lung | SCLC |
| ICC15 | 8.803 | Bile Duct Cancer | Bile Duct | Cholangiocarcinoma |
| NCIH2171 | 8.792 | Lung Cancer | Lung | SCLC |
| | | Head and Neck | Upper | |
| HSC4 | 8.791 | Cancer | Aerodigestive | Upper Aerodigestive Squamous |
| NCIH69 | 8.784 | Lung Cancer | Lung | SCLC |
| KMBC2 | 8.781 | Bladder Cancer | Urinary Tract | Bladder Carcinoma |
| | | Head and Neck | Upper | |
| H103 | 8.773 | Cancer | Aerodigestive | Upper Aerodigestive Squamous |
| HEPG2 | 8.740 | Liver Cancer | Liver | Hepatocellular Carcinoma |
| HUH7 | 8.738 | Liver Cancer | Liver | Hepatocellular Carcinoma |
| CORL279 | 8.734 | Lung Cancer | Lung | SCLC |
| 697 | 8.718 | Leukemia | Blood | ALL |
| HSKTC | 8.717 | Ovarian Cancer | Ovary | Krukenberg Tumor |
| ZR7530 | 8.705 | Breast Cancer | Breast | Breast Ductal Carcinoma |
| CORL311 | 8.702 | Lung Cancer | Lung | SCLC |
| YAPC | 8.688 | Pancreatic Cancer | Pancreas | Exocrine |
| SUM190PT | 8.685 | Breast Cancer | Breast Peripheral | Breast Carcinoma |
| NB1 | 8.683 | Neuroblastoma | Nervous System | Neuroblastoma |
| NCIH2110 | 8.683 | Lung Cancer | Lung | NSCLC |
| HEL9217 | 8.678 | Leukemia | Blood | AML |
| NCIH727 | 8.677 | Lung Cancer | Lung | Lung Carcinoid |
| PEER | 8.669 | Leukemia | Blood | ALL |
| PL21 | 8.664 | Leukemia | Blood | AML |
| | | Head and Neck | Upper | |
| SNU1076 | 8.656 | Cancer | Aerodigestive | Upper Aerodigestive Squamous |
| KP2 | 8.654 | Pancreatic Cancer | Pancreas | Exocrine |
| MDAMB134VI | 8.640 | Breast Cancer | Breast | Breast Ductal Carcinoma |
| SF767 | 8.629 | Cervical Cancer | Cervix | Cervical Squamous |
| PANC0504 | 8.619 | Pancreatic Cancer | Pancreas | Exocrine |
| NCIH520 | 8.618 | Lung Cancer | Lung | NSCLC |
| JHH7 | 8.586 | Liver Cancer | Liver | Hepatocellular Carcinoma |
| DND41 | 8.578 | Leukemia | Blood | ALL |
| CAL51 | 8.574 | Breast Cancer | Breast | Breast Carcinoma |
| UMUC7 | 8.567 | Bladder Cancer | Urinary Tract | Bladder Carcinoma |
| HCS2 | 8.563 | Cervical Cancer | Cervix | Cervical Squamous |
| NCIH1876 | 8.559 | Lung Cancer | Lung Peripheral | SCLC |
| IMR32 | 8.555 | Neuroblastoma | Nervous System | Neuroblastoma |
| ZR751 | 8.550 | Breast Cancer | Breast | Breast Ductal Carcinoma |
| NCIH1694 | 8.549 | Lung Cancer | Lung | SCLC |
| UMUC9 | 8.546 | Bladder Cancer | Urinary Tract | Bladder Carcinoma |
| SKMEL1 | 8.545 | Skin Cancer | Skin | Melanoma |
| UPCISCC152 | 8.534 | Head and Neck | Upper | Upper Aerodigestive Squamous |
| | | Cancer | Aerodigestive | |
| PFEIFFER | 8.528 | Lymphoma | Lymphocyte | Non Hodgkin Lymphoma |
| DMS79 | 8.516 | Lung Cancer | Lung | SCLC |
| HCC202 | 8.515 | Breast Cancer | Breast | Breast Ductal Carcinoma |
| ABC1 | 8.512 | Lung Cancer | Lung | NSCLC |
| | | Endometrial/Uteri | | |
| HEC59 | 8.512 | ne Cancer | Uterus | Endometrial Adenocarcinoma |
| SUM229PE | 8.503 | Breast Cancer | Breast | Breast Carcinoma |
| | | Head and Neck | Upper | |
| SCC4 | 8.502 | Cancer | Aerodigestive | Upper Aerodigestive Squamous |
| HKGZCC | 8.498 | Bile Duct Cancer | Bile Duct Central Nervous | Cholangiocarcinoma |
| SF539 | 8.485 | Brain Cancer | System | Glioma |
| Y79 | 8.483 | Eye Cancer | Eye | Retinoblastoma |
| | | Colon/Colorectal | | |
| SNU503 | 8.483 | Cancer | Colorectal | Colorectal Adenocarcinoma |
| RPMI8402 | 8.482 | Leukemia | Blood | ALL |
| PACADD165 | 8.469 | Pancreatic Cancer | Pancreas | Exocrine |
| JHOS4 | 8.452 | Ovarian Cancer | Ovary | Ovary Adenocarcinoma |
| NCIH596 | 8.450 | Lung Cancer | Lung | NSCLC |
| NCIH1623 | 8.449 | Lung Cancer | Lung | NSCLC |
| THP1 | 8.438 | Leukemia | Blood | AML |
| MDAMB415 | 8.436 | Breast Cancer | Breast | Breast Carcinoma |
| CMLT1 | 8.435 | Leukemia | Blood | CML |
| RT4 | 8.430 | Bladder Cancer | Urinary Tract | Bladder Carcinoma |
| RERFGC1B | 8.426 | Gastric Cancer | Gastric | Gastric Adenocarcinoma |
| JHH5 | 8.425 | Liver Cancer | Liver | Hepatocellular Carcinoma |
| HCC33 | 8.417 | Lung Cancer | Lung | SCLC |
| | | Colon/Colorectal | | |
| SNUC2A | 8.415 | Cancer | Colorectal | Colorectal Adenocarcinoma |
| MONOMAC1 | 8.413 | Leukemia | Blood | AML |
| RCHACV | 8.405 | Leukemia | Blood | ALL |
| NCIH2228 | 8.401 | Lung Cancer | Lung | NSCLC |
| | | Head and Neck | Upper | |
| BICR56 | 8.391 | Cancer | Aerodigestive | Upper Aerodigestive Squamous |
| BEN | 8.387 | Lung Cancer | Lung | NSCLC |
| HCC1954 | 8.382 | Breast Cancer | Breast | Breast Ductal Carcinoma |
| | | Endometrial/Uteri | | |
| JHUEM2 | 8.378 | ne Cancer | Uterus | Endometrial Adenocarcinoma |
| OCIAML4 | 8.373 | Leukemia | Blood | AML |
| MDAMB468 | 8.371 | Breast Cancer | Breast | Breast Carcinoma |
| | | Endometrial/Uteri | | |
| RL952 | 8.354 | ne Cancer | Uterus | Endometrial Adenosquamous |
| TALL1 | 8.354 | Leukemia | Blood | ALL |
| KKU213 | 8.323 | Bile Duct Cancer | Bile Duct | Cholangiocarcinoma |
| MONOMAC6 | 8.321 | Leukemia | Blood | AML |
| | | Head and Neck | Upper | |
| SCC9 | 8.319 | Cancer | Aerodigestive | Upper Aerodigestive Squamous |
| NCIH1385 | 8.307 | Lung Cancer | Lung | NSCLC |
| JHOM2B | 8.293 | Ovarian Cancer | Ovary | Ovary Adenocarcinoma |
| | | Head and Neck | Upper | |
| BICR16 | 8.292 | Cancer | Aerodigestive | Upper Aerodigestive Squamous |
| SKBR3 | 8.290 | Breast Cancer | Breast | Breast Carcinoma |
| TOLEDO | 8.290 | Lymphoma | Lymphocyte | Non Hodgkin Lymphoma |
| HPBALL | 8.285 | Leukemia | Blood | ALL |
| | | Head and Neck | Upper | |
| CAL33 | 8.282 | Cancer | Aerodigestive | Upper Aerodigestive Squamous |
| NCIH2106 | 8.280 | Lung Cancer | Lung | NSCLC |
| HCC1500 | 8.280 | Breast Cancer | Breast | Breast Ductal Carcinoma |
| ECC10 | 8.277 | Gastric Cancer | Gastric | Gastric Small Cell |
| RDES | 8.277 | Bone Cancer | Bone | Ewing Sarcoma |
| SCLC21H | 8.272 | Lung Cancer | Lung | SCLC |
| NCIH1930 | 8.268 | Lung Cancer | Lung | SCLC |
| GSS | 8.255 | Gastric Cancer | Gastric | Gastric Adenocarcinoma |
| HUNS1 | 8.250 | Myeloma | Plasma Cell | Multiple Myeloma |
| EWS502 | 8.246 | Bone Cancer | Bone | Ewing Sarcoma |
| HCC1806 | 8.246 | Breast Cancer | Breast | Breast Ductal Carcinoma |
| JM1 | 8.246 | Leukemia | Blood | ALL |
| PECAPJ34CLONE | | Head and Neck | Upper | |
| C12 | 8.239 | Cancer | Aerodigestive | Upper Aerodigestive Squamous |
| HUH28 | 8.218 | Bile Duct Cancer | Bile Duct | Cholangiocarcinoma |
| | | Endometrial/Uteri | | |
| EN | 8.212 | ne Cancer | Uterus | Endometrial Adenocarcinoma |
| REH | 8.211 | Leukemia | Blood | ALL |
| HCC38 | 8.211 | Breast Cancer | Breast | Breast Ductal Carcinoma |
| OCIAML2 | 8.210 | Leukemia | Blood | AML |
| DMS273 | 8.208 | Lung Cancer | Lung | SCLC |
| NALM19 | 8.202 | Leukemia | Blood | ALL |
| SW900 | 8.201 | Lung Cancer | Lung | NSCLC |
| NCIH1618 | 8.193 | Lung Cancer | Lung | SCLC |
| HS860T | 8.193 | Bone Cancer | Bone | Osteosarcoma |
| NCIH1184 | 8.189 | Lung Cancer | Lung | SCLC |
| NCIH1105 | 8.188 | Lung Cancer | Lung | SCLC |
| KARPAS299 | 8.187 | Lymphoma | Lymphocyte | Non Hodgkin Lymphoma |
| HCC2814 | 8.183 | Lung Cancer | Lung | NSCLC |
| MAPACHS77 | 8.182 | Pancreatic Cancer | Pancreas | |
| HSC1 | 8.181 | Skin Cancer | Skin | Skin Squamous |
| HH | 8.177 | Lymphoma | Lymphocyte | Non Hodgkin Lymphoma |
| CBAGPN | 8.170 | Bone Cancer | Bone | Ewing Sarcoma |
| HDQP1 | 8.165 | Breast Cancer | Breast | Breast Ductal Carcinoma |
| | | Head and Neck | Upper | |
| OSC20 | 8.163 | Cancer | Aerodigestive | Upper Aerodigestive Squamous |
| OVK18 | 8.161 | Ovarian Cancer | Ovary | Ovary Adenocarcinoma |
| GDM1 | 8.160 | Leukemia | Blood | AML |
| NCIH1963 | 8.159 | Lung Cancer | Lung | SCLC |
| | | Colon/Colorectal | | |
| HCC56 | 8.155 | Cancer | Colorectal | Colorectal Adenocarcinoma |
| HLFA | 8.154 | Non-Cancerous | Fibroblast | Fibroblast Lung |
| NCIH2227 | 8.151 | Lung Cancer | Lung | SCLC |
| KG1 | 8.145 | Leukemia | Blood | AML |
| JHH1 | 8.125 | Liver Cancer | Liver | Hepatocellular Carcinoma |
| WM4235 | 8.107 | Skin Cancer | Skin Central Nervous | Melanoma |
| D458 | 8.107 | Brain Cancer | System | Medulloblastoma |
| YSCCC | 8.103 | Bile Duct Cancer | Bile Duct Central Nervous | Cholangiocarcinoma |
| SNU489 | 8.098 | Brain Cancer | System | Glioma |
| RT11284 | 8.097 | Bladder Cancer | Urinary Tract | Bladder Carcinoma |
| SKES1 | 8.091 | Bone Cancer | Bone | Ewing Sarcoma |
| SNU869 | 8.087 | Bile Duct Cancer | Bile Duct | Cholangiocarcinoma |
| TE159T | 8.086 | Non-Cancerous | Fibroblast | Fibroblast Soft Tissue |
| P2URK562 | 8.080 | Leukemia | Blood | CML |
| SCC3 | 8.080 | Lymphoma | Lymphocyte Peripheral | Non Hodgkin Lymphoma |
| SKNBE2 | 8.072 | Neuroblastoma | Nervous System | Neuroblastoma |
| KYSE30 | 8.069 | Esophageal Cancer | Esophagus | Esophagus Squamous |
| | | Endometrial/Uteri | | |
| KLE | 8.064 | ne Cancer | Uterus | Endometrial Adenocarcinoma |
| TO14 | 8.050 | Ovarian Cancer | Ovary Peripheral | Ovary Adenocarcinoma |
| KELLY | 8.048 | Neuroblastoma | Nervous System | Neuroblastoma |
| HUT78 | 8.047 | Lymphoma | Lymphocyte | Non Hodgkin Lymphoma |
| T3M4 | 8.042 | Pancreatic Cancer | Pancreas | Exocrine |
| CAL72 | 8.037 | Bone Cancer | Bone | Osteosarcoma |
| MV411 | 8.035 | Leukemia | Blood | AML |
| HCC1187 | 8.009 | Breast Cancer | Breast | Breast Ductal Carcinoma |
| | | Head and Neck | Upper | |
| BICR6 | 8.009 | Cancer | Aerodigestive | Upper Aerodigestive Squamous |
| SEM | 8.007 | Leukemia | Blood | ALL |
| HS840T | 8.000 | Non-Cancerous | Fibroblast | Fibroblast Upper Aerodigestive |
| NCIH2029 | 7.998 | Lung Cancer | Lung | SCLC |
| CORL24 | 7.993 | Lung Cancer | Lung | SCLC |
| | | Head and Neck | Upper | |
| SNU1066 | 7.982 | Cancer | Aerodigestive | Upper Aerodigestive Squamous |
| HCC1937 | 7.968 | Breast Cancer | Breast | Breast Ductal Carcinoma |
| UMUC14 | 7.968 | Bladder Cancer | Urinary Tract | Bladder Carcinoma |
| ISHIKAWAHERAK | | Endometrial/Uteri | | |
| LIO02ER | 7.953 | ne Cancer | Uterus | Endometrial Adenocarcinoma |
| SKNO1 | 7.947 | Leukemia | Blood | AML |
| | | Head and Neck | Upper | |
| SCC15 | 7.942 | Cancer | Aerodigestive | Upper Aerodigestive Squamous |
| OVKATE | 7.940 | Ovarian Cancer | Ovary | Ovary Adenocarcinoma |
| HCC1599 | 7.939 | Breast Cancer | Breast | Breast Ductal Carcinoma |
| NCIH1436 | 7.937 | Lung Cancer | Lung | SCLC |
| NCIH441 | 7.936 | Lung Cancer | Lung | NSCLC |
| CHLA32 | 7.935 | Bone Cancer | Bone | Ewing Sarcoma |
| SCLC22H | 7.930 | Lung Cancer | Lung | SCLC |
| WERIRB1 | 7.929 | Eye Cancer | Eye | Retinoblastoma |
| NCIH1836 | 7.927 | Lung Cancer | Lung | SCLC |
| DL40 | 7.924 | Lymphoma | Lymphocyte | Lymphoma Unspecified |
| NCIH2081 | 7.920 | Lung Cancer | Lung | SCLC |
| ME1 | 7.919 | Leukemia | Blood | AML |
| HEP3B217 | 7.919 | Liver Cancer | Liver | Hepatocellular Carcinoma |
| CHL1DM | 7.917 | Skin Cancer | Skin | Melanoma |
| L540 | 7.913 | Lymphoma | Lymphocyte | Hodgkin Lymphoma |
| JHUEM1 | 7.902 | Endometrial/Uteri | Uterus | Endometrial Adenocarcinoma |
| | | ne Cancer | | |
| KYSE150 | 7.893 | Esophageal Cancer | Esophagus | Esophagus Squamous |
| UMUC5 | 7.890 | Bladder Cancer | Urinary Tract | Bladder Carcinoma |
| LK2 | 7.884 | Lung Cancer | Lung | NSCLC |
| PC14 | 7.880 | Lung Cancer | Lung | NSCLC |
| | | Head and Neck | Upper | |
| UPCISCC131 | 7.874 | Cancer | Aerodigestive | Upper Aerodigestive Squamous |
| DOTC24510 | 7.870 | Cervical Cancer | Cervix Peripheral | Cervical Adenocarcinoma |
| NCCMPNST2C1 | 7.864 | Unknown | Nervous System | MPNST |
| COGE352 | 7.864 | Bone Cancer | Bone | Ewing Sarcoma |
| | | Colon/Colorectal | | |
| C2BBE1 | 7.860 | Cancer | Colorectal | Colorectal Adenocarcinoma |
| TE9 | 7.849 | Esophageal Cancer | Esophagus | Esophagus Squamous |
| KASUMI1 | 7.842 | Leukemia | Blood | AML |
| ONCODG1 | 7.841 | Ovarian Cancer | Ovary | Ovary Adenocarcinoma |
| | | Head and Neck | Upper | |
| HO1U1 | 7.838 | Cancer | Aerodigestive | Upper Aerodigestive Squamous |
| LU135 | 7.833 | Lung Cancer | Lung | SCLC |
| COLO824 | 7.831 | Breast Cancer | Breast | |
| LI7 G292CLONEA141 | 7.830 | Liver Cancer | Liver | |
| B1 | 7.826 | Bone Cancer | Bone | Osteosarcoma |
| SNU668 | 7.823 | Gastric Cancer | Gastric | Gastric Adenocarcinoma |
| | | Head and Neck | Upper | |
| DETROIT562 | 7.823 | Cancer | Aerodigestive | Upper Aerodigestive Squamous |
| DMS53 | 7.818 | Lung Cancer | Lung | SCLC |
| WPE1NA22 | 7.817 | Prostate Cancer | Prostate | Prostate Adenocarcinoma |
| MORCPR | 7.816 | Lung Cancer | Lung | NSCLC |
| NCIH2196 | 7.816 | Lung Cancer | Lung | SCLC |
| | | Endometrial/Uteri | | |
| EMTOKA | 7.815 | ne Cancer | Uterus | Uterine Carcinosarcoma |
| MHHCALL2 | 7.806 | Leukemia | Blood | ALL |
| SUM159PT | 7.802 | Breast Cancer | Breast | Breast Carcinoma |
| NCIH1341 | 7.789 | Lung Cancer | Lung | SCLC |
| NCIH510 | 7.788 | Lung Cancer | Lung | SCLC |
| KOPN8 | 7.787 | Leukemia | Blood | ALL |
| HS934T | 7.786 | Non-Cancerous | Fibroblast | Fibroblast Skin |
| NALM6 | 7.785 | Leukemia | Blood | ALL |
| | | Colon/Colorectal | | |
| SW1417 | 7.784 | Cancer | Colorectal | Colorectal Adenocarcinoma |
| OCILY19 | 7.779 | Lymphoma | Lymphocyte | Non Hodgkin Lymphoma |
| TE5 | 7.774 | Esophageal Cancer | Esophagus Peripheral | Esophagus Squamous |
| KPNYN | 7.761 | Neuroblastoma | Nervous System | Neuroblastoma |
| HS852T | 7.760 | Skin Cancer | Skin | Melanoma |
| NCIH1155 | 7.759 | Lung Cancer | Lung | NSCLC |
| IGROV1 | 7.758 | Ovarian Cancer | Ovary | Ovary Adenocarcinoma |
| OV17R | 7.757 | Ovarian Cancer | Ovary | Ovary Adenocarcinoma |
| HCC78 | 7.754 | Lung Cancer | Lung | NSCLC |
| NCIH146 | 7.746 | Lung Cancer | Lung | SCLC |
| RD | 7.744 | Sarcoma | Soft Tissue | Rhabdomyosarcoma |
| LS | 7.744 | Neuroblastoma | Peripheral Nervous System | Neuroblastoma |
| TT | 7.740 | Thyroid Cancer | Thyroid | Thyroid Squamous |
| PK1 | 7.740 | Pancreatic Cancer | Pancreas | Exocrine |
| HUH6 | 7.737 | Liver Cancer | Liver | Hepatoblastoma |
| | | Endometrial/Uteri | | |
| MFE296 | 7.733 | ne Cancer | Uterus Peripheral | Endometrial Adenocarcinoma |
| CHP212 | 7.727 | Neuroblastoma | Nervous System | Neuroblastoma |
| | | Head and Neck | Upper | |
| PECAPJ15 | 7.727 | Cancer | Aerodigestive | Upper Aerodigestive Squamous |
| HB1119 | 7.724 | Leukemia | Blood | ALL |
| NCIH889 | 7.724 | Lung Cancer | Lung | SCLC |
| MHHES1 | 7.722 | Bone Cancer | Bone | Ewing Sarcoma |
| KO52 | 7.722 | Leukemia | Blood | AML |
| KYSE70 | 7.716 | Esophageal Cancer | Esophagus | Esophagus Squamous |
| NB4 | 7.714 | Leukemia | Blood | AML |
| SIGM5 | 7.712 | Leukemia | Blood | AML |
| HS839T | 7.708 | Non-Cancerous | Fibroblast Peripheral | Fibroblast Skin |
| SKNDZ | 7.706 | Neuroblastoma | Nervous System | Neuroblastoma |
| CCLFPEDS0003T | 7.702 | Sarcoma | Soft Tissue | Undifferentiated Sarcoma |
| | | Colon/Colorectal | | |
| CCK81 | 7.693 | Cancer | Colorectal Peripheral | Colorectal Adenocarcinoma |
| KPNRTBM1 | 7.693 | Neuroblastoma | Nervous System | Neuroblastoma |
| TDOTT | 7.692 | Esophageal Cancer | Esophagus | Esophagus Squamous |
| CORL47 | 7.684 | Lung Cancer | Lung | SCLC |
| ML1 | 7.684 | Thyroid Cancer | Thyroid | Thyroid Carcinoma |
| NCIH647 | 7.682 | Lung Cancer | Lung | NSCLC |
| A673 | 7.681 | Bone Cancer | Bone | Ewing Sarcoma |
| CAOV4 | 7.680 | Ovarian Cancer | Ovary | Ovary Adenocarcinoma |
| NCIH3122 | 7.673 | Lung Cancer | Lung | NSCLC |
| A431 | 7.672 | Skin Cancer | Skin | Skin Squamous |
| | | Endometrial/Uteri | | |
| MFE280 | 7.667 | ne Cancer | Uterus | Endometrial Adenocarcinoma |
| RH36 | 7.663 | Sarcoma | Soft Tissue Peripheral | Rhabdomyosarcoma |
| SKNFI | 7.655 | Neuroblastoma | Nervous System | Neuroblastoma |
| HS274T | 7.647 | Non-Cancerous | Fibroblast | Fibroblast Breast |
| KCIMOH1 | 7.645 | Pancreatic Cancer | Pancreas | Exocrine |
| 5637 | 7.640 | Bladder Cancer | Urinary Tract | Bladder Carcinoma |
| | | Colon/Colorectal | | |
| CL14 | 7.639 | Cancer | Colorectal | Colorectal Adenocarcinoma |
| OCIM2 | 7.636 | Leukemia | Blood Peripheral | AML |
| LAN2 | 7.630 | Neuroblastoma | Nervous System Central Nervous | Neuroblastoma |
| SW1088 | 7.629 | Brain Cancer | System | Glioma |
| SNU213 | 7.624 | Pancreatic Cancer | Pancreas | Exocrine |
| RI1 | 7.624 | Lymphoma | Lymphocyte | Non Hodgkin Lymphoma |
| HS675T | 7.621 | Non-Cancerous | Fibroblast | Fibroblast Colorectal |
| HS600T | 7.619 | Non-Cancerous | Fibroblast Central Nervous | Fibroblast Skin |
| CCFSTTG1 | 7.617 | Brain Cancer | System | Glioma |
| KYSE270 | 7.615 | Esophageal Cancer | Esophagus | Esophagus Squamous |
| HCC4006 | 7.613 | Lung Cancer | Lung Peripheral | NSCLC |
| CHP126 | 7.609 | Neuroblastoma | Nervous System | Neuroblastoma |
| MG63 | 7.608 | Bone Cancer | Bone | Osteosarcoma |
| HS888T | 7.605 | Non-Cancerous | Fibroblast | Fibroblast Bone |
| KYSE450 | 7.604 | Esophageal Cancer | Esophagus Peripheral | Esophagus Squamous |
| NH6 | 7.600 | Neuroblastoma | Nervous System | Neuroblastoma |
| HDLM2 | 7.596 | Lymphoma | Lymphocyte | Hodgkin Lymphoma |
| NCIH1650 | 7.596 | Lung Cancer | Lung | NSCLC |
| NCIH2170 | 7.592 | Lung Cancer | Lung Central Nervous | NSCLC |
| U251MG | 7.583 | Brain Cancer | System | Glioma |
| NCIH1944 | 7.581 | Lung Cancer | Lung | NSCLC |
| PSN1 | 7.574 | Pancreatic Cancer | Pancreas | Exocrine |
| MOLM13 | 7.570 | Leukemia | Blood | AML |
| NCIH1648 | 7.567 | Lung Cancer | Lung | NSCLC |
| NALM1 | 7.567 | Leukemia | Blood | CML |
| RH4 | 7.566 | Sarcoma | Soft Tissue | Rhabdomyosarcoma |
| NCIH2122 | 7.561 | Lung Cancer | Lung Central Nervous | NSCLC |
| U251MGDM | 7.557 | Brain Cancer | System | Glioma |
| SNU478 | 7.557 | Bile Duct Cancer | Bile Duct | Cholangiocarcinoma |
| TC138 | 7.557 | Bone Cancer | Bone | Ewing Sarcoma |
| | | Colon/Colorectal | | |
| SNU175 | 7.554 | Cancer | Colorectal | Colorectal Adenocarcinoma |
| CHLA9 | 7.552 | Bone Cancer | Bone | Ewing Sarcoma |
| DMS114 | 7.551 | Lung Cancer | Lung Central Nervous | SCLC |
| HS683 | 7.550 | Brain Cancer | System | Glioma |
| CHLA10 | 7.538 | Bone Cancer | Bone Central Nervous | Ewing Sarcoma |
| SNB75 | 7.531 | Brain Cancer | System | Glioma |
| JHESOAD1 | 7.530 | Esophageal Cancer | Esophagus | Esophagus Adenocarcinoma |
| RMSYM | 7.519 | Sarcoma | Soft Tissue Peripheral | Rhabdomyosarcoma |
| MHHNB11 | 7.516 | Neuroblastoma | Nervous System | Neuroblastoma |
| RH41 | 7.512 | Sarcoma | Soft Tissue | Rhabdomyosarcoma |
| SUM44PE | 7.511 | Breast Cancer | Breast | Breast Carcinoma |
| | | Colon/Colorectal | | |
| LS180 | 7.511 | Cancer | Colorectal | Colorectal Adenocarcinoma |
| P30OHK | 7.509 | Leukemia | Blood | ALL |
| | | Colon/Colorectal | | |
| ECC4 | 7.508 | Cancer | Colorectal | Colorectal Adenocarcinoma |
| | | Colon/Colorectal | | |
| SNU1033 | 7.505 | Cancer | Colorectal | Colorectal Adenocarcinoma |
| MPP89 | 7.499 | Lung Cancer | Lung | Mesothelioma |
| LN382 | 7.484 | Brain Cancer | Central Nervous | Glioma |
| HELA | 7.477 | Cervical Cancer | System Cervix | Cervical Carcinoma |
| 22RV1 | 7.474 | Prostate Cancer | Prostate | Prostate Adenocarcinoma |
| SMSCTR | 7.472 | Sarcoma | Soft Tissue | Rhabdomyosarcoma |
| T24 | 7.471 | Bladder Cancer | Urinary Tract | Bladder Carcinoma |
| MUTZ5 | 7.469 | Leukemia | Blood | ALL |
| SCMCRM2 | 7.468 | Sarcoma | Soft Tissue | Rhabdomyosarcoma |
| | | Head and Neck | Upper | |
| FADU | 7.462 | Cancer | Aerodigestive | Upper Aerodigestive Squamous |
| | | Head and Neck | Upper | |
| PECAPJ49 | 7.461 | Cancer | Aerodigestive Peripheral | Upper Aerodigestive Squamous |
| KPNSI9S | 7.460 | Neuroblastoma | Nervous System | Neuroblastoma |
| UMUC4 | 7.457 | Bladder Cancer | Urinary Tract | Bladder Carcinoma |
| NCIH2882 | 7.456 | Lung Cancer | Lung | NSCLC |
| ONE58 | 7.455 | Lung Cancer | Lung | Mesothelioma |
| SNU119 | 7.452 | Ovarian Cancer | Ovary | Ovary Adenocarcinoma |
| MM253 | 7.452 | Skin Cancer | Skin | Melanoma |
| U2904 | 7.447 | Lymphoma | Lymphocyte | Lymphoma Unspecified |
| PANC0213 | 7.445 | Pancreatic Cancer | Pancreas | Exocrine |
| DANG | 7.443 | Pancreatic Cancer | Pancreas | Exocrine |
| LC1F | 7.440 | Lung Cancer | Lung | NSCLC |
| OCIAML3 | 7.437 | Leukemia | Blood | AML |
| RH28 | 7.437 | Sarcoma | Soft Tissue | Rhabdomyosarcoma |
| | | Colon/Colorectal | | |
| HT29 | 7.436 | Cancer | Colorectal | Colorectal Adenocarcinoma |
| | | Endometrial/Uteri | | |
| JHUEM3 | 7.436 | ne Cancer | Uterus | Endometrial Adenocarcinoma |
| MAC2A | 7.435 | Lymphoma | Lymphocyte Central Nervous | Lymphoma Unspecified |
| D425 | 7.433 | Brain Cancer | System | Medulloblastoma |
| SNU308 | 7.422 | Gallbladder Cancer | Bile Duct | Gallbladder Adenocarcinoma |
| JR | 7.421 | Sarcoma | Soft Tissue Peripheral | Rhabdomyosarcoma |
| GOTO | 7.421 | Neuroblastoma | Nervous System | Neuroblastoma |
| HCSC1 | 7.417 | Cervical Cancer | Cervix | Cervical Carcinoma |
| LNCAPCLONEFGC | 7.414 | Prostate Cancer | Prostate | Prostate Adenocarcinoma |
| NCIH292 | 7.413 | Lung Cancer | Lung | NSCLC |
| HCC515 | 7.405 | Lung Cancer | Lung | NSCLC |
| | | Colon/Colorectal | | |
| SNU407 | 7.404 | Cancer | Colorectal | Colorectal Adenocarcinoma |
| ECGI10 | 7.401 | Esophageal Cancer | Esophagus | Esophagus Squamous |
| MUTZ8 | 7.399 | Leukemia | Blood | AML |
| OVCAR5 | 7.395 | Ovarian Cancer | Ovary | Ovary Adenocarcinoma |
| SKMEL19 | 7.392 | Skin Cancer | Skin | Melanoma |
| OVSAHO | 7.385 | Ovarian Cancer | Ovary | Ovary Adenocarcinoma |
| CAL148 | 7.380 | Breast Cancer | Breast | Breast Ductal Carcinoma |
| MUTZ3 | 7.379 | Leukemia | Blood | AML |
| | | Colon/Colorectal | | |
| LS411N | 7.378 | Cancer | Colorectal | Colorectal Adenocarcinoma |
| MCC26 | 7.377 | Skin Cancer | Skin | Merkel Cell Carcinoma |
| TGW | 7.374 | Neuroblastoma | Peripheral | Neuroblastoma |
| NCIH524 | 7.373 | Lung Cancer | Nervous System Lung | SCLC |
| JHOS2 | 7.372 | Ovarian Cancer | Ovary | Ovary Adenocarcinoma |
| COLO680N | 7.366 | Esophageal Cancer | Esophagus | Esophagus Squamous |
| KYSE180 | 7.364 | Esophageal Cancer | Esophagus | Esophagus Squamous |
| MKN45 | 7.364 | Gastric Cancer | Gastric | Gastric Adenocarcinoma |
| LUDLU1 | 7.358 | Lung Cancer | Lung | NSCLC |
| SU8686 | 7.356 | Pancreatic Cancer | Pancreas | Exocrine |
| MEL290 | 7.354 | Eye Cancer | Eye | Uveal Melanoma |
| HCC2279 | 7.351 | Lung Cancer | Lung | NSCLC |
| COV362 | 7.349 | Ovarian Cancer | Ovary | Ovary Adenocarcinoma |
| | | Endometrial/Uteri | | |
| JAR | 7.342 | ne Cancer | Uterus | Choriocarcinoma |
| OMM1 | 7.341 | Eye Cancer | Eye | Uveal Melanoma |
| NCCIT | 7.339 | Embryonal Cancer | Embryo | Embryo Carcinoma |
| MDAPCA2B | 7.334 | Prostate Cancer | Prostate | Prostate Adenocarcinoma |
| HS618T | 7.331 | Non-Cancerous | Fibroblast | Fibroblast Lung |
| EGI1 | 7.329 | Bile Duct Cancer | Bile Duct | Cholangiocarcinoma |
| SNU387 | 7.329 | Liver Cancer | Liver | Hepatocellular Carcinoma |
| NCIH196 | 7.329 | Lung Cancer | Lung | SCLC |
| TOV112D | 7.328 | Ovarian Cancer | Ovary | Ovary Adenocarcinoma |
| MHHCALL4 | 7.327 | Leukemia | Blood Central Nervous | ALL |
| LN235 | 7.323 | Brain Cancer | System | Glioma |
| KURAMOCHI | 7.312 | Ovarian Cancer | Ovary | Ovary Adenocarcinoma |
| LPS27 | 7.311 | Liposarcoma | Soft Tissue Central Nervous | Liposarcoma |
| SUMB002 | 7.310 | Brain Cancer | System | Medulloblastoma |
| YUHOIN0650 | 7.307 | Unknown | Unknown | |
| | | Colon/Colorectal | | |
| CACO2 | 7.303 | Cancer | Colorectal | Colorectal Adenocarcinoma |
| HS343T | 7.297 | Non-Cancerous | Fibroblast | Fibroblast Breast |
| SKNEP1 | 7.292 | Bone Cancer | Bone | Ewing Sarcoma |
| KNS62 | 7.291 | Lung Cancer | Lung | NSCLC |
| OVCAR4 | 7.285 | Ovarian Cancer | Ovary | Ovary Adenocarcinoma |
| SKNMC | 7.283 | Bone Cancer | Bone | Ewing Sarcoma |
| TC71 | 7.277 | Bone Cancer | Bone | Ewing Sarcoma |
| SNU398 | 7.276 | Liver Cancer | Liver | Hepatocellular Carcinoma |
| CAMA1 | 7.271 | Breast Cancer | Breast | Breast Carcinoma |
| BPH1 | 7.268 | Prostate Cancer | Prostate | Prostate Hyperplasia |
| CALU6 | 7.268 | Lung Cancer | Lung | NSCLC |
| BV173 | 7.266 | Leukemia | Blood Peripheral | CML |
| SKNAS | 7.263 | Neuroblastoma | Nervous System | Neuroblastoma |
| COLO679 | 7.263 | Skin Cancer | Skin | Melanoma |
| OAW42 | 7.261 | Ovarian Cancer | Ovary | Ovary Carcinoma |
| CHLA99 | 7.260 | Bone Cancer | Bone | Ewing Sarcoma |
| SKUT1 | 7.259 | Sarcoma | Soft Tissue | Leiomyosarcoma |
| NOS1 | 7.252 | Bone Cancer | Bone | Osteosarcoma |
| | | Colon/Colorectal | | |
| JVE127 | 7.249 | Cancer | Colorectal | Colorectal Adenocarcinoma |
| TO175T | 7.244 | Non-Cancerous | Fibroblast | Fibroblast Lymphocyte |
| NCIH1437 | 7.239 | Lung Cancer | Lung | NSCLC |
| LPS067 | 7.239 | Liposarcoma | Soft Tissue | Liposarcoma |
| MB1 | 7.238 | Thyroid Cancer | Thyroid | Thyroid Carcinoma |
| NCIH209 | 7.237 | Lung Cancer | Lung | SCLC |
| NCIN87 | 7.236 | Gastric Cancer | Gastric | Gastric Adenocarcinoma |
| PATU8988T | 7.236 | Pancreatic Cancer | Pancreas | Exocrine |
| JEKO1 | 7.235 | Lymphoma | Lymphocyte | Non Hodgkin Lymphoma |
| OCILY3 | 7.235 | Lymphoma | Lymphocyte | Non Hodgkin Lymphoma |
| TGBC11TKB | 7.234 | Gastric Cancer | Gastric | Gastric Adenocarcinoma |
| OELE | 7.231 | Non-Cancerous Colon/Colorectal | Ovary | |
| SNU1040 | 7.229 | Cancer | Colorectal | Colorectal Adenocarcinoma |
| 9505BIK | 7.227 | Pancreatic Cancer | Pancreas | Exocrine |
| EFO21 | 7.225 | Ovarian Cancer | Ovary | Ovary Adenocarcinoma |
| HS606T | 7.225 | Non-Cancerous | Fibroblast Central Nervous | Fibroblast Breast |
| 8MGBA | 7.224 | Brain Cancer | System | Glioma |
| HS688AT | 7.223 | Non-Cancerous | Fibroblast | Fibroblast Skin |
| HCC1833 | 7.222 | Lung Cancer | Lung | NSCLC |
| HCC1569 | 7.222 | Breast Cancer | Breast | Breast Carcinoma |
| UBLC1 | 7.218 | Bladder Cancer | Urinary Tract | Bladder Carcinoma |
| HSOS1 | 7.209 | Bone Cancer Head and Neck | Bone Upper | Osteosarcoma |
| A253 | 7.209 | Cancer | Aerodigestive Central Nervous | Upper Aerodigestive Squamous |
| SNU201 | 7.202 | Brain Cancer | System | Glioma |
| HS229T | 7.200 | Non-Cancerous | Fibroblast | Fibroblast Lung |
| CALU1 | 7.199 | Lung Cancer Colon/Colorectal | Lung | NSCLC |
| HCT116 | 7.199 | Cancer | Colorectal | Colorectal Adenocarcinoma |
| SLR25 | 7.196 | Kidney Cancer | Kidney | Renal Cell Carcinoma |
| TE15 | 7.186 | Esophageal Cancer | Esophagus | Esophagus Squamous |
| SUPT1 | 7.180 | Leukemia | Blood | ALL |
| RERFLCMS | 7.173 | Lung Cancer Head and Neck | Lung Upper | NSCLC |
| SAT | 7.173 | Cancer | Aerodigestive | Upper Aerodigestive Squamous |
| LU99 | 7.167 | Lung Cancer | Lung | NSCLC |
| SCS214 | 7.167 | Sarcoma | Soft Tissue | Synovial Sarcoma |
| MEL285 | 7.163 | Eye Cancer Colon/Colorectal | Eye | Uveal Melanoma |
| SW948 | 7.157 | Cancer | Colorectal | Colorectal Adenocarcinoma |
| NCIH3255 | 7.155 | Lung Cancer | Lung | NSCLC |
| TE11 | 7.148 | Esophageal Cancer | Esophagus | Esophagus Squamous |
| UACC812 | 7.148 | Breast Cancer | Breast | Breast Ductal Carcinoma |
| UMUC1 | 7.147 | Bladder Cancer Colon/Colorectal | Urinary Tract | Bladder Carcinoma |
| SKCO1 | 7.147 | Cancer | Colorectal | Colorectal Adenocarcinoma |
| COV318 | 7.145 | Ovarian Cancer | Ovary Peripheral | Ovary Adenocarcinoma |
| NMB | 7.140 | Neuroblastoma | Nervous System | Neuroblastoma |
| JHOC5 | 7.138 | Ovarian Cancer | Ovary | Ovary Adenocarcinoma |
| HS616T | 7.136 | Non-Cancerous | Fibroblast | Fibroblast Lymphocyte |
| S462 | 7.133 | Sarcoma | Peripheral Nervous System | MPNST |
| QGP1 | 7.127 | Pancreatic Cancer | Pancreas | Endocrine |
| SJSA1 | 7.126 | Bone Cancer | Bone Central Nervous | Osteosarcoma |
| NHAHTDD | 7.116 | Non-Cancerous | System | |
| LC1SQSF | 7.115 | Lung Cancer | Lung Epidermoid | NSCLC |
| A388 | 7.111 | Skin Cancer | Carcinoma | Skin Squamous |
| BHT101 | 7.100 | Thyroid Cancer | Thyroid | Thyroid Carcinoma |
| VCAP | 7.096 | Prostate Cancer | Prostate | Prostate Adenocarcinoma |
| SNU16 | 7.092 | Gastric Cancer | Gastric | Gastric Adenocarcinoma |
| SHP77 | 7.086 | Lung Cancer | Lung | SCLC |
| | | Endometrial/Uteri | | |
| JEG3 | 7.082 | ne Cancer | Uterus | Choriocarcinoma |
| UMUC10 | 7.077 | Bladder Cancer | Urinary Tract | Bladder Carcinoma |
| HS695T | 7.074 | Skin Cancer | Skin | Melanoma |
| NCIH1666 | 7.067 | Lung Cancer | Lung | NSCLC |
| | | Endometrial/Uteri | | |
| MFE319 | 7.066 | ne Cancer | Uterus | Endometrial Adenocarcinoma |
| AGS | 7.065 | Gastric Cancer | Gastric | Gastric Adenocarcinoma |
| COLO792 | 7.065 | Skin Cancer | Skin | Melanoma |
| JJN3 | 7.064 | Myeloma | Plasma Cell Central Nervous | Multiple Myeloma |
| HSPSS | 7.054 | Sarcoma | System | MPNST |
| HOKUG | 7.051 | Cervical Cancer | Cervix Central Nervous | Glassy Cell Carcinoma |
| A1207 | 7.051 | Brain Cancer | System | Glioma |
| | | Colon/Colorectal | | |
| SW837 | 7.050 | Cancer | Colorectal Peripheral | Colorectal Adenocarcinoma |
| COGN278 | 7.036 | Neuroblastoma | Nervous System | Neuroblastoma |
| SUDHL1 | 7.035 | Lymphoma | Lymphocyte | Non Hodgkin Lymphoma |
| | | Endometrial/Uteri | | |
| SNU1077 | 7.031 | ne Cancer | Uterus | Mullerian Carcinoma |
| | | Colon/Colorectal | | |
| CL40 | 7.029 | Cancer | Colorectal | Colorectal Adenocarcinoma |
| HUPT3 | 7.029 | Pancreatic Cancer | Pancreas | Exocrine |
| SW954 | 7.028 | Cervical Cancer | Cervix | Cervical Squamous |
| RHJT | 7.025 | Sarcoma | Soft Tissue | Rhabdomyosarcoma |
| TE1 | 7.023 | Esophageal Cancer | Esophagus | Esophagus Squamous |
| NALM16 | 7.021 | Leukemia | Blood | ALL |
| PACADD119 | 7.021 | Pancreatic Cancer | Pancreas Peripheral | Exocrine |
| NB1643 | 7.020 | Neuroblastoma | Nervous System Central Nervous | Neuroblastoma |
| ONDA7 | 7.020 | Brain Cancer | System | Glioma |
| FUOV1 | 7.019 | Ovarian Cancer | Ovary | Ovary Adenocarcinoma |
| RPMI7951 | 7.016 | Skin Cancer | Skin | Melanoma |
| M040416 | 6.998 | Skin Cancer | Skin | Melanoma |
| HS255T | 6.997 | Non-Cancerous | Fibroblast | Fibroblast Colorectal |
| NIHOVCAR3 | 6.997 | Ovarian Cancer | Ovary | Ovary Adenocarcinoma |
| NUDUL1 | 6.993 | Lymphoma Colon/Colorectal | Lymphocyte | Non Hodgkin Lymphoma |
| SW620 | 6.992 | Cancer Colon/Colorectal | Colorectal | Colorectal Adenocarcinoma |
| CL11 | 6.990 | Cancer | Colorectal | Colorectal Adenocarcinoma |
| PA1 | 6.988 | Ovarian Cancer Colon/Colorectal | Ovary | Mixed Germ Cell |
| NCIH684 | 6.986 | Cancer | Colorectal | Colorectal Adenocarcinoma |
| NCIH226 | 6.986 | Lung Cancer | Lung | NSCLC |
| 8305C | 6.985 | Thyroid Cancer | Thyroid | Thyroid Carcinoma |
| OC314 | 6.978 | Ovarian Cancer | Ovary | Ovary Adenocarcinoma |
| JOPACA1 | 6.975 | Pancreatic Cancer | Pancreas | Exocrine |
| ICC2 | 6.975 | Bile Duct Cancer | Bile Duct | Cholangiocarcinoma |
| UWB1289 | 6.975 | Ovarian Cancer | Ovary | Ovary Adenocarcinoma |
| HS834T | 6.973 | Non-Cancerous | Fibroblast | Fibroblast Skin |
| NCIH1651 | 6.970 | Lung Cancer | Lung | NSCLC |
| KMRC20 | 6.967 | Kidney Cancer | Kidney | Renal Cell Carcinoma |
| MOLM1 | 6.963 | Leukemia | Blood | CML |
| SSP25 | 6.960 | Bile Duct Cancer | Bile Duct Central Nervous | Cholangiocarcinoma |
| T98G | 6.959 | Brain Cancer | System | Glioma |
| SKRC20 | 6.956 | Kidney Cancer | Kidney | Renal Cell Carcinoma |
| NZM3 | 6.953 | Skin Cancer | Skin Central Nervous | Melanoma |
| LN340 | 6.952 | Brain Cancer | System | Glioma |
| MDAMB436 | 6.946 | Breast Cancer | Breast | Breast Carcinoma |
| TE14 | 6.944 | Esophageal Cancer | Esophagus Central Nervous | Esophagus Squamous |
| HSSCH2 | 6.939 | Sarcoma | System | MPNST |
| LCLC97TM1 | 6.936 | Lung Cancer Colon/Colorectal | Lung | NSCLC |
| SW626 | 6.934 | Cancer | Colorectal Central Nervous | Colorectal Adenocarcinoma |
| SNU1105 | 6.930 | Brain Cancer | System | Glioma |
| YAMATO | 6.930 | Sarcoma | Soft Tissue | Synovial Sarcoma |
| HS751T | 6.928 | Non-Cancerous | Fibroblast | Fibroblast Lymphocyte |
| CHLA218 | 6.925 | Bone Cancer | Bone Central Nervous | Ewing Sarcoma |
| NP5 | 6.924 | Brain Cancer | System | Glioma |
| HCC1438 | 6.921 | Lung Cancer | Lung | NSCLC |
| SW982 | 6.921 | Sarcoma | Soft Tissue | Synovial Sarcoma |
| RERFLCAD1 | 6.917 | Lung Cancer | Lung | NSCLC |
| PANC0813 | 6.915 | Pancreatic Cancer | Pancreas | Exocrine |
| HOP62 | 6.908 | Lung Cancer | Lung | NSCLC |
| KP4 | 6.906 | Pancreatic Cancer | Pancreas | Exocrine |
| SUDHL5 | 6.906 | Lymphoma | Lymphocyte | Non Hodgkin Lymphoma |
| MS751 | 6.906 | Cervical Cancer | Cervix | Cervical Squamous |
| ICC137 | 6.905 | Bile Duct Cancer | Bile Duct | Cholangiocarcinoma |
| NCIH1568 | 6.900 | Lung Cancer | Lung | NSCLC |
| KYSE140 | 6.898 | Esophageal Cancer | Esophagus | Esophagus Squamous |
| HS944T | 6.895 | Skin Cancer | Skin | Melanoma |
| MDAMB157 | 6.895 | Breast Cancer | Breast | Breast Ductal Carcinoma |
| MEWO | 6.894 | Skin Cancer | Skin | Melanoma |
| | | Colon/Colorectal | | |
| SW403 | 6.890 | Cancer | Colorectal Peripheral | Colorectal Adenocarcinoma |
| SKNSH | 6.889 | Neuroblastoma | Nervous System | Neuroblastoma |
| VP229 | 6.883 | Breast Cancer | Breast | Breast Ductal Carcinoma |
| HUH1 | 6.879 | Liver Cancer | Liver | Hepatocellular Carcinoma |
| MERO84 | 6.877 | Lung Cancer | Lung | Mesothelioma |
| COV413A | 6.870 | Ovarian Cancer | Ovary | Ovary Carcinoma |
| KYSE510 | 6.865 | Esophageal Cancer | Esophagus | Esophagus Squamous |
| SNU449 | 6.862 | Liver Cancer | Liver | Hepatocellular Carcinoma |
| CFPAC1 | 6.862 | Pancreatic Cancer | Pancreas | Exocrine |
| NCIH2452 | 6.861 | Lung Cancer | Lung | Mesothelioma |
| MERO25 | 6.860 | Lung Cancer | Lung | Mesothelioma |
| HUG1N | 6.860 | Gastric Cancer | Gastric | Gastric Adenocarcinoma |
| HGC27 | 6.859 | Gastric Cancer | Gastric | Gastric Adenocarcinoma |
| HMY1 | 6.859 | Skin Cancer | Skin | Melanoma |
| SNU8 | 6.858 | Ovarian Cancer | Ovary | Ovary Adenocarcinoma |
| HS729 | 6.844 | Unknown | Soft Tissue | Rhabdomyosarcoma, Embryonal |
| NCIH1792 | 6.842 | Lung Cancer | Lung | NSCLC |
| DV90 | 6.830 | Lung Cancer | Lung | NSCLC |
| HCC1171 | 6.828 | Lung Cancer | Lung | NSCLC |
| KMS11 | 6.827 | Myeloma | Plasma Cell | Multiple Myeloma |
| PACADD137 | 6.824 | Pancreatic Cancer | Pancreas | Exocrine |
| HT3 | 6.823 | Cervical Cancer | Cervix | Cervical Carcinoma |
| MEC1 | 6.823 | Leukemia | Blood | CLL |
| NCIH2085 | 6.821 | Lung Cancer | Lung | NSCLC |
| TIG3TD | 6.820 | Non-Cancerous | Fibroblast | Fibroblast Lung |
| | | Endometrial/Uteri | | |
| HEC151 | 6.817 | ne Cancer | Uterus | Endometrial Adenocarcinoma |
| SKMES1 | 6.815 | Lung Cancer | Lung Central Nervous | NSCLC |
| 42MGBA | 6.812 | Brain Cancer | System | Glioma |
| CAL62 | 6.810 | Thyroid Cancer | Thyroid | Thyroid Carcinoma |
| NCIH2291 | 6.804 | Lung Cancer | Lung | NSCLC |
| UMUC16 | 6.804 | Bladder Cancer | Urinary Tract | Bladder Carcinoma |
| OC316 | 6.797 | Ovarian Cancer | Ovary | Ovary Adenocarcinoma |
| | | Colon/Colorectal | | |
| CL34 | 6.796 | Cancer | Colorectal | Colorectal Adenocarcinoma |
| LPS141 | 6.794 | Liposarcoma | Soft Tissue | Liposarcoma |
| | | Head and Neck | Upper | |
| SNU46 | 6.782 | Cancer | Aerodigestive Peripheral | Upper Aerodigestive Squamous |
| NGP | 6.769 | Neuroblastoma | Nervous System | Neuroblastoma |
| | | Colon/Colorectal | | |
| COLO320 | 6.765 | Cancer | Colorectal | Colorectal Adenocarcinoma |
| SKMEL5 | 6.763 | Skin Cancer | Skin Central Nervous | Melanoma |
| SF268 | 6.761 | Brain Cancer | System Central Nervous | Glioma |
| GI1 | 6.761 | Brain Cancer | System | Glioma |
| ESS1 | 6.761 | Endometrial/Uteri | Uterus | Endometrial Stromal Sarcoma |
| | | ne Cancer | | |
| MCC13 | 6.761 | Skin Cancer | Skin | Merkel Cell Carcinoma |
| MP46 | 6.758 | Eye Cancer | Eye | Uveal Melanoma |
| PSS131R | 6.753 | Bone Cancer | Bone | Osteosarcoma |
| HL60 | 6.752 | Leukemia | Blood | AML |
| | | Endometrial/Uteri | | |
| SNU685 | 6.749 | ne Cancer | Uterus | MMMT |
| SKPNDW | 6.747 | Bone Cancer | Bone Peripheral | Ewing Sarcoma |
| TN2 | 6.747 | Neuroblastoma | Nervous System Peripheral | Neuroblastoma |
| TASK1 | 6.743 | Brain Cancer | Nervous System | PNET |
| HS870T | 6.742 | Non-Cancerous | Fibroblast | Fibroblast Bone |
| SNU423 | 6.738 | Liver Cancer | Liver | Hepatocellular Carcinoma |
| ISTMES2 | 6.729 | Lung Cancer | Lung Peripheral | Mesothelioma |
| COGN305 | 6.727 | Neuroblastoma | Nervous System | Neuroblastoma |
| STM9101 | 6.727 | Rhabdoid | Soft Tissue | Malignant Rhabdoid Tumor |
| HS281T | 6.725 | Non-Cancerous | Fibroblast | Fibroblast Breast |
| SNU1079 | 6.721 | Bile Duct Cancer | Bile Duct | Cholangiocarcinoma |
| NCIH28 | 6.720 | Lung Cancer | Lung | Mesothelioma |
| RERFLCAI | 6.719 | Lung Cancer | Lung | NSCLC |
| SNU475 | 6.716 | Liver Cancer | Liver Peripheral | Hepatocellular Carcinoma |
| SIMA | 6.714 | Neuroblastoma | Nervous System | Neuroblastoma |
| | | Endometrial/Uteri | | |
| TEN | 6.713 | ne Cancer | Uterus | Clear Cell Carcinoma |
| | | Head and Neck | Upper | |
| OSC19 | 6.710 | Cancer | Aerodigestive | Upper Aerodigestive Squamous |
| NCIH1563 | 6.709 | Lung Cancer | Lung | NSCLC |
| PLCPRF5 | 6.709 | Liver Cancer | Liver | Hepatocellular Carcinoma |
| PC9 | 6.707 | Lung Cancer | Lung | NSCLC |
| PEA1 | 6.699 | Ovarian Cancer | Ovary | Ovary Adenocarcinoma |
| NCIH2444 | 6.694 | Lung Cancer | Lung | NSCLC |
| TOV21G | 6.694 | Ovarian Cancer | Ovary | Ovary Adenocarcinoma |
| RC2 | 6.691 | Sarcoma | Soft Tissue | Rhabdomyosarcoma |
| SCABER | 6.687 | Bladder Cancer | Urinary Tract | Bladder Carcinoma |
| MM160113 | 6.687 | Skin Cancer | Skin | Melanoma |
| MMAC | 6.685 | Skin Cancer | Skin Central Nervous | Melanoma |
| DAOY | 6.684 | Brain Cancer | System | Medulloblastoma |
| SKMEL3 | 6.683 | Skin Cancer | Skin | Melanoma |
| M140325 | 6.683 | Skin Cancer | Skin | Melanoma |
| HCC2157 | 6.682 | Breast Cancer | Breast | Breast Ductal Carcinoma |
| SW1573 | 6.680 | Lung Cancer | Lung | NSCLC |
| OVISE | 6.675 | Ovarian Cancer | Ovary | Ovary Adenocarcinoma |
| UPMD1 | 6.672 | Unknown | Unknown | Melanoma |
| NUDHL1 | 6.664 | Lymphoma | Lymphocyte | Non Hodgkin Lymphoma |
| SLR24 | 6.656 | Kidney Cancer | Kidney | Renal Cell Carcinoma |
| EW8 | 6.654 | Bone Cancer | Bone | Ewing Sarcoma |
| | | Colon/Colorectal | | |
| LOVO | 6.653 | Cancer | Colorectal | Colorectal Adenocarcinoma |
| C396 | 6.652 | Bone Cancer | Bone | Osteosarcoma |
| | | Head and Neck | Upper | |
| KOSC2 | 6.651 | Cancer | Aerodigestive | Upper Aerodigestive Squamous |
| DM3 | 6.641 | Non-Cancerous | Fibroblast | Fibroblast Lung |
| MDAMB231 | 6.622 | Breast Cancer | Breast | Breast Carcinoma |
| SUDHL8 | 6.620 | Lymphoma | Lymphocyte | Non Hodgkin Lymphoma |
| MDAMB453 | 6.619 | Breast Cancer | Breast Central Nervous | Breast Carcinoma |
| GOS3 | 6.617 | Brain Cancer | System | Glioma |
| RPE1SS77 | 6.597 | Non-Cancerous | Eye | |
| | | Colon/Colorectal | | |
| TGBC18TKB | 6.596 | Cancer | Colorectal | Colorectal Adenocarcinoma |
| HS737T | 6.592 | Non-Cancerous | Fibroblast | Fibroblast Bone |
| TE4 | 6.583 | Esophageal Cancer | Esophagus | Esophagus Squamous |
| | | Colon/Colorectal | | |
| LS513 | 6.578 | Cancer | Colorectal | Colorectal Adenocarcinoma |
| VMCUB1 | 6.575 | Bladder Cancer | Urinary Tract | Bladder Carcinoma |
| NCIH2347 | 6.575 | Lung Cancer | Lung | NSCLC |
| NUGC3 | 6.571 | Gastric Cancer | Gastric Peripheral | Gastric Adenocarcinoma |
| CHLA15 | 6.569 | Neuroblastoma | Nervous System | Neuroblastoma |
| MCF7 | 6.569 | Breast Cancer | Breast | Breast Carcinoma |
| | | Colon/Colorectal | | |
| NCIH747 | 6.566 | Cancer | Colorectal | Colorectal Adenocarcinoma |
| | | Head and Neck | Upper | |
| YD8 | 6.565 | Cancer | Aerodigestive | Upper Aerodigestive Squamous |
| G361 | 6.564 | Skin Cancer | Skin Central Nervous | Melanoma |
| U178 | 6.559 | Brain Cancer | System | Glioma |
| HS940T | 6.555 | Non-Cancerous | Fibroblast | Fibroblast Skin |
| TE8 | 6.555 | Esophageal Cancer | Esophagus Peripheral | Esophagus Squamous |
| SHSY5Y | 6.553 | Neuroblastoma | Nervous System | Neuroblastoma |
| SALE | 6.548 | Non-Cancerous | Lung | |
| TC106 | 6.541 | Bone Cancer | Bone | Ewing Sarcoma |
| JL1 | 6.537 | Lung Cancer | Lung | Mesothelioma |
| | | Colon/Colorectal | | |
| GP2D | 6.537 | Cancer | Colorectal | Colorectal Adenocarcinoma |
| HNT34 | 6.534 | Leukemia | Blood | AML |
| BDCM | 6.534 | Leukemia | Blood | AML |
| TT2609C02 | 6.534 | Thyroid Cancer | Thyroid | Thyroid Carcinoma |
| PATU8902 | 6.532 | Pancreatic Cancer | Pancreas | Exocrine |
| | | Colon/Colorectal | | |
| OUMS23 | 6.531 | Cancer | Colorectal Peripheral | Colorectal Adenocarcinoma |
| CHP134 | 6.530 | Neuroblastoma | Nervous System | Neuroblastoma |
| 95T1000 | 6.521 | Liposarcoma | Soft Tissue | Liposarcoma |
| | | Head and Neck | Upper | |
| HSC2 | 6.520 | Cancer | Aerodigestive | Upper Aerodigestive Squamous |
| GSU | 6.518 | Gastric Cancer | Gastric | Gastric Adenocarcinoma |
| HCC1143 | 6.518 | Breast Cancer | Breast | Breast Ductal Carcinoma |
| LS123 | 6.513 | Colon/Colorectal | Colorectal | Colorectal Adenocarcinoma |
| | | Cancer | | |
| SMZ1 | 6.512 | Lymphoma | Lymphocyte | Lymphoma Unspecified |
| RMGI | 6.508 | Ovarian Cancer | Ovary | Ovary Adenocarcinoma |
| A427 | 6.505 | Lung Cancer | Lung | NSCLC |
| OACM51 | 6.504 | Esophageal Cancer | Esophagus | Esophagus Adenocarcinoma |
| RH18 | 6.504 | Sarcoma | Soft Tissue | Rhabdomyosarcoma |
| HS746T | 6.503 | Gastric Cancer | Gastric Central Nervous | Gastric Adenocarcinoma |
| 170MGBA | 6.502 | Brain Cancer | System | Glioma |
| SNU601 | 6.496 | Gastric Cancer | Gastric | Gastric Adenocarcinoma |
| NCIH1755 | 6.493 | Lung Cancer | Lung | NSCLC |
| CAPAN2 | 6.484 | Pancreatic Cancer | Pancreas | Exocrine |
| SAOS2 | 6.479 | Bone Cancer | Bone | Osteosarcoma |
| HOS | 6.472 | Bone Cancer | Bone | Osteosarcoma |
| COV504 | 6.460 | Ovarian Cancer | Ovary | Ovary Adenocarcinoma |
| HAP1 | 6.460 | Leukemia | Blood | CML |
| | | Head and Neck | Upper | |
| HSC3 | 6.455 | Cancer | Aerodigestive | Upper Aerodigestive Squamous |
| 59M | 6.453 | Ovarian Cancer | Ovary | Ovary Adenocarcinoma |
| OCIC4P | 6.451 | Ovarian Cancer | Ovary | Ovary Adenocarcinoma |
| CAL78 | 6.438 | Bone Cancer | Bone Central Nervous | Chondrosarcoma |
| CAS1 | 6.436 | Brain Cancer | System | Glioma |
| | | Head and Neck | Upper | |
| BHY | 6.435 | Cancer | Aerodigestive | Upper Aerodigestive Squamous |
| 8505C | 6.430 | Thyroid Cancer | Thyroid | Thyroid Carcinoma |
| PACADD135 | 6.430 | Pancreatic Cancer | Pancreas | Exocrine |
| P4E6 | 6.430 | Prostate Cancer | Prostate Central Nervous | Prostate Adenocarcinoma |
| ONDA8 | 6.426 | Brain Cancer | System | Glioma |
| MM426 | 6.425 | Skin Cancer | Skin Central Nervous | Melanoma |
| SW1783 | 6.421 | Brain Cancer | System | Glioma |
| HS822T | 6.418 | Non-Cancerous | Fibroblast | Fibroblast Bone |
| MERO48A | 6.418 | Lung Cancer | Lung | Mesothelioma |
| HS739T | 6.412 | Non-Cancerous | Fibroblast | Fibroblast Breast |
| | | Head and Neck | Upper | |
| SNU1214 | 6.409 | Cancer | Aerodigestive | Upper Aerodigestive Squamous |
| T173 | 6.406 | Non-Cancerous | Fibroblast | Fibroblast Bone |
| VMRCRCZ | 6.406 | Kidney Cancer | Kidney | Renal Cell Carcinoma |
| EPLC272H | 6.403 | Lung Cancer | Lung | NSCLC |
| JVM2 | 6.401 | Lymphoma | Lymphocyte | Non Hodgkin Lymphoma |
| MERO95 | 6.399 | Lung Cancer | Lung | Mesothelioma |
| NCIH2023 | 6.397 | Lung Cancer | Lung | NSCLC |
| RKN | 6.391 | Sarcoma | Soft Tissue | Leiomyosarcoma |
| SUPB15 | 6.391 | Leukemia | Blood | ALL |
| UACC257 | 6.390 | Skin Cancer | Skin | Melanoma |
| | | Head and Neck | Upper | |
| SAS | 6.385 | Cancer | Aerodigestive | Upper Aerodigestive Squamous |
| ICC108 | 6.382 | Bile Duct Cancer | Bile Duct | Cholangiocarcinoma |
| | | Colon/Colorectal | | |
| SNU283 | 6.371 | Cancer | Colorectal | Colorectal Adenocarcinoma |
| TTC442 | 6.371 | Sarcoma | Soft Tissue | Rhabdomyosarcoma |
| KYSE520 | 6.366 | Esophageal Cancer | Esophagus | Esophagus Squamous |
| COLO800 | 6.364 | Skin Cancer | Skin | Melanoma |
| | | Head and Neck | Upper | |
| UPCISCC200 | 6.363 | Cancer | Aerodigestive | Upper Aerodigestive Squamous |
| COGAR359 | 6.362 | Rhabdoid | Soft Tissue | ATRT |
| JHOM1 | 6.360 | Ovarian Cancer | Ovary | Ovary Adenocarcinoma |
| HEKTE | 6.357 | Non-Cancerous | Kidney Central Nervous | |
| GAMG | 6.348 | Brain Cancer | System | Glioma |
| SNU410 | 6.343 | Pancreatic Cancer | Pancreas | Exocrine |
| BT20 | 6.343 | Breast Cancer | Breast | Breast Ductal Carcinoma |
| KPL1 | 6.341 | Breast Cancer | Breast | Breast Carcinoma |
| C33A | 6.340 | Cervical Cancer | Cervix Central Nervous | Cervical Carcinoma |
| LN464 | 6.339 | Brain Cancer | System Central Nervous | Glioma |
| CHLA57 | 6.332 | Unknown | System | PNET |
| NAMALWA | 6.328 | Lymphoma | Lymphocyte | Non Hodgkin Lymphoma |
| SHI1 | 6.324 | Leukemia | Blood | AML |
| HS819T | 6.320 | Non-Cancerous | Fibroblast | Fibroblast Bone |
| | | Colon/Colorectal | | |
| SNUC5 | 6.319 | Cancer | Colorectal | Colorectal Adenocarcinoma |
| SNU324 | 6.313 | Pancreatic Cancer | Pancreas Central Nervous | Exocrine |
| AM38 | 6.309 | Brain Cancer | System | Glioma |
| MERO82 | 6.303 | Lung Cancer | Lung Central Nervous | Mesothelioma |
| NMCG1 | 6.301 | Brain Cancer | System | Glioma |
| SEMK2 | 6.298 | Leukemia | Blood | ALL |
| WM1799 | 6.293 | Skin Cancer | Skin | Melanoma |
| | | Colon/Colorectal | | |
| HCT15 | 6.290 | Cancer | Colorectal | Colorectal Adenocarcinoma |
| RH30 | 6.288 | Sarcoma | Soft Tissue | Rhabdomyosarcoma |
| | | Colon/Colorectal | | |
| SW48 | 6.281 | Cancer | Colorectal | Colorectal Adenocarcinoma |
| | | Head and Neck | Upper | |
| H413 | 6.281 | Cancer | Aerodigestive | Upper Aerodigestive Squamous |
| HS895T | 6.276 | Non-Cancerous | Fibroblast | Fibroblast Skin |
| HCC2218 | 6.271 | Breast Cancer | Breast | Breast Ductal Carcinoma |
| RS5 | 6.266 | Non-Cancerous | Fibroblast | Fibroblast Lung |
| PC3 | 6.265 | Prostate Cancer | Prostate | Prostate Adenocarcinoma |
| OSRC2 | 6.264 | Kidney Cancer | Kidney | Renal Cell Carcinoma |
| NCIH1734 | 6.263 | Lung Cancer | Lung | NSCLC |
| OMM25 | 6.263 | Eye Cancer | Eye | Uveal Melanoma |
| HS821T | 6.262 | Non-Cancerous | Fibroblast | Fibroblast Bone |
| TC205 | 6.260 | Bone Cancer | Bone | Ewing Sarcoma |
| | | Colon/Colorectal | | |
| HT55 | 6.259 | Cancer | Colorectal | Colorectal Adenocarcinoma |
| NCIH2009 | 6.258 | Lung Cancer | Lung | NSCLC |
| NCIH1703 | 6.251 | Lung Cancer | Lung | NSCLC |
| NCIH1793 | 6.249 | Lung Cancer | Lung | NSCLC |
| CALU3 | 6.248 | Lung Cancer | Lung | NSCLC |
| RPE1SS111 | 6.248 | Non-Cancerous | Eye | |
| IPC298 | 6.246 | Skin Cancer | Skin | Melanoma |
| TE125T | 6.243 | Non-Cancerous | Fibroblast | Fibroblast Soft Tissue |
| JHH2 | 6.242 | Liver Cancer | Liver | Hepatocellular Carcinoma |
| TGBC52TKB | 6.235 | Bile Duct Cancer | Bile Duct Central Nervous | Cholangiocarcinoma |
| U87MG | 6.234 | Brain Cancer | System Central Nervous | Glioma |
| ONS76 | 6.229 | Brain Cancer | System | Medulloblastoma |
| | | Head and Neck | Upper | |
| YD38 | 6.229 | Cancer | Aerodigestive | Upper Aerodigestive Squamous |
| PANC0203 | 6.227 | Pancreatic Cancer | Pancreas | Exocrine |
| RPE1SS48 | 6.224 | Non-Cancerous | Eye | |
| BCPAP | 6.216 | Thyroid Cancer | Thyroid | Thyroid Carcinoma |
| CADOES1 | 6.215 | Bone Cancer | Bone Central Nervous | Ewing Sarcoma |
| A172 | 6.212 | Brain Cancer | System | Glioma |
| OE33 | 6.211 | Esophageal Cancer | Esophagus | Esophagus Adenocarcinoma |
| JHH4 | 6.210 | Liver Cancer | Liver | Hepatocellular Carcinoma |
| MKN1 | 6.210 | Gastric Cancer | Gastric | Gastric Adenocarcinoma |
| ESO26 | 6.206 | Esophageal Cancer | Esophagus | Esophagus Adenocarcinoma |
| MM1S | 6.198 | Myeloma | Plasma Cell | Multiple Myeloma |
| HCC827 | 6.198 | Lung Cancer | Lung | NSCLC |
| CAPAN1 | 6.197 | Pancreatic Cancer | Pancreas | Exocrine |
| NOMO1 | 6.193 | Leukemia | Blood | AML |
| SNU620 | 6.192 | Gastric Cancer | Gastric | Gastric Adenocarcinoma |
| MERO41 | 6.189 | Lung Cancer | Lung | Mesothelioma |
| J82 | 6.188 | Bladder Cancer | Urinary Tract | Bladder Carcinoma |
| 143B | 6.186 | Bone Cancer | Bone | Osteosarcoma |
| KMS26 | 6.182 | Myeloma | Plasma Cell | Multiple Myeloma |
| SKGT4 | 6.181 | Esophageal Cancer | Esophagus Central Nervous | Esophagus Adenocarcinoma |
| IOMMLEE | 6.180 | Brain Cancer | System | Meningioma |
| HEYA8 | 6.180 | Ovarian Cancer | Ovary | Ovary Adenocarcinoma |
| PECAPJ41CLONE | | Head and Neck | Upper | |
| D2 | 6.169 | Cancer | Aerodigestive | Upper Aerodigestive Squamous |
| NUGC4 | 6.166 | Gastric Cancer | Gastric | Gastric Adenocarcinoma |
| NCIH2030 | 6.164 | Lung Cancer | Lung | NSCLC |
| | | Head and Neck | Upper | |
| SCC25 | 6.161 | Cancer | Aerodigestive | Upper Aerodigestive Squamous |
| UMUC11 | 6.159 | Bladder Cancer | Urinary Tract | Bladder Carcinoma |
| PK45H | 6.159 | Pancreatic Cancer | Pancreas | Exocrine |
| CAOV3 | 6.145 | Ovarian Cancer | Ovary | Ovary Adenocarcinoma |
| HS863T | 6.143 | Non-Cancerous | Fibroblast | Fibroblast Bone |
| JIMT1 | 6.142 | Breast Cancer | Breast Peripheral | Breast Ductal Carcinoma |
| NH12 | 6.138 | Neuroblastoma | Nervous System | Neuroblastoma |
| PACADD188 | 6.127 | Pancreatic Cancer | Pancreas Peripheral | Exocrine |
| GIMEN | 6.125 | Neuroblastoma | Nervous System | Neuroblastoma |
| YD15 | 6.121 | Head and Neck | Upper | Upper Aerodigestive Squamous |
| | | Cancer | Aerodigestive Central Nervous | |
| GMS10 | 6.117 | Brain Cancer | System | Glioma |
| ICC5 | 6.117 | Bile Duct Cancer | Bile Duct | Cholangiocarcinoma |
| K029AX | 6.116 | Skin Cancer | Skin | Melanoma |
| COLO783 | 6.115 | Skin Cancer | Skin Central Nervous | Melanoma |
| TM31 | 6.113 | Brain Cancer | System | Glioma |
| ICC8 | 6.111 | Bile Duct Cancer | Bile Duct Peripheral | Cholangiocarcinoma |
| MPNST724 | 6.106 | Sarcoma | Nervous System | MPNST |
| MSTO211H | 6.099 | Lung Cancer | Lung | Mesothelioma |
| CHLA266 | 6.097 | Rhabdoid | Soft Tissue | ATRT |
| | | Colon/Colorectal | | |
| SNU1197 | 6.096 | Cancer | Colorectal | Colorectal Adenocarcinoma |
| 639V | 6.095 | Bladder Cancer | Urinary Tract | Bladder Carcinoma |
| | | Head and Neck | Upper | |
| UPCISCC111 | 6.093 | Cancer | Aerodigestive | Upper Aerodigestive Squamous |
| MFM223 | 6.092 | Breast Cancer | Breast | Breast Ductal Carcinoma |
| C4II | 6.091 | Cervical Cancer | Cervix | Cervical Carcinoma |
| NCIH1435 | 6.087 | Lung Cancer | Lung Central Nervous | NSCLC |
| LN443 | 6.085 | Brain Cancer | System | Glioma |
| GCT | 6.078 | Sarcoma | Soft Tissue | Pleomorphic Sarcoma |
| HT | 6.074 | Lymphoma | Lymphocyte | Non Hodgkin Lymphoma |
| HCA1 | 6.074 | Cervical Cancer | Cervix | Cervical Adenocarcinoma |
| LXF289 | 6.073 | Lung Cancer | Lung | NSCLC |
| SW1990 | 6.071 | Pancreatic Cancer | Pancreas | Exocrine |
| HCC1195 | 6.068 | Lung Cancer | Lung | NSCLC |
| WSUNHL | 6.067 | Lymphoma | Lymphocyte | Lymphoma Unspecified |
| SYO1 | 6.065 | Sarcoma | Soft Tissue | Synovial Sarcoma |
| PANC0327 | 6.059 | Pancreatic Cancer | Pancreas | Exocrine |
| | | Colon/Colorectal | | |
| SNUC4 | 6.056 | Cancer | Colorectal | Colorectal Adenocarcinoma |
| ISTMES1 | 6.054 | Lung Cancer | Lung Central Nervous | Mesothelioma |
| SNU626 | 6.051 | Brain Cancer | System | Glioma |
| MERO14 | 6.047 | Lung Cancer | Lung | Mesothelioma |
| HDMYZ | 6.047 | Unknown | Unknown | |
| TFK1 | 6.046 | Bile Duct Cancer | Bile Duct | Cholangiocarcinoma |
| TE441T | 6.046 | Sarcoma | Soft Tissue | Rhabdomyosarcoma |
| MERO83 | 6.045 | Lung Cancer | Lung | Mesothelioma |
| SUIT2 | 6.044 | Pancreatic Cancer | Pancreas | Exocrine |
| SNU719 | 6.044 | Gastric Cancer | Gastric | Gastric Adenocarcinoma |
| NCIH1838 | 6.039 | Lung Cancer | Lung | NSCLC |
| MM415 | 6.038 | Skin Cancer | Skin | Melanoma |
| SNU761 | 6.036 | Liver Cancer | Liver | Hepatocellular Carcinoma |
| | | Head and Neck | Upper | |
| UPCISCC040 | 6.036 | Cancer | Aerodigestive | Upper Aerodigestive Squamous |
| | | Endometrial/Uteri | | |
| EFE184 | 6.032 | ne Cancer | Uterus | Endometrial Adenocarcinoma |
| SNU886 | 6.030 | Liver Cancer | Liver | Hepatocellular Carcinoma |
| UOK101 | 6.024 | Kidney Cancer | Kidney | Renal Cell Carcinoma |
| | | Endometrial/Uteri | | |
| JHUEM7 | 6.022 | ne Cancer | Uterus | Endometrial Adenocarcinoma |
| IGR1 | 6.020 | Skin Cancer | Skin | Melanoma |
| SW1710 | 6.016 | Bladder Cancer | Urinary Tract | Bladder Carcinoma |
| RPE1SS119 | 6.013 | Non-Cancerous | Eye | |
| ECC2 | 6.008 | Bile Duct Cancer | Bile Duct | Cholangiocarcinoma |
| ICC106 | 6.005 | Bile Duct Cancer | Bile Duct | Cholangiocarcinoma |
| NO36 | 6.004 | Lung Cancer | Lung | Mesothelioma |
| NCIH358 | 5.996 | Lung Cancer | Lung | NSCLC |
| ICC12 | 5.992 | Bile Duct Cancer | Bile Duct | Cholangiocarcinoma |
| SNU1272 | 5.991 | Kidney Cancer | Kidney | Renal Cell Carcinoma |
| SKLMS1 | 5.989 | Sarcoma | Soft Tissue | Leiomyosarcoma |
| OS252 | 5.981 | Bone Cancer | Bone | Osteosarcoma |
| | | Head and Neck | Upper | |
| RPMI2650 | 5.980 | Cancer | Aerodigestive Central Nervous | Upper Aerodigestive Squamous |
| SF126 | 5.977 | Brain Cancer | System | Glioma |
| | | Endometrial/Uteri | | |
| HEC1B | 5.976 | ne Cancer | Uterus | Endometrial Adenocarcinoma |
| SKHEP1 | 5.975 | Liver Cancer | Liver Central Nervous | Hepatocellular Carcinoma |
| LN215 | 5.974 | Brain Cancer | System | Glioma |
| SKOV3 | 5.972 | Ovarian Cancer | Ovary | Ovary Adenocarcinoma |
| SLR26 | 5.961 | Kidney Cancer | Kidney | Renal Cell Carcinoma |
| 94T778 | 5.950 | Liposarcoma | Soft Tissue | Liposarcoma |
| LCLC103H | 5.945 | Lung Cancer | Lung | NSCLC |
| | | Endometrial/Uteri | | |
| HEC265 | 5.940 | ne Cancer | Uterus | Endometrial Adenocarcinoma |
| | | Endometrial/Uteri | | |
| HEC1A | 5.935 | ne Cancer | Uterus | Endometrial Adenocarcinoma |
| | | Colon/Colorectal | | |
| SW1463 | 5.926 | Cancer | Colorectal | Colorectal Adenocarcinoma |
| MEL270 | 5.925 | Eye Cancer | Eye | Uveal Melanoma |
| PEO1 | 5.913 | Ovarian Cancer | Ovary | Ovary Adenocarcinoma |
| BC3C | 5.910 | Bladder Cancer | Urinary Tract | Bladder Carcinoma |
| MALME3M | 5.908 | Skin Cancer | Skin | Melanoma |
| | | Head and Neck | Upper | |
| H357 | 5.906 | Cancer | Aerodigestive | Upper Aerodigestive Squamous |
| CAL54 | 5.901 | Kidney Cancer | Kidney | Renal Cell Carcinoma |
| CW9019 | 5.899 | Sarcoma | Soft Tissue | Rhabdomyosarcoma |
| NCIH1395 | 5.897 | Lung Cancer | Lung Central Nervous | NSCLC |
| KG1C | 5.889 | Brain Cancer | System | Glioma |
| PRECLH | 5.888 | Non-Cancerous | Prostate | |
| SW1353 | 5.884 | Bone Cancer | Bone | Chondrosarcoma |
| NCIH838 | 5.883 | Lung Cancer | Lung | NSCLC |
| PK59 | 5.877 | Pancreatic Cancer | Pancreas | Exocrine |
| ES2 | 5.877 | Ovarian Cancer | Ovary | Ovary Adenocarcinoma |
| RMUGS | 5.866 | Ovarian Cancer | Ovary | Ovary Adenocarcinoma |
| HS698T | 5.858 | Non-Cancerous | Fibroblast | Fibroblast Colorectal |
| RERFLCSQ1 | 5.849 | Lung Cancer | Lung | NSCLC |
| FEPD | 5.848 | Lymphoma | Lymphocyte | Lymphoma Unspecified |
| HARA | 5.847 | Lung Cancer | Lung | NSCLC |
| SW579 | 5.840 | Thyroid Cancer | Thyroid Central Nervous | Thyroid Squamous |
| GB1 | 5.835 | Brain Cancer | System | Glioma |
| 647V | 5.831 | Bladder Cancer | Urinary Tract | Bladder Carcinoma |
| CME1 | 5.830 | Sarcoma | Soft Tissue | Synovial Sarcoma |
| NCIH1573 | 5.829 | Lung Cancer | Lung | NSCLC |
| KMRC3 | 5.828 | Kidney Cancer | Kidney | Renal Cell Carcinoma |
| SIHA | 5.820 | Cervical Cancer | Cervix | Cervical Carcinoma |
| | | Head and Neck | Upper | |
| BICR78 | 5.810 | Cancer | Aerodigestive | Upper Aerodigestive Squamous |
| L82 | 5.806 | Lymphoma | Lymphocyte | Lymphoma Unspecified |
| UMUC13 | 5.804 | Bladder Cancer | Urinary Tract | Bladder Carcinoma |
| VAESBJ | 5.796 | Sarcoma | Soft Tissue Central Nervous | Epithelioid Sarcoma |
| LN229 | 5.780 | Brain Cancer | System | Glioma |
| SKMEL2 | 5.778 | Skin Cancer | Skin | Melanoma |
| | | Colon/Colorectal | | |
| SW480 | 5.775 | Cancer | Colorectal Central Nervous | Colorectal Adenocarcinoma |
| NP3 | 5.772 | Brain Cancer | System | Glioma |
| HA1E | 5.771 | Non-Cancerous | Kidney Central Nervous | |
| DBTRG05MG | 5.766 | Brain Cancer | System | Glioma |
| KP3 | 5.763 | Pancreatic Cancer | Pancreas | Exocrine |
| REC1 | 5.761 | Lymphoma | Lymphocyte | Non Hodgkin Lymphoma |
| HUO9 | 5.754 | Bone Cancer | Bone | Osteosarcoma |
| SNU1196 | 5.754 | Bile Duct Cancer | Bile Duct | Cholangiocarcinoma |
| HS766T | 5.752 | Pancreatic Cancer | Pancreas | Exocrine |
| RPE1SS6 | 5.743 | Non-Cancerous | Eye | |
| NCIH322 | 5.741 | Lung Cancer | Lung | NSCLC |
| HOTHC | 5.740 | Thyroid Cancer | Thyroid | Thyroid Carcinoma |
| NCIH23 | 5.740 | Lung Cancer | Lung | NSCLC |
| FLO1 | 5.738 | Esophageal Cancer | Esophagus Central Nervous | Esophagus Adenocarcinoma |
| DKMG | 5.736 | Brain Cancer | System | Glioma |
| JVM3 | 5.732 | Leukemia | Blood | CLL |
| SNU840 | 5.731 | Ovarian Cancer | Ovary | Brenner Tumor |
| MCC142 | 5.725 | Skin Cancer | Skin | Merkel Cell Carcinoma |
| | | Colon/Colorectal | | |
| KP363T | 5.716 | Cancer | Colorectal | Colorectal Adenocarcinoma |
| BCP1 | 5.715 | Lymphoma | Lymphocyte | Non Hodgkin Lymphoma |
| PACADD161 | 5.713 | Pancreatic Cancer | Pancreas | Exocrine |
| 127399 | 5.708 | Sarcoma | Soft Tissue | Synovial Sarcoma |
| MDAMB435S | 5.705 | Skin Cancer | Skin | Melanoma |
| | | Colon/Colorectal | | |
| NCIH716 | 5.703 | Cancer | Colorectal Central Nervous | Colorectal Adenocarcinoma |
| SF172 | 5.701 | Brain Cancer | System | Glioma |
| BT549 | 5.701 | Breast Cancer | Breast | Breast Ductal Carcinoma |
| JHH6 | 5.693 | Liver Cancer | Liver | Hepatocellular Carcinoma |
| RPMI8226 | 5.688 | Myeloma | Plasma Cell | Multiple Myeloma |
| WM983B | 5.686 | Skin Cancer | Skin Central Nervous | Melanoma |
| LN18 | 5.684 | Brain Cancer | System Central Nervous | Glioma |
| KNS81 | 5.682 | Brain Cancer | System | Glioma |
| NOZ | 5.663 | Gallbladder Cancer | Bile Duct | Gallbladder Adenocarcinoma |
| NCIH460 | 5.653 | Lung Cancer | Lung | NSCLC |
| MEL202 | 5.649 | Eye Cancer | Eye | Uveal Melanoma |
| MELHO | 5.648 | Skin Cancer | Skin | Melanoma |
| MESSA | 5.641 | Sarcoma | Soft Tissue | Uterine Sarcoma |
| | | Colon/Colorectal | | |
| C80 | 5.622 | Cancer | Colorectal | Colorectal Adenocarcinoma |
| HKA1 | 5.622 | Skin Cancer | Skin | Skin Squamous |
| FTC238 | 5.612 | Thyroid Cancer | Thyroid | Thyroid Carcinoma |
| NCIH1373 | 5.605 | Lung Cancer | Lung | NSCLC |
| SW756 | 5.601 | Cervical Cancer | Cervix | Cervical Squamous |
| NCIH1819 | 5.599 | Lung Cancer | Lung | NSCLC |
| | | Colon/Colorectal | | |
| SNU61 | 5.597 | Cancer | Colorectal | Colorectal Adenocarcinoma |
| OE19 | 5.594 | Esophageal Cancer | Esophagus | Esophagus Squamous |
| | | Colon/Colorectal | | |
| RCM1 | 5.592 | Cancer | Colorectal | Colorectal Adenocarcinoma |
| MTA | 5.592 | Lymphoma | Lymphocyte | Lymphoma Unspecified |
| | | Head and Neck | Upper | |
| H376 | 5.583 | Cancer | Aerodigestive | Upper Aerodigestive Squamous |
| ACHN | 5.581 | Kidney Cancer | Kidney | Renal Cell Carcinoma |
| TUHR4TKB | 5.580 | Kidney Cancer | Kidney | Renal Cell Carcinoma |
| SLVL | 5.579 | Lymphoma | Lymphocyte | Lymphoma Unspecified |
| MOLM14 | 5.563 | Leukemia | Blood | AML |
| GB2 | 5.559 | Gallbladder Cancer | Bile Duct Central Nervous | Gallbladder Adenocarcinoma |
| M059K | 5.559 | Brain Cancer | System Central Nervous | Glioma |
| CH157MN | 5.553 | Brain Cancer | System | Meningioma |
| LPS510 | 5.550 | Liposarcoma | Soft Tissue | Liposarcoma |
| | | Endometrial/Uteri | | |
| HEC6 | 5.542 | ne Cancer | Uterus | Endometrial Adenocarcinoma |
| CAL12T | 5.536 | Lung Cancer | Lung | NSCLC |
| MOLP2 | 5.532 | Myeloma | Plasma Cell | Multiple Myeloma |
| MINO | 5.529 | Lymphoma | Lymphocyte | Non Hodgkin Lymphoma |
| TE10 | 5.529 | Esophageal Cancer | Esophagus | Esophagus Squamous |
| KU1919 | 5.528 | Bladder Cancer | Urinary Tract | Bladder Carcinoma |
| TE6 | 5.517 | Esophageal Cancer | Esophagus | Esophagus Squamous |
| | | Colon/Colorectal | | |
| RKO | 5.502 | Cancer | Colorectal | Colorectal Adenocarcinoma |
| RS411 | 5.501 | Leukemia | Blood | ALL |
| SNU638 | 5.499 | Gastric Cancer | Gastric | Gastric Adenocarcinoma |
| BT474 | 5.494 | Breast Cancer | Breast | Breast Ductal Carcinoma |
| PANC0403 | 5.489 | Pancreatic Cancer | Pancreas | Exocrine |
| LPS853 | 5.484 | Liposarcoma | Soft Tissue | Liposarcoma |
| HS939T | 5.472 | Skin Cancer | Skin | Melanoma |
| SKMEL24 | 5.464 | Skin Cancer | Skin | Melanoma |
| NCIH2073 | 5.460 | Lung Cancer | Lung | NSCLC |
| MCAS | 5.456 | Ovarian Cancer | Ovary | Ovary Adenocarcinoma |
| COLO829 | 5.447 | Skin Cancer | Skin | Melanoma |
| HCC2429 | 5.446 | Lung Cancer | Lung | NSCLC |
| SKMEL30 | 5.443 | Skin Cancer | Skin | Melanoma |
| PANC1 | 5.434 | Pancreatic Cancer | Pancreas | Exocrine |
| KIJK | 5.433 | Lymphoma | Lymphocyte | Non Hodgkin Lymphoma |
| | | Colon/Colorectal | | |
| GP5D | 5.432 | Cancer | Colorectal | Colorectal Adenocarcinoma |
| | | Colon/Colorectal | | |
| TT1TKB | 5.432 | Cancer | Colorectal | Colorectal Adenocarcinoma |
| CCLFUPGI0005T | 5.427 | Gastric Cancer | Gastric | Gastric Adenocarcinoma |
| | | Colon/Colorectal | | |
| DLD1 | 5.417 | Cancer | Colorectal Central Nervous | Colorectal Adenocarcinoma |
| NP2 | 5.415 | Brain Cancer | System | Glioma |
| DAUDI | 5.415 | Lymphoma | Lymphocyte | Non Hodgkin Lymphoma |
| MM370 | 5.410 | Skin Cancer | Skin | Melanoma |
| 921 | 5.408 | Eye Cancer | Eye | Uveal Melanoma |
| KMS28PE | 5.405 | Myeloma | Plasma Cell | Multiple Myeloma |
| C4I | 5.402 | Cervical Cancer | Cervix | Cervical Carcinoma |
| | | Colon/Colorectal | | |
| NCIH508 | 5.401 | Cancer | Colorectal | Caecum Adenocarcinoma |
| OV90 | 5.397 | Ovarian Cancer | Ovary | Ovary Adenocarcinoma |
| | | Head and Neck | Upper | |
| UPCISCC026 | 5.397 | Cancer | Aerodigestive | Upper Aerodigestive Squamous |
| 93T449 | 5.395 | Liposarcoma | Soft Tissue | Liposarcoma |
| UO31 | 5.394 | Kidney Cancer | Kidney Central Nervous | Renal Cell Carcinoma |
| NP8 | 5.392 | Brain Cancer | System | Glioma |
| | | Colon/Colorectal | | |
| JVE253 | 5.382 | Cancer | Colorectal | Colorectal Adenocarcinoma |
| KYSE410 | 5.366 | Esophageal Cancer | Esophagus | Esophagus Squamous |
| MKN7 | 5.358 | Gastric Cancer | Gastric | Gastric Adenocarcinoma |
| NCIH2052 | 5.354 | Lung Cancer | Lung Central Nervous | Mesothelioma |
| ANGMCSS | 5.341 | Brain Cancer | System | Glioma |
| | | Colon/Colorectal | | |
| C75 | 5.322 | Cancer | Colorectal | Colorectal Adenocarcinoma |
| OCIP5X | 5.315 | Ovarian Cancer | Ovary | Ovary Adenocarcinoma |
| SLR20 | 5.313 | Bladder Cancer | Urinary Tract | Bladder Carcinoma |
| HCC461 | 5.307 | Lung Cancer | Lung | NSCLC |
| SUSA | 5.300 | Teratoma | Embryo | Teratoma |
| ICC3 | 5.298 | Bile Duct Cancer | Bile Duct | Cholangiocarcinoma |
| UMUC6 | 5.297 | Bladder Cancer | Urinary Tract | Bladder Carcinoma |
| SNU1 | 5.296 | Gastric Cancer | Gastric | Gastric Adenocarcinoma |
| GRANTA519 | 5.292 | Lymphoma | Lymphocyte Central Nervous | Non Hodgkin Lymphoma |
| YH13 | 5.283 | Brain Cancer | System | Glioma |
| HPAFII | 5.265 | Pancreatic Cancer | Pancreas | Exocrine |
| BOKU | 5.264 | Cervical Cancer | Cervix | Cervical Squamous |
| IALM | 5.248 | Lung Cancer | Lung | NSCLC |
| MOLP8 | 5.241 | Myeloma | Plasma Cell | Multiple Myeloma |
| COLO794 | 5.232 | Skin Cancer | Skin | Melanoma |
| | | Colon/Colorectal | | |
| T84 | 5.231 | Cancer | Colorectal | Colorectal Adenocarcinoma |
| RCK8 | 5.230 | Lymphoma | Lymphocyte Central Nervous | Lymphoma Unspecified |
| YKG1 | 5.230 | Brain Cancer | System Central Nervous | Glioma |
| BECKER | 5.217 | Brain Cancer | System | Glioma |
| | | Head and Neck | Upper | |
| YD10B | 5.217 | Cancer | Aerodigestive | Upper Aerodigestive Squamous |
| KATOIII | 5.190 | Gastric Cancer | Gastric | Gastric Adenocarcinoma |
| OCILY18 | 5.182 | Lymphoma | Lymphocyte | Non Hodgkin Lymphoma |
| DU4475 | 5.180 | Breast Cancer | Breast | Breast Carcinoma |
| RPE1SS51 | 5.172 | Non-Cancerous | Eye | |
| HS742T | 5.161 | Non-Cancerous | Fibroblast | Fibroblast Breast |
| | | Endometrial/Uteri | | |
| SKGII | 5.161 | ne Cancer | Uterus | Endometrial Squamous |
| SUM52PE | 5.146 | Breast Cancer | Breast | Breast Carcinoma |
| KE97 | 5.130 | Myeloma | Plasma Cell | Multiple Myeloma |
| CJM | 5.129 | Skin Cancer | Skin | Melanoma |
| KMS34 | 5.128 | Myeloma | Plasma Cell | Multiple Myeloma |
| A101D | 5.125 | Skin Cancer | Skin | Melanoma |
| NCIH841 | 5.119 | Lung Cancer | Lung | SCLC |
| CCSW1 | 5.119 | Bile Duct Cancer | Bile Duct | Cholangiocarcinoma |
| HS172T | 5.095 | Non-Cancerous | Fibroblast | Fibroblast Urinary Tract |
| KARPAS384 | 5.090 | Lymphoma | Lymphocyte | Lymphoma Unspecified |
| HCC2450 | 5.073 | Lung Cancer | Lung | NSCLC |
| | | Colon/Colorectal | | |
| C125PM | 5.060 | Cancer | Colorectal | Colorectal Adenocarcinoma |
| OE21 | 5.052 | Esophageal Cancer | Esophagus | Esophagus Squamous |
| | | Endometrial/Uteri | | |
| HEC251 | 5.051 | ne Cancer | Uterus | Endometrial Adenocarcinoma |
| OCIMY5 | 5.040 | Myeloma | Plasma Cell | Multiple Myeloma |
| CORL105 | 5.021 | Lung Cancer | Lung | NSCLC |
| NCIH1693 | 5.004 | Lung Cancer | Lung | NSCLC |
| | | Colon/Colorectal | | |
| C99 | 4.984 | Cancer | Colorectal | Colorectal Adenocarcinoma |
| C32 | 4.969 | Skin Cancer | Skin | Melanoma |
| | | Endometrial/Uteri | | |
| HOUAI | 4.960 | ne Cancer | Uterus | Endometrial Adenocarcinoma |
| SNU349 | 4.935 | Kidney Cancer | Kidney | Renal Cell Carcinoma |
| OV56 | 4.920 | Ovarian Cancer | Ovary | Ovary Adenocarcinoma |
| EKVX | 4.916 | Lung Cancer | Lung | NSCLC |
| WM3772F | 4.874 | Eye Cancer | Eye | Uveal Melanoma |
| ICC4 | 4.873 | Bile Duct Cancer | Bile Duct | Cholangiocarcinoma |
| PSS008 | 4.849 | Bone Cancer | Bone | Osteosarcoma |
| PACADD159 | 4.848 | Pancreatic Cancer | Pancreas | Exocrine |
| A2780 | 4.836 | Ovarian Cancer | Ovary | Ovary Adenocarcinoma |
| LPS6 | 4.829 | Liposarcoma | Soft Tissue | Liposarcoma |
| 253J | 4.823 | Bladder Cancer | Urinary Tract | Bladder Carcinoma |
| | | Colon/Colorectal | | |
| COLO201 | 4.820 | Cancer | Colorectal | Colorectal Adenocarcinoma |
| | | Endometrial/Uteri | | |
| HEC50B | 4.820 | ne Cancer | Uterus | Endometrial Adenocarcinoma |
| HT144 | 4.818 | Skin Cancer | Skin | Melanoma |
| NCIH1299 | 4.817 | Lung Cancer | Lung | NSCLC |
| KARPAS1718 | 4.813 | Lymphoma | Lymphocyte | Non Hodgkin Lymphoma |
| HCC364 | 4.810 | Lung Cancer | Lung | NSCLC |
| ICC10 | 4.810 | Bile Duct Cancer | Bile Duct | Cholangiocarcinoma |
| NCIH1975 | 4.801 | Lung Cancer | Lung | NSCLC |
| HS611T | 4.785 | Lymphoma | Lymphocyte Central Nervous | Hodgkin Lymphoma |
| H4 | 4.784 | Brain Cancer | System | Glioma |
| SW156 | 4.784 | Kidney Cancer | Kidney | Renal Cell Carcinoma |
| PEO4 | 4.782 | Ovarian Cancer | Ovary | Ovary Adenocarcinoma |
| | | Colon/Colorectal | | |
| LS1034 | 4.768 | Cancer | Colorectal | Colorectal Adenocarcinoma |
| CAL120 | 4.765 | Breast Cancer | Breast | Breast Carcinoma |
| CII | 4.741 | Leukemia | Blood | Unspecified Leukemia |
| RVH421SKINFV1 | 4.737 | Skin Cancer | Skin | Melanoma |
| MELJUSO | 4.737 | Skin Cancer | Skin Central Nervous | Melanoma |
| PFSK1 | 4.737 | Brain Cancer | System | PNET |
| PATU8988S | 4.734 | Pancreatic Cancer | Pancreas | Exocrine |
| KMH2 | 4.729 | Lymphoma | Lymphocyte | Hodgkin Lymphoma |
| SLR23 | 4.713 | Kidney Cancer | Kidney | Renal Cell Carcinoma |
| | | Head and Neck | Upper | |
| KON | 4.691 | Cancer | Aerodigestive | Upper Aerodigestive Squamous |
| 253JBV | 4.688 | Bladder Cancer | Urinary Tract | Bladder Carcinoma |
| NCCLMS1C1 | 4.679 | Unknown | Soft Tissue | Leiomyosarcoma |
| T3M5 | 4.676 | Thyroid Cancer | Thyroid | Thyroid Squamous |
| RO82W1 | 4.672 | Thyroid Cancer | Thyroid | Thyroid Carcinoma |
| MJ | 4.671 | Lymphoma | Lymphocyte | Non Hodgkin Lymphoma |
| A549 | 4.667 | Lung Cancer | Lung | NSCLC |
| SUM102PT | 4.666 | Breast Cancer | Breast | Breast Carcinoma |
| RCC10RGB | 4.664 | Kidney Cancer | Kidney | Renal Cell Carcinoma |
| | | Endometrial/Uteri | | |
| HEC108 | 4.662 | ne Cancer | Uterus | Endometrial Adenocarcinoma |
| WM3211 | 4.660 | Skin Cancer | Skin | Melanoma |
| SUM185PE | 4.653 | Breast Cancer | Breast Central Nervous | Breast Carcinoma |
| KNS60 | 4.641 | Brain Cancer | System | Glioma |
| AMO1 | 4.638 | Myeloma | Plasma Cell | Multiple Myeloma |
| NCIH2405 | 4.605 | Lung Cancer | Lung | NSCLC |
| CAKI2 | 4.603 | Kidney Cancer | Kidney | Renal Cell Carcinoma |
| IHH4 | 4.598 | Thyroid Cancer | Thyroid | Thyroid Carcinoma |
| TKKK | 4.593 | Bile Duct Cancer | Bile Duct | Cholangiocarcinoma |
| | | Colon/Colorectal | | |
| CW2 | 4.581 | Cancer | Colorectal | Colorectal Adenocarcinoma |
| NCIH2887 | 4.578 | Lung Cancer | Lung Central Nervous | NSCLC |
| LN428 | 4.556 | Brain Cancer | System | Glioma |
| | | Colon/Colorectal | | |
| HT115 | 4.540 | Cancer | Colorectal | Colorectal Adenocarcinoma |
| SKRC31 | 4.537 | Kidney Cancer | Kidney | Renal Cell Carcinoma |
| KARPAS620 | 4.520 | Myeloma | Plasma Cell | Multiple Myeloma |
| HCC366 | 4.513 | Lung Cancer | Lung | NSCLC |
| NCIH2172 | 4.498 | Lung Cancer | Lung | NSCLC |
| MM127 | 4.487 | Skin Cancer | Skin | Melanoma |
| LO68 | 4.485 | Lung Cancer | Lung | Mesothelioma |
| SW1271 | 4.481 | Lung Cancer | Lung | SCLC |
| KMS28BM | 4.478 | Myeloma | Plasma Cell | Multiple Myeloma |
| SNU216 | 4.474 | Gastric Cancer | Gastric | Gastric Adenocarcinoma |
| IGR37 | 4.452 | Skin Cancer | Skin | Melanoma |
| | | Colon/Colorectal | | |
| KM12 | 4.447 | Cancer | Colorectal Central Nervous | Colorectal Adenocarcinoma |
| KALS1 | 4.419 | Brain Cancer | System | Glioma |
| LP1 | 4.409 | Myeloma | Plasma Cell | Multiple Myeloma |
| | | Colon/Colorectal | | |
| SW1116 | 4.407 | Cancer | Colorectal Central Nervous | Colorectal Adenocarcinoma |
| LNZ308 | 4.396 | Brain Cancer | System | Glioma |
| OCIC5X | 4.393 | Ovarian Cancer | Ovary | Ovary Adenocarcinoma |
| | | Colon/Colorectal | | |
| COLO678 | 4.385 | Cancer | Colorectal | Colorectal Adenocarcinoma |
| | | Colon/Colorectal | | |
| SNU81 | 4.343 | Cancer | Colorectal | Colorectal Adenocarcinoma |
| HCC1359 | 4.308 | Lung Cancer | Lung | NSCLC |
| TTC709 | 4.297 | Rhabdoid | Soft Tissue | Malignant Rhabdoid Tumor |
| U266B1 | 4.259 | Myeloma | Plasma Cell | Multiple Myeloma |
| HS294T | 4.216 | Skin Cancer | Skin | Melanoma |
| TCCPAN2 | 4.201 | Pancreatic Cancer | Pancreas | Exocrine |
| A375 | 4.156 | Skin Cancer | Skin | Melanoma |
| WAOSEL | 4.155 | Leukemia | Blood | CLL |
| UMUC3 | 4.106 | Bladder Cancer | Urinary Tract | Bladder Carcinoma |
| A4FUK | 4.100 | Lymphoma | Lymphocyte | Non Hodgkin Lymphoma |
| A704 | 4.092 | Kidney Cancer | Kidney | Renal Cell Carcinoma |
| | | Endometrial/Uteri | | |
| HEC1 | 4.082 | ne Cancer | Uterus | Endometrial Adenocarcinoma |
| HT144SKINFV1 | 4.074 | Skin Cancer | Skin | Melanoma |
| HT1080 | 4.073 | Sarcoma | Soft Tissue | Fibrosarcoma |
| HLF | 4.040 | Liver Cancer | Liver | Hepatocellular Carcinoma |
| WM115 | 3.996 | Skin Cancer | Skin | Melanoma |
| SQ1 | 3.973 | Lung Cancer | Lung | NSCLC |
| | | Endometrial/Uteri | | |
| SNGM | 3.971 | ne Cancer | Uterus | Endometrial Adenocarcinoma |
| RAJI | 3.964 | Lymphoma | Lymphocyte | Non Hodgkin Lymphoma |
| KHYG | 3.955 | Lymphoma | Lymphocyte | Lymphoma Unspecified |
| IGR39 | 3.923 | Skin Cancer | Skin | Melanoma |
| KMS12BM | 3.914 | Myeloma | Plasma Cell | Multiple Myeloma |
| EHEB | 3.903 | Lymphoma | Lymphocyte | Lymphoma Unspecified |
| WM88 | 3.831 | Skin Cancer | Skin | Melanoma |
| SUM149PT | 3.818 | Breast Cancer | Breast | Breast Ductal Carcinoma |
| KMCH1 | 3.797 | Bile Duct Cancer | Bile Duct | Cholangiocarcinoma |
| | | Head and Neck | Upper | |
| HSQ89 | 3.788 | Cancer | Aerodigestive | Upper Aerodigestive Squamous |
| PANC1005 | 3.776 | Pancreatic Cancer | Pancreas | Exocrine |
| A375SKINCJ2 | 3.766 | Skin Cancer | Skin | Melanoma |
| RVH421 | 3.759 | Skin Cancer | Skin | Melanoma |
| HCC1395 | 3.752 | Breast Cancer | Breast | Breast Ductal Carcinoma |
| CASKI | 3.748 | Cervical Cancer | Cervix | Cervical Squamous |
| S117 | 3.709 | Sarcoma | Soft Tissue | Thyroid Sarcoma |
| A375SKINCJ3 | 3.696 | Skin Cancer | Skin | Melanoma |
| OVMANA | 3.669 | Ovarian Cancer | Ovary | Ovary Adenocarcinoma |
| KMRC2 | 3.655 | Kidney Cancer | Kidney | Renal Cell Carcinoma |
| CORL23 | 3.639 | Lung Cancer | Lung | NSCLC |
| JMSU1 | 3.619 | Bladder Cancer | Urinary Tract | Bladder Carcinoma |
| KML1 | 3.606 | Lymphoma | Lymphocyte | Non Hodgkin Lymphoma |
| HT144SKINFV3 | 3.604 | Skin Cancer | Skin | Melanoma |
| | | Endometrial/Uteri | | |
| SKGI | 3.587 | ne Cancer | Uterus | Endometrial Squamous |
| | | Endometrial/Uteri | | |
| HEC116 | 3.573 | ne Cancer | Uterus | Endometrial Adenocarcinoma |
| MM383 | 3.543 | Skin Cancer | Skin | Melanoma |
| NCCSTCK140 | 3.541 | Gastric Cancer | Gastric | Gastric Adenocarcinoma |
| | | Colon/Colorectal | | |
| C84 | 3.507 | Cancer | Colorectal | Colorectal Adenocarcinoma |
| SKMM2 | 3.502 | Myeloma | Plasma Cell | Multiple Myeloma |
| A375SKINCJ1 | 3.480 | Skin Cancer | Skin | Melanoma |
| NCIH1581 | 3.471 | Lung Cancer | Lung | NSCLC |
| NCIH929 | 3.465 | Myeloma | Plasma Cell | Multiple Myeloma |
| HCC44 | 3.459 | Lung Cancer | Lung Central Nervous | NSCLC |
| SF295 | 3.444 | Brain Cancer | System | Glioma |
| 2313287 | 3.438 | Gastric Cancer | Gastric | Gastric Adenocarcinoma |
| G415 | 3.432 | Gallbladder Cancer | Bile Duct | Gallbladder Adenocarcinoma |
| MM485 | 3.430 | Skin Cancer | Skin | Melanoma |
| WM2664 | 3.424 | Skin Cancer | Skin | Melanoma |
| SLR21 | 3.410 | Kidney Cancer | Kidney Central Nervous | Renal Cell Carcinoma |
| UW228 | 3.395 | Brain Cancer | System | Medulloblastoma |
| FTC133 | 3.390 | Thyroid Cancer | Thyroid | Thyroid Carcinoma |
| VMRCRCW | 3.376 | Kidney Cancer | Kidney | Renal Cell Carcinoma |
| TGBC1TKB | 3.355 | Gallbladder Cancer | Bile Duct | Gallbladder Adenocarcinoma |
| KMRC1 | 3.306 | Kidney Cancer | Kidney | Renal Cell Carcinoma |
| HS578T | 3.281 | Breast Cancer | Breast | Breast Carcinoma |
| HT144SKINFV2 | 3.274 | Skin Cancer | Skin | Melanoma |
| NCIH1869 | 3.237 | Lung Cancer | Lung | NSCLC |
| SKMEL28 | 3.227 | Skin Cancer | Skin | Melanoma |
| HUT102 | 3.225 | Lymphoma | Lymphocyte | Lymphoma Unspecified |
| ICC9 | 3.197 | Bile Duct Cancer | Bile Duct | Cholangiocarcinoma |
| | | Endometrial/Uteri | | |
| HHUA | 3.126 | ne Cancer | Uterus | Endometrial Adenocarcinoma |
| CCLP1 | 3.093 | Bile Duct Cancer | Bile Duct | Cholangiocarcinoma |
| EBC1 | 3.072 | Lung Cancer | Lung | NSCLC |
| A2058 | 3.069 | Skin Cancer | Skin | Melanoma |
| HT1197 | 3.057 | Bladder Cancer | Urinary Tract | Bladder Carcinoma |
| NCIH522 | 3.055 | Lung Cancer | Lung | NSCLC |
| | | Head and Neck | Upper | |
| UPCISCC116 | 3.032 | Cancer | Aerodigestive Central Nervous | Upper Aerodigestive Squamous |
| U118MG | 3.021 | Brain Cancer | System | Glioma |
| TCCSUP | 3.007 | Bladder Cancer | Urinary Tract | Bladder Carcinoma |
| TUHR14TKB | 2.918 | Kidney Cancer | Kidney | Renal Cell Carcinoma |
| OVTOKO | 2.860 | Ovarian Cancer | Ovary | Ovary Adenocarcinoma |
| RBE | 2.836 | Bile Duct Cancer | Bile Duct | Cholangiocarcinoma |
| COV434 | 2.770 | Ovarian Cancer | Ovary | Ovary Adenocarcinoma |
| TUHR10TKB | 2.740 | Kidney Cancer | Kidney | Renal Cell Carcinoma |
| MM386 | 2.733 | Skin Cancer | Skin | Melanoma |
| ASH3 | 2.731 | Thyroid Cancer | Thyroid | Thyroid Carcinoma |
| 769P | 2.696 | Kidney Cancer | Kidney | Renal Cell Carcinoma |
| EJM | 2.667 | Myeloma | Plasma Cell Central Nervous | Multiple Myeloma |
| ONDA9 | 2.635 | Brain Cancer | System | Glioma |
| NCIH661 | 2.449 | Lung Cancer | Lung | NSCLC |
| SUPT11 | 2.409 | Leukemia | Blood | ALL |
| KKU100 | 2.398 | Bile Duct Cancer | Bile Duct | Cholangiocarcinoma |
| HUTU80 | 2.287 | Gastric Cancer | Gastric | Duodenal Adenocarcinoma |
| LOUNH91 | 2.260 | Lung Cancer | Lung | NSCLC |
| SNU245 | 2.251 | Bile Duct Cancer | Bile Duct Central Nervous | Cholangiocarcinoma |
| F5 | 2.217 | Brain Cancer | System | Meningioma |
| | | Endometrial/Uteri | | |
| HTMMT | 2.189 | ne Cancer | Uterus | MMMT |
| OCILY132 | 2.183 | Lymphoma | Lymphocyte | Lymphoma Unspecified |
| MIAPACA2 | 2.167 | Pancreatic Cancer | Pancreas | Exocrine |

**Table 5: Protein relative expression value as available on Cancer Genome Atlas**

| Cell Line Name | Primary Disease | Lineage Subtype | Protein Expression | RNA |
|---|---|---|---|---|
| JHH1 | Liver Cancer | Hepatocellular Carcinoma | 3.680 | 8.12 |
| KP2 | Pancreatic Cancer | Exocrine | 3.553 | 8.65 |
| EFM19 | Breast Cancer | Breast Ductal Carcinoma | 3.275 | 9.08 |
| J82 | Bladder Cancer | Bladder Carcinoma Malignant Rhabdoid | 3.078 | 6.19 |
| G401 | Rhabdoid | Tumor | 2.963 | 9.42 |
| HEL | Leukemia | AML | 2.908 | 9.82 |
| F36P | Leukemia | AML | 2.808 | 9.18 |
| 697 | Leukemia | ALL | 2.751 | 8.72 |
| U937 | Leukemia | AML | 2.748 | 9.86 |
| | Endometrial/Uterine | Endometrial | | |
| JHUEM2 | Cancer | Adenocarcinoma | 2.705 | 8.38 |
| PC14 | Lung Cancer | NSCLC | 2.649 | 7.88 |
| KARPAS299 | Lymphoma | Non Hodgkin Lymphoma | 2.536 | 8.19 |
| CORL88 | Lung Cancer | SCLC | 2.464 | 9.58 |
| CMK | Leukemia | AML | 2.459 | 10.59 |
| NCIH2291 | Lung Cancer | NSCLC | 2.450 | 6.80 |
| ISHIKAWAHE | Endometrial/Uterine | Endometrial | | |
| RAKLIO02ER | Cancer | Adenocarcinoma | 2.410 | 7.95 |
| AU565 | Breast Cancer | Breast Adenocarcinoma | 2.301 | 9.44 |
| NCIH1650 | Lung Cancer | NSCLC | 2.290 | 7.60 |
| EFM192A | Breast Cancer | Breast Adenocarcinoma | 2.271 | 9.96 |
| NALM6 | Leukemia | ALL | 2.240 | 7.79 |
| NCIH1155 | Lung Cancer | NSCLC | 2.234 | 7.76 |
| HT1376 | Bladder Cancer | Bladder Carcinoma | 2.227 | 9.87 |
| VCAP | Prostate Cancer | Prostate Adenocarcinoma | 2.203 | 7.10 |
| NCIH2066 | Lung Cancer | SCLC Colorectal | 2.157 | |
| SW1417 | Colon/Colorectal Cancer | Adenocarcinoma | 2.154 | 7.78 |
| HUH7 | Liver Cancer | Hepatocellular Carcinoma | 2.139 | 8.74 |
| SKES1 | Bone Cancer | Ewing Sarcoma | 2.028 | 8.09 |
| HCC70 | Breast Cancer | Breast Ductal Carcinoma | 1.999 | 9.29 |
| NCIH2126 | Lung Cancer | NSCLC | 1.928 | 10.61 |
| HEPG2 | Liver Cancer | Hepatocellular Carcinoma Colorectal | 1.925 | 8.74 |
| LS411N | Colon/Colorectal Cancer | Adenocarcinoma | 1.899 | 7.38 |
| CORL47 | Lung Cancer | SCLC | 1.846 | 7.68 |
| NCIH2122 | Lung Cancer | NSCLC | 1.842 | 7.56 |
| HDQP1 | Breast Cancer | Breast Ductal Carcinoma | 1.828 | 8.16 |
| NCIH446 | Lung Cancer | SCLC | 1.807 | 9.10 |
| HEP3B217 | Liver Cancer | Hepatocellular Carcinoma | 1.771 | 7.92 |
| NCIH2170 | Lung Cancer | NSCLC | 1.769 | 7.59 |
| CHAGOK1 | Lung Cancer | NSCLC | 1.728 | 9.50 |
| NCIH647 | Lung Cancer | NSCLC | 1.726 | 7.68 |
| NCIH1703 | Lung Cancer | NSCLC | 1.719 | 6.25 |
| DANG | Pancreatic Cancer | Exocrine | 1.718 | 7.44 |
| EOL1 | Leukemia | AML | 1.678 | 8.98 |
| HS944T | Skin Cancer | Melanoma | 1.606 | 6.90 |
| MDAMB468 | Breast Cancer | Breast Carcinoma | 1.600 | 8.37 |
| ZR751 | Breast Cancer | Breast Ductal Carcinoma | 1.598 | 8.55 |
| DU145 | Prostate Cancer | Prostate Adenocarcinoma | 1.594 | |
| JEKO1 | Lymphoma | Non Hodgkin Lymphoma Colorectal | 1.589 | 7.24 |
| CCK81 | Colon/Colorectal Cancer | Adenocarcinoma | 1.574 | 7.69 |
| CALU6 | Lung Cancer | NSCLC | 1.552 | 7.27 |
| NCIH2110 | Lung Cancer | NSCLC | 1.520 | 8.68 |
| SU8686 | Pancreatic Cancer | Exocrine | 1.498 | 7.36 |
| DMS273 | Lung Cancer | SCLC Colorectal | 1.449 | 8.21 |
| SNUC5 | Colon/Colorectal Cancer | Adenocarcinoma | 1.446 | 6.32 |
| HCC1937 | Breast Cancer | Breast Ductal Carcinoma | 1.439 | 7.97 |
| LNCAPCLONEFGC | Prostate Cancer | Prostate Adenocarcinoma | 1.407 | 7.41 |
| CAL27 | Head and Neck Cancer | Upper Aerodigest. Squam. | 1.378 | 9.37 |
| HUH6 | Liver Cancer | Hepatoblastoma | 1.374 | 7.74 |
| OCILY3 | Lymphoma | Non Hodgkin Lymphoma | 1.372 | 7.23 |
| K562 | Leukemia | CML | 1.372 | 9.95 |
| NCIH292 | Lung Cancer | NSCLC | 1.322 | 7.41 |
| CAL51 | Breast Cancer | Breast Carcinoma | 1.308 | 8.57 |
| KYSE30 | Esophageal Cancer | Esophagus Squamous | 1.299 | 8.07 |
| SKMEL3 | Skin Cancer | Melanoma | 1.297 | 6.68 |
| ABC1 | Lung Cancer | NSCLC | 1.273 | 8.51 |
| RCHACV | Leukemia | ALL | 1.269 | 8.40 |
| COLO679 | Skin Cancer | Melanoma | 1.265 | 7.26 |
| JHH7 | Liver Cancer | Hepatocellular Carcinoma | 1.259 | 8.59 |
| MDAMB453 | Breast Cancer | Breast Carcinoma | 1.217 | 6.62 |
| MCF7 | Breast Cancer | Breast Carcinoma | 1.216 | 6.57 |
| JHH5 | Liver Cancer | Hepatocellular Carcinoma | 1.209 | 8.42 |
| KYSE180 | Esophageal Cancer | Esophagus Squamous | 1.200 | 7.36 |
| HUPT4 | Pancreatic Cancer | Exocrine Upper Aerodigestive | 1.197 | 8.95 |
| FADU | Head and Neck Cancer | Squamous | 1.182 | 7.46 |
| MKN45 | Gastric Cancer | Gastric Adenocarcinoma | 1.160 | 7.36 |
| 22RV1 | Prostate Cancer | Prostate Adenocarcinoma | 1.152 | 7.47 |
| NCIH1435 | Lung Cancer | NSCLC Colorectal | 1.151 | 6.09 |
| HCT116 | Colon/Colorectal Cancer | Adenocarcinoma | 1.126 | 7.20 |
| NCIH3255 | Lung Cancer | NSCLC | 1.122 | 7.15 |
| RH41 | Sarcoma | Rhabdomyosarcoma | 1.073 | 7.51 |
| BXPC3 | Pancreatic Cancer | Exocrine | 1.067 | 9.29 |
| KYSE450 | Esophageal Cancer | Esophagus Squamous | 1.061 | 7.60 |
| OCIAML5 | Leukemia | AML | 1.054 | 10.20 |
| KYSE150 | Esophageal Cancer | Esophagus Squamous Colorectal | 1.035 | 7.89 |
| HT29 | Colon/Colorectal Cancer | Adenocarcinoma | 1.030 | 7.44 |
| KMS11 | Myeloma | Multiple Myeloma | 1.020 | 6.83 |
| NCIH196 | Lung Cancer | SCLC Colorectal | 1.019 | 7.33 |
| HCT15 | Colon/Colorectal Cancer | Adenocarcinoma | 0.957 | 6.29 |
| KASUMI2 | Leukemia | ALL Colorectal | 0.954 | 8.88 |
| HCC56 | Colon/Colorectal Cancer | Adenocarcinoma | 0.951 | 8.15 |
| IGR1 | Skin Cancer | Melanoma | 0.947 | 6.02 |
| NCIH1944 | Lung Cancer | NSCLC | 0.947 | 7.58 |
| HUH1 | Liver Cancer | Hepatocellular Carcinoma Colorectal | 0.940 | 6.88 |
| SW620 | Colon/Colorectal Cancer | Adenocarcinoma | 0.927 | 6.99 |
| HCC1806 | Breast Cancer | Breast Ductal Carcinoma | 0.911 | 8.25 |
| NCIH1048 | Lung Cancer | SCLC | 0.909 | 9.73 |
| KMRC20 | Kidney Cancer | Renal Cell Carcinoma | 0.909 | 6.97 |
| SKMEL5 | Skin Cancer | Melanoma Colorectal | 0.906 | 6.76 |
| SKCO1 | Colon/Colorectal Cancer | Adenocarcinoma | 0.874 | 7.15 |
| JHH4 | Liver Cancer | Hepatocellular Carcinoma Colorectal | 0.866 | 6.21 |
| SNUC2A | Colon/Colorectal Cancer Endometrial/Uterine | Adenocarcinoma Endometrial | 0.845 | 8.41 |
| HEC59 | Cancer | Adenocarcinoma | 0.835 | 8.51 |
| NCIH226 | Lung Cancer | NSCLC | 0.824 | 6.99 |
| HCC1500 | Breast Cancer | Breast Ductal Carcinoma | 0.806 | 8.28 |
| HUPT3 | Pancreatic Cancer | Exocrine Colorectal | 0.752 | 7.03 |
| SW837 | Colon/Colorectal Cancer | Adenocarcinoma Colorectal | 0.736 | 7.05 |
| NCIH716 | Colon/Colorectal Cancer | Adenocarcinoma | 0.707 | 5.70 |
| SEM | Leukemia | ALL | 0.704 | 8.01 |
| IPC298 | Skin Cancer | Melanoma | 0.700 | 6.25 |
| MKN1 | Gastric Cancer | Gastric Adenocarcinoma | 0.690 | 6.21 |
| SKNAS | Neuroblastoma | Neuroblastoma | 0.687 | 7.26 |
| COV362 | Ovarian Cancer | Ovary Adenocarcinoma | 0.663 | 7.35 |
| TE11 | Esophageal Cancer | Esophagus Squamous | 0.637 | 7.15 |
| FUOV1 | Ovarian Cancer | Ovary Adenocarcinoma | 0.619 | 7.02 |
| TE4 | Esophageal Cancer | Esophagus Squamous | 0.601 | 6.58 |
| BT20 | Breast Cancer | Breast Ductal Carcinoma | 0.573 | 6.34 |
| RD | Sarcoma | Rhabdomyosarcoma | 0.573 | 7.74 |
| DV90 | Lung Cancer | NSCLC Colorectal | 0.571 | 6.83 |
| SW948 | Colon/Colorectal Cancer | Adenocarcinoma | 0.547 | 7.16 |
| JM1 | Leukemia | ALL | 0.519 | 8.25 |
| HCC95 | Lung Cancer | NSCLC | 0.517 | 9.88 |
| PC3 | Prostate Cancer | Prostate Adenocarcinoma | 0.512 | 6.26 |
| NCIH2009 | Lung Cancer | NSCLC Colorectal | 0.512 | 6.26 |
| LS513 | Colon/Colorectal Cancer | Adenocarcinoma | 0.499 | 6.58 |
| SNU119 | Ovarian Cancer | Ovary Adenocarcinoma | 0.457 | 7.45 |
| KASUMI1 | Leukemia | AML | 0.455 | 7.84 |
| KATOIII | Gastric Cancer | Gastric Adenocarcinoma | 0.453 | 5.19 |
| NCIH358 | Lung Cancer | NSCLC | 0.439 | 6.00 |
| PANC0203 | Pancreatic Cancer | Exocrine | 0.427 | 6.23 |
| DMS114 | Lung Cancer | SCLC | 0.384 | 7.55 |
| RT4 | Bladder Cancer | Bladder Carcinoma | 0.381 | 8.43 |
| TF1 | Leukemia | AML | 0.363 | 9.63 |
| DAOY | Brain Cancer | Medulloblastoma Colorectal | 0.355 | 6.68 |
| CL34 | Colon/Colorectal Cancer | Adenocarcinoma | 0.351 | 6.80 |
| KP4 | Pancreatic Cancer | Exocrine | 0.329 | 6.91 |
| MSTO211H | Lung Cancer Endometrial/Uterine | Mesothelioma Endometrial | 0.310 | 6.10 |
| HEC50B | Cancer | Adenocarcinoma | 0.305 | 4.82 |
| KYSE70 | Esophageal Cancer Endometrial/Uterine | Esophagus Squamous Endometrial | 0.293 | 7.72 |
| MFE296 | Cancer | Adenocarcinoma Colorectal | 0.292 | 7.73 |
| SW403 | Colon/Colorectal Cancer | Adenocarcinoma | 0.271 | 6.89 |
| HCC1187 | Breast Cancer | Breast Ductal Carcinoma | 0.264 | 8.01 |
| JHH6 | Liver Cancer | Hepatocellular Carcinoma | 0.258 | 5.69 |
| SHP77 | Lung Cancer | SCLC | 0.253 | 7.09 |
| SNB19 | Brain Cancer | Glioma Upper Aerodigestive | 0.252 | |
| BICR6 | Head and Neck Cancer | Squamous | 0.243 | 8.01 |
| MEWO | Skin Cancer | Melanoma | 0.233 | 6.89 |
| UACC257 | Skin Cancer | Melanoma | 0.229 | 6.39 |
| RMUGS | Ovarian Cancer | Ovary Adenocarcinoma | 0.224 | 5.87 |
| NCIN87 | Gastric Cancer | Gastric Adenocarcinoma | 0.217 | 7.24 |
| U87MG | Brain Cancer | Glioma Colorectal | 0.213 | 6.23 |
| LS180 | Colon/Colorectal Cancer | Adenocarcinoma | 0.191 | 7.51 |
| GB1 | Brain Cancer | Glioma | 0.190 | 5.84 |
| MOLM16 | Leukemia | AML | 0.186 | 9.20 |
| NUGC3 | Gastric Cancer | Gastric Adenocarcinoma | 0.180 | 6.57 |
| T24 | Bladder Cancer | Bladder Carcinoma | 0.178 | 7.47 |
| KYSE510 | Esophageal Cancer | Esophagus Squamous | 0.175 | 6.87 |
| HEL9217 | Leukemia | AML | 0.154 | 8.68 |
| JHOS2 | Ovarian Cancer | Ovary Adenocarcinoma | 0.147 | 7.37 |
| 8505C | Thyroid Cancer | Thyroid Carcinoma | 0.111 | 6.43 |
| TE1 | Esophageal Cancer | Esophagus Squamous | 0.108 | 7.02 |
| SW1783 | Brain Cancer | Glioma | 0.106 | 6.42 |
| HCC38 | Breast Cancer | Breast Ductal Carcinoma | 0.099 | 8.21 |
| SW1573 | Lung Cancer | NSCLC | 0.090 | 6.68 |
| SNU423 | Liver Cancer | Hepatocellular Carcinoma | 0.068 | 6.74 |
| OV90 | Ovarian Cancer | Ovary Adenocarcinoma | 0.063 | 5.40 |
| PECAPJ34 | | Upper Aerodigestive | | |
| CLONEC12 | Head and Neck Cancer | Squamous | 0.059 | 8.24 |
| A2780 | Ovarian Cancer | Ovary Adenocarcinoma | 0.018 | 4.84 |
| TUHR4TKB | Kidney Cancer | Renal Cell Carcinoma | 0.000 | 5.58 |

## Claims

1. An *in vitro* method for evaluating whether a substance is a glutamine synthetase modulator in mammal cells, said method comprising:
(a) culturing mammal cells in presence or absence of at least one candidate substance whose ability to act as glutamine synthetase modulator is sought to be determined;
(b) stopping the culturing of the cells of step (a);
(c) determining levels of one or more glutamine markers;
(d) comparing levels of said one or more glutamine marker in the cells cultured with the candidate substance to those cells cultured without said candidate substance;
(e) identifying the candidate substance as glutamine synthetase modulator on the basis of the comparison at step (d), and
wherein the glutamine synthetase modulator is a glutamine synthetase inhibitor or a glutamine synthetase activator.

2. The method of claim 1, wherein the one or more glutamine marker is selected from the group comprising citrate, a fatty acid-derived lipid, and a metabolite derived from the mevalonate pathway downstream of HMGCR, and
wherein step (e) comprises:
identifying the tested candidate substance as a glutamine synthetase inhibitor if the level of citrate in the cells cultured with said candidate substance is at least 10% lower compared to cells cultured without said candidate substance, and/or the level of said fatty acid-derived lipid in the cells cultured with said candidate substance is at least 10% lower compared to cells cultured without said candidate substance and/or the level of the metabolite derived from the mevalonate pathway downstream of HMGCR in the cells cultured with said candidate substance is at least 10% lower compared to cells cultured without said candidate substance, or
identifying the tested candidate substance as a glutamine synthetase activator if the level of citrate in the cells cultured with said candidate substance is at least 10% higher to cells cultured without said candidate substance, and/or the level of said fatty acid-derived lipid in the cells cultured with said candidate substance is at least 10% higher compared to cells cultured without said candidate substance and/or the level of the metabolite derived from the mevalonate pathway downstream of HMGCR in the cells cultured with said candidate substance is at least 10% higher compared to cells cultured without said candidate substance.

3. The method of claim 2, wherein the metabolite derived from the mevalonate pathway downstream of HMGCR is selected from the group comprising: HMG-CoA, mevalonate, mevalonate-5-P, mevalonate-5-PP, Isopentyl-5-PP, farnesyl-PP, squalene, lanosterol, dihydrolanosterol (DHL), desmosterol, epoxycholesterol (EC), dehydrocholesterols, cholesterol, geranyl-PP, geranylgeranyl-PP, dolicyl-PP, dolichol, coenzyme Q10, Vitamin K2, Vitamin D, steroid hormones, bile acid, Heme A; and/or
wherein the fatty acid-derived lipid is selected from the group comprising a fatty acid with a carbon chain length between 16 and 24 carbon atoms, a phospholipid comprising acyl groups with a carbon chain length between 16 and 24 carbon atoms, a diacylglycerol comprising acyl groups with a carbon chain length between 16 and 24 carbon atoms, a triglyceride comprising acyl groups with a carbon chain length between 16 and 24 carbon atoms, and a sphingolipid comprising acyl groups with a carbon chain length between 16 and 24 carbon atoms.

4. The method of claim 2 or 3, wherein the fatty acid -derived lipid is selected from the group comprising palmitic acid, a phosphatidylcholine, phosphatidylserine, phosphatidylethanoline, phosphatidylglycerol, phosphatidylinositol, diacylglycerol, triglyceride, sphingomyelin, wherein said phosphatidylcholine, phosphatidylserine, phosphatidylethanoline, phosphatidylglycerol, phosphatidylinositol, diacylglycerol, triglyceride, and sphingomyelin, comprise acyl groups with a carbon chain length between 16 and 24 carbon atoms.

5. The method of anyone of the claims 1 - 4, wherein the one or more glutamine marker is a glutamine stimulated mRNA selected from the group comprising *FASN,* HSPA1A, CAVIN2, HSPA1B, HMGCR, JAKMIP2, NYNRIN, SH3TC2, INSIG1, ZBED2, CD180, TNFRSF11B, FAM111B, PNPLA3, THBS1, BMP4, MIAT, HSPA8, SORBS2, MAP1A, EPHB3, NES, LINC02615, SLC2A12, DPYSL2, HSPB1, CENPI, SAMD9L, TMEM97, FDFT1, HMMR, GAS6-AS1, ANXA6, BLACAT1, RHOBTB1, SQLE, PCYT2, TET1, TRIB2, MSMO1, NSDHL, ITGB8, CRISPLD1, UACA, ROR1, LSS, SYNC, LPCAT1, IDI1, PIK3R3, SOX2, MVK, PDGFB, SNAI3-AS1, DUBR, CSGALNACT1, PLD5P1, TUBA1A, IGSF10, SAMD9, SNX21, TNFSF9, ACSS2, PARP9, RTKN, PLCH1, NREP, ZMYM3, HEXIM1, MAGED1, TTC3P1, PGP, SLCO4C1, USP51, DHCR24, and/or a glutamine inhibited mRNA selected from the group comprising TRIB3, UPP1, SPX, TSLP, MAFF, CLDN1, MYC, ATF3, RAB39B, CREB5, PPP1R15A, ERRFI1, ALOXE3, GADD45A, CDC42EP1, CTH, SESN2, ARC, DDIT3, CHRNA9, BTG1, KDM7A, SLC22A15, N4BP3, EGR1, DDIT4, NR1D1, ESRP1, INHBE, CXCL8, PTGS2, HK2, KDM6B, AREG, TNFRSF9, MYCT1, GEM, CHIC2, SNHG17, ASNS, FOSL1, FLNC, CD274, FHL2, FAM107B, SERPINE1, CCL4, ZNF697, EPAS1, PER2, ITPRIP, MTHFD2, ROCK1P1, MXD1, CEBPB, UNC5B, MAP2K3, RELB, ARHGEF2, PTX3, AKNATRIM25, NSC, SLC7A1, LIMA1, CHAC1, GPAT3, SLC38A1, EGFR, CNKSR3, RPLP0P2, LAT2, BEND7, TNFAIP3, ETV5, UHRF1BP1, SLC3A2, NFIL3, ABCB1, FIBIN, LINC01667, GPT2, RASGEF1C, LDAF1, IL1RL1, GDF15, NGF, NR4A1, TAF4B, SNHG1, GOT1, SLC6A9, ARID5A, SPNS2, CEBPG, LETM2, ADM2, EPB41L4A-AS1, MMP3, SNAPC1, DUSP2, NUPR1, VEGFA, OSBPL6, MAP1LC3B, SYNE1, FAM83G, PKD1P6, KCTD16, LINC00973, DUSP1, EBF3, HRK, PER1, CLUHP4, RAPH1, SPHK1, PKD1P1, PSAT1, SARS1, FGF21, DUSP5, IL6R, PRKCE, ABL2, GARS1, TSC22D3, PXK, TXNIP, SINHCAF, FGF2, FAM167A, NFATC1, SH3TC1, UAP1, RCL1, SLC1A5, SPRR2D, SLC1A4, SLC30A1, C3orf52, SMOX, CECR2, GABARAPL1, SYP, CBARP, ETS1, SNHG15, PFDN2, NIPAL4, LONRF2, IL31RA, EIF1B, CDO1, IL11, SERPINB8, SLC9A1, CSRNP1, CARS1, FUT1, RND3, TNFRSF10B, OLIG2, FAM241A, WDR43, DDR2, VGLL3, CYTOR, HBEGF, DUSP8, KLF10, ATXN1, DAGLB, AVPI1, GTPBP2, TMEM268, SH2B3, FBXO31, B3GALT5, SPOCD1, TIMM44, PHLDA2, SDC4, AEN, EGR2, MIDEAS, TRIB1, CYLD, CDK7, MOCOS, STK40, PTHLH, PCID2, ZFPM2-AS1, GPR137B, GAS5, SGMS2, DDX21, EREG, FYN, ZFAS1, BEX2, FOXO3, YARS1, ICOSLG, FKBP9, DUX4L26, IRF2BP2, PSPH,
and wherein step (e) further comprises:
identifying the tested candidate substance as glutamine synthetase inhibitor if the level of a glutamine inhibited mRNA in the cells cultured with the candidate substance is higher than + 1.5 and/or the level of a glutamine stimulated mRNA in the cells cultured with the candidate substance is lower than 1.5 compared to cells cultured without said candidate substance; or
identifying the tested candidate substance as glutamine synthetase activator if the level of a glutamine inhibited mRNA in the cells cultured with the candidate substance is lower than - 1.5 and/or the level of a glutamine stimulated mRNA in the cells cultured with the candidate substance is higher than 1.5 compared to cells cultured without said candidate substance; and wherein the level of said mRNA is calculated as log2FC between the cells cultured with said candidate substance and the cells cultured without said candidate substance.

6. The method of claim 5, wherein the one or more glutamine marker is glutamine stimulated mRNA *HMGCR.*

7. The method of anyone of the claims 1 - 6, wherein the one or more glutamine marker is a glutamine stimulated protein selected from SREBP2, HMGCR, FDFT1, c-MYC, FASN, HMGCS1, FDPS, LDLR and LSS, and wherein step (e) further comprises:
identifying the tested candidate substance as a glutamine synthetase inhibitor if the level of said "glutamine- stimulated proteins" is at least 10% lower compared to cells cultured without said candidate substance, or
identifying the tested candidate substance as a glutamine synthetase activator if the level of said "glutamine-- stimulated proteins" is at least 10% higher compared to cells cultured without said candidate substance.

8. The method of claim 7, wherein step (c) further comprises determining proteolytic activation of the glutamine stimulated protein and/or the glutamine stimulated protein is SREBP2.

9. The method of any one of the claims 1 - 8, wherein the mammal cells are mammal cell lines having a glutamine synthetase protein relative expression value higher than 0.5 and/or a glutamine synthetase mRNA expression value higher than 2.0, wherein the mRNA expression value is calculated as log2 of transcripts per million.

10. The method of any one of the claims 1 - 9, wherein the mammal cells are HepG2.

11. A glutamine synthetase inhibitor identifiable by the *in vitro* method of any one of the claims 1 - 10 for use in treatment of a disease associated with high levels of a mevalonate pathway derived metabolite, and/or with high levels of a fatty acid-derived lipid.

12. The glutamine synthetase inhibitor for use according to claim 11, wherein the disease is selected from the group comprising or consisting of steatosis, cirrhosis, renal dysfunction, testosterone deficiency, glioblastoma, multiple myeloma, leukemia, cancer, premature aging, infectious disease, neurodegenerative disease including Alzheimer's disease, arthritis, cardiovascular disease, Hutchinson-Gilford progeria syndrome (HGPS), cerebral cavernous malformations (CCMs), neuroinflammatory disorders, autoimmune disorders, diabetes, atherosclerosis, hyperlipidemia, immuno-inflammatory diseases, acquired immune deficiency syndrome (AIDS), allograft rejection, adult respiratory distress syndrome, arthritis, asthma, atherosclerosis, autoimmune disorders, cerebral palsy, eczema, glomerulonephritis, heart failure, herpes dementia, immune complex diseases, inflammatory bowel disease, ischemia , metabolic syndrome, migraine, multiple sclerosis, myocarditis, organ transplant/bypass disorders, osteoporosis, oxidant induced lung injury, Paget's disease, hyperimmunoglobulin D syndrome, mevalonate kinase deficiency, pain, peritonitis, reperfusion injury, retinitis, sepsis, septic shock, sickle cell anemia, stroke, toxic shock syndrome, uveitis, X-adenoleukodystrophy (X-ALD), Alzheimer's disease, Parkinson's disease, Landry-Guillain-Barre-Strohl syndrome, multiple sclerosis, viral encephalitis, AIDS-related dementia, amyotrophic lateral sclerosis, brain trauma, spinal cord disorders, atherosclerosis, coronary artery insufficiency, coronary heart disease, myocardial infarction, hypertriglyceridemia, lymphoma, Cushing syndrome, porphyria, hypothyroidism, renal disease, cholestatic liver disorders, lymphoma, bipolar disorders, schizophrenia, neurodegenerative diseases, Niemann-Pick and Huntington diseases, multiple sclerosis, lipid storage disease.

13. The glutamine synthetase inhibitor for use according to claim 11 or 12, wherein the glutamine synthetase inhibitor is selected from L-methionine sulfoximine, a phosphinothricin analogue, a derivative of aminomethylenebisphosphonic acid, and γ-hydroxylysine.

14. A glutamine synthetase activator identifiable by the *in vitro* method of any one of the claims 1 - 10, for use in treatment of a disease associated with low levels of a mevalonate pathway derived metabolite, and/or with low levels of a fatty acid-derived lipid.

15. A pharmaceutical composition comprising a glutamine synthetase modulator identifiable by the method of any one of the claims 1 - 10, together with at least one pharmaceutically acceptable carrier, excipient and/or diluent.
